(19) Europäisches Patentamt
European Patent Office
Office européen des brevets

(11) EP 2 999 345 B1

(12) **EUROPEAN PATENT SPECIFICATION**

(45) Date of publication and mention of the grant of the patent:
**10.08.2022 Bulletin 2022/32**

(21) Application number: **14735722.2**

(22) Date of filing: **21.05.2014**

(51) International Patent Classification (IPC):
***A01P 1/00*** (2006.01) ***A01N 33/04*** (2006.01)
***A01N 37/18*** (2006.01) ***A01N 43/10*** (2006.01)
***A01N 43/30*** (2006.01) ***A01N 43/54*** (2006.01)
***A01N 59/16*** (2006.01) ***A61P 31/04*** (2006.01)
***C08G 73/02*** (2006.01)

(52) Cooperative Patent Classification (CPC):
(C-Sets available)
**A01N 33/04; A01N 37/18; A01N 43/10; A01N 43/30; A01N 43/54; A01N 59/16; A61P 31/04; C08G 73/02** (Cont.)

(86) International application number:
**PCT/US2014/039039**

(87) International publication number:
**WO 2014/190096 (27.11.2014 Gazette 2014/48)**

(54) **COMPOSITIONS AND METHODS COMPRISING A POLYAMINE**

ZUSAMMENSETZUNGEN UND VERFAHREN, DIE EIN POLYAMIN UMFASSEN

COMPOSITIONS ET PROCÉDÉS COMPRENANT UNE POLYAMINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**22.05.2013 US 201361826453 P**
**23.05.2013 US 201361826761 P**
**12.06.2013 US 201361834149 P**
**18.06.2013 US 201361836555 P**
**04.10.2013 US 201361887267 P**
**08.11.2013 US 201314076149**
**08.11.2013 US 201314076143**
**08.11.2013 US 201361902135 P**
**10.02.2014 US 201461938111 P**

(43) Date of publication of application:
**30.03.2016 Bulletin 2016/13**

(73) Proprietors:
- **Curza Global LLC**
  **Provo, Utah 84604 (US)**
- **University of Utah Research Foundation**
  **Salt Lake City UT 84112 (US)**

(72) Inventors:
- **LOOPER, Ryan**
  **Provo, Utah 84604 (US)**
- **WILLIAMS, Dustin**
  **Provo, Utah 84604 (US)**
- **JEYAPALINA, Sujeevini**
  **Provo, Utah 84604 (US)**
- **HAUSSENER, Travis**
  **Provo, Utah 84604 (US)**
- **SEBAHAR, Paul, R.**
  **Provo, Utah 84604 (US)**
- **REDDY, Hariprasada R. Kanna**
  **Provo, Utah 84604 (US)**

(74) Representative: **AWA Sweden AB**
**Box 45086**
**104 30 Stockholm (SE)**

(56) References cited:
**WO-A1-94/19311**
**WO-A1-2005/105729**
**WO-A1-2008/137195**
**WO-A1-2012/151554**
**WO-A2-2010/148390**
**FR-A1- 2 860 129**
**US-A1- 2007 208 082**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



**(Cont. next page)**

- **MARTIN B ET AL: "N-benzylpolyamines as vectors of boron and fluorine for cancer therapy and imaging: Synthesis and biological evaluation", JOURNAL OF MEDICINAL CHEMISTRY, AMERICAN CHEMICAL SOCIETY, US, vol. 44, no. 22, 25 October 2001 (2001-10-25), pages 3653-3664, XP002314543, ISSN: 0022-2623, DOI: 10.1021/JM010897Q**
- **DENGFENG DOU ET AL: "Design and synthesis of inhibitors of noroviruses by scaffold hopping", BIOORGANIC & MEDICINAL CHEMISTRY, PERGAMON, GB, vol. 19, no. 19, 15 August 2011 (2011-08-15), pages 5749-5755, XP028294114, ISSN: 0968-0896, DOI: 10.1016/J.BMC.2011.08.032 [retrieved on 2011-08-22]**


(52) Cooperative Patent Classification (CPC): (Cont.)

C-Sets
**A01N 59/16, A01N 33/04, A01N 37/18, A01N 43/10, A01N 43/30, A01N 43/54, A01N 2300/00**

**Description**

FIELD OF THE INVENTION

The present invention is directed to polyamine compounds, compositions, and methods, which preferably have antimicrobial or dispersing activity against a variety of bacterial strains capable of forming biofilms. Various aspects and embodiments relate generally to polyamine compounds and to methods of preparing or using such compounds.

BACKGROUND OF THE INVENTION

Antimicrobial compounds, such as traditional antibiotics, have the ability to kill or to retard the growth of bacteria, fungi, and other microorganisms. Some antimicrobial compounds also are effective against viruses. Antimicrobial compounds are used in a wide variety of clinical settings, industrial applications, food production facilities and environmental applications all across the globe in an effort to reduce the risk of, for example, bacterial colonization and development of disease in people.

Traditional antibiotics are primarily derivatives or synthetic mimics of natural compounds secreted by bacteria, plants, or fungi. These compounds typically have very specific methods of action against a cell wall/membrane component of bacteria, or an enzyme/protein in a metabolic pathway. Examples of traditional antibiotics on the market include penicillin, oxacillin, vancomycin, gentamicin, rifampicin and amoxicillin, among others.

Because bacteria have the ability to develop resistance genes to these antibiotics as a result of genetic mutations or acquired defense mechanisms that target the specific activity of the antibiotics, bacteria typically have the ability to develop resistance to traditional antibiotics. Increasingly more prevalent bacterial resistance has made traditional antibiotics to become less and less effective in a variety of applications.

Bacterial resistance to antibiotics represents one of the most underappreciated threats to modern society. *See* Zhang et al., Antibiotic resistance as a global threat: Evidence from China, Kuwait and the United States, Global Health 2, 6 (2006). Currently, more than 90% of clinical isolates of *Staphylococcus aureus* display resistance to penicillin. *See* Balaban et al., Control of Biofilm Infections by Signal Manipulation, Ch. 1, 1-11 (Springer, 2008). Recent reports have even indicated that bacteria in natural ecosystems metabolize antibiotics as an energy source. *See* Leslie, Germs Take a Bite Out of Antibiotics, Science 320, 33 (2008). The trend of bacterial resistance continues to increase as indicated by almost daily scientific publications and world news reports of antibiotic resistant superbugs such as carbapenem-resistant Enterobacteriacea, vancomycin-resistant Enterococci, multidrug-resistant *Pseudomonas aeruginosa* and methicillin-resistant *Staphylococcus aureus* (MRSA). *See e.g.,* FoxNews.com. Europe in the Grip of Drug-Resistant Superbugs (2011); Melnick, M., TIME (2010); Arias et al., The rise of the Enterococcus: beyond vancomycin resistance, Nat Rev Microbiol 10, 266-278 (2012); Jain, R. et al., Veterans affairs initiative to prevent methicillin-resistant Staphylococcus aureus infections, N Engl J Med 364, 1419-1430 (2011); Nordmann et al., The real threat of Klebsiella pneumoniae carbapenemase-producing bacteria, Lancet Infect Dis 9, 228-236 (2009); Aloush et al., Multidrug-resistant Pseudomonas aeruginosa: risk factors and clinical impact, Antimicrob Agents Chem 50, 43-48 (2006). In addition to adversely affecting civilian patients, antibiotic-resistant bacteria affect injured military personnel. Multiple reports from Operation Iraqi Freedom/Operation Enduring Freedom have indicated that multidrug-resistant bacteria and antibiotic resistance constitute one of the most disconcerting aspects of military theater treatment. *See e.g.,* Calhoun et al., Multidrug-resistant Organisms in Military Wounds from Iraq and Afghanistan, Clinical Orthopaedics and Related Research 466, 1356-1362 (2008); Murray et al., Bacteriology of War Wounds at the Time of Injury, Military Medicine 171, 826-829 (2006); Hujer et al., Analysis of Antibiotic Resistance Genes in Multidrug-Resistant Acinetobacter sp. Isolates from Military and Civilian Patients Treated at the Walter Reed Army Medical Center, Antmicrobial Agents and Chemotherapy 50, 4114-4123 (2006).

Multiple factors contribute to bacterial cells' ability to resist the effects of antibiotics. *See e.g.,* Morita et al., Antibiotic Inducibility of the MexXY Multidrug Efflux System of Pseudomonas aeruginosa: Involvement of the Antibiotic-Inducible PA5471 Gene Product, Journal of Bacteriology 188, 1847-1855 (2006); Tran et al., Heat-Shock Protein ClpL/HSP 100 Increases Penicillin Tolerance in Streptococcus pneumoniae, Advances in Oto-rhino-laryngology 72, 126-128 (2011); Livorsi et al., Virulence Factors of Gram-Negative Bacteria in Sepsis With a Focus on Neisseria meningitidis, Contributions to Microbiology 17, 31-47 (2011); Nostro, et al., Specific Ion Effects on the Growth Rates of Staphylococus aureus and Pseudomonas aeruginosa, Physical Biology 2, 1-7 (2005). Amongst these factors is the ability of bacteria to develop a biofilm. *See e.g.,* Costerton et al., How bacteria stick, Sci Am 238, 86-95 (1978); Lawrence et al., Optical sectioning of microbial biofilms, J Bacteriol 173, 6558-6567 (1991); ZoBell, The Effect of Solid Surfaces upon Bacterial Activity, Journal of Bacteriology 46, 39-56 (1943). Biofilms have unique characteristics that allow them to withstand, or defend themselves against a variety of perturbations including exposure to antibiotics.

Biofilms are surface-attached communities of bacteria, often polymicrobial, that produce a slimy, extracellular polysaccharide substance (EPS) that encapsulates them. The EPS provides protection, Leid et al., The Exopolysacharide Alginate Protects Pseudomonas aeruginosa Biofilm Bacteria from IFN-γ-Mediated Macrophage Killing, The Journal of

Immunology 175, 7512-7518 (2005), as well as a reserve of nutrients, water and trace elements to sustain life. Costerton et al., The Bacterial Glycocalyx in Nature and Disease, Annual Review of Microbiology 35, 299-324 (1981). Biofilms are the predominant phenotype of bacteria in natural ecosystems. Gram-negative bacteria, Gram-positive bacteria, and mycobacteria, in addition to other unicellular organisms, can produce biofilms.

Within the biofilm community, bacteria may have several methods of defending themselves against the biocidal effects of antibiotics. First, they have strength in numbers. Biofilms may contain millions or trillions of cells in a very small volume. Second, bacteria in a biofilm have the ability to rapidly transfer genetic material, such as plasmids, that specifically code for the production of molecules that protect them against antibiotics. Lujan etal., Disrupting Antibiotic Resistance Propagation by Inhibiting the Conjugative DNA Relaxase, PNAS 104, 12282-12287 (2007); Lederberg et al., Gene Recombination in Escherichia coli. Nature 158, 529-564 (1946). Rates of plasmid transfer in biofilms have been shown to be much higher than amongst planktonic bacteria, which are free-floating in an environment. Hausner et al., High Rates of Conjugation in Bacterial Biofilms as Determined by Quantitative In Situ Analysis, Applied and Environmental Microbiology 65, 3710-3713 (1999). Third, as a biofilm community matures, it creates an oxygen gradient such that an oxygen-rich environment exists on the outer edges of a biofilm, whereas an oxygen-deprived, or anaerobic, area exists in the deepest portions of a biofilm. Walters et al., Contributions of Antibiotic Penetration, Oxygen Limitation, and Low Metabolic Activity to Tolerance of Pseudomonas aeruginosa biofilms to Ciprofloxacin and Tobramycin, Antimicrobial Agents and Chemotherapy 47, 317-323 (2003); Borriello et al., Oxygen Limitation Contributes to Antibiotic Tolerance of Pseudomonas aeruginosa in Biofilms, Antimicrobial Agents and Chemotherapy 48, 2659-2664 (2004). This may result in reduced metabolic activity in those cells that dwell in the interior of the biofilm. Importantly, traditional antibiotics are typically effective against bacterial cells that are rapidly dividing, i.e., in a logarithmic phase of growth. Mandell, Interaction of Intraleukocytic Bacteria and Antibiotics, The Journal of Clinical Investigation 52, 1673-1673 (1973); Gilbert et al., Influence of Growth Rate on Susceptibility to Antimicrobial Agents: Biofilms, Cell Cycle, Dormancy, and Stringent Response, Antimicrobial Agents and Chemotherapy 34, 1865-1868 (1990). Fourth, in a mature biofilm, water channels form throughout the community. Stoodley et al., Liquidflow in biofilm systems, App Env Microbiol 60, 2711-2716 (1994). These water channels have the ability to diffuse, remove or prevent toxic byproducts as well as antibiotics from interacting with cells in the biofilm. For novel antimicrobial agents to be effective over the long term, addressing each of these four characteristics may increase the potential for success in a variety of applications including healthcare, industrial, environmental, agricultural and sanitation industries. Furthermore, biofilms tend to secrete proteoglycan materials that create an extracellular matrix, which has the ability to potentially bind and hinder the activity of antibiotics.

Alternative approaches to killing bacteria include the use of antimicrobial agents that have fast-acting and nonspecific mode of activity against the cell membrane of bacteria. These alternate compounds include detergents, squalamine, quaternary ammonium compounds, and naturally occurring antimicrobial peptides, among others. By attacking and depolarizing the cell membrane in a nonspecific fashion at a faster rate, agents that attack the cell membrane globally can kill bacteria before they have time to upregulate their defense mechanisms. In addition, modes of action of these alternate antimicrobials are not limited to a specific protein or enzyme within a metabolic pathway.

However, as important as it is to kill bacteria and prevent their ability to cause infections in humans or animals, or contaminate unwanted processes in industrial, agricultural or environmental applications, when bacteria are attached to a surface, it sometimes may be more beneficial to not only kill bacteria, but also to cause them to "fall off" of a surface as well, e.g. disperse or dislodge bacteria in a biofilm community. In certain aspects, the present invention provides compounds, compositions, and methods that have shown the ability to disperse or dislodge bacterial cells in a biofilm, such that the cells are no longer able to reattach and form new biofilm communities, and, notably, the same compounds, compositions, and methods kill substantially all bacteria cells in a biofilm.

By dispersing a biofilm and killing the cells within it, at least two benefits are provided. This may be particularly important when considering the fact that although bacteria in a biofilm, which may be attached to a surface, can be killed by an antimicrobial agent, the dead cells and extracellular matrix residues may provide an attachment point for viable bacteria to re-adhere and form a biofilm once again with greater affinity. If biofilms are dispersed and killed, viable bacteria that are introduced to a surface will have reduced ability to preferentially adhere to that area. This can be particularly important in industrial applications wherein the formation of biofilms on a surface can be problematic, as well as medical applications wherein bacteria may adhere to the surface of a medical device. It has been surprisingly discovered that compositions of the present invention have significant potential to eradicate bacteria within a biofilm as well as cause the biofilm to disperse or dislodge, resulting in a variety of potential applications across multiple settings. WO 2012/151554 discloses the treatment of biofilms with polyamines.

Thus, there is a need for novel compounds, compositions, and methods that have potent antimicrobial and anti-biofilm activity against a variety of bacterial strains, especially at high bacterial concentrations and against antibiotic-resistant bacteria.

SUMMARY OF THE INVENTION

It is an object of the present invention to provide novel compounds, compositions, and methods having antimicrobial activity and dispersing activity against a wide variety of bacterial strains capable of forming biofilms. In some preferred aspects, the invention provides compounds, compositions, and methods that are effective against antibiotic-resistant bacterial biofilms.

It has been discovered that compounds, compositions, and methods described herein rapidly disperse biofilms and kill microorganisms such as bacteria, so that the microorganisms do not have an opportunity to upregulate their defense mechanisms. Thus, there may be a reduced risk of bacteria developing resistance to the compounds, compositions, and methods descibed herein. Furthermore, such compounds, compositions, and methods may not be limited to eradicating bacteria that are in log-phase growth. The ability of compounds, compositions, and methods to disperse biofilms while demonstrating antimicrobial activity may address many of the characteristics that make biofilm communities difficult to treat using traditional antibiotics. More specifically, by dispersing and killing bacteria in a biofilm, water channels and the bacterial community as a whole may be broken apart, allowing for broader distribution of antimicrobial agent(s) to a greater number, or even substantially all, of the cells within a biofilm.

Aspects of this disclosure feature non-therapeutic methods of killing, dispersing, dislodging, treating, and reducing biofilms as well as preventing or inhibiting biofilm formation.

In a first embodiment, the present invention provides a polyamine compound for use in therapeutic dispersing or killing of a biofilm, wherein the polyamine compound is selected from the group consisting of

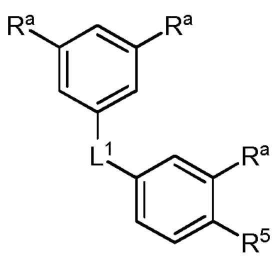 , 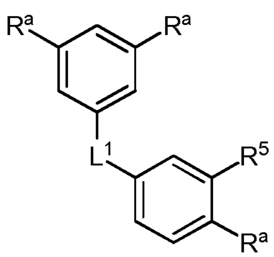 , 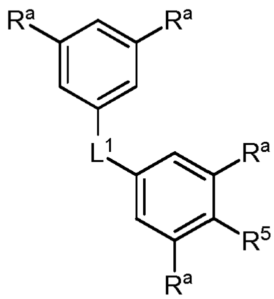 , 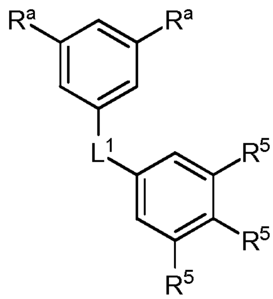 ,

and a salt thereof;
wherein:

- each $L^1$ is selected from the group consisting of a bond and -O-;
- each $R^a$ $-CH_2[NH(CH_2)_n]_pNHR^4$;
- each n is an integer independently selected from 3 to 12;
- each p is an integer independently selected from 1 to 3;
- each $R^4$ is a member independently selected from the group consisting of hydrogen, alkyl, arylalkyl and cycloalkylalkyl; and
- each $R^5$ is a member independently selected from the group consisting of hydrogen, alkyl, hydroxyl, alkoxy, halo, fluoroalkyl, fluoroalkyloxy, and hydroxyalkyl,

wherein the polyamine compound comprises at least four primary or secondary amino groups.

In a second embodiment, the present invention provides an anti-biofilm composition, the composition comprising a polyamine compound as set forth in the first embodiment.

In a third embodiment, the present invention provides a non-therapeutic method for dispersing or killing a biofilm, the method comprising a step of treating the biofilm with an anti-biofilm composition, thereby effectively dispersing or killing the biofilm composition comprising the polyamine compound, as set forth in the first embodiment.

In a fourth embodiment, the present invention provides a non-therapeutic method for inhibiting formation of a biofilm, the method comprising a step of treating planktonic bacteria with the anti-biofilm composition comprising the polyamine compound, as set forth in the first embodiment, thereby inhibiting incorporation of the planktonic bacteria into the biofilm.

The invention is defined by the appended claims..

BRIEF DESCRIPTION OF THE DRAWINGS

Various instances are shown and described in reference to the numbered drawings.

FIG. 1 shows a simplified schematic representation of a class of polyamine compounds.
FIG. 2A through FIG. 2H show exemplary starting materials or reactants that may be used to prepare certain specific polyamine compounds as set forth herein.
FIG. 3 shows exemplary polyamine chains that may be used to prepare certain specific polyamine compounds as set forth herein.
FIG. 4A through FIG. 4P show exemplary polyamine compounds (not part of the invention).
FIG. 5A through FIG. 5B show exemplary polyamine compounds (not part of the invention).
FIG. 6A through FIG. 6E show exemplary polyamine compounds (not part of the invention).
FIG. 7 shows an exemplary synthesis strategy to produce polyamine compounds (not part of the invention).
FIG. 8 shows another exemplary synthesis strategy to produce polyamine compounds (not part of the invention).
FIG. 9 shows still another exemplary synthesis strategy to produce polyamine compounds (not part of the invention).
FIG. 10 shows yet another exemplary synthesis strategy to produce polyamine compounds (not part of the invention).
FIG. 11 shows the minimum inhibitory concentration ("MIC") of polyamines and novel polyamine compounds (not part of the invention).
FIG. 12A is a table showing certain polyamine compounds (not part of the invention);
FIG. 12B is a table showing the effect biofilm eradication concentration ("EBEC") of certain polyamine compounds represented in FIG. 8A and EBECs of certain (not part of the invention).
FIG. 13 shows an example of microbial contamination in the oil and gas industry.
FIG. 14A shows a representative bacteria biofilm.
FIG. 14B shows a representative bacteria biofilm, such as the biofilm shown in FIG. 10A, treated according to certain standards in the oil and gas industry.
FIG. 14C shows a representative bacteria biofilm, such as the biofilm shown in FIG. 10A, treated with a polyamine compound.
FIG. 15 shows the results of treating biofilms of *Alcanivorax borkumensis* grown on the surface of galvanized steel with a polyamine compound. (not part of the invention).
FIG. 16 shows a photograph demonstrating the ability of Vanicream-based cream to elute compounds CZ-25, CZ-52, and polymyxin B polytherapy formula and to eradicate bacteria as indicated by zones of clearing around the cream. The second row of plates is from a duplicate test of the same cream. CZ-25 and CZ-52 are not part of the invention.
FIG. 17 shows scanning electron microscopy (SEM) images of Ti coupons with MRSA biofilms exposed to CZ-25 (left), CZ-52 (middle), or water only (right). The compounds CZ-25 and CZ-52 demonstrated the ability to disperse the majority of biofilms such that a monolayer of cells remained on the surface of the Ti. Those samples treated with water only had biofilm communities that remained in all areas of the CZ-25 and CZ-52 are not part of the invention.
FIG. 18 shows photographs of MRSA biofilms being dispersed by the compound CZ-25. (Left) Biofilms of MRSA grown on the surface of Ti coupons in a CDC biofilm reactor exposed to water for 2 hours. (Right) Biofilms of MRSA grown on the surface of Ti coupons exposed to the compound CZ-25 in water for 2 hours. Note the strings of biofilm dispersing from the surface of the metal. CZ-25 is not part of the invention.
FIG. 19 shows SEM images of biofilms of *Alcanivorax borkumensis* grown on the surface of galvanized steel in a flow system, then exposed to the compound CZ-25 (also known as PBC-25). CZ-25 is not part of the invention.
FIG. 20 shows photographs SEM images of biofilms of *Alcanivorax borkumensis* grown on the surface of galvanized steel in a flow system, then exposed to the compound CZ-7 (also known as PBC-7; not part of the invention). Glutaraldehyde, the standard of treatment in oil and gas facilities, was compared as a control.
FIG. 21 shows a schematic of the stir tank biofilm reactor.
FIG.22 shows a photograph of a glass slide removed after 6 days in the stir tank bioreactor.
FIG. 23 shows a hypothetical mode of binding of spermidine to an exopolysaccharide.
FIG. 24 shows several polyamine-containing natural products with antimicrobial activity.
FIG. 25 shows a method for making several embodiments of the polyamine compound (not part of the invention).
FIG. 26 shows a method for making yet another embodiment of the polyamine compound.
FIG. 27 shows a method for making still another embodiment of the polyamine compound.
FIG. 28 shows a method for making several other embodiments of the polyamine compound (CZ-52 and CZ-25 are not part of the invention).
FIG. 29 shows a pH profile of selected polyamine compounds’ hydrochloride salts in deionized water.
FIGS. 30A to 30I show the effects of the polyamine compounds CZ-86 and CZ-110 (CZ-110 is not part of the invention) on various bacterial cell cultures. FIG. 30B shows a control without added polyamine compounds.

DETAILED DESCRIPTION OF THE INVENTION

## DEFINITIONS

Unless otherwise defined, all technical and scientific terms used herein have the same meaning as commonly understood by one of ordinary skill in the art to which this invention belongs. In addition, the materials, methods, and examples are illustrative only and not intended to be limiting. All publications, patent applications, patents, and other references mentioned herein include U.S. Appl. Nos. 61/482,522; 61/482523; 61/591,601; 61/616,944; 61/826,453; 61/826,761; 61/836,555; 61/834,149; 13/379,191; 14/076,143; and 14/076,149 as well as Int'l Pat. Publ. Nos. WO 2010/148390, 2012/151555, and 2013/148230. In case of conflict, the present specification, including these definitions, will control.

The terms "a," "an," and "the" as used herein not only includes aspects with one member, but also includes aspects with more than one member. For example, an embodiment including "a polyamine compound and an excipient" should be understood to present certain aspects with at least a second polyamine compound, at least a second excipient, or both.

The term "about" as used herein to modify a numerical value indicates a defined range around that value. If "X" were the value, "about X" would generally indicate a value from 0.95X to 1.05X. Any reference to "about X" specifically indicates at least the values X, 0.95X, 0.96X, 0.97X, 0.98X, 0.99X, 1.01X, 1.02X, 1.03X, 1.04X, and 1.05X. Thus, "about X" is intended to teach and provide written description support for a claim limitation of, *e.g.,* "0.98X." When the quantity "X" only includes whole-integer values *(e.g.,* "X carbons"), "about X" indicates from (X-1) to (X+1). In this case, "about X" as used herein specifically indicates at least the values X, X-1, and X+1.

When "about" is applied to the beginning of a numerical range, it applies to both ends of the range. Thus, "from about 5 to 20%" is equivalent to "from about 5% to about 20%." When "about" is applied to the first value of a set of values, it applies to all values in that set. Thus, "about 7, 9, or 11%" is equivalent to "about 7%, about 9%, or about 11%."

The term "acyl" as used herein includes an alkanoyl, aroyl, heterocycloyl, or heteroaroyl group as defined herein. Examples of acyl groups include, but are not limited to, acetyl, benzoyl, and nicotinoyl.

The term "alkanoyl" as used herein includes an alkyl-C(O)- group wherein the alkyl group is as defined herein. Examples of alkanoyl groups include, but are not limited to, acetyl and propanoyl.

The term "agent" as used herein includes a compound or mixture of compounds that, when added to a composition, tend to produce a particular effect on the composition's properties. For example, a composition comprising a thickening agent is likely to be more viscous than an otherwise identical comparative composition that lacks the thickening agent.

The term "alkenyl" as used herein includes a straight or branched chain hydrocarbon containing at least one carbon-carbon double bond. The chain may contain an indicated number of carbon atoms. For example, "$C_1$-$C_{12}$ alkenyl" indicates that the group may have from 1 to 12 (inclusive) carbon atoms and at least one carbon-carbon double bond. When the indicated number of carbon atoms is 1, then the $C_i$ alkenyl is double bonded to a carbon *(i.e.,* a carbon equivalent to an oxo group). In certain aspects, the chain includes 1 to 12, about 2 to 15, about 2 to 12, about 2 to 8, or about 2 to 6 carbon atoms. Examples of an alkenyl group may include, but are not limited to, ethenyl *(i.e.,* vinyl), allyl, propenyl, butenyl, crotyl, pentenyl, hexenyl, heptenyl, octenyl, nonenyl, decenyl, dodecenyl, cyclopentenyl, cyclohexenyl, 2-isopentenyl, allenyl, butadienyl, pentadienyl, 3-(1,4-pentadienyl), and hexadienyl.

An alkenyl group can be unsubstituted or optionally substituted. When optionally substituted, one or more hydrogen atoms of the alkenyl group (e.g., from 1 to 4, from 1 to 2, or 1) may be replaced with a moiety independently selected from fluoro, hydroxy, alkoxy, amino, alkylamino, acylamino, thio, and alkylthio, with the proviso that no hydrogen atom substituent on the carbon-carbon double bond is replaced by a hydroxy, amino, or thio group. In some aspects, the alkenyl group is unsubstituted or not optionally substituted.

The term "alkyl" as used herein includes an aliphatic hydrocarbon chain that may be straight chain or branched. The chain may contain an indicated number of carbon atoms: For example, $C_1$-$C_{12}$ indicates that the group may have from 1 to 12 (inclusive) carbon atoms in it. If not otherwise indicated, an alkyl group contains from 1 to about 20 carbon atoms. In some aspects, alkyl groups have 1 to about 12 carbon atoms in the chain. In some aspects, alkyl groups ("lower alkyl") have 1 to about 6 carbon atoms in the chain. Examples may include, but are not limited to, methyl, ethyl, propyl, isopropyl (iPr), 1-butyl, 2-butyl, isobutyl (iBu), tert-butyl, pentyl, 2-methylbutyl, 1,1-dimethylpropyl, hexyl, heptyl, octyl, nonyl, decyl, docecyl, cyclopentyl, or cyclohexyl.

An alkyl group can be unsubstituted or optionally substituted. When optionally substituted, one or more hydrogen atoms of the alkyl group (e.g., from 1 to 4, from 1 to 2, or 1) may be replaced with a moiety independently selected from fluoro, hydroxy, alkoxy, amino, alkylamino, acylamino, thio, and alkylthio. In some aspects, the alkyl group is unsubstituted or not optionally substituted.

The term "alkoxy" as used herein includes a straight or branched chain saturated or unsaturated hydrocarbon containing at least one oxygen atom in an ether group (e.g., EtO-). The chain may contain an indicated number of carbon

atoms. For example, "$C_1$-$C_{12}$ alkoxy" indicates that the group may have from 1 to 12 (inclusive) carbon atoms and at least one oxygen atom. Examples of a $C_1$-$C_{12}$ alkoxy group include, but are not limited to, methoxy, ethoxy, isopropoxy, butoxy, n-pentoxy, isopentoxy, neopentoxy, and hexoxy.

An alkoxy group can be unsubstituted or optionally substituted. When optionally substituted, one or more hydrogen atoms of the alkoxy group (e.g., from 1 to 4, from 1 to 2, or 1) may be replaced with a moiety independently selected from fluoro, hydroxy, alkoxy, amino, alkylamino, acylamino, thio, and alkylthio, with the proviso that no hydrogen atom alpha to the ether oxygen is replaced by a hydroxy, amino, or thio group. In some aspects, the alkoxy group is unsubstituted or not optionally substituted.

The term "alkynyl" as used herein includes a straight, branched, or cyclic hydrocarbon containing at least one carbon-carbon triple bond. Examples may include, but are not limited to, ethynyl, propargyl, propynyl, butynyl, pentynyl, hexynyl, heptynyl, octynyl, nonynyl, decynyl, or decynyl.

An alkynyl group can be unsubstituted or optionally substituted. When optionally substituted, one or more hydrogen atoms of the alkynyl group (e.g., from 1 to 4, from 1 to 2, or 1) may be replaced with a moiety independently selected from fluoro, hydroxy, alkoxy, amino, alkylamino, acylamino, thio, and alkylthio, with the proviso that no sp-hybridized hydrogen atom substituent is replaced by a hydroxy, amino, or thio group. In some aspects, the alkynyl group is unsubstituted or not optionally substituted.

The term "aroyl" as used herein includes an aryl-CO- group wherein aryl is as defined herein. Examples include, but are not limited to, benzoyl, naphth-1-oyl and naphth-2-oyl.

The term "aryl" as used herein includes cyclic aromatic carbon ring systems containing from 6 to 18 carbons. Examples of an aryl group include, but are not limited to, phenyl, naphthyl, anthracenyl, tetracenyl, biphenyl and phenanthrenyl.

An aryl group can be unsubstituted or optionally substituted. When optionally substituted, one or more hydrogen atoms of the aryl group (e.g., from 1 to 5, from 1 to 2, or 1) may be replaced with a moiety independently selected from alkyl, cyano, acyl, halo, hydroxy, alkoxy, amino, alkylamino, acylamino, thio, and alkylthio. In some aspects, the alkoxy group is unsubstituted or not optionally substituted.

The term "arylalkyl" or "aralkyl" as used herein includes an alkyl group as defined herein where at least one hydrogen substituent has been replaced with an aryl group as defined herein. Examples include, but are not limited to, benzyl, 1-phenylethyl, 4-methylbenzyl, and 1,1,-dimethyl-1-phenylmethyl.

A arylalkyl or aralkyl group can be unsubstituted or optionally substituted as per its component groups. For example, but without limitation, the aryl group of an arylalkyl group can be substituted, such as in 4-methylbenzyl.. In some aspects, the group is unsubstituted or not optionally substituted, especially if including a defined substituent, such as a hydroxyalkyl or alkylaminoalkoxy group.

The term "cycloalkyl" as used herein includes a cyclic hydrocarbon group that may contain an indicated number of carbon atoms: For example, $C_3$-$C_{12}$ indicates that the group may have from 3 to 12 (inclusive) carbon atoms in it. If not otherwise indicated, a cycloalkyl group includes about 3 to about 20 carbon atoms. In some aspects, cyclo alkyl groups have 3 to about 12 carbon atoms in the group. In some aspects, cycloalkyl groups have 3 to about 7 carbon atoms in the group. Examples may include, but are not limited to, cyclopropyl, cyclobutyl, cyclopentyl, cyclohexyl, 4,4-dimethylcyclohexyl, and cycloheptyl.

A cycloalkyl group can be unsubstituted or optionally substituted. When optionally substituted, one or more hydrogen atoms of the cycloalkyl group *(e.g.,* from 1 to 4, from 1 to 2, or 1) may be replaced with a moiety independently selected from fluoro, hydroxy, alkoxy, amino, alkylamino, acylamino, thio, and alkylthio. In some aspects, a substituted cycloalkyl group can incorporate an exo- or endocyclic alkene (e.g., cyclohex-2-en-1-yl). In some aspects, a cycloalkyl group is unsubstituted or not optionally substituted.

The terms "disorder," "disease," and "condition" are used herein interchangeably for a condition in a subject. A disorder is a disturbance or derangement that affects the normal function of the body of a subject. A disease is a pathological condition of an organ, a body part, or a system resulting from various causes, such as infection, genetic defect, or environmental stress that is characterized by an identifiable group of symptoms. A disorder or disease can refer to a biofilm-related disorder or disorder caused by a planktonic bacterial phenotype that is characterized by a disease-related growth of bacteria.

The term "effective amount" or "effective dose" as used herein includes an amount sufficient to achieve the desired result and accordingly will depend on the ingredient and its desired result. Nonetheless, once the desired effect is identified, determining the effective amount is within the skill of a person skilled in the art.

As used herein, "fluoroalkyl" includes an alkyl group wherein the alkyl group includes one or more fluoro-substituents. Examples include, but are not limited to, trifluoromethyl.

As used herein, "geminal" substitution includes two or more substituents that are directly attached to the same atom. An example is 3,3-dimethyl substitution on a cyclohexyl or spirocyclohexyl ring.

As used herein, "halo" or "halogen" includes fluoro, chloro, bromo, or iodo.

The term "heteroaryl" includes mono and bicyclic aromatic groups of about 4 to about 14 ring atoms (e.g., 4 to

10 or 5 to 10 atoms) containing at least one heteroatom. Heteroatom as used in the term heteroaryl refers to oxygen, sulfur and nitrogen. A nitrogen atom of a heteroaryl is optionally oxidized to the corresponding N-oxide. Examples include, but are not limited to, pyrazinyl, furanyl, thienyl, pyridyl, pyrimidinyl, isoxazolyl, isothiazolyl, oxazolyl, thiazolyl, pyrazolyl, furazanyl, pyrrolyl, pyrazolyl, triazolyl, 1,2,4-thiadiazolyl, pyrazinyl, pyridazinyl, quinoxalinyl, phthalazinyl, imidazo[1,2-a]pyridine, imidazo[2,1-b]thiazolyl, benzofurazanyl, indolyl, azaindolyl, benzimidazolyl, benzothienyl, quinolinyl, imidazolyl, thienopyridyl, quinazolinyl, thienopyrimidyl, pyrrolopyridyl, imidazopyridyl, isoquinolinyl, benzoazaindolyl, 1,2,4-triazinyl, and benzothiazolyl.

A heteroaryl group can be unsubstituted or optionally substituted. When optionally substituted, one or more hydrogen atoms of the heteroaryl group (e.g., from 1 to 5, from 1 to 2, or 1) may be replaced with a moiety independently selected from alkyl, cyano, acyl, halo, hydroxy, alkoxy, amino, alkylamino, acylamino, thio, and alkylthio. In some aspects, the heteroaryl group is unsubstituted or not optionally substituted.

The term "heteroaroyl" as used herein includes a heteroaryl-C(O)- group wherein heteroaryl is as defined herein. Heteroaroyl groups include, but are not limited to, thiophenoyl, nicotinoyl, pyrrol-2-ylcarbonyl, and pyridinoyl.

The term "heterocycloyl" as used herein includes a heterocyclyl-C(O)- group wherein heterocyclyl is as defined herein. Examples include, but are not limited to, N-methyl prolinoyl and tetrahydrofuranoyl.

As used herein, "heterocyclyl" includes a non-aromatic saturated monocyclic or multicyclic ring system of about 3 to about 10 ring atoms (e.g., 5 to about 10 ring atoms, or 3 to about 6 ring atoms), in which one or more of the atoms in the ring system is an element or elements other than carbon, e.g., nitrogen, oxygen or sulfur. A heterocyclyl group optionally comprises at least one $sp^2$-hybridized atom *(e.g.,* a ring incorporating an carbonyl, endocyclic olefin, or exocyclic olefin). In some embodiments, a nitrogen or sulfur atom of the heterocyclyl is optionally oxidized to the corresponding N-oxide, S-oxide or S,S-dioxide. Examples of monocycylic heterocyclyl rings include, but are not limited to, piperidyl, pyrrolidinyl, piperazinyl, morpholinyl, thiomorpholinyl, thiazolidinyl, 1,3-dioxolanyl, 1,4-dioxanyl, tetrahydrofuranyl, tetrahydrothiophenyl, and tetrahydrothiopyranyl.

A heterocycyl group can be unsubstituted or optionally substituted. When optionally substituted, one or more hydrogen atoms of the group *(e.g.,* from 1 to 4, from 1 to 2, or 1) may be replaced with a moiety independently selected from fluoro, hydroxy, alkoxy, amino, alkylamino, acylamino, thio, and alkylthio. In some aspects, a substituted heterocycyl group can incorporate an exo- or endocyclic alkene (e.g., cyclohex-2-en-1-yl). In some aspects, the heterocycyl group is unsubstituted or not optionally substituted.

The term "hydrophobic moiety" or "hydrophobic group" as used herein includes a moiety or a functional group that repels water. Examples may include, but are not limited to, a non-polar alkyl moiety, such as an unsubstituted alkyl group having more than five carbons; a phenyl group; and an anthracenyl group.

As used herein, the terms "hydrophilic moiety" or "hydrophilic group" includes a moiety or a functional group that has a strong affinity to water. Examples may include, but are not limited to, a charged moiety, such as a cationic moiety or an anionic moiety, or a polar uncharged moiety, such as an alkoxy group or an amine group.

As used herein, the term "hydroxyalkyl" includes an alkyl group where at least one hydrogen substituent has been replaced with an alcohol (-OH) group. In certain aspects, the hydroxyalkyl group has one alcohol group. In certain aspects, the hydroxyalkyl group has one or two alcohol groups, each on a different carbon atom. In certain aspects, the hydroxyalkyl group has 1, 2, 3, 4, 5, or 6 alcohol groups. Examples may include, but are not limited to, hydroxymethyl, 2-hydroxyethyl, and 1-hydroxyethyl.

When any two substituent groups or any two instances of the same substituent group are "independently selected" from a list of alternatives, the groups may be the same or different. For example, if $R^a$ and $R^b$ are independently selected from alkyl, fluoro, amino, and hydroxyalkyl, then a molecule with two $R^a$ groups and two $R^b$ groups could have all groups be an alkyl group (e.g., four different alkyl groups). Alternatively, the first $R^a$ could be alkyl, the second $R^a$ could be fluoro, the first $R^b$ could be hydroxyalkyl, and the second $R^b$ could be amino (or any other substituents taken from the group). Alternatively, both $R^a$ and the first $R^b$ could be fluoro, while the second $R^b$ could be alkyl *(i.e.,* some pairs of substituent groups may be the same, while other pairs may be different).

As used herein, "polyamine" includes a compound that has at least two amine groups, which may be the same or different. The amine group may be a primary amine, a secondary amine, a tertiary amine, or quaternary ammonium salt. Examples may include, but are not limited to, 1,3-diaminopropane, 1,4-diaminobutane, hexamethylenediamine, dodecan-1,12-diamine, spermine, spermidine, norspermine, and norspermidine.

As used herein, "or" should in general be construed non-exclusively. For example, an embodiment of "a composition comprising A or B" would typically present an aspect with a composition comprising both A and B, and an embodiment of "a method to disperse or kill biofilms" could disperse, kill, or a combination of both. "Or" should, however, be construed to exclude those aspects presented that cannot be combined without contradiction (e.g., a composition pH that is between 9 and 10 or between 7 and 8).

As used herein, "spirocycloalkyl" includes a cycloalkyl in which geminal substituents on a carbon atom are replaced to join in forming a 1,1-substituted ring. For example, but without limitation, for a $-C(R^1)(R^2)-$ group that was part of a longer carbon chain, if $R^1$ and $R^2$ joined to form a cyclopropyl ring incorporating the carbon to which $R^1$ and

$R^2$ were bonded, this would be a spirocycloalkyl group (i.e., spirocyclopropyl).

As used herein, "spiroheterocyclyl" includes a heterocycloalkyl in which geminal substituents on a carbon atom are replaced to join in forming a 1,1-substituted ring. For example, but without limitation, for a -C($R^1$)($R^2$)- group that was part of a longer carbon chain, if $R^1$ and $R^2$ joined to form a pyrrolidine ring incorporating the carbon to which $R^1$ and $R^2$ were bonded, this would be a spiroheterocyclyl group.

As used herein, the term "treat," "treating," or "treatment" includes administering or applying a composition (e.g., a composition described herein) in an amount, manner (e.g., schedule of administration), and mode *(e.g.,* route of administration) that is effective to improve a disorder or a symptom thereof, or to prevent, to retard, or to slow the progression of a disorder or a symptom thereof. Such improvements can include, but are not limited to, alleviation or amelioration of one or more symptoms or conditions, diminishment of the extent of a disease, stabilizing *(i.e.,* not worsening) the state of disease, prevention of a disease's transmission or spread, delaying or slowing of disease progression, amelioration or palliation of the disease state, diminishment of the reoccurrence of disease, and remission, whether partial or total and whether detectable or undetectable.

This can be evidenced by, *e.g.,* an improvement in a parameter associated with a biofilm or with a biofilm-related disorder or an indication or symptom thereof, a biofilm-related industrial, agricultural, environmental, etc. condition, *e.g.,* to a statistically significant degree or to a degree detectable to one skilled in the art. An effective amount, manner, or mode can vary depending on the surface, application, or subject and may be tailored to the surface, application, or subject. By eradicating a biofilm or preventing or slowing progression of a biofilm or of a biofilm-related disorder or an indication or symptom thereof, or a biofilm-related industrial, agricultural, environmental, etc. condition, a treatment can prevent or slow deterioration or corrosion resulting from a biofilm or from a biofilm-related disorder or an indication or symptom thereof on an affected surface or in an affected or diagnosed subject.

"Treating" and "treatment" as used herein also include prophylactic treatment.. The administering step may consist of a single administration or may comprise a series of administrations. The length of the treatment period depends on a variety of factors, such as the severity of the condition, the age of the patient, the concentration of active agent in the composition, the activity of the compositions used in the treatment, or a combination thereof. It will also be appreciated that the effective dosage of an agent used for the treatment or prophylaxis may increase or decrease over the course of a particular treatment or prophylaxis regime. Changes in dosage may result and become apparent by standard diagnostic assays known in the art. In some aspects, chronic administration may be required. For example, the compositions are administered to the subject in an amount, and for a duration, sufficient to treat the patient.

As used herein, a reference to a composition of formula A, B, C, or a salt thereof may indicate A, a salt of A, B, a salt of B, C, or a salt of C.

## POLYAMINE COMPOUNDS

Aliphatic polyamines, for example, spermine, spermidine, norspermine, norspermidine, hexamethylenediamine, 1,12-diaminododecane, 1,3-diaminopropane, etc., have been shown to have some activity against a variety of bacterial strains when used alone. In particular, it has been shown that norspermine and norspermidine may be effective in dispersing various biofilm strains. However, it does not appear that these polyamines have sufficient bactericidal activity.

It has been surprisingly discovered that by attaching one or more polyamine chains to a lipophilic or hydrophobic moiety, examples of which may include cyclic or aromatic backbone molecules, results in novel compounds that are capable of dispersing biofilms and that also have substantial antimicrobial activity against a variety of strains of bacteria (e.g., by killing the bacteria).

Referring now to FIG. 1, there is shown a simplified schematic of an exemplary polyamine compound, generally indicated at 10. The polyamine compound 10 may be generally characterized as including a lipophilic or hydrophobic moiety 20, and one or more cationic residues 30. Tthe polyamine compound 10 may include one or more cationic residues 30 comprising primary amines 40 and one or more secondary amines 50. The polyamine compound 10 may include one or more cationic residues 30 having a tertiary or quaternary amine (not shown).

As depicted in FIG. 1, exemplary polyamine compounds 10 of the present invention may be amphipathic, e.g. the cationic residues 30 may be generally disposed along one face of the molecule, while the lipophilic or hydrophobic moiety 20 is generally disposed along the opposite face of the molecule.

In certain embodiments, the present invention provides polyamine compounds and compositions and methods comprising such compounds. Polyamine compounds comprise one or more polyamine side chains (*e.g*., 1, 2, 3, 4, 5, 6, 7, 8, ... n polyamine moieties/side chains) that may be the same or different. The amino groups of the one or more polyamine side chains may be ionizable. The amino groups may be positively charged. The polyamine side chains are branched. Sometimes, the polyamine chains are linear.

FIGs. 2A-2H show exemplary backbone molecules, which may be used to prepare certain novel polyamine compounds. It will be appreciated by those skilled in the art that alternative backbone molecules may be used to produce polyamine compounds according to certain aspects of the present invention. Without being bound by theory, it is believed

that the lipophilicity or hydrophobicity of a backbone molecule of novel polyamine compounds may contribute to the antimicrobial activity or biofilm dispersing activity of these novel compounds.

FIG. 3 shows exemplary polyamine chains, which may be used to prepare certain novel polyamine compounds. It will be appreciated by those skilled in the art that polyamine chains are intended to only be representative. Without being bound by theory, it is believed that increasing the number of primary or secondary amines of polyamine compounds of the present invention may increase the antimicrobial activity or biofilm dispersing activity of these novel compounds.

FIGs. 4A-4P show exemplary polyamine compounds comprising a benzene backbones. FIGs. 5A and 5B show exemplary polyamine compounds comprising an anthracene backbone. FIGs. 6A-6E show exemplary polyamine compounds comprising a Vitamin D backbone. These compounds are not part of the invention.

FIG. 7 through FIG. 10 show exemplary chemical synthesis strategies for preparing a variety of polyamine compounds (not part of the invention).

The one or more polyamine moieties may have at least three amino groups separated by three atoms either in a straight chain or cyclic molecule. The one or more polyamine moiety or moieties may comprise norspermidine (also known as N-(3-aminopropyl)propane-1,3-diamine), norspermine (N'-[3-(3-aminopropylamino)propyl]propane-1,3-diamine), or a combination thereof.

Exemplary compositions may include a compound comprising a single polyamine chain or multiple polyamine chains attached to a base backbone compound to improve their antimicrobial activities or improve certain other features or aspects not present when polyamines are used alone such that the compositions may be designed for use in a specific application. Furthermore, it has been surprisingly discovered that by increasing the number of primary and secondary amines within a single molecule an increase in the antimicrobial activity of a compound may result. Furthermore, exemplary novel polyamines may be used in combination (*e.g.* formulated as a composition) with already approved, commercial antimicrobial products (*e.g.* chlorhexidine gluconate) to improve the dispersing or killing ability of such compositions.

In some aspects, the invention provides a compound for use in therapeutic dispersing or killing of a biofilm or an anti-bio-film composition that comprises, consists essentially of, or consists of a polyamine compound or composition.

The polyamine compound comprises at least four primary or secondary amino groups.

In some aspects, the polyamine compound is of formula

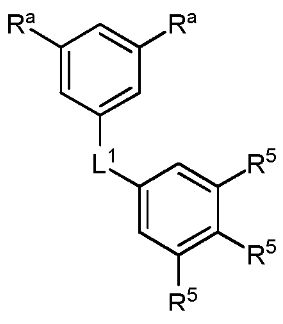

or a salt thereof.

In some aspects, $R^a$ is $-CH_2[NH(CH_2)_3]_2NH_2$.

In some aspects, $R^5$ is hydrogen.

In some aspects, each $R^4$ is a member independently selected from hydrogen and alkyl.

In some aspects, the polyamine compound is selected from

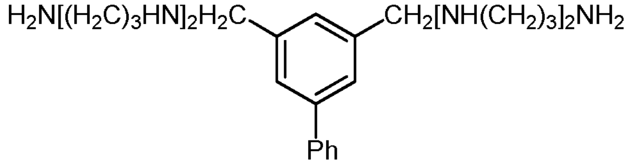

,

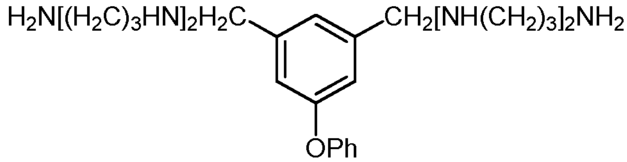

,

and a salt thereof.

In some aspects, $L^1$ is a bond.

In some aspects, the polyamine compound is selected from

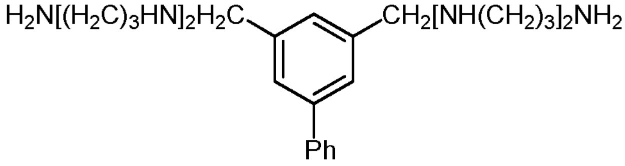

,

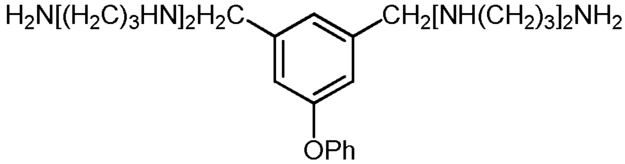

,

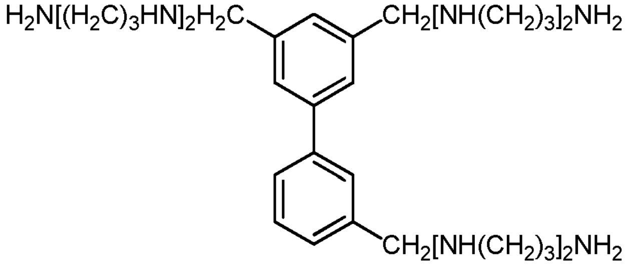

,

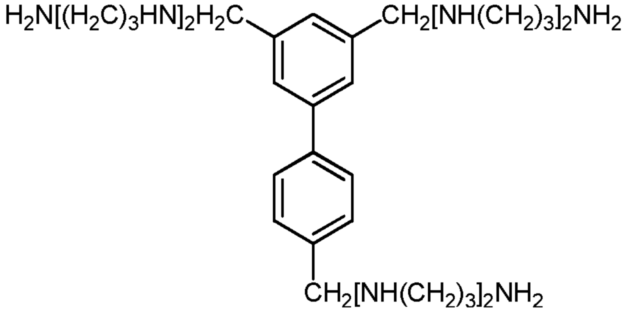

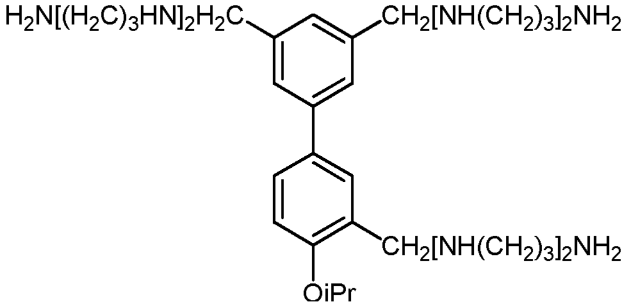

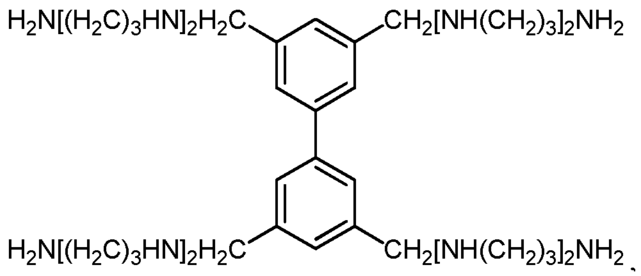

and a salt thereof.

The polyamine compound comprises at least four primary or secondary amino groups. In some aspects, the polyamine compound comprises at least six primary or secondary amino groups.

$R^a$ is $-CH_2[NH(CH_2)_n]_pNH_2$; each n is an integer independently selected from 3 to 12; and each p is an integer independently selected from 1 to 3. In some aspects, $R^5$ is selected from hydrogen, phenyl, and phenyloxy.

The polyamine compound is selected from

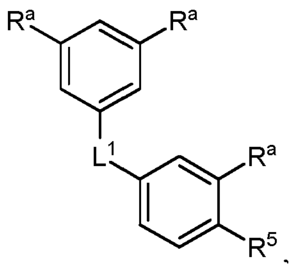

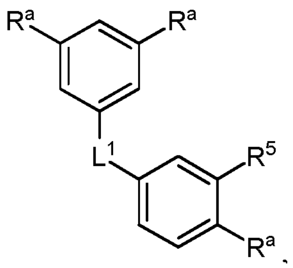

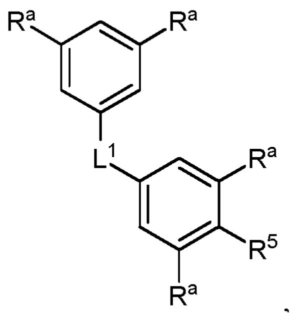

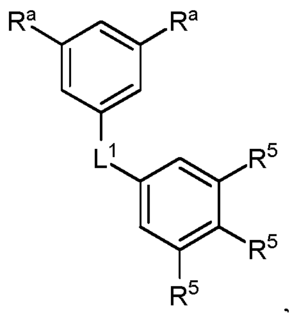

and a salt thereof.

In some aspects, $R^5$ is hydrogen. $L^1$ is selected from a bond and O

$R^a$ is $-CH_2[NH(CH_2)_n]_pNH_2$; each n is an integer independently selected from 3 to 12; and each p is an integer independently selected from 1 to 3.

In some aspects, the polyamine compound is selected from

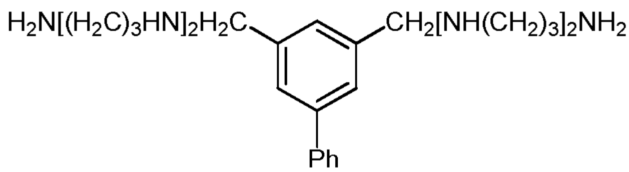

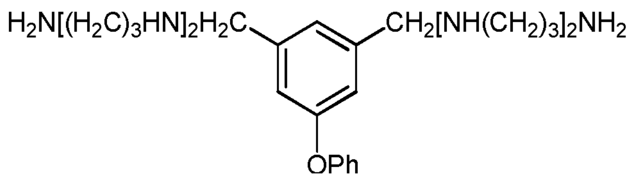

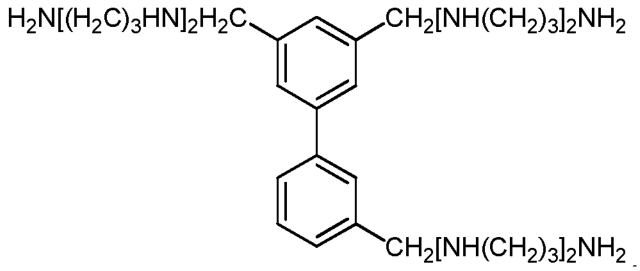

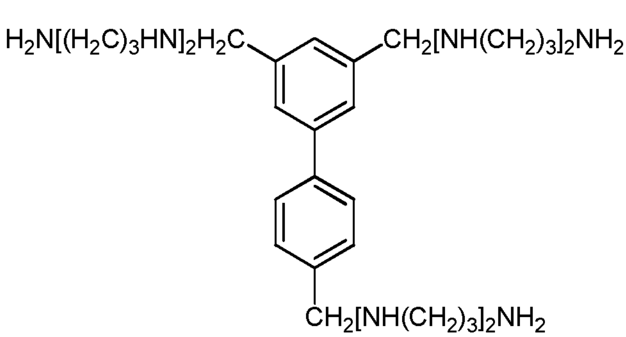

,

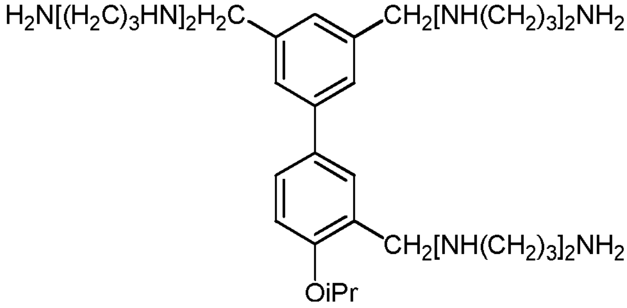

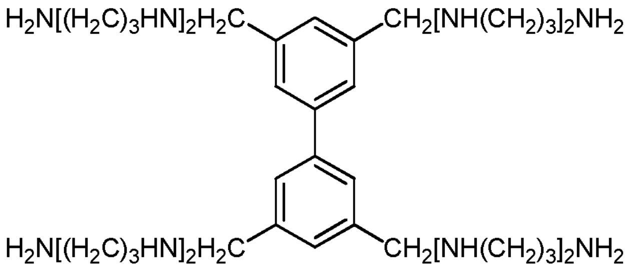

,

and a salt thereof.

In some aspects, $R^5$ is hydroxyl, alkoxy, or cycloalkoxy. Preferably, $R^5$ is alkoxy.

$R^a$ is $-CH_2[NH(CH_2)_n]_pNHR^4$; each n is an integer independently selected from 3 to 12; and each p is an integer independently selected from 1 to 3. Preferably, $R^4$ is alkyl.

In some aspects, $R^a$ is $-CH_2[NH(CH_2)_n]_pNHR^4$; each n is an integer independently selected from 3 to 12; and each p is an integer independently selected from 1 to 3. Preferably, n is 3. More preferably, $R^4$ is not hydrogen.

In some aspects, the polyamine compound is:

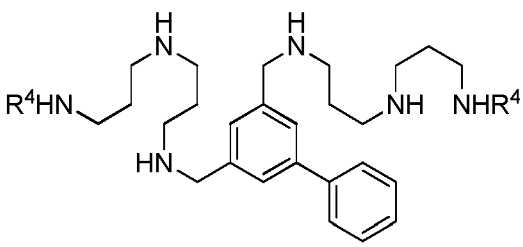

or a salt thereof. Preferably, $R^4$ is isobutyl.

Each $L^1$ is a member independently selected from a bond and -O-.

Each $R^5$ is a member independently selected from hydrogen, alkyl, hydroxyl, alkoxy, halo, fluoroalkyl, fluoroalkyloxy, and hydroxyalkyl.

In some aspects, the polyamine compound is

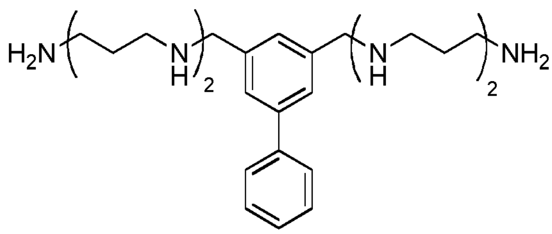

or a salt thereof.

In some aspects, the polyamine compound is

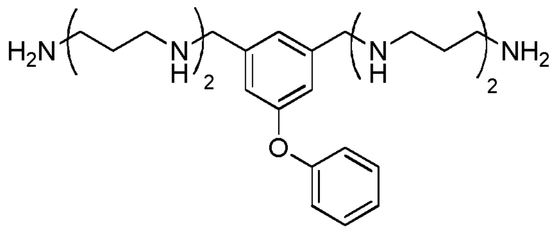

or a salt thereof.

In certain aspects, the compounds of the present invention are antimicrobial and provide triple action against bacteria and biofilms. Advantageously, the antimicrobial compounds of the present invention have specific activity against biofilms.

Organisms residing in biofilms present a complex extracellular matrix of polysaccharides (exopolysaccharides) and proteins. As a result of this complex matrix, nutrient-limiting conditions exist that alter the normal or planktonic metabolic state. These conditions reduce the efficacy of traditional antibiotic agents, rendering them up to 1,000x less active against biofilms. A hallmark of these exopolysaccharides is the presentation of acidic residues from repeated glucoronic acid motifs and pyruvate derived acetals. A recent study by Losick and co-workers demonstrated that the simple polyamines spermine and norspermidine (FIG. 23A) were naturally occurring inhibitors of biofilm formation, endogenously produced at high concentrations (50-80 μM) in response to nutrient limiting conditions and waste accumulation in mature pellicles (Kolodkin-Gal, I. et al., A self-produced trigger for biofilm disassemby that targets exopolysaccharide. Cell 149 (2012)). In this study, they were able to demonstrate that norspermidine could inhibit biofilm formation at 25 μM and showed that, at similar concentrations, it could disperse the exopolysaccharide component of the matrix but not the protein component. Interestingly, spermidine was only active at much higher concentrations (~1 mM) leading them to propose a rationale for this activity in the ability of the polyamines to engage the acidic residues in the matrix at regular intervals (FIG. 23B).

In certain aspects, compounds of the present invention having increased numbers of chains, produce a more effective compound against *A. baumannii.* For example, compounds with four polyamine chains can be generated with Pd(II) mediated dimerization of 5-bromoisopthalaldehyde followed by reductive amination (FIG. 26).

Certain compounds combine a hydrophobic backbone with a cationic tail that have the functionality to inhibit biofilm formation, disrupt established biofilms, and kill the emerging planktonic bacteria. Certain of the compounds are set forth in FIGS. 27 and 28.

In a particular embodiment, the polyamine compound may comprise a hydrophobic moiety head and at least one hydrophilic moiety tail comprising a polyamine group. When the polyamine compound comprises more than one hydrophilic moiety tails, the hydrophilic moiety tails may be the same, or alternatively, the hydrophilic moiety tails may be different.

As discussed above, the exemplary polyamine compounds shown herein are not intended to be limiting.

## SYNTHESIS

The synthesis of diaminopropane substituted backbones is straightforward and results in CZ-4, 12, 32 (FIG. 25) from the known mono-Boc protected diaminopropane and commercially available aldehydes (benzaldehyde, isopthalaldehyde or 1,3,5-benzenetricarboxaldehyde). This three-step synthetic procedure proceeds via reductive amination (Baxter, E. W. & Reitz, A. B. Reductive Aminations of Carbonyl Compounds with Borohydride and Borane Reducing Agents. Org Reac 1, 59 (2004)) and acidic removal of the Boc group. The norspermidine series *(e.g.,* CZ-7, CZ-25, and CZ-52) can be prepared in a similar manner from the mono-Boc protected norspermidine (FIG. 25). No purification is

required until a final recrystallization of the HCl salt, which has allowed easy preparation of these compounds on larger scale.

In some embodiments, the polyamine compound may be produced by the reductive amination method of General Synthetic Scheme 1.

## General Synthetic Scheme 1

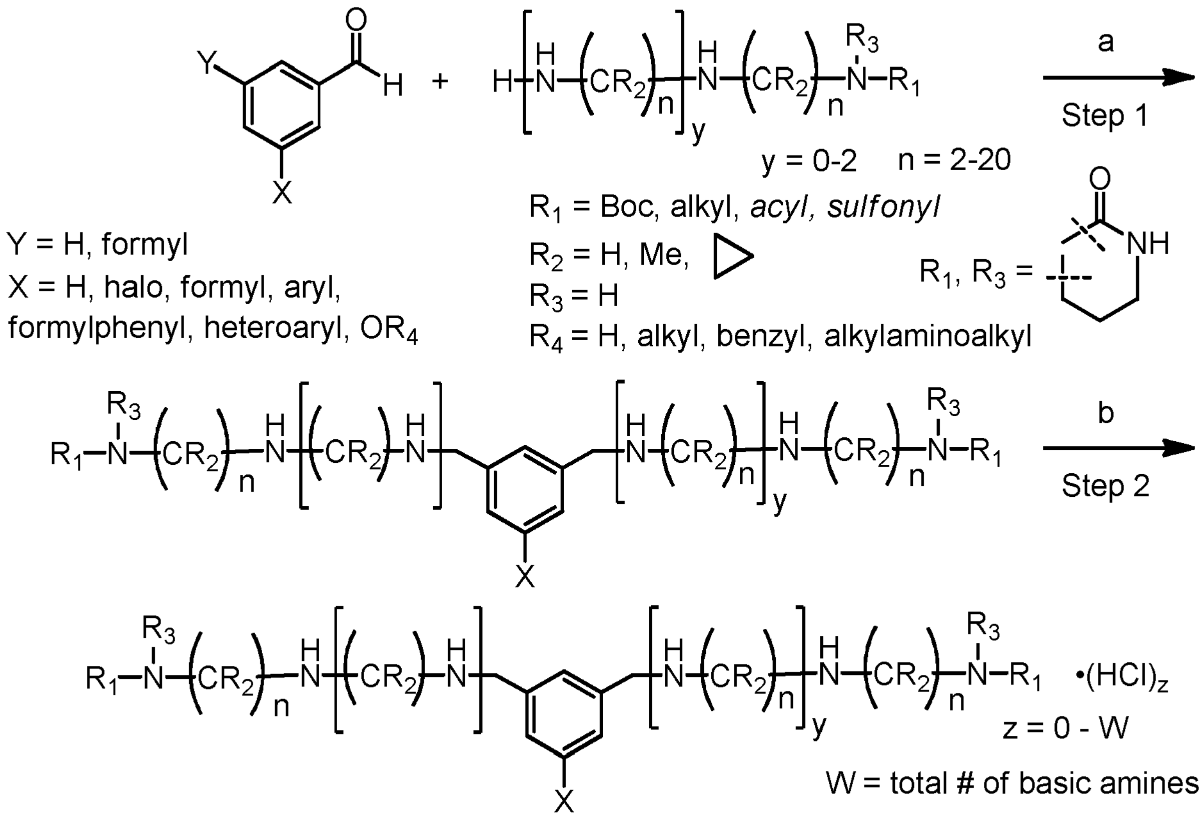


Reagents: (a) MeOH, 3 Å molecular sieves, rt, 1-28 h; $NaBH_4$; (b) HCl, MeOH, rt 1-4 h.

The General Synthetic Schemes included herein set forth methods of preparing some compounds within the aspects and embodiments disclosed herein. In some alternative aspects, these methods can be used to produce a compound of the one of the aspects and embodiments disclosed herein. In some alternative aspects, these methods can be used to produce a precursor or starting material for producing such a compound.

In General Synthetic Schemes 1, 2, 3, and 5, "$CR_2$" indicates a methylene with two substituents, which may be hydrogen, methyl (or, alternatively, lower alkyl), or spirocyclopropane *(i.e.,* the two substituents are the spirocyclic ring).

In some embodiments, the polyamine compound may be produced by the acylation method of General Synthetic Scheme 2.

## General Synthetic Scheme 2

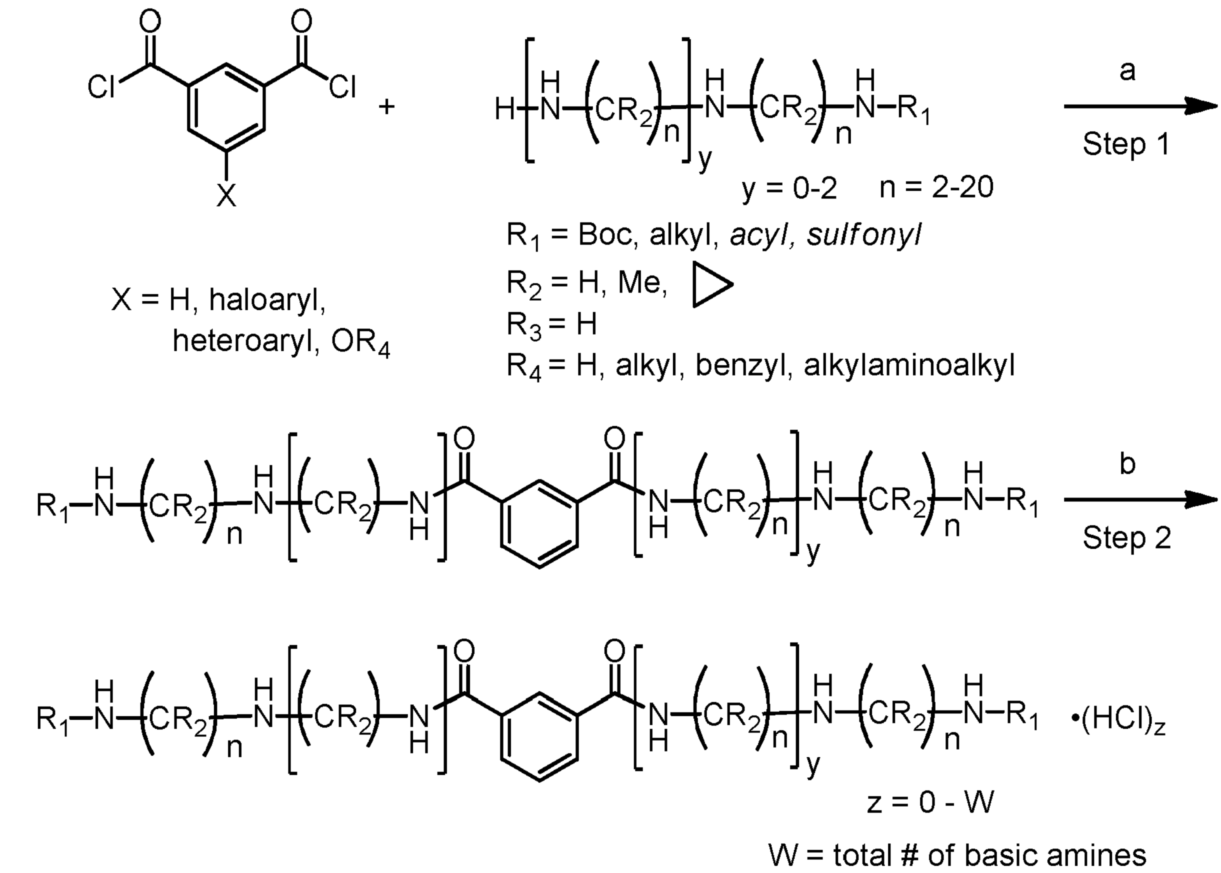


Reagents: (a) TEA, $CH_2Cl_2$, rt, 1-28 h; (b) HCl, MeOH, rt 1-4 h.

In some embodiments, the polyamine compound may be produced by the reductive amination method of General Synthetic Scheme 3.

## General Synthetic Scheme 3

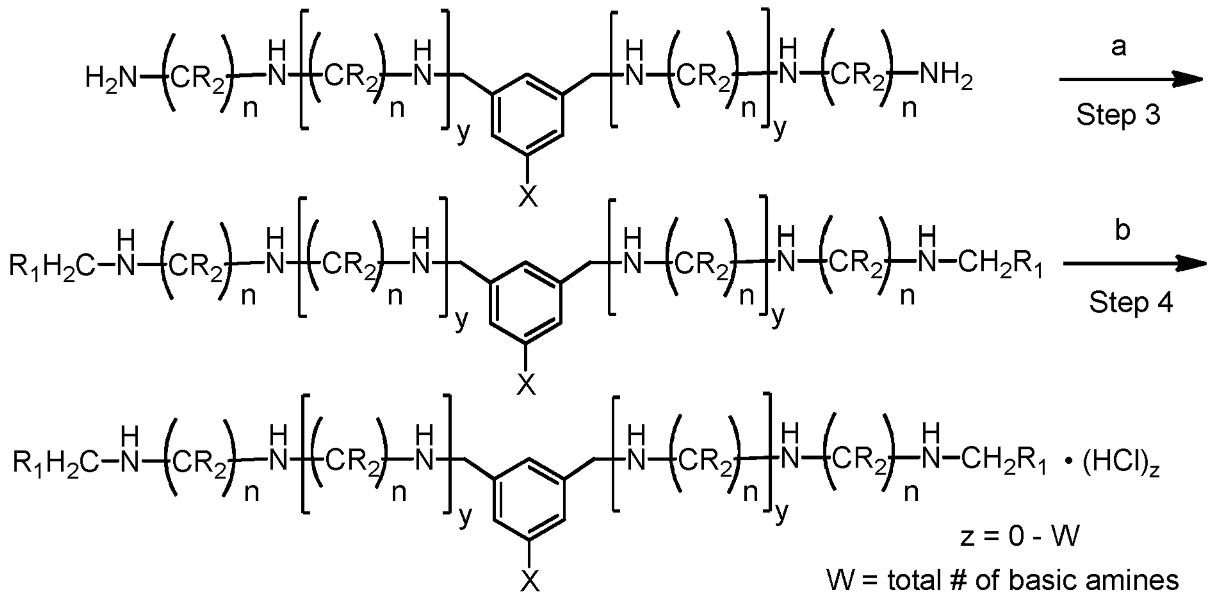

Reagents: (a) $R_1CHO$, MeOH, 3 Å molecular sieves, rt, 1-28 h; $NaBH_4$; (b) HCl, MeOH, rt 1-4 h.

In some embodiments, the polyamine compound may be produced by the reductive amination method of General Synthetic Scheme 4.

**General Synthetic Scheme 4**

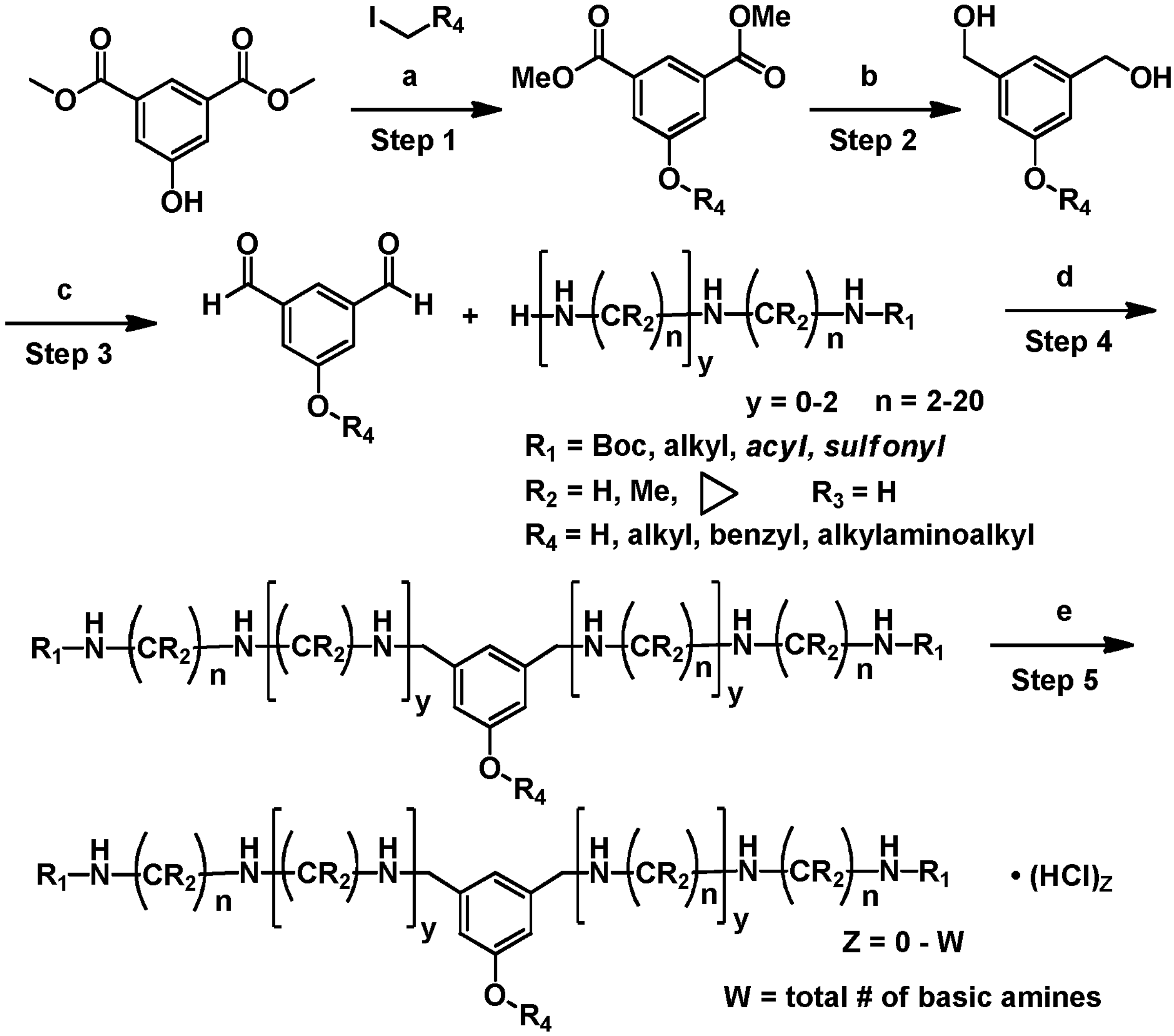


Reagents: (a) $CH_3CN$, $Cs_2CO_3$, rt, 16 h; (b) THF, $LiAlH_4$, rt, 8 h; (c) PCC, $CH_2Cl_2$, rt 1-4 h; (d) MeOH, 3 Å molecular sieves, rt, 1-28 h; $NaBH_4$; (e) HCl, MeOH, rt 1-4 h.

In some aspects, the $R_4CH_2I$ can include a different leaving group for the nucleophilic substitution reaction of (a). Other leaving groups useful for phenol alkylation include Br, Cl, tosylate, mesylate, triflate, and the like.

The precursor compounds shown below were made by the method of Steps 1 to 3 in the General Synthetic Scheme 4 above.

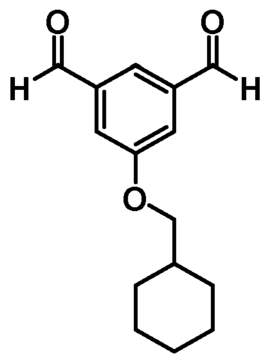

5-(Cyclohexylmethoxy)isophthalaldehyde: $^1$H NMR (300 MHz, $CDCl_3$) δ ppm 10.02 (s, 2H), 7.91 (s, 1H), 7.61 (s, 2H), 3.84 (d, $J$ = 6.0 Hz, 2H), 1.87-1.67 (m, 6H), 1.31-1.03 (m, 5H). $^{13}$C NMR (75 MHz, $CDCl_3$) δ ppm 191.4, 160.9, 138.6, 124.3, 120.2, 74.6, 37.9, 30.1, 26.7, 26.0.

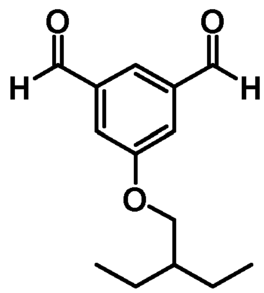

5-(2-Ethylbutoxy)isophthalaldehyde: $^1$H NMR (500 MHz, $CDCl_3$) δ ppm 10.01 (s, 2H), 7.90 (s, 1H), 7.62 (s, 2H), 3.94 (d, $J$ = 5.5 Hz, 2H), 1.68 (hept, $J$ = 6.5 Hz, 1H), 1.46 (dec, $J$ = 6.5 Hz, 4H), 0.91 (t, J = 7.5 Hz, 6H). $^{13}$C NMR (125 MHz, $CDCl_3$) δ ppm 191.1, 160.7, 138.5, 124.1, 120.0, 71.1, 40.9, 23.5, 11.3.

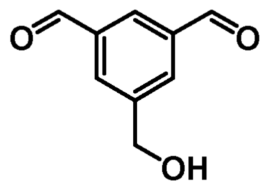

5-(Hydroxymethyl)isophthalaldehyde: $^1$H NMR (500 MHz, $CDCl_3$) δ ppm 10.09 (s, 2H), 8.27 (s, 1H), 8.15 (s, 2H), 4.88 (s, 2H), 2.54 (br s, 1H). $^{13}$C NMR (125 MHz, $CDCl_3$) δ ppm 191.4, 143.7, 137.4, 132.8, 130.3, 63.9.

In some embodiments, the polyamine compound may be produced by the method of Scheme 5, wherein $R^1$ may be hydrogen or a $C_1$-$C_{13}$ alkyl group.

## Scheme 5

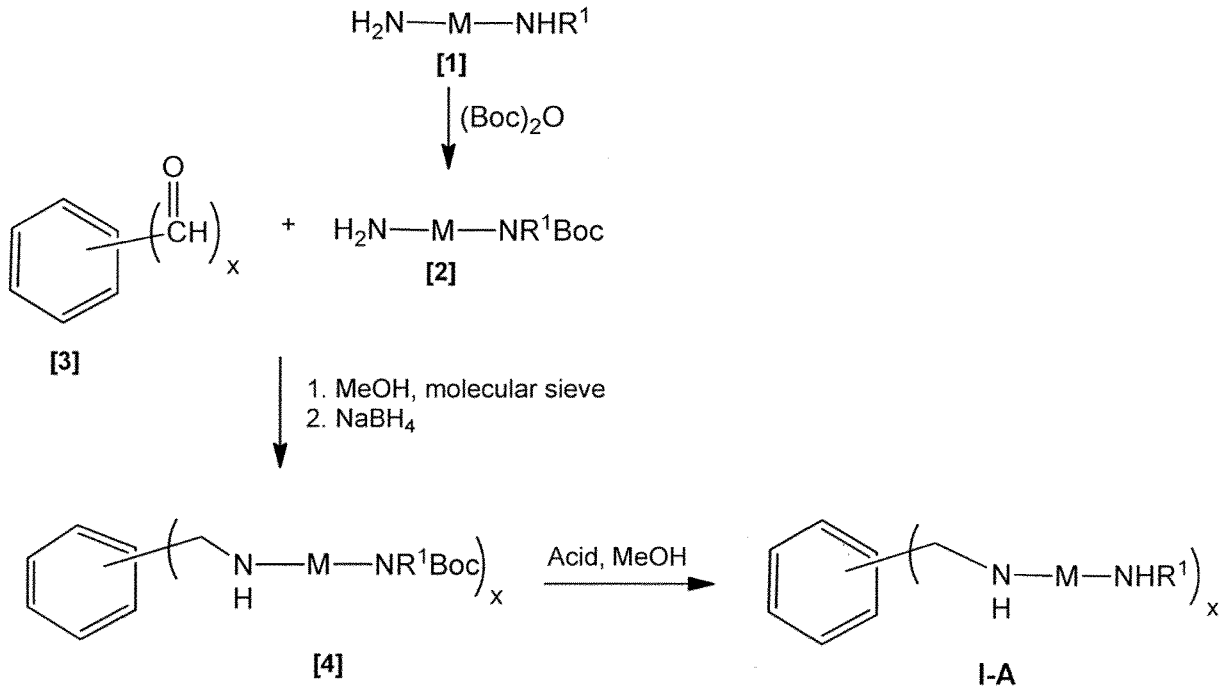


[1] [2] [3] [4] I-A

The method of Scheme 6 may include reacting a polyamine of formula **[1]** with di-t-butyldicarbonate compound [$(Boc)_2O$] to protect at least one terminal amine group of the polyamine **[1],** while leaving at least one terminal amine group of the polyamine **[1]** unprotected. The resulting polyamine of formula **[2]** having at least one Boc-protected terminal amine is reacted with a substituted benzadehyde of formula **[3].** Then, the resulting product is reduced, such as by a hydride reducing agent (e.g., $NaBH_4$ or $LiAlH_4$) to provide a corresponding polyamine conjugate of formula **[4]** having the terminal amine group on at least one hydrophilic polyamine chain Boc-protected. The Boc-protected terminal amine group is then deprotected, such as by acid hydrolysis, to provide the polyamine compound of Formula I-A comprising a hydrophobic phenyl group and at least one hydrophilic polyamine chain.

In some embodiments, the polyamine compound of formula VIII-P may be produced by the method of Scheme 6, wherein $R^1$ may independently be hydrogen or $C_1$-$C_{13}$ alkyl group.

## Scheme 6

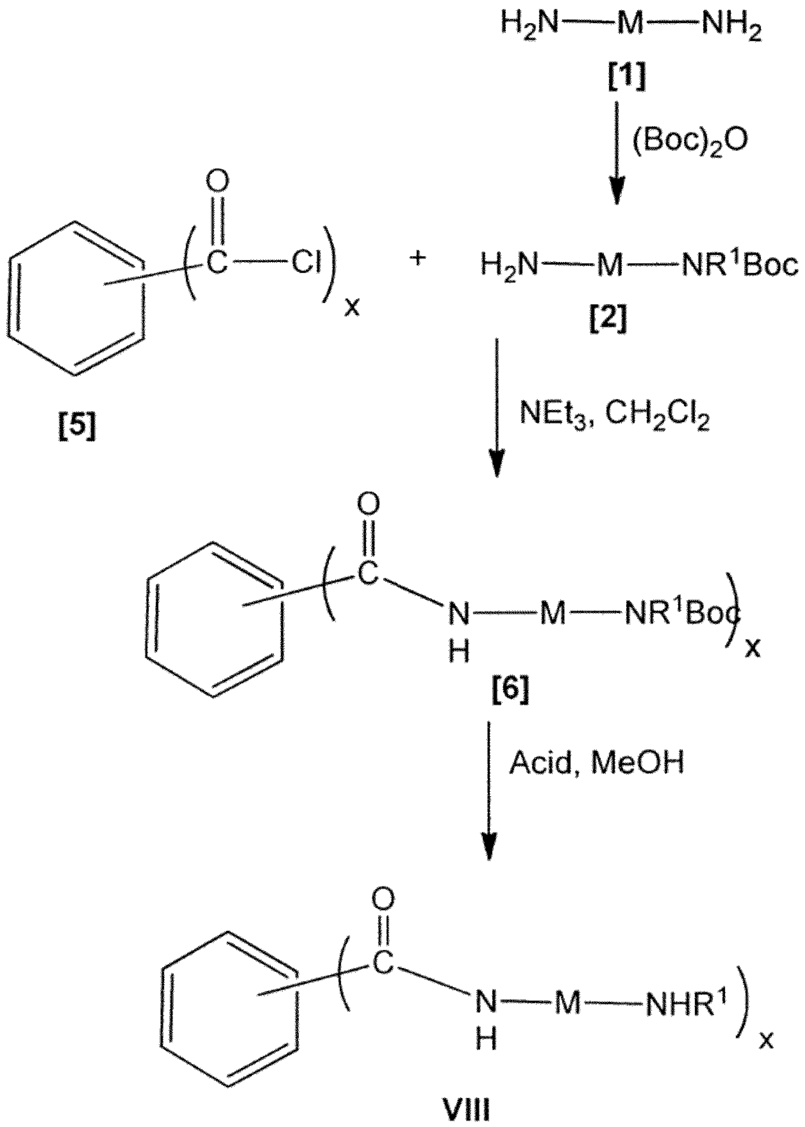


## VIII-P

The method of Scheme 7 may include reacting the substituted benzoyl chloride of formula **[5]** with a polyamine of formula **[2]** having at least one terminal amine group protected with a Boc-protecting group, in a presence of base such as triethylamine ($NEt_3$) to provide a corresponding substituted benzyl amide of formula **[6].** The compound of formula **[6]** comprises a hydrophobic phenyl group and at least one hydrophilic polyamine chain having a Boc-protected terminal amine group. Then, the Boc-protected terminal amine group of the hydrophilic polyamine chain is deprotected, such as by acid hydrolysis, to provide the polyamine compound of Formula VIII-P.

The polyamine compounds exhibit unexpectedly superior ability in treating biofilms, compared to most known antimicrobial compounds. The microorganisms in the biofilm community may be eradicated effectively and rapidly such that they have minimum, if any, opportunity to upregulate their defense mechanism and develop resistance against the polyamine compound. Without being bound by any theory, it is believed that the unexpectedly superior biofilms treatment is due to the beneficial synergistic of hydrophobicity and hydrophilicity of the polyamine compound. It is believed that the hydrophobic moiety of the polyamine compound facilitates the dispersion of the microorganisms in biofilms, while the hydrophilic polyamine moiety provides an antimicrobial effect on the dispersed microorganisms.

The polyamine compounds may eradicate the biofilms, reduce the formation of biofilms, or inhibit the formation of biofilms. The hydrophobicity and hydrophilicity of the polyamine compounds may be designed by tailoring the hydrophobic moieties and hydrophilic moieties of the polyamine compounds such that the desired level of antimicrobial effect on the biofilms may be achieved. One skilled in the arts recognizes the parameters controlling the hydrophobicity/hydrophilicity effect, and, therefore, may readily modify the teachings of present disclosure to produce various polyamine compounds without departing from the scope of present disclosure. As non-limiting examples, the hydrophilicity effect of the polyamine compound may be modified by varying numbers of the hydrophilic polyamine chains in the polyamine compound, numbers of amine groups in the hydrophilic polyamine chains, numbers of carbons between amine groups in the hydrophilic polyamine chains, *etc.* As non-limiting examples, the hydrophobicity effect of the polyamine compound may be modified by varying positions of the hydrophilic polyamine chains on the hydrophobic groups, altering chemical structures of the hydrophobic groups to other known non-polar functional group, *etc.*

The polyamine compounds may exhibit enhanced antimicrobial effect on biofilms comprised of Gram-negative or Gram-positive bacteria. The polyamine compounds may exhibit enhanced antimicrobial effect on biofilms consisting of mycobacteria.

In one embodiment, the antimicrobial composition may comprise a polyamine compound and at least one additive. Various additives may be used for the antimicrobial composition. By way of non-limiting examples, the additives may further enhance the dispersion of microorganisms in biofilms, impart the antimicrobial effect against the dispersed microorganisms, facilitate the application/administration of the antimicrobial composition to the biofilms, improve the stability of the antimicrobial composition, control the release/application rate of the antimicrobial composition to the biofilms, *etc.* Nonlimiting examples of additives for further enhancing the antimicrobial effect may be biocide and other bactericide. By way of non-limiting examples, the additives for facilitating the administration of the antimicrobial composition may include a pharmaceutically acceptable carrier typically used for medical or pharmaceutical applications, an emulsifier or dispersant typically used for industrial applications.

The antimicrobial composition may be formulated to provide the desired level of antimicrobial effect on the biofilms by selecting a polyamine compound and other additives as well as by adjusting the amount of each component in the antimicrobial composition. In some embodiments, the antimicrobial composition may be formulated to inhibit the formation of biofilms. In some embodiment, the antimicrobial composition may be formulated to disrupt the biofilms. In still other embodiments, the antimicrobial composition may be formulated to eradicate substantially all microorganisms in the biofilms.

Any suitable amount of polyamine can be used in the compositions and methods of the invention. In general, the polyamines are used in concentrations ranging from about 1 ppm to about 100,000 ppm, or higher. The concentration of a polyamine used in a composition or method of the invention can be, for example, from about 1 to about 100,000 ppm, or from about 10 to about 10,000 ppm, or from about 100 to about 1,000 ppm, or from about 1 to about 100 ppm, or from about 1,000 to about 10,000 ppm, or from about 10,000 to about 100,000 ppm. The concentration of a polyamine can be about 1; 2; 3; 4; 5; 6; 7; 8; 9; 10; 15; 20; 25; 30; 35; 40; 45; 50; 55; 60; 65; 70; 75; 80; 85; 90; 95; 100; 125; 150; 175; 200; 225; 250; 275; 300; 325; 350; 375; 400; 425; 450; 475; 500; 525; 550; 575; 600; 625; 650; 675; 700; 725; 750; 775; 800; 825; 850; 875; 900; 925; 950; 975; 1000; 1500; 2000; 2500; 3000; 3500; 4000; 4500; 5000; 5500; 6000; 6500; 7000; 7500; 8000; 8500; 9000; 9500; 10,000; 12,500; 15,000; 17,500; 20,000; 22,500; 25,000; 27,500; 30,000; 32,500; 35,000; 37,500; 40,000; 42,500; 45,000; 47,500; 50,000; 52,500; 55,000; 57,500; 60,000; 62,500; 65,000; 67,500; 70,000; 72,500; 75,000; 77,500; 80,000; 82,500; 85,000; 87,500; 90,000; 92,500; 95,000; 97,500; or about 100,000 ppm. Other concentrations of polyamines can be useful in the compositions and methods of the invention, depending in part on factors including the specific polyamine used, the presence of potentiating agents if any, or the species of microorganisms that are targeted.

In certain aspects, increasing number of polyamine chains on the hydrophobic backbone systematically increase the antimicrobial activity. For example, it is demonstrated herein that with a single chain of diaminopropane added to a benzyl backbone (CZ-4, FIG. 25), the MIC (as defined by the Clinical and Laboratory Standards Institute (CLSI)) against MRSA is greater than 1,200 μg/mL, whereas two chains decrease the MIC to 300 μg/mL (CZ-12) and three chains reduce it further to 45 μg/mL (CZ-32). Similarly, with a single chain of norspermidine attached to a benzyl backbone (CZ-7, FIG. 25), the MIC against MRSA is greater than 1,200 μg/mL. By adding a second chain of norspermidine (CZ-25), the MIC is 45 μg/mL. With a third chain of norspermidine attached (CZ-52), the MIC becomes 3 μg/mL (FIG. 25).

This trend of enhancing antimicrobial activity by increasing the number of polyamine chains attached to a backbone is effective for treating MRSA, but modulating this trend with increased hydrophobicity enhances the activity of CZ compounds against A. *baumannii.* Interestingly, CZ-52, with three norspermidine chains, has greater activity against MRSA biofilms than against *A. baumannii,* whereas CZ-58 (FIG. 27) has greater activity (10-fold increase) against *A. baumannii* biofilms than MRSA biofilms (see Table 4 below).

## APPLICATIONS

As described herein, biofilms can also affect a wide variety of biological, medical, and processing operations. Methods and treatments using a polyamine compound, or a combination of a polyamine compound with another compound, may include killing, dispersing, treating, reducing biofilms or preventing or inhibiting biofilm formation.

In one embodiment, the invention provides a non-therapeutical method for dispersing or killing a biofilm, the method comprising a step of treating the biofilm with an anti-biofilm composition, thereby effectively dispersing or killing the biofilm; wherein the method comprises, consists essentially of, or consists of using a polyamine compound or composition as set forth in any of the embodiments or aspects described herein.

In some aspects, the step of treating the biofilm with an anti-biofilm composition effectively disperses the biofilm.

In another embodiment, the invention provides a non-therapeutic method for inhibiting formation of a biofilm, the method comprising a step of treating planktonic bacteria with a polyamine composition as set forth in any of the embodiments or aspects herein, thereby inhibiting incorporation of the planktonic bacteria into the biofilm.

In some aspects, the formation of a biofilm is inhibited. In other aspects, a previously formed biofilm is dispersed. In still other aspects, substantially all of the cells comprising a biofilm are killed.

A surface may comprise a medical device, a wound dressing, a contact lens, or an oral device. Such medical device is selected from a clamp, forceps, scissors, skin hook, tubing, needle, retractor, scaler, drill, chisel, rasp, saw, catheter, orthopedic device, artificial heart valve, prosthetic joint, voice prosthetic, stent, shunt, pacemaker, surgical pin, respirator, ventilator, and an endoscope and combinations thereof.

In some aspects, the method described herein comprises, consists essentially of, or consists of using the polyamine compound or composition described in any of the embodiments or aspects herein.

In some aspects, the invention provides a method that comprises, consists essentially of, or consists of using a polyamine compound or composition from any of the embodiments or aspects described herein.

In some embodiments, the invention provides a compound for use in a method for dispersing or killing a biofilm, the method comprising a step of treating the biofilm with an anti-biofilm composition, thereby effectively dispersing or killing the biofilm; wherein the anti-biofilm composition comprises, consists essentially of, or consists of a polyamine compound selected from

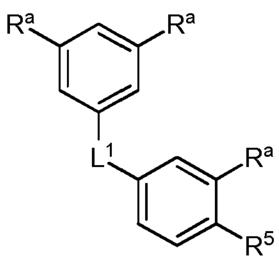 , 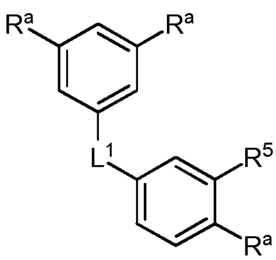 , 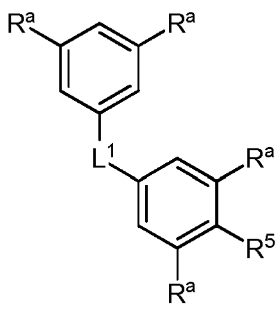 , 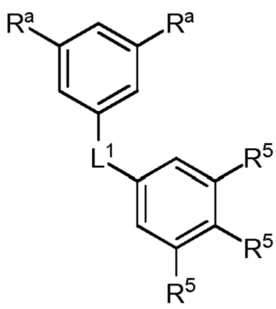 ,

and a salt thereof;
wherein:

- each $L^1$ is selected from the group consisting of a bond and -O-;
- each $R^a$ is $-CH_2[NH(CH_2)_n]_pNHR^4$;
- each n is an integer independently selected from 3 to 12;
- each p is an integer independently selected from 1 to 3;
- each $R^4$ is a member independently selected from the group consisting of hydrogen, alkyl, arylalkyl, and cycloalkylalkyl; and
- each $R^5$ is a member independently selected from the group consisting of hydrogen, alkyl, hydroxyl, alkoxy, halo, fluoroalkyl, fluoroalkyloxy, and hydroxyalkyl,

wherein the polyamine compound comprises at least four primary or secondary amino groups..

Each $L^1$ is a member independently selected from a bond and -O-.

Each $R^4$ is a member independently selected from hydrogen, alkyl, arylalkyl, and cycloalkylalkyl. In some aspects, $R^4$ is hydrogen, butyl, isobutyl, hexyl, or octyl.

In some aspects, at least one $R^4$ is a member independently selected from alkyl, arylalkyl, and cycloalkylalkyl. In some aspects, at least one $R^4$ is an independently selected alkyl. In some aspects, at least one $R^4$ is not hydrogen. In some aspects, at least one $R^4$ is butyl, isobutyl, hexyl, or octyl.

In some aspects, at least one pair of $R^4$ are both a member independently selected from alkyl, arylalkyl, and cycloalkylalkyl. In some aspects, at least one pair of $R^4$ are both an alkyl. In some aspects, at least one pair of $R^4$ is not hydrogen. In some aspects, at least one pair of $R^4$ is butyl, isobutyl, hexyl, or octyl.

In some aspects, $R^5$ is hydrogen.

The polyamine compound comprises at least four primary or secondary amino groups. In some aspects, the polyamine compound comprises at least six primary or secondary amino groups.

$R^a$ is $-CH_2[NH(CH_2)_n]_pNH_2$; each n is an integer independently selected from 3 to 12; and each p is an integer independently selected from 1 to 3.

In some aspects, the polyamine compound is selected from:

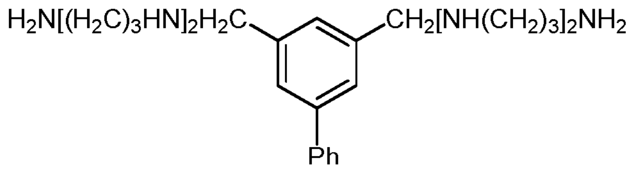

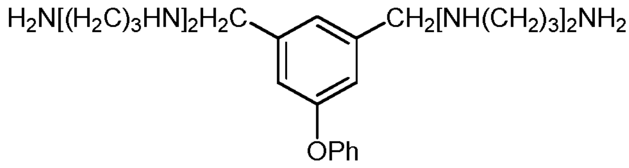

,

and a salt thereof.

The polyamine compound is selected from

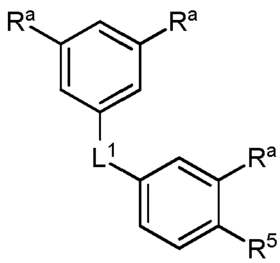
,
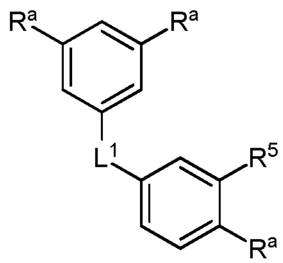
,
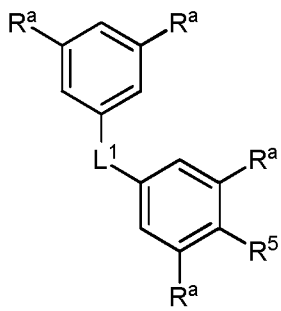
,
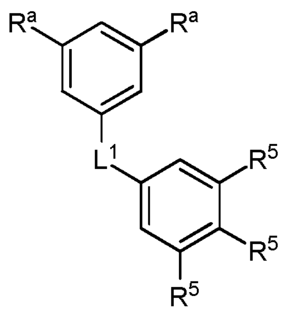
,

and a salt thereof.

In some aspects, $R^5$ is hydrogen. $L^1$ is selected from a bond and O

In some aspects, the polyamine compound is selected from

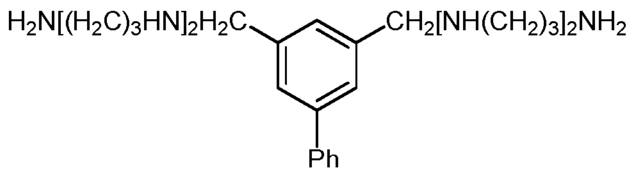

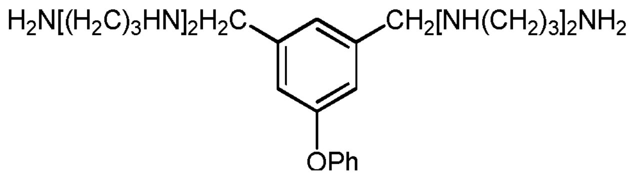

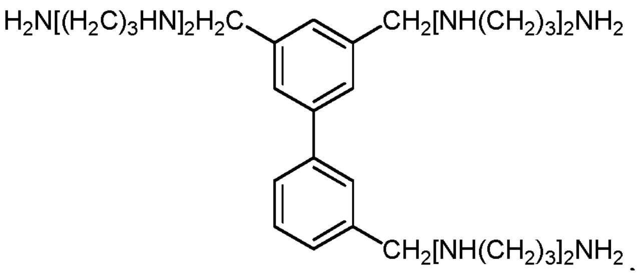

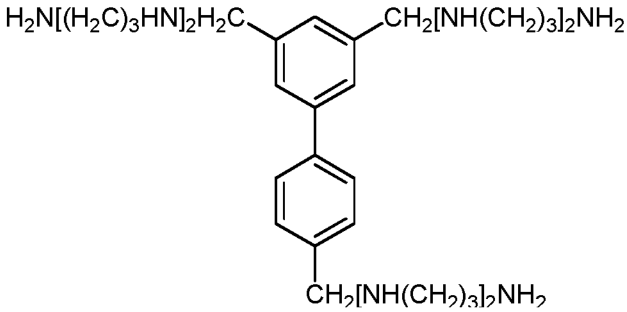

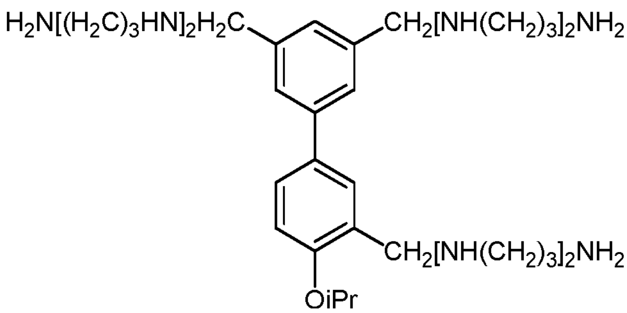

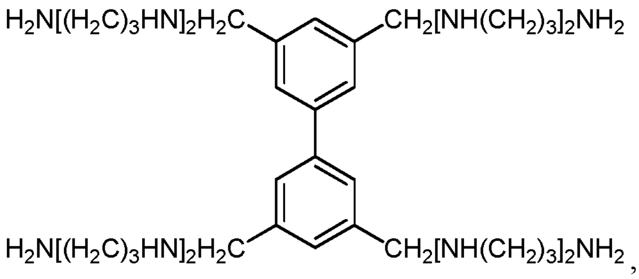

and a salt thereof.

$R^a$ is $-CH_2[NH(CH_2)_n]_pNH_2$; each n is an integer independently selected from 3 to 12; and each p is an integer independently selected from 1 to 3. In some aspects, n is 3. In some aspects, n is 4. In some aspects, n is 5, 6, 7, 8, 9, 10, 11, or 12.

In some aspects, the polyamine compound is

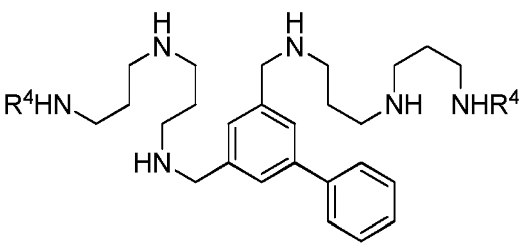

or a salt thereof

In some aspects, $R^4$ is alkyl. In some aspects, $R^4$ is isobutyl.

In some embodiments, the polyamine compound for use or combination of a polyamine compound for use and at least one other composition may be used to treat Gram negative and Gram positive bacteria (including strains that are resistant to conventional antibiotics), mycobacteria (including *Mycobacterium tuberculosis),* enveloped viruses, fungi and even transformed or cancerous cells.

Compounds, compositions, and methods described herein can be used to kill, disperse, treat, reduce biofilms, or prevent or inhibit biofilm formation. In exemplary methods, the biofilms are formed by biofilm-forming bacteria. The bacteria can be a gram-negative bacterial species or a gram-positive bacterial species. Nonlimiting examples of such bacteria include a member of the genus *Actinobacillus* (such as *Actinobacillus actinomycetemcomitans*), a member of the genus *Acinetobacter* (such as *Acinetobacter baumannii*), a member of the genus *Aeromonas,* a member of the genus *Bordetella* (such as *Bordetella pertussis, Bordetella bronchiseptica,* or *Bordetella parapertussis*), a member of the genus *Brevibacillus,* a member of the genus *Brucella,* a member of the genus *Bacteroides* (such as *Bacteroides fragilis*), a member of the genus *Burkholderia* (such as *Burkholderia cepacia* or *Burkholderia pseudomallei*), a member of the genus *Borelia* (such as *Borelia burgdorferi*), a member of the genus *Bacillus* (such as *Bacillus anthracis* or *Bacillus subtilis*), a member of the genus *Campylobacter* (such as *Campylobacter jejuni*), a member of the genus *Capnocytophaga,* a member of the genus *Cardiobacterium* (such as *Cardiobacterium hominis*), a member of the genus *Citrobacter,* a member of the genus *Clostridium* (such as *Clostridium tetani* or *Clostridium dijficile*), a member of the genus *Chlamydia* (such as *Chlamydia trachomatis, Chlamydia pneumoniae,* or *Chlamydia psiffaci),* a member of the genus *Eikenella* (such as *Eikenella corrodens*), a member of the genus *Enterobacter,* a member of the genus *Escherichia* (such as *Escherichia coli*), a member of the genus *Francisella* (such as *Francisella tularensis*), a member of the genus *Fusobacterium,* a member of the genus *Flavobacterium,* a member of the genus *Haemophilus* (such as *Haemophilus ducreyi* or *Haemophilus influenzae*), a member of the genus *Helicobacter* (such as *Helicobacter pylori*), a member of the genus *Kingella* (such as *Kingella kingae*), a member of the genus *Klebsiella* (such as *Klebsiella pneumoniae*), a member of the genus *Legionella* (such as *Legionella pneumophila*), a member of the genus *Listeria* (such as *Listeria monocytogenes*), a member of the genus *Leptospirae,* a member of the genus *Moraxella* (such as *Moraxella catarrhalis*), a member of the genus *Morganella,* a member of the genus *Mycoplasma* (such as *Mycoplasma hominis* or *Mycoplasma pneumoniae*), a member of the genus *Mycobacterium* (such as *Mycobacterium tuberculosis* or *Mycobacterium leprae*), a member of the genus *Neisseria* (such as *Neisseria gonorrhoeae* or *Neisseria meningitidis*), a member of the genus *Pasteurella* (such as *Pasteurella multocida*), a member of the genus *Proteus* (such as *Proteus vulgaris* or *Proteus mirablis*), a member of the genus *Prevotella,* a member of the genus *Plesiomonas* (such as *Plesiomonas shigelloides*), a member of the genus *Pseudomonas* (such as *Pseudomonas aeruginosa*), a member of the genus *Providencia,* a member of the genus *Rickettsia* (such as *Rickettsia rickettsii* or *Rickettsia typhi*), a member of the genus *Stenotrophomonas* (such as *Stenotrophomonas maltophila*), a member of the genus *Staphylococcus* (such as *Staphylococcus aureus* or *Staphylococcus epidermidis),* a member of the genus *Streptococcus* (such as *Streptococcus viridans, Streptococcus pyogenes* (group A), *Streptococcus agalactiae* (group B), *Streptococcus bovis,* or *Streptococcus pneumoniae*), a member of the genus *Streptomyces* (such as *Streptomyces hygroscopicus*), a member of the genus *Salmonella* (such as *Salmonella enteriditis, Salmonella typhi,* or *Salmonella typhimurium*), a member of the genus *Serratia* (such as *Serratia marcescens*), a member of the genus *Shigella,* a member of the genus *Spirillum* (such as *Spirillum minus*), a member of the genus *Treponema* (such as *Treponema pallidum*), a member of the genus *Veillonella,* a member of the genus *Vibrio* (such as Vibrio *cholerae, Vibrio parahaemolyticus,* or *Vibrio vulnificus*), a member of the genus *Yersinia* (such as *Yersinia enter ocolitica, Yersinia pestis,* or *Yersinia pseudotuberculosis*), and a member of the genus *Xanthomonas* (such as *Xanthomonas maltophilia*).

In some embodiments, the biofilm exposed to the compounds, compositions, or methods of the present invention may comprise Gram-negative or Gram-positive bacteria. In some embodiments, the bacteria are mycobacteria.

In some aspects, the biofilm comprises an antibiotic-resistant bacterial species.

The antimicrobial compounds, compositions, and methods comprising a polyamine compound may be used to control, prevent or kill biofilms in various environments. Sometimes, they may be used for treating biofilms in subjects that include human or other animals. In some embodiments, they may be used for treating biofilms in medical applications such as medical devices, wound dressings, contact lens, oral devices, *etc.* In some embodiments, they may be used for treating or preventing a biofilm-related disorder. In some embodiments, they may be used for treating biofilms in industrial applications such as oil pipelines, water pipelines, water treatment at manufacturing sites, industrial flush solution, industrial wash water, industrial coatings, *etc.* In some embodiments, they may be used for household and hygiene applications. In some embodiments, they may be used for agricultural applications, such as water remediation, crop treatment, etc. In some embodiments, they may be used for food preparation applications, such as meat sprays, fruit and vegetable sanitizers.

In some aspects, the method comprises a step of coating an object with the anti-biofilm composition. In some aspects, the method comprises a step of treating a contact lens with the anti-biofilm composition.

In some embodiments, the polyamine compound or combination of a polyamine compound and at least one

other composition are directed for use in industrial applications, for example oil pipelines, water treatment, water pipelines, fracking water sanitation, milk production facility pipeline flush solution, oil fields, paper and pulp production, machining fluids, ship coatings, shipping, paint, handrail sanitizers, water filtration, biofouling and biocorrosion, natural gas pipeline treatment, HVAC units, etc.

In some embodiments, the polyamine compound or combination of a polyamine compound and at least one other composition are directed for use in household applications, for example, sanitizing wipes, cleansers, toilet bowl inserts, baby care products, toys, etc.

In some embodiments, the polyamine compound or combination of a polyamine compound and at least one other composition are directed for use in environmental applications, for example, agriculture, water remediation, water treatment, crop treatment, etc.

In some aspects, the non-therapeutical method comprises a step of treating a pipe with the anti-biofilm composition. In some aspects, the method comprises a step of treating a heating or cooling tower with the anti-biofilm composition.

In some embodiments, the polyamine compound or combination of a polyamine compound and at least one other composition are directed for use in food production, for example, fruit and vegetable sanitizers, water systems in food production facilities, meat sprays, cooling system sanitizers, air filtration units, feed, packaging, etc.

In some aspects, the anti-biofilm composition is a paint.

Some aspects of this disclosure is directed to compounds for use in methods of treating a biofilm-related disorder in a subject in need thereof, the method comprising administering to the subject an effective amount of a polyamine compound of the present invention.

In some embodiments, the composition is administered to a surface of the subject selected from the group of dermal and mucosal surfaces and combinations thereof. In other embodiments, the surface is an oral surface, a skin surface, a urinary tract surface, a vaginal tract surface, or a lung surface.

In some embodiments, the composition is administered to the subject via subcutaneous, intra-muscular, intraperitoneal, intravenous, oral, nasal, or topical administration, and a combination thereof.

In some aspects, a subject is treated. A subject can be a mammal including, but not limited to, a primate (e.g., a monkey, such as a cynomolgous monkey, a chimpanzee, and a human). A subject can be a non-human animal such as a bird (e.g., a quail, chicken, or turkey), a farm animal (e.g., a cow, goat, horse, pig, or sheep), a pet (e.g., a cat, dog, or guinea pig, rat, or mouse), or laboratory animal (e.g., an animal model for a disorder). Nonlimiting representative subjects can be a human infant, a pre-adolescent child, an adolescent, an adult, or a senior/elderly adult.

In some embodiments, the subject is a human.

In some instances, a subject in need of treatment can be one afflicted with one or more of the infections or disorders described herein. In some aspects, the subject is at risk of developing a biofilm on or in a biologically relevant surface, or already has developed such a biofilm. Such a subject at risk can be a candidate for treatment with a polyamine compound, or combination of a polyamine compound with another compound, in order to inhibit the development or onset of a biofilm-production-related disorder/condition or prevent the recurrence, onset, or development of one or more symptoms of a biofilm-related disorder or condition. Such a subject can be harboring an immature biofilm that is clinically evident or detectable to the skilled artisan, but that has not yet fully formed. A subject at risk of developing a biofilm can also be one in which implantation of an indwelling device, such as a medical device, is scheduled. The risk of developing a biofilm can also be due to a propensity of developing a biofilm-related disease (such as the presence of a channel transporter mutation associated with cystic fibrosis). In such subjects, a biofilm-related disorder can be at an early stage, *e.g.,* no bacterial infection or biofilm formation is yet detected.

In certain embodiments a biofilm-related disorder is selected from pneumonia, cystic fibrosis, otitis media, chronic obstructive pulmonary disease, and a urinary tract infection and combinations thereof. In other embodiments, the biofilm-related disorder is a medical device-related infection. In further embodiments, the biofilm-related disorder is a periodontal disease, such as gingivitis, periodontitis or breath malodor. In still further embodiments, the biofilm-related disorder is caused by bacteria. In some embodiments, the bacteria are Gram-negative or Gram-positive bacteria. In still other embodiments, the bacteria are of the genus *Actinobacillus, Acinetobacter, Aeromonas, Bordetella, Brevibacillus, Brucella, Bacteroides, Burkholderia, Borelia, Bacillus, Campylobacter, Capnocytophaga, Cardiobacterium, Citrobacter, Clostridium, Chlamydia, Eikenella, Enterobacter, Escherichia, Entembacter, Francisella, Fusobacterium, Flavobacterium, Haemophilus, Helicobacter, Kingella, Klebsiella, Legionella, Listeria, Leptospirae, Moraxella, Morganella, Mycoplasma, Mycobacterium, Neisseria, Pasteurella, Proteus, Prevotella, Plesiomonas, Pseudomonas, Providencia, Rickettsia, Stenotrophomonas, Staphylococcus, Streptococcus, Streptomyces, Salmonella, Serratia, Shigella, Spirillum, Treponema, Veillonella, Vibrio, Yersinia,* or *Xanthomonas*.

Non-limiting examples of biofilm-related disorders include otitis media, prostatitis, cystitis, bronchiectasis, bacterial endocarditis, osteomyelitis, dental caries, periodontal disease, infectious kidney stones, acne, Legionnaire's disease, chronic obstructive pulmonary disease (COPD), and cystic fibrosis. In one specific example, subjects with cystic fibrosis display an accumulation of biofilm in the lungs and digestive tract. Subjects afflicted with COPD, such as emphysema and chronic bronchitis, display a characteristic inflammation of the airways wherein airflow through such airways, and subsequently out of the lungs, is chronically obstructed.

Biofilm-related disorders can also encompass infections derived from implanted/inserted devices, medical device-related infections, such as infections from biliary stents, orthopedic implant infections, and catheter-related infections (kidney, vascular, peritoneal). An infection can also originate from sites where the integrity of the skin or soft tissue has been compromised. Non-limiting examples include dermatitis, ulcers from peripheral vascular disease, a burn injury, and trauma. For example, a Gram-positive bacterium, such as *S. pneumoniae,* can cause opportunistic infections in such tissues. The ability of *S. pneumoniae* to infect burn wound sites, e.g., is enhanced due to the breakdown of the skin, burn-related immune defects, and antibiotic selection.

In yet other embodiments, a biofilm-related disorder is pneumonia, cystic fibrosis, otitis media, chronic obstructive pulmonary disease, or a urinary tract infection. In some embodiments, the biofilm-related disorder is a medical device-related infection.

In other aspects, this disclosure features compounds, compositions, or methods, such as industrial, therapeutic or pharmaceutical compositions, comprising polyamine compounds in combination with one or more additional active compositions.

In some instances a polyamine compound can be administered alone or in combination with a second agent, *e.g.* a biocide, an antibiotic, or an antimicrobial agent, to thereby kill, disperse, treat, reduce prevent, or inhibit bacterial biofilms. An antibiotic can be co-administered with the polyamine compound either sequentially or simultaneously.

The antibiotic can be any compound known to one of ordinary skill in the art that can inhibit the growth of, or kill, bacteria. Useful, non-limiting examples of antibiotics include lincosamides (clindomycin); chloramphenicols; tetracyclines (such as tetracycline, chlortetracycline, demeclocycline, methacycline, doxycycline, minocycline); aminoglycosides (such as gentamicin, tobramycin, netilmicin, smikacin, kanamycin, streptomycin, neomycin); beta-lactams (such as penicillins, cephalosporins, imipenem, aztreonam); glycopeptide antibiotics (such as vancomycin); polypeptide antibiotics (such as bacitracin); macrolides (erythromycins), amphotericins; sulfonamides (such as sulfanilamide, sulfamethoxazole, sulfacetamide, sulfadiazine, sulfisoxazole, sulfacytine, sulfadoxine, mafenide, p-aminobenzoic acid, trimethoprim-sulfamethoxazole); methenamin; nitrofurantoin; phenazopyridine; trimethoprim; rifampicins; metronidazoles; cefazolins; lincomycin; spectinomycin; mupirocins; quinolones (such as nalidixic acid, cinoxacin, norfloxacin, ciprofloxacin, perfloxacin, ofloxacin, enoxacin, fleroxacin, levofloxacin); novobiocins; polymixins; gramicidins; and antipseudomonals (such as carbenicillin, carbenicillin indanyl, ticarcillin, azlocillin, mezlocillin, piperacillin) or any salts or variants thereof. Such antibiotics are commercially available, e.g., from Daiichi Sankyo, Inc. (Parsipanny, NJ), Merck (Whitehouse Station, NJ), Pfizer (New York, NY), Glaxo Smith Kline (Research Triangle Park, NC), Johnson & Johnson (New Brunswick, NJ), AstraZeneca (Wilmington, DE), Novartis (East Hanover, NJ), and Sanofi-Aventis (Bridgewater, NJ). The antibiotic used will depend on the type of bacterial infection.

Additional known biocides include biguanide, chlorhexidine, triclosan, chlorine dioxide, and the like.

Useful examples of antimicrobial agents include, but are not limited to, Pyrithiones, especially the zinc complex (ZPT); Octopirox®; dimethyldimethylol hydantoin (Glydant®); methylchloroisothiazolinone/methylisothiazolinone (Kathon CG®); sodium sulfite; sodium bisulfite; imidazolidinyl urea (Germall 115®), diazolidinyl urea (Germaill II®); benzyl alcohol; 2-bromo-2-nitropropane-1,3-diol (Bronopol®); formalin (formaldehyde); iodopropenyl butylcarbamate (Polyphase PI 00®); chloroacetamide; methanamine; methyldibromonitrile glutaronitrile (1,2-dibromo-2,4-dicyanobutane or Tektamer®); glutaraldehyde; 5-bromo-5-nitro-1,3-dioxane (Bronidox®); phenethyl alcohol; *o*-phenylphenol/sodium o-phenylphenol; sodium hydroxymethylglycinate (Suttocide A®); polymethoxy bicyclic oxazolidine (Nuosept C®); dimethoxane; thimersal; dichlorobenzyl alcohol; captan; chlorphenenesin; dichlorophene; chlorbutanol; glyceryl laurate; halogenated diphenyl ethers; 2,4,4'-trichloro-2'-hydroxy-diphenyl ether (Triclosan®. or TCS); 2,2'-dihydroxy-5,5'-dibromo-diphenyl ether; phenolic compounds; phenol; 2-methylphenol; 3-methylphenol; 4-methylphenol; 4-ethylphenol; 2,4-dimethylphenol; 2,5-dimethylphenol; 3,4-dimethylphenol; 2,6-dimethylphenol; 4-n-propylphenol; 4-n-butylphenol; 4-n-amylphenol; 4-tert-amylphenol; 4-n-hexylphenol; 4-n-heptylphenol; mono- and poly-alkyl and aromatic halophenols; p-chlorophenol; methyl p-chlorophenol; ethyl p-chlorophenol; n-propyl p-chlorophenol; n-butyl p-chlorophenol; n-amyl p-chlorophenol; sec-amyl p-chlorophenol; cyclohexyl p-chlorophenol; n-heptyl p-chlorophenol; n-octyl p-chlorophenol; o-chlorophenol; methyl o-chlorophenol; ethyl o-chlorophenol; n-propyl o-chlorophenol; n-butyl o-chlorophenol; n-amyl o-chlorophenol; tert-amyl o-chlorophenol; n-hexyl o-chlorophenol; n-heptyl o-chlorophenol; o-benzyl p-chlorophenol; o-benxyl-m-methyl p-chlorophenol; o-benzyl-m,m-dimethyl-p-chlorophenol; o-phenylethyl-p-chlorophenol; o-phenylethyl-m-methyl p-chlorophenol; 3-methyl p-chlorophenol; 3,5-dimethyl p-chlorophenol; 6-ethyl-3 - methyl p-chlorophenol; 6-n-propyl-3-methyl-p-chlorophenol; 6-isopropyl-3-methyl-p-chlorophenol; 2-ethyl-3,5-dimethyl p-chlorophenol; 6-sec-butyl-3-methyl p-chlorophenol; 2-isopropyl-3, 5 -dimethyl p-chlorophenol; 6-diethylmethyl-3 -methyl p-chlorophenol; 6-isopropyl-2-ethyl-3-methyl p-chlorophenol; 2-sec-amyl-3, 5-dimethyl p-chlorophenol; 2-diethylmethyl-3, 5 -dimethyl p-chlorophenol; 6-sec-octyl-3-methyl p-chlorophenol; p-chloro-m-cresol: p-bromophenol; methyl p-bromophenol; ethyl p-bromophenol; n-propyl p-bromophenol; n-butyl p-bromophenol; n-amyl p-bromophenol; sec-amyl p-bromophenol; n-hexyl p-bromophenol; cyclohexyl p-bromophenol; o-bromophenol; tert-amyl o-bromophenol; n-hexyl o-bromophenol; n-propyl-m,m-dimethyl-o-bromophenol; 2-phenylphenol; 4-chloro-2-methylphenol; 4-chloro-3-methyl phenol; 4-chloro-3,5-dimethyl phenol; 2,4-dichloro-3,5-dimethylphenol; 3,4,5,6-tetrabromo-2-methyl-phenol; 5-methyl-2-pentylphenol; 4-isopropyl-3-methylphenol; p-chloro-m-xylenol (PCMX); chlorothymol; phenoxyethanol; phenoxyisopropanol; 5-chloro-2-hydroxydiphenylmethane; resorcinol and its derivatives; resorcinol; methyl resorcinol; ethyl resorcinol; n-propyl resorcinol; n-butyl resorcinol; n-amyl resorcinol; n-hexyl resorcinol; n-heptyl resorcinol; n-octyl resorcinol; n-nonyl resorcinol; phenyl resorcinol; benzyl resorcinol; phenylethyl resorcinol; phenylpropyl resorcinol; p-chlorobenzyl resorcinol; 5-chloro 2,4-dihydroxydiphenyl methane; 4'-chloro 2,4-dihydroxydiphenyl methane; 5-bromo 2,4-dihydroxydiphenyl methane; 4'-bromo 2,4-dihydroxydiphenyl methane; bisphenolic compounds; 2,2'-methylene bis-(4-chlorophenol); 2,2'-methylene bis-(3,4,6-trichlorophenol); 2,2'-methylene bis(4-chloro-6-bromophenol); bis(2-hydroxy-3,5-dichlorophenyl)sulfide; bis(2-hydroxy-5-chlorobenzyl)sulfide; benzoic esters (parabens); methylparaben; propylparaben; butylparaben; ethylparaben; isopropylparaben; isobutylparaben; benzylparaben; sodium methylparaben; sodium propylparaben; halogenated carbanilides; 3,4,4'-trichlorocarbanilides (e.g., Triclocarban® or TCC); 3-trifluoromethyl-4,4'-dichlorocarbanilide; 3,3',4-trichlorocarbanilide; chlorohexidine and its digluconate; diacetate and dihydrochloride; undecenoic acid; thiabendazole, hexetidine; and poly(hexamethylenebiguanide) hydrochloride (Cosmocil®).

In some embodiments of any methods described herein, the method further comprises administering a biocide. In some embodiments, the biocide is an antibiotic.

In instances where a polyamine compound, or combination of a polyamine compound with another compound, is to be administered to a subject, the compound or composition herein can be incorporated into pharmaceutical compositions. The polyamine compound, or combination of a polyamine compound with another compound, can be incorporated into pharmaceutical compositions as pharmaceutically acceptable salts or derivatives. As used herein, a "pharmaceutically acceptable carrier" means a carrier that can be administered to a subject together with a polyamine compound, or combination of a polyamine compound with another compound, described herein, which does not destroy the pharmacological activity thereof. Pharmaceutically acceptable carriers include, for example, solvents, binders, dispersion media, coatings, preservatives, colorants, isotonic and absorption delaying agents, and the like, compatible with pharmaceutical administration. Supplementary active compounds can also be incorporated into the compositions.

Non-limiting examples of pharmaceutically acceptable carriers that can be used include poly(ethylene-co-vinyl acetate), PVA, partially hydrolyzed poly(ethylene-co-vinyl acetate), poly(ethylene-co-vinyl acetate-co-vinyl alcohol), a cross-linked poly(ethylene-co-vinyl acetate), a cross-linked partially hydrolyzed poly(ethylene-co-vinyl acetate), a cross-linked poly(ethylene-co-vinyl acetate-co-vinyl alcohol), poly-D,L-lactic acid, poly-L-lactic acid, polyglycolic acid, PGA, copolymers of lactic acid and glycolic acid (PLGA), polycaprolactone, polyvalerolactone, poly (anhydrides), copolymers of polycaprolactone with polyethylene glycol, copolymers of polylactic acid with polyethylene glycol, polyethylene glycol; and combinations and blends thereof.

Other carriers include, e.g., an aqueous gelatin, an aqueous protein, a polymeric carrier, a cross-linking agent, or a combination thereof. In other instances, the carrier is a matrix. In yet another instances, the carrier includes water, a pharmaceutically acceptable buffer salt, a pharmaceutically acceptable buffer solution, a pharmaceutically acceptable antioxidant, ascorbic acid, one or more low molecular weight pharmaceutically acceptable polypeptides, a peptide comprising about 2 to about 10 amino acid residues, one or more pharmaceutically acceptable proteins, one or more pharmaceutically acceptable amino acids, an essential-to-human amino acid, one or more pharmaceutically acceptable carbohydrates, one or more pharmaceutically acceptable carbohydrate-derived materials, a non-reducing sugar, glucose, sucrose, sorbitol, trehalose, mannitol, maltodextrin, dextrins, cyclodextrin, a pharmaceutically acceptable chelating agent, EDTA, DTP A, a chelating agent for a divalent metal ion, a chelating agent for a trivalent metal ion, glutathione, pharmaceutically acceptable nonspecific serum albumin, or combinations thereof.

In other embodiments, the compositions can also comprise a pharmaceutically acceptable carrier. In still other embodiments the effective amount is an amount effective to treat or prevent a biofilm-related disorder. In some embodiments, an effective amount comprises and amount effective to treat or prevent a biofilm on a surface.

In some embodiments, the compositions discussed herein further comprises an agent suitable for application to the surface. In other embodiments, the composition is formulated as a wash solution, a dressing, a wound gel, or a synthetic tissue. In further embodiments, the composition is formulated as tablets, pills, troches, capsules, aerosol spray, solutions, suspensions, gels, pastes, creams, or foams. In some embodiments, the composition is formulated for parenteral *(e.g.,* intravenous), intradermal, subcutaneous, oral (*e.g.,* inhalation), transdermal (topical), transmucosal, vaginal, or rectal administration.

In some embodiments, the polyamine compound or combination of a polyamine compound and at least one other composition is formulated as a slow-release formulation.

A pharmaceutical composition containing a polyamine compound, or combination of a polyamine compound with another compound, can be formulated to be compatible with its intended route of administration as known by those of ordinary skill in the art. Nonlimiting examples of routes of administration include parenteral, e.g., intravenous, intradermal, subcutaneous, oral {e.g., inhalation), transdermal (topical), transmucosal, vaginal and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water-soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, the composition can be sterile and can be fluid to the extent that easy syringability exists. It should be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. It may be desirable to include isotonic agents, for example, sugars, polyalcohols such as mannitol, sorbitol, or sodium chloride in the composition. Prolonged absorption of the injectable compositions can be accomplished by including in the composition an agent that delays absorption, for example, aluminum monostearate and gelatin (see, e.g., Remington: The Science and Practice of Pharmacy, 21st edition, Lippincott Williams & Wilkins, Gennaro, ed. (2006)).

Sterile injectable solutions can be prepared by incorporating a polyamine compound, or combination of a polyamine compound with another compound, in the required amount in an appropriate solvent with one or a combination of ingredients enumerated above, as required, followed by filtered sterilization. Generally, dispersions are prepared by incorporating an active compound into a sterile vehicle that contains a basic dispersion medium and the required other ingredients from those enumerated above. In the case of sterile powders for the preparation of sterile injectable solutions, the methods of preparation include, without limitation, vacuum drying and freeze-drying which yields a powder of the active ingredient plus any additional desired ingredient from a previously sterile-filtered solution thereof.

Oral compositions may include an inert diluent or an edible carrier or binders. For the purpose of oral therapeutic administration, a polyamine, or a combination of a polyamine compound, or combination of a polyamine compound with another compound, can be incorporated with excipients and used in the form of tablets, pills, troches, or capsules, e.g., gelatin capsules. Oral compositions can also be prepared using a fluid carrier for use as a mouthwash. Pharmaceutically compatible binding agents, or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, polyamine compound, or combination of a polyamine compound with another compound, can be delivered in the form of an aerosol spray from pressured container or dispenser that contains a suitable propellant, *e.g.,* a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, but are not limited to, for example, for transmucosal administration, detergents, bile salts, and fusidic acid derivatives. Transmucosal administration can be accomplished through the use of nasal sprays or suppositories. For transdermal administration, the active compounds and compositions are formulated into pharmaceutically acceptable formulation embodiments, such as ointments, salves, gels, or creams as generally known in the art.

For treatment of acute or chronic wounds, polyamine compound, or combination of a polyamine compound with another compound, can be formulated as a dressing, a wash solution, gel, or a synthetic tissue, etc.

The pharmaceutical compositions containing a polyamine compound, or combination of a polyamine compound with another compound, can also be prepared in the form of suppositories (e.g., with conventional suppository bases such as cocoa butter and other glycerides) or retention enemas for rectal delivery.

Some pharmaceutical compositions containing a polyamine compound, or combination of a polyamine compound with another compound, can be prepared with a carrier that protects the polyamine compound, or combination of a polyamine compound with another compound, against rapid elimination from the body, such as a controlled release formulation, including implants and microencapsulated delivery systems as described, *e.g.*, in Tan et al., Pharm. Res. 24:2297-2308 (2007).

Additionally, biodegradable, biocompatible polymers can be used, such as ethylene vinyl acetate, polyanhydrides, polyglycolic acid, collagen, polyorthoesters, and polylactic acid. Methods for preparation of such formulations are apparent to those skilled in the art. The materials can also be obtained commercially (e.g., from Alza Corp., Mountain View, Calif). Liposomal suspensions (including liposomes targeted to particular cells with monoclonal antibodies to cell surface antigens) can also be used as pharmaceutically acceptable carriers. These can be prepared according to methods known to those skilled in the art, *e.g.,* as described in U.S. Pat. No. 4,522,811.

Toxicity and therapeutic efficacy of such compounds and compositions can be determined by standard pharmaceutical procedures in cell cultures or experimental animals, e.g., for determining the $LD_{50}$ (the dose lethal to 50%> of the population) and the $ED_{50}$ (the dose therapeutically effective in 50% of the population). The dose ratio between toxic and therapeutic effects is the therapeutic index and it can be expressed as the ratio $LD_{50}/ED_{50}$. While compounds and compositions that exhibit toxic side effects can be used, care should be taken to design a delivery system that targets active components to the site of affected tissue in order to minimize potential damage to normal cells and, thereby, reduce side effects.

The data obtained from the cell culture assays and animal studies can be used in formulating a range of dosage for use in humans. The dosage of such compounds and compositions lies generally within a range of circulating concentrations that include the $ED_{50}$ with little or no toxicity. The dosage can vary within this range depending upon the dosage form employed and the route of administration utilized. For any compounds or compositions used in the methods described herein, the therapeutically effective dose can be estimated initially from cell culture assays. A dose can be formulated in animal models to achieve a circulating plasma concentration range that includes the $IC_{50}$ (i.e., the concentration of the test compound or composition that achieves a half-maximal inhibition of symptoms) as determined in cell culture. Such information can be used to more accurately determine useful doses in humans. Levels in plasma can be measured, for example, by high performance liquid chromatography. Information for preparing and testing such compositions are known in the art. *See, e.g.,* Remington: The Science and Practice of Pharmacy, 21st ed., Lippincott Williams & Wilkins, Gennaro, ed. (2006).

A physician will appreciate that certain factors may influence the dosage required to effectively treat a subject, including but not limited to the severity of the disease or disorder, previous treatments, the general health or age of the subject, and other diseases present. Moreover, treatment of a subject with a therapeutically effective amount of a polyamine compound, or combination of a polyamine compound with another compound, can include a single treatment or a series of treatments.

The compounds or pharmaceutical compositions can be included in a container, pack, or dispenser together with instructions for administration. A person of ordinary skill in the art will appreciate that the compounds or pharmaceutical compositions described herein can be formulated as single-dose vials.

Polyamine compounds, or combination of a polyamine compound with another compound, may be suitable as antibiofilm active substances in personal care preparations, for example shampoos, bath additives, hair care preparations, liquid and solid soaps (based on synthetic surfactants and salts of saturated or unsaturated fatty acids), lotions and creams, deodorants, other aqueous or alcoholic solutions, e.g. cleansing solutions for the skin, moist cleaning cloths, oils or powders.

Any suitable amount of polyamine can be used in the compositions and methods of the invention. In general, the polyamines are used in concentrations ranging from about 1 ppm to about 100,000 ppm, or higher. The concentration of a polyamine used in a composition or method of the invention can be, for example, from about 1 to about 100,000 ppm, or from about 10 to about 10,000 ppm, or from about 100 to about 1,000 ppm, or from about 1 to about 100 ppm, or from about 1,000 to about 10,000 ppm, or from about 10,000 to about 100,000 ppm. The concentration of a polyamine can be about 1; 2; 3; 4; 5; 6; 7; 8; 9; 10; 15; 20; 25; 30; 35; 40; 45; 50; 55; 60; 65; 70; 75; 80; 85; 90; 95; 100; 125; 150; 175; 200; 225; 250; 275; 300; 325; 350; 375; 400; 425; 450; 475; 500; 525; 550; 575; 600; 625; 650; 675; 700; 725; 750; 775; 800; 825; 850; 875; 900; 925; 950; 975; 1000; 1500; 2000; 2500; 3000; 3500; 4000; 4500; 5000; 5500; 6000; 6500; 7000; 7500; 8000; 8500; 9000; 9500; 10,000; 12,500; 15,000; 17,500; 20,000; 22,500; 25,000; 27,500; 30,000; 32,500; 35,000; 37,500; 40,000; 42,500; 45,000; 47,500; 50,000; 52,500; 55,000; 57,500; 60,000; 62,500; 65,000; 67,500; 70,000; 72,500; 75,000; 77,500; 80,000; 82,500; 85,000; 87,500; 90,000; 92,500; 95,000; 97,500; or about 100,000 ppm. Other concentrations of polyamines can be useful in the compositions and methods of the invention, depending in part on factors including the specific polyamine used, the presence of other active agents if any, or the species of microorganisms that are targeted.

There is thus disclosed compounds, compositions, or methods comprising novel polyamine compounds, or combinations of polyamine compounds with other compounds, that have antimicrobial activity and dispersing activity against a variety of bacterial strains capable of forming biofilms, and methods of using the same.

## EXAMPLES

The following examples serve to explain embodiments of the present disclosure in more detail. Compounds CZ-58, CZ-62, CZ-65, CZ-66, CZ-67, CZ-69, CZ-70, CZ-73, CZ-74, CZ-75, CZ-77, CZ-83, CZ86, CZ-86 Free Base, CZ-92, CZ-94, CZ-96, CZ-99, CZ-111, CZ-114, CZ-115, CZ-116, CZ-117 CZ-118 are part of the invention. The other compounds mentioned are not.

The following material and methods were used.

Bacterial strains. A clinical strain of MRSA, isolated from a patient who underwent arthroscopic knee surgery and characterized by ARUP Laboratories, Salt Lake City, UT, was used for this study in addition to *Pseudomonas aeruginosa* ATCC 27853 and *Alcanivorax borkumensis* ATCC 700651. Both ATCC strains were purchased as freeze-dried pellets from ATCC. *P. aeruginosa* was resuspended in BHI broth, grown overnight at 37° C and transferred to fresh BHI with 30% glycerol for storage at -80° C. The MRSA isolate was likewise stored in BHI with 30% glycerol at -80° C. Notably, the clinical MRSA isolate was not passaged more than three times prior to or during the study. Prior to performing MIC analysis and biofilm experiments, the frozen stocks of MRSA and *P. aeruginosa* were streaked onto Columbia blood agar plates and grown overnight at 37° C. *A. borkumensis* ATCC 700651 was resuspended from a lyophilized pellet into marine broth, grown overnight at 30° C and passaged on marine agar plates prior to experimentation.

**Example 1**

MIC Determination. To determine the MIC of the polyamine chains alone and the synthesized compounds against MRSA and *P. aeruginosa* ATCC 27853, a modified protocol from the Clinical and Laboratory Standards Institute (CLSI) guideline M26-A was used. Consistent with this guideline, the MIC was defined as the concentration of antimicrobial that was required to reduce approximately $5 \times 10^5$ cells per mL to approximately $5 \times 10^2$ per mL in a 24-hour period. Each MIC experiment was performed n=10 times for each antimicrobial. All data was collected using cation adjusted MHB for standardization.

As mentioned, MRSA and *P. aeruginosa* ATCC 27853 were grown on Columbia blood agar plates prior to experimentation. From the blood agar plates, a 0.5 McFarland standard was made of each isolate. This equated to approximately $5 \times 10^7$ cells/mL for the MRSA isolate and approximately $3 \times 10^7$ cells/mL for the *P. aeruginosa* ATCC 27853 isolate. Of the 0.5 McFarland stock solution, 50 μL were added to MHB. All of the antimicrobials were kept in PBS stock solutions at 10 mg/mL and diluted in the MHB to reach the desired concentration that would determine the MIC value. All tests were performed such that a final volume of 5 mL of MHB were obtained. This provided a final bacterial concentration of approximately $5 \times 10^5$ cells/mL.

Each sample was incubated in glass test tubes at 37° C for 24 hours, then plated in duplicate on tryptic soy agar (TSA) using a 10-fold dilution series. TSA plates were further incubated 24 hours at 37° C. The number of colony forming units (CFU) that grew were counted in order to calculate the number of bacteria that were present per mL in the original MHB solution. The concentration of compound that resulted in a reduction of approximately $5 \times 10^5$ cells/mL to approximately $5 \times 10^2$ cells/mL in a 24-hour period was defined as the MIC.

For comparison to a current standard of care, the MICs of vancomycin against the MRSA isolate and tobramycin against the *P. aeruginosa* ATCC 27853 isolate were also determined. MIC results for various compounds are shown in FIG. 7.

MRSA and *P. aeruginosa* Biofilm Eradication. Biofilms of MRSA and *P. aeruginosa* ATCC 27853 were grown on the surface of PEEK membranes using a membrane biofilm reactor. Data demonstrating the design and repeatability of this reactor have been published previously. Williams et al., In Vivo Efficacy of a Silicone - Cationic Steroid Antimicrobial Coating to Prevent Implant-Related Infection, Biomaterials 33, 8641-8656 (2012); Williams et al., Use of delrin plastic in a modified CDC biofilm reactor, Res J Microbiol 6, 425-429 (2011); Williams et al, Experimental model of biofilm implant-related osteomyelitis to test combination biomaterials using biofilms as initial inocula, J Biomed Mat Res A 100, 1888-1900 (2012); Williams et al., A modified CDC biofilm reactor to produce mature biofilms on the surface of PEEK membranes for an in vivo animal model application, Curr Microbiol 62, 1657-1663 (2011). Prior to growing the biofilms, PEEK membranes were first sonicated for 10 min in medical grade detergent, rinsed with running reverse osmosis water for 10 min, sonicated in reverse osmosis water for 10 min and rinsed once again using 70% ethanol.

Four guillotine-like holders were designed to hold eight PEEK membranes (two per holder). All components were washed, assembled and the reactor autoclaved prior to each use. Following American Society for Testing and Materials (ASTM) standard E2562-07, the modified reactor was run under the following conditions: approximately $5 \times 10^7$ bacterial cells were inoculated into 500 mL of BHI in the biofilm reactor. A paddle in the base of the reactor was stirred at 130 rpm. The unit was placed on a hot plate set at 33° C for 24 hrs. A 10% BHI broth solution was then flowed through the reactor at 6.94 mL/min for an additional 24 hrs.

Following the 48-hour growth period, six of the PEEK membranes were aseptically removed and placed into 5 mL of MHB that contained varying concentrations of the synthesized compounds. For comparison, vancomycin was also tested against MRSA biofilms and tobramycin was tested against *P. aeruginosa* ATCC 27853 biofilms. The membranes and broth were incubated in glass test tubes at 37° C for 24 hours. The test tubes containing membranes were vortexed for 1 minute, sonicated at 42 kHz for 10 minutes and plated in duplicate on TSA using a 10-fold dilution series and incubated at 37° C for 24 hours. This process allowed for the determination of what was defined as the effective

biofilm eradication concentration (EBEC) of the selected compounds. In this case, the EBEC was defined as the concentration of compound required to reduce the number of cells in the biofilms from approximately $10^9$ cells/PEEK membrane to $10^5$ cells/membrane. A level of $10^9$ bacteria in the biofilms represented an amount of bacteria that may be present in one gram of soil in a natural ecosystem. *See e.g.,* Bakken, Separation and Purification of Bacteria from Soil, Appl Environ Microbiol 49, 1482-1487 (1985); Torsvik et al., High Diversity in DNA of Soil Bacteria, Applied and Environmental Microbiology 56, 782-787 (1990).

Two membranes from each run of the reactor were aseptically removed and quantified to serve as positive controls of biofilm growth. All of the biofilm eradication studies were repeated n=5 times. EBEC results are summarized in FIGs. 12A and 12B.

Model oil pipeline contamination using *Alcanivorax borkumensis.* FIG. 13 shows an example of microbial contamination in the oil and gas industry. To test the ability and efficacy of the novel synthesized compounds to disperse and kill biofilms that may exist in a variety of settings, a model oil pipeline system was prepared to grow biofilms of *A. borkumensis,* a gram-negative organism that metabolizes alkane chains. In cases of oil spills, this organism may be beneficial for bioremediation, but in this instance, it was used to represent bacteria in an oil field or pipeline that may have adverse or unwanted effects of oil degradation.

FIG. 14B shows a representative bacteria biofilm, such as the biofilm shown in FIG. 14A, treated according to certain standards in the oil and gas industry. The biofilm shown in FIG. 14B was treated with a solution comprising 0.25% glutaraldehyde. A similar representative biofilm was treated with a solution comprising 0.5% of a polyamine compound described herein, and is shown in FIG. 14C.

FIG. 15 shows the results of treating biofilms of *Alcanivorax borkumensis* grown on the surface of galvanized steel with a solution comprising 0.25% of a polyamine compound.

Combination treatment. Data collected with benzylnorspermidine (CZ-7; PBC-7) at 0.25% mixed with chlorhexidine gluconate at 0.25% has shown significant dispersal of a biofilm of MRSA that consisted of $10^{11}$ cells within one hour.

## Example 2

A compound was screened for its ability to create zones of inhibition on cation adjusted Mueller Hinton agar. It is believed that the compound of Example 2 may be represented by the structure disclosed in FIG. 4H. To perform this test, lawns of methicillin-resistant *Staphylococcus aureus* (MRSA) or *Pseudomonas aeruginosa* ATCC 27853 were made on cation adjusted Mueller Hinton agar using standard microbiological techniques. Immediately after plating bacteria on agar, a 50 μL drop of the compound was placed onto the agar surface. The compound was tested in duplicate at 100 μg/mL, 50 μg/mL, 25 μg/mL and 10 μg/mL. All plates were incubated at 37° C for 24 hours. Results indicated that total clearing was seen against MRSA at 10 μg/mL. Partial clearing was seen against P. *aeruginosa* at 25 μg/mL and total clearing at 50 μg/mL.

The second test determined the minimum inhibitory concentration (MIC) of the compound against MRSA and *P. aeruginosa* ATCC 27853. In this instance, the MIC was defined as the amount of antimicrobial required to reduce $10^5$ cells/mL to $10^2$ cells/mL in a 24-hour period. This definition was consistent with the Clinical and Laboratory Standards Institute (CLSI). More specifically, the microdilution method as outlined in the M26-A CLSI guideline was used. All tests were performed with cation adjusted Mueller Hinton broth. MIC data are presented in Table 1.

In addition to determining the MIC, the minimum biofilm eradication concentration (MBEC) of PBC-51 was also determined. The MBEC was determined using the MBEC assay, formerly known as the Calgary Biofilm Device, developed by Innovotech. In this instance, the MBEC was defined as the amount of antimicrobial required to eradicate 100% of detectable biofilm after being grown in the MBEC device. To grow the biofilms within this device, 150 μL of solution containing a concentration of $10^5$ cells/mL were inoculated into each well of a 96-well plate. A lid containing 96 pegs was placed onto the plate and incubated for 24 hours at 37° C while being shaken at 110 rpm. Biofilms developed on the surface of the polystyrene pegs within the 96-well plate. After 24 hours, each peg contained between $10^5$ and $10^6$ cells in well-established biofilms. The lid with pegs was transferred to a 96-well plate that contained 200 μL of varying concentrations of the compound and was incubated at 37° C for an additional 24 hours. The plate was then sonicated, and the biofilms quantified using a 10-fold dilution series to determine the MBEC. Data are presented in Table 1.

**Table** 1: MIC, MBEC and EBEC of PBC-51 Against MRSA and *P. aeruginosa* ATCC 27853. Data are presented in μg/mL and μM concentrations.

| Organism | MIC (μg/mL / μM) | MBEC (μg/mL / μM) | EBEC (μg/mL / μM) |
|---|---|---|---|
| MRSA | ~1/~1.88 | <25/<47 | ~250/~470.88 |
| *P. aeruginosa* | ~6/~11.30 | ~35/~65.92 | <100/<188.33 |

The fourth test that was performed was the effective biofilm eradication concentration (EBEC). To determine the EBEC, biofilms of MRSA and *P. aeruginosa* ATCC 27853 were grown on the surface of PEEK membranes using a previously established protocol. Briefly, 500 mL of brain heart infusion (BHI) broth were inoculated with 105 cells/mL of MRSA or *P. aeruginosa.* BHI broth was placed into a membrane biofilm reactor and placed on a hot plate set at 34° C for 24 hours with a stir bar rotating at 130 rpm. Following the initial 24-hour growth period, a 10% BHI broth solution was flowed through the reactor at a rate of 6.944 mL/min for another 24 hours. After 48 hours of growth, each PEEK membrane had biofilm growth on it. In this experiment, there were ~2 $\times$ $10^{11}$ cells of MRSA per PEEK membrane and ~2 $\times$ $10^{10}$ cells of *P. aeruginosa* per PEEK membrane. The EBEC was defined as the concentration of the compound that was required to eradicate all detectable amounts (100%) of bacteria within the biofilms. Data are presented in Table 1.

In addition to determining the antimicrobial efficacy of the compound, an initial test was performed to determine its hemolytic nature. To do so, the compound was suspended in concentrations of 100 $\mu$g/mL, 50 $\mu$g/mL and 25 $\mu$g/mL. Twenty $\mu$L of each concentration was placed onto the surface of Columbia blood agar and incubated at 37° C for 24 hours. Results indicated that none of these concentrations displayed hemolytic activity.

Initial tests based on visual observation were performed to determine the ability of the compound to disperse biofilms. This was done by observing the biofilms of MRSA and *P. aeruginosa* that had grown on the surface of PEEK membranes after they had been placed in three separate concentrations of the compound (300 $\mu$g/mL, 200 $\mu$g/mL and 100 $\mu$g/mL). Biofilms were observed every 30 minutes to see if they separated from the PEEK membrane. Within 2 hours, biofilms began to separate from the PEEK membranes, by 4 hours approximately half of the biofilms had separated and by 24 hours, all detectable amounts of biofilm had separated from the PEEK membrane. For comparison, it is the understanding of the inventors hereof that this dispersal effect is not seen with other antimicrobials such as chlorhexidine gluconate, glutaraldehyde, or benzethonium chloride.

The MIC, MBEC and EBEC of vancomycin were also determined for comparison, and the results are presented in Table 2.

**Table** 2: MIC, MBEC and EBEC of Vancomycin Against MRSA.

| Organism | MIC ($\mu$g/mL / $\mu$M) | MBEC ($\mu$g/mL / $\mu$M) | EBEC ($\mu$g/mL / $\mu$M) |
|---|---|---|---|
| MRSA | 10/6.73 | >500/>336.50 | >20,000/>13,460 |

**Example 3**

*MIC Analysis*

To determine the MIC of polyamine compounds, the protocol described herein was used. The MIC is defined as being the concentration of antimicrobial (in $\mu$g/mL) required to reduce the number of bacteria in a solution from $10^5$ colony forming units (CFU)/mL to $10^2$ CFU/mL in a 24-hour period.

In brief, a 0.5 McFarland of each bacterial isolate was made. A 0.5 McFarland is a measure of turbidity in a liquid sample that contains approximately 1 $\times$ $10^{\wedge 8}$ CFU/mL. The 0.5 McFarland standard was diluted in cation adjusted Mueller Hinton Broth (CAMHB), and 50 $\mu$L of broth were added to a well of a 96-well plate. In addition, 50 $\mu$L of CAMHB that contained a desired concentration of antimicrobial were also added to the well for a final volume of 100 $\mu$L and a final concentration of approximately 5 $\times$ $10^4$ CFU/well (which equated to approximately 5 $\times$ $10^5$ CFU/mL). Each well contained a desired amount of polyamine compound in order to experimentally determine the MIC. Each 96-well plate was incubated at 37° C for 24 hours. The contents of each well were plated on tryptic soy agar (TSA). TSA plates were incubated for 37° C for 24 hours after which the number of CFU were counted and used to calculate the CFU/mL that remained after exposure to varying concentrations of compound. This procedure was repeated n=8 times for each concentration of antimicrobial. The concentration of polyamine compound that reduced bacteria from $10^5$ CFU/mL to $10^2$ CFU/mL in 24 hours was considered the MIC.

MICs from representative polyamine compounds as well as select antibiotics are provided in Table 3A and 3B. Consistent with what was mentioned previously, these data indicate that with an increase in the number of polyamine chains attached to a lipophilic backbone, the MIC is lowered, indicating it has greater antimicrobial potential. CZ-7 has one chain of norspermidine attached, CZ-25 has two chains of norspermidine and CZ-51 and CZ-52 have three chains attached.

**Table 3A:** MICs of Polyamine Compounds and Select Traditional Antibiotics ($\mu$g/mL)

| **Compound** | *MRSA* | *P. aeruginosa* | *A. baumannii* | *MRSA* | *P. aeruginosa* |
|---|---|---|---|---|---|
| CZ-7 | >500 | >500 | >500 | >500 | >5,000 |

(continued)

| Compound | MRSA | P. aeruginosa | A. baumannii | MRSA | P. aeruginosa |
|---|---|---|---|---|---|
| CZ-25 | 40 | 500 | 150 | 25 | >500 |
| CZ-52 | 3 | 17 | -25 | 20 | 400 |
| Vancomycin | 10 | - | - | >500 | - |
| Tobramycin | -* | 2 | - | - | 100 |
| Polymyxin B sulfate | - | - | 2 | - | - |
| *Not yet determined<br>**No change | | | | | |

**Table 3B:** MICs of Polyamine Compounds and Select Traditional Antibiotics (μg/mL) II

| Compound | A. baumannii | MRSA | P. aeruginosa | A. baumannii |
|---|---|---|---|---|
| CZ-7 | >500 | >5,000 | >5,000 | >5,000 |
| CZ-25 | 450 | >5,000 | >5,000 | >5,000 |
| CZ-52 | 300 | 250 | >5,000 | >5,000 |
| Vancomycin | - | >25,000 | - | - |
| Tobramycin | - | - | 2,000 | - |
| Polymyxin B sulfate | 50 | - | - | 1,000 |
| *Not yet determined<br>**No change | | | | |

To determine the MBEC of each polyamine compound, the MBEC Inoculation Tray by Innovotech, formerly known as the Calgary biofilm device, was used. Within this device, biofilms grow on the surface of polystyrene pegs, 96 of which are attached to a lid. These pegs are inserted into a flat bottom 96-well plate. In this instance, the MBEC of a molecule was defined as the concentration of compound (in μg/mL) required to reduce $10^5$ or $10^6$ CFU/peg (biofilm levels varied by isolate) to $10^2$ CFU/peg in a 24-hour period.

Following the manufacturer's guidelines, biofilms were grown on the surface of each peg by first making a 0.5 McFarland of each isolate. The 0.5 McFarland was diluted 1:100 in CAMHB. Into each well of a flat bottom 96-well plate, 150 μL of broth were pipetted. The plate was shaken at 100 rpm for 24 hours (*P. aeruginosa* and *A. baumannii*) or 48 hours (MRSA). The pegs were then placed into a separate flat bottom 96-well plate for 10 seconds with 200 μL of phosphate buffered saline (PBS) in each well to remove nonadherent cells. The lid was then placed into a 96-well plate that contained varying concentrations of antimicrobial with 200 μL per well. The plate was incubated for 24 hours at 37° C after which time 100 μL of broth were plated on TSA. TSA plates were incubated 24 hours at 37° C and the number of CFU counted to calculate the CFU/peg. In this instance, the MBEC was defined as the concentration of antimicrobial required to reduce $10^5$ or $10^6$ CFU/peg to $10^2$ CFU/peg in a 24 hour period.

MBEC data are presented in Table 4. Similar to the MIC data, the trend of increasing antimicrobial activity by increasing the number of polyamine chains attached to a backbone was confirmed against low number biofilms.

*EBEC Analysis*

In addition to determining the MIC and the MBEC of polyamine compounds against planktonic bacteria and low number biofilms, our group wanted to determine the efficacy of polyamine compounds against high number biofilms. To do so, biofilms were grown on the surface of polyetheretherketone (PEEK) membranes using a membrane biofilm reactor. This reactor is similar to the CDC biofilm reactor, but rather than growing biofilms on coupon surfaces, the reactor was modified to hold PEEK membranes. In short, to grow biofilms within this system, 500 mL of brain heart infusion (BHI) broth were inoculated with 1 mL of a 0.5 McFarland. The reactor was placed on a hot plate set at 34° C and the bacteria were grown under batch conditions for 24 hours. Following this protocol, biofilms typically grow to $10^9$

CFU/PEEK membrane. each PEEK membrane has high number biofilms A solution of 10% BHI was then flowed through the reactor at a rate of 6.94 mL/min for an additional 24 hours. PEEK membranes were then removed and placed into 2 mL of CAMHB that contained a desired concentration of polyamine compound or antibiotic. The EBEC was defined as the concentration of antimicrobial required to reduce a biofilm from approximately $10^9$ CFU/PEEK membrane to approximately $10^2$ CFU/PEEK membrane in a 24-hour period.

EBEC data are presented in Table 4. One of the most striking results was the difference in efficacy between CZ-52 and vancomycin. At 25,000 μg/mL, vancomycin did not have the ability to reduce biofilms of MRSA by even 1 $log_{10}$ unit. In contrast, at 250 μg/mL CZ-52 was able to reduce MRSA biofilms by greater than 7 $log_{10}$ units in a 24-hour period.

**Example 4**

*Release of Polyamine Compounds from Carrier Products*

To collect proof-of-concept/preliminary data that carrier(s) will release these products in sufficient amounts to have antimicrobial efficacy, a polytherapy formula was prepared. A Vanicream-based cream from the University of Utah compounding pharmacy was used as the formulation base, and active ingredients were added to create a cream with final concentrations of 0.25% CZ-52, 0.25% CZ-25 and 0.1% Polymyxin B sulfate. To test its efficacy, 0.5 McFarland standards of MRSA, *P. aeruginosa,* and *A. baumannii* were made, and a lawn of each isolate was spread on Columbia blood agar. Approximately 20 mg of cream was placed in the center of each lawn. The plates were incubated 24 hours at 37° C.

Zones of clearing in each experiment provided a preliminary indication that the antimicrobials will elute out of the cream in sufficient quantities to eradicate each bacterial isolate (FIG. 16). Notably, the cream alone (negative control) created no zones of inhibition, and polyamine compounds used alone in the cream likewise created zones of clearing. In broth samples, the polytherapy composition has shown similar results by eradicating all detectable amounts of each bacterial isolate after they had been grown as biofilms on PEEK membranes with approximately $10^9$ CFU/PEEK membrane. In short, these data indicate that the polytherapy antimicrobial agents will elute out of carrier product(s) and eradicate bacteria, including those in biofilms.

**Example 5**

*Biofilm Dispersion*

CZ-25 and CZ-52 were tested for their ability to disperse biofilms of MRSA and *P. aeruginosa.* To test for dispersion, biofilms of each isolate were grown on the surface of commercially pure titanium (Ti) coupons (1/2" diameter × 1/8" height) in a CDC biofilm reactor. The growth conditions for this reactor were the same as those for the membrane biofilm reactor. After biofilms were grown for 48 hours, each Ti coupon was aseptically removed and placed into 2 mL of CAMHB for 2 hours. The CAMHB had a final concentration of 0.25% (2.5 mg/mL) CZ-25 or CZ-52. Each compound was tested n=3 times.

Following the 2-hour exposure, each coupon was fixed in 0.25% glutaraldehyde for 24 hours, dehydrated using ascending concentrations of ethanol (70%, 95%, 100%) with 3x 10 min. changes and desiccated. One side of each coupon was carbon coated and imaged using a JEOL JSM-6610 SEM to directly observe the surface of the coupons and determine the ability of polyamine compounds to disperse biofilms compared to positive controls that had no treatment.

When compared to untreated controls, data have indicated that CZ-25 and CZ-52 have the ability to disperse biofilms from the surface of a material (FIG. 17) such that a monolayer of cells or reduced communities remain.

The MRSA biofilms were grown on the surface of titanium (Ti) coupons using a CDC biofilm reactor. For this reactor, an initial inoculum of ~5 × 10^5 cells/mL were put into 500 mL of brain heart infusion (BHI) broth. The reactor was placed on a hot plate set at 34 C for 24 hours and the bacteria were allowed to grow in batch culture. For an additional 24 hours, 10% BHI broth was flowed through the reactor at a rate of 6.94 mL/min. After the 48-hour growth period, the coupons were aseptically removed and placed into 2 mL of cation adjusted Mueller Hinton broth (CAMHB) for 2 hours. Digital images of the biofilm dispersion process were collected after 30 minutes of exposure to polyamine compounds. After 2 hours, the same procedure as above was used to fix the biofilms and image them by SEM.

Digital images of MRSA biofilms being dispersed from the surface of the Ti coupon also help to highlight the dispersive ability of CZ-25 (FIG. 18).

Data has also been collected to determine the dispersive capacity of polyamine compounds against *Alcanivorax borkumensis,* an oil-eating bacterium (FIGS. 19 and 20).

For the dispersal data with the Alcanivorax borkumensis, 1/2 inch diameter galvanized steel pipe that was

approximately 6 inches long was purchased. Two strips of galvanized sheet metal were cut into 1/2 cm × 2 cm strips, and two of those were placed into the pipe. The pipe was connectedto silicone tubing, and marine broth was run through the pipes with an inoculum of 5 × 10^5 cells/mL. The broth was flowed through the pipes at a rate of 6.94 mL/min using a peristaltic pump. Biofilms were grown on the surface of the galvanized steel for 48 hours, then exposed to polyamine compounds or glutaraldehyde for two hours. The strips of galvanized steel were then removed, fixed in 0.25% glutaraldehyde for 2 hours (those that had already been exposed to glutaraldehyde were not fixed twice), and dehydrated in ascending concentrations of ethanol (70%, 95%, 100%) with 3x 10 minute exchanges of each. Lastly, the strips were carbon-coated and were imaged using a JEOL JSM-6610 scanning electron microscope (SEM) with a lanthanum hexaboride ($LaB_6$) filament.

**Example 6**

One of the most promising aspects of polyamine compounds is that preliminary data suggests that these compounds address the characteristics of biofilms that have made them resistant to antibiotic treatment. Specifically, polyamine compounds have potent antimicrobial activity against very high inocula of bacteria and kill bacteria in a rapid fashion. Without meaning to be bound by theory, initial characterization suggests that polyamine compounds are membrane-active, meaning they are nonspecific in nature. This nonspecific action reduces the potential for bacteria to upregulate their defense mechanisms and reduces the risk of resistance development. From the data collected to date, it does not appear that polyamine compounds are limited to eradicating bacteria that are in log-phase growth as indicated by their activity against bacteria in the biofilm phenotype. Bacteria in a biofilm have reduced metabolic activity, which is one of the primary contributing factors that allows biofilms to resist traditional antibiotic therapy. Typically, antibiotics affect bacteria in log-phase growth. Finally, polyamine compounds have the ability to disperse biofilms while demonstrating antimicrobial kill. By dispersing and killing bacteria in a biofilm, it is hypothesized that polyamine compounds interrupt water channels and the community as a whole, allowing for distribution of polyamine compounds to a greater number, if not all, of the cells within a biofilm.

A key aspect of polyamine compounds is the process by which they are synthesized to provide triple action against bacteria and biofilms. The ability to synthesize antimicrobial compounds with specific activity against biofilms is significant. Traditional approaches to discovering or developing antibiotics has primarily been done through cumbersome, high-throughput screening methods with limited results wherein perhaps 1 in 1,000 compounds may be of interest. Using the below method, our group was able to produce a lead product after synthesizing approximately 20 compounds.

Organisms residing in biofilms present a complex extracellular matrix of polysaccharides (exopolysaccharides) and proteins. As a result of this complex matrix, nutrient limiting conditions may exist that alter the normal or planktonic metabolic state. As stated above, this produces to a reduced efficacy of traditional antibiotic agents, rendering them up to 1,000 times less active. A hallmark of these exopolysachharides is the presentation of acidic residues from repeated glucoronic acid motifs and pyruvate derived acetals. A recent study by Losick and co-workers demonstrated that the simple polyamines spermine and norspermidine were naturally occurring inhibitors of biofilm formation, endogenously produced at high concentrations (50-80 μM) in response to nutrient limiting conditions and waste accumulation in mature pellicles. In this study they were able to demonstrate that norspermidine could inhibit biofilm formation at 25 μM and showed that at similar concentrations it could disperse the exopolysaccharide component of the matrix but not the protein component. This does suggest, however, that these agents might be capable of disassembling established biofilms. Interestingly, spermidine was only active at much higher concentrations (~ 1 mM) leading them to propose a rationale for this activity in the ability of the polyamines to engage the acidic residues in the matrix at regular intervals.

Given the lack of activity for spermidine vs. norspermidine, it is interesting to note that many secondary metabolites containing the polyamine frameworks have been identified as potent antimicrobials against planktonic bacteria. Most notably squalamine, which contains a spermidine tail, and the related natural product incorporating a spermine tail show potent antimicrobial activity against Gram-positive and Gram-negative bacteria (FIG. 24). Lysine-rich cationic peptides such as magainin and pexiganin are also potent anti-microbials against a wide range of organisms and have been developed as topical antimicrobials for the treatment of diabetic foot ulcers.

Of note is the incorporation of hydrophobic and cationic residues that are present in these antimicrobial "kill" compounds, without adhering to the strict hydrogen bond donor-acceptor pattern of the biofilm disruptors. Thus, an initial study was made to investigate the utility of combining a simplified hydrophobic backbone with a cationic tail that might impart molecules with the ability to inhibit biofilm formation, disrupt established biofilms and kill the emerging planktonic bacteria. The study began with the synthesis of benzylic substituted polyamines (FIG. 25). The synthesis of diaminopropane substituted backbones is straightforward to provide CZ-4,12,32 from the known mono-Boc protected diaminopropane and the commercially available aldehydes (benzaldehyde, isopthalaldehyde or 1,3,5-benzenetricarboxaldehyde). This three-step synthetic procedure proceeds via reductive amination and acidic removal of the Boc group. Of note is that no purification is required until a final recrystallization of the HCl salt. The norspermidine series (CZ-7,25,52) can be prepared in a similar manner from the mono-Boc protected norspermidine. Of note is that no purification is

required until a final recrystallization of the HCl salt, which allows easy preparation of these compounds on at least a ~ 10 g scale.

In some cases, increasing number of polyamine chains on the hydrophobic backbone systematically increases activity. For example, with a single chain of diaminopropane added to a benzyl backbone (CZ-4, FIG. 25), the minimum inhibitory concentration (MIC; as defined by the Clinical and Laboratory Standards Institute (CLSI)) against MRSA is greater than 1,200 μg/mL, whereas two chains decrease the MIC to 300 μg/mL (CZ-12) and three chains reduce it further to 45 μg/mL (CZ-32). Similarly, with a single chain of norspermidine attached to a benzyl backbone (CZ-7, FIG. 25), the MIC against MRSA is greater than 1,200 μg/mL. By adding a second chain of norspermidine (CZ-25), the MIC is 45 μg/mL. With a third chain of norspermidine attached (CZ-52), the MIC becomes 3 μg/mL.

Concurrently to the other goals of this project, a focused synthetic effort will continue to exploit this trend and generate related chemotypes with increased efficacy (e.g., compounds with four polyamine chains). This is expected to be accomplished, for example, via Pd(II) mediated dimerization of 5-bromoisopthalaldehyde followed by reductive amination (FIG. 26).

**Example 7**

*Evaluation of Biofilm Remediation and Removal Chemistries*

A series of experiments is conducted to investigate the impact of biofilm remediation and removal chemistries on mimetic multispecies biofilms commonly associated with anthropogenic heating and cooling water systems.

The criteria for success is to demonstrate that these chemistries:

(1) Remove a multispecies biofilm from a copper, mild steel, galvanized steel, and stainless steel substrate to a greater extent than that which can be achieved by the existing water treatment chemistries.

(2) Do not aggressively corrode the metal substrates when used in an optimum concentration.

(3) Avoid material compatibility issues with the existing treatment chemicals used to minimize scale formation and prevent corrosion of the metal parts of the anthropogenic heating and cooling system.

**Biofilm Generation on the Metal Coupons**

Normally, monoculture bacteria belonging to the *Pseudomonas* genus have been used to investigate the physical and mechanical properties of biofilm on different heat exchanger surfaces and substrates under changing flow conditions. However, to more accurately represent conditions that would be found in the real environment, a mixture of *Pseudomonas fluorescens* and *Pseudomonas putida* together with a 0.1% mixture of NCH 3010 *Bacillus subtilis,* NCH 3032 *Bacillus subtilis,* NCH 3016 *Bacillus licheniformis,* and NCH 3040 *Bacillus thuringiensis* all of which are naturally occurring soil bacteria is used. The microorganisms will be provided in pellet form from Eco-Bionics, and each pellet contains a starter culture and appropriate nutrients that produce actively growing bacteria with the addition of water.

The experimental test procedure is a modified version of the single tube method. Biofilm are grown on the metal coupons using a biofilm reactor designed around a continuous stir tank reactor (FIG. 21). Prior to operation the reaction chamber and the individual components are disassembled, soaked in a 10% bleach solution, then scrubbed in hot soapy water and rinsed in distilled water. Once cleaned, the stir tank reactor is charged with 2 liters of DI water, and 20 g of the Free Flow pellets will be added to the water. Coupons are placed onto the coupon holding rods, which is then inserted into the Free Flow pellet solution. The reactor runs for 6 days with the Free Flow solution being replaced every 2 days.

Biofilm growth isvisually observed and documented photographically. To quantify the presence of viable microorganisms within the biofilm structure formed on the metal coupons (see FIG. 17 for an example of the typical biofilm formed on the substrate) the biofilm covered coupons is removed from the reactor and lightly rinsed with sterile buffer to remove any loose debris from the surface. The coupons are then transferred to a container with 40 ml of Phosphate buffer solution (PBS) and vortexed for 30 sec then placed in a sonication bath (Branson model 3200) for 30 sec. After sonication, the container with the buffer and coupon is removed and vortexed again for 30 sec before being returned to the sonication bath for an additional 30 sec of processing. A third and final vortexing step ensures that the adhered biofilm will be thoroughly removed. After processing to remove the bioflim the PBS solution will be serially diluted onto tryptic soy agar (TSA) plates which will be incubated at 30 °C for up to 36 hours before being enumerated.

The biofilm coupons are exposed to a high, medium, and low concentration of the product (based on the recommended dosing rates) for a prescribed time. After treatment, the coupons are removed, and the eluant and treatment solutions are assayed to enumerate viable bacteria A standard dispersant used for biofilm removal, together with a standard biocide (oxidizing and non-oxidizing) used at the recommended label use concentrations, are used as controls.

**Corrosion Testing**

Treatment solution of the same concentrations used in the experiments above is prepared with standard corrosion inhibitor products and calcium deposit products in regular tap water and tested in a standard corrosion test. The solution is added to a treatment tank that is stirred continually for two weeks, after which the coupons are removed and analyzed for corrosion using standard corrosion test methods. Corrosion rates of less than 3.0 mpy for mild steel and 0.2 mpy for copper are considered non corrosive. A standard dispersant used for biofilm removal together with a standard biocide (oxidizing and non-oxidizing) used at the recommended label use concentrations are used as controls.

**Conclusions**

We have been able to determine minimum amounts of our polyamine compounds that will have activity against biofilms of P. aeruginosa. In general, the polyamine compounds do better against Bacilli than P. aeruginosa, so what is active against P. aeruginosa is likely active against Bacilli.
These formulas are made in PBS, water or broth.

**Example 8**

***Methods for MIC, MBEC and EBEC Analysis***

To determine the MIC of compounds the following procedure was used.
A 0.5 McFarland of *A. baumannii* is made. A 0.5 McFarland is a measure of turbidity in a liquid sample that contains approximately $7.5 \times 10^7$ CFU/mL. The 0.5 McFarland standard is diluted in CAMHB and 50 $\mu$L of broth is added to a well of a 96-well plate. In addition, 50 $\mu$L of CAMHB that contains a desired concentration of antimicrobial is added to the well for a final volume of 100 $\mu$L and a final concentration of approximately $5 \times 10^4$ CFU/well (which equates to approximately $5 \times 10^5$ CFU/mL). Each well contains a desired amount of CZ compound in order to experimentally determine the MIC. Each 96-well plate is incubated at 37° C for 24 hours. The contents of each well is plated on tryptic soy agar (TSA). TSA plates is incubated at 37° C for 24 hours after which the number of CFUs is counted and used to calculate the CFU/mL that remain after exposure to varying concentrations of compound. This procedure is repeated n=8 times for each concentration of antimicrobial. The concentration of CZ compound that reduces bacteria from $10^5$ CFU/mL to $10^2$ CFU/mL in 24 hours is considered the MIC.
To determine the MBEC, the MBEC Inoculation Tray by Innovotech, formerly known as the Calgary biofilm device, is used. Within this device, biofilms grow on the surface of polystyrene pegs, 96 of which are attached to a lid. These pegs are inserted into a flat bottom 96-well plate. In this instance, the MBEC of a molecule is defined as the concentration of compound required to reduce approximately $10^6$ CFU/peg to approximately $10^2$ CFU/peg in a 24-hour period.
Following the manufacturer's guidelines, biofilms are grown on the surface of each peg by first making a 0.5 McFarland of *A. baumannii.* The 0.5 McFarland is diluted 1:100 in CAMHB broth. Into each well of a flat bottom 96-well plate, 150 $\mu$L of broth is pipetted. The plate is shaken at 100 rpm for 24 hours. The pegs are placed into a separate flat bottom 96-well plate for 10 seconds with 200 $\mu$L of phosphate buffered saline (PBS) in each well to remove nonadherent cells. The lid is placed into a 96-well plate that contains varying concentrations of antimicrobial with 200 $\mu$L of broth per well. The plate is incubated for 24 hours at 37° C after which time 100 $\mu$L of broth is plated on TSA. TSA plates is incubated 24 hours at 37° C and the number of CFU counted to calculate the CFU/peg.
In addition to determining the MIC and the MBEC of compounds against planktonic bacteria and low number biofilms, the efficacy of the compounds of the invention against high number biofilms is determined. To do so, biofilms are grown on the surface of PEEK membranes using a membrane biofilm reactor (Williams, D. L., Haymond, B. S. & Bloebaum, R. D. Use of delrin plastic in a modified CDC biofilm reactor. Res J Microbiol 6, 425-429 (2011); Williams, D. L., Woodbury, K. L., Haymond, B. S., Parker, A. E. & Bloebaum, R. D. A modified CDC biofilm reactor to produce mature biofilms on the surface of PEEK membranes for an in vivo animal model application. Curr Microbiol 62, 1657-1663 (2011); Williams, D. L. et al., In Vivo Efficacy of a Silicone - Cationic Steroid Antimicrobial Coating to Prevent Implant-Related Infection. Biomaterials 33, 8641-8656 (2012); Williams, D. L. et al., Experimental model of biofilm implant-related osteomyelitis to test combination biomaterials using biofilms as initial inocula. JBiomedMat Res A 100, 1888-1900 (2012)). This reactor is similar to the CDC biofilm reactor, but rather than growing biofilms on coupon surfaces, the reactor was modified to hold PEEK membranes. In short, to grow biofilms within this system, 500 mL of brain heart infusion (BHI) broth is inoculated with 1 mL of a 0.5 McFarland. The reactor is placed on a hot plate set at 34° C and the bacteria grown under batch conditions for 24 hours. A solution of 10% BHI will then be flowed through the reactor at a rate of 6.94 mL/min for an additional 24 hours. Following this protocol, biofilms typically grow to $10^9$ CFU/PEEK membrane. PEEK membranes is removed and placed into 2 mL of CAMHB that contain a desired concentration of the

test compound. The EBEC is defined as the concentration of antimicrobial required to reduce a biofilm from approximately $10^9$ CFU/PEEK membrane to approximately $10^2$ CFU/PEEK membrane in a 24-hour period.

***MIC, MBEC and EBEC Analysis***

To determine the MIC of the test compounds and traditional antibiotics, a protocol which defines the MIC as being the concentration of antimicrobial required to reduce the number of bacteria in a solution from $10^5$ colony forming units (CFU)/mL to $10^2$ CFU/mL in a 24-hour period. MICs for tests compounds as well as for select antibiotics are provided in Table 4. Consistent with what was mentioned previously, these data indicate that, with an increase in the number of polyamine chains attached to a lipophilic backbone, the MIC is lowered, indicating it has greater antimicrobial potential. CZ-7 has one chain of norspermidine attached, CZ-25 has two chains of norspermidine, and CZ-52 has three chains attached (FIG. 25).

The trend of increasing antimicrobial activity by increasing the number of polyamine chains attached to a backbone while also modulating the hydrophobicity of that backbone shows promising activity against *A. baumannii,* more specifically in the case of CZ-58 (FIG. 27). The analysis below shows that compounds of the invention have increased efficacy relative to current therapies (e.g., polymyxin B).

MBEC data were collected using the MBEC Inoculation Tray by Innovotech. The MBEC was defined in these preliminary results as the concentration of antimicrobial required to reduce $10^5$ or $10^6$ CFU/peg to $10^2$ CFU/peg in a 24-hour period. MBEC data are presented in Table 4.

**Table 4:** MICs, MBECs and EBECs of CZ compounds and traditional antibiotics. Data reported in μg/mL and μM concentrations.

| | **MIC μg/mL (μM)** | | **MBEC μg/mL (μM)** | | **EBEC μm/mL (μM)** | |
|---|---|---|---|---|---|---|
| **Compound** | *MRSA* | *A. baumannii* | *MRSA* | *A. baumannii* | *MRSA* | *A. baumannii* |
| **CZ-7** | >500 (>2,259) | >500 (>2,259) | >500 (>2,259) | >500 (>2,259) | >5,000 (>22,590) | >10,000 (>45,180) |
| **CZ-25** | 40 (110) | 150 (411) | 25 (69) | 450 (1234) | >5,000 (13,715) | ~7,500 |
| **CZ-52** | 3 (6) | 400 (800) | 20 (40) | >500 (995) | 250 (500) | ~7,000 (~ 14,000) |
| **CZ-58** | 6 (13.6) | 30 (68) | 30 (68) | >50 (>113) | 1,500 (3,403) | 400 (908) |
| **Vancomycin** | 10 (6.9) | - | >500 (>345) | - | >>25,000 (>>17,225) | - |
| **Polymyxin B sulfate** | - | 1 (0.7) | - | 50 (36) | - | 1,000 (722) |

EBEC data were collected to determine the efficacy of CZ-52, CZ-58 and traditional antibiotics against high number biofilms containing approximately $10^9$ CFU grown on the surface of a polyetheretherketone (PEEK) membrane. Results are shown in Table 4. EBEC data showed striking differences in efficacy between CZ-52 and vancomycin (Table 4). At 25,000 μg/mL, vancomycin did not have the ability to reduce biofilms of MRSA by even 1 $\log_{10}$ unit. In contrast, at 250 μg/mL CZ-52 was able to reduce MRSA biofilms by greater than 7 $\log_{10}$ units in a 24-hour period. Although the MIC of CZ-58 was lower against MRSA compared to *A. baumannii,* CZ-58 had greater activity against biofilms of *A. baumannii* than against MRSA biofilms in the EBEC assay. The EBEC of polymyxin B against *A. baumannii* was 1,000× greater than the MIC, whereas the EBEC for CZ-58 was only ~13x greater than the MIC. Although the MIC of CZ-58 was "high" compared to MICs of traditional antibiotics, the efficacy against biofilms was promising.

Table 5 provides MIC results for a variety of compounds against MRSA, *Pseudomonas aeruginosa,* and *Acinetobacter baumannii.* The compounds CZ-58, CZ-62, CZ-65, CZ-66, CZ-67, CZ-69, CZ-70, CZ-73, CZ-74, CZ-75, CZ-77, CZ-83, CZ-86, CZ-86 Free base, CZ-92, CZ-94, CZ-96, CZ-99, CZ-111, CZ-114, CZ-115, CZ-116, CZ-117 CZ-118 in table 5 are part of the invention. The other compounds appearing in table 5 are not.

**Table 5:** MICs of CZ compounds.

| CZ No. | Compound | MIC (MRSA) | MIC (P. *aeruginosa)* | MIC (A. *bumannii)* |
| --- | --- | --- | --- | --- |
| CZ-3 | 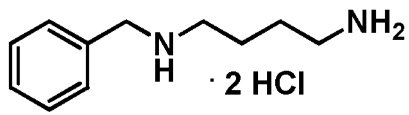 | + | + | |
| CZ-4 | 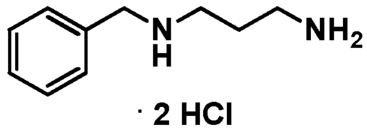 | + | + | |
| CZ-5 | 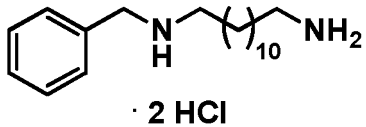 | + | | |
| CZ-6 | 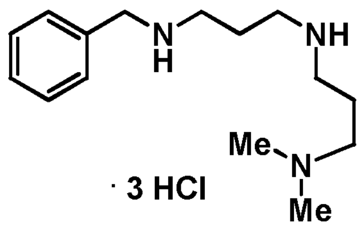 | + | | |
| CZ-7 | 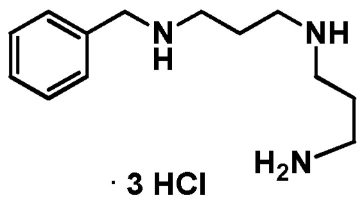 | + | + | |
| CZ-8 | 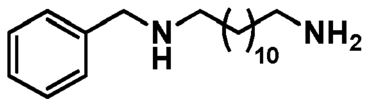 | + | | |

(continued)

| CZ No. | Compound | MIC (MRSA) | MIC (P. *aeruginosa)* | MIC (A. *bumannii)* |
|---|---|---|---|---|
| CZ-9 | 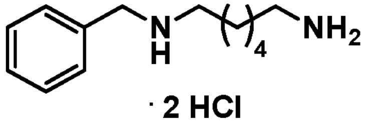 · 2 HCl | + | | |
| CZ-10 | 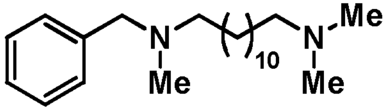 · 2 HCl | + | | |
| CZ-11 | 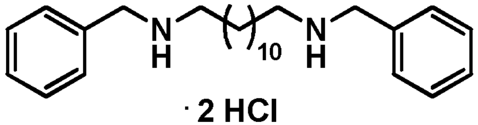 · 2 HCl | + | | |
| CZ-12 | 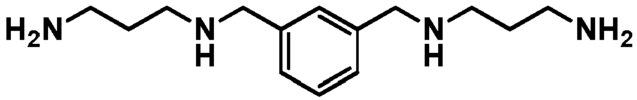 · 4 HCl | + | ++ | |
| CZ-13 | 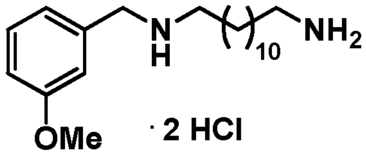 · 2 HCl | | | |
| CZ-14 | 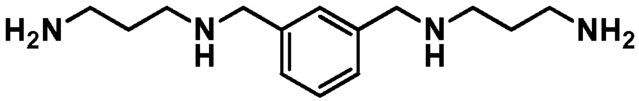 | + | | |

(continued)

| CZ No. | Compound | MIC (MRSA) | MIC (P. *aeruginosa)* | MIC (A. *bumannii)* |
|---|---|---|---|---|
| CZ-15 | 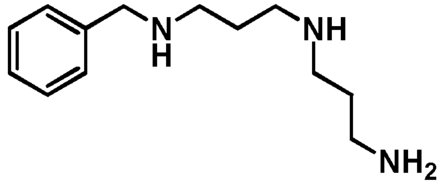 | + | | |
| CZ-16 | 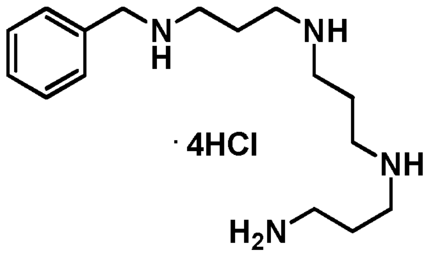 · 4HCl | + | | |
| CZ-19 | 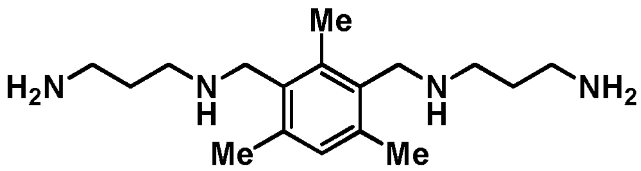 · 4HCl | + | | |
| CZ-21 | 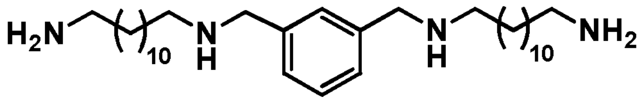 | +++ | | + |
| CZ-22 | 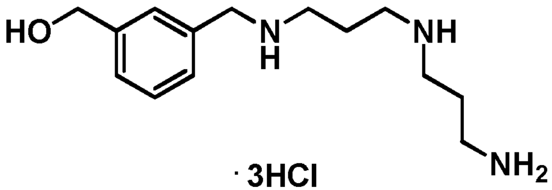 · 3HCl | ++ | | |

(continued)

| CZ No. | Compound | MIC (MRSA) | MIC (P. *aeruginosa)* | MIC (A. *bumannii)* |
|---|---|---|---|---|
| CZ-24 | 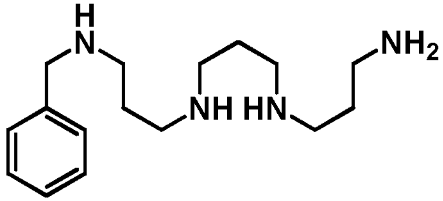 | | | |
| CZ-25 | 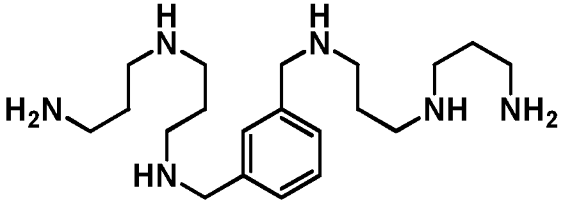 · 6 HCl | +++ | + | + |
| CZ-25 (Bz) | 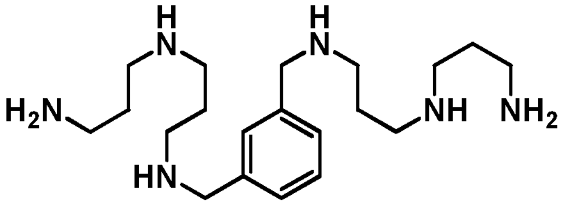 · 6 HOBz | +++ | | |
| CZ-26 | 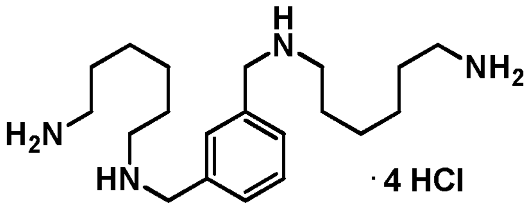 · 4 HCl | ++ | + | |

(continued)

| CZ No. | Compound | MIC (MRSA) | MIC (P. *aeruginosa)* | MIC (A. *bumannii)* |
|---|---|---|---|---|
| CZ-27 | 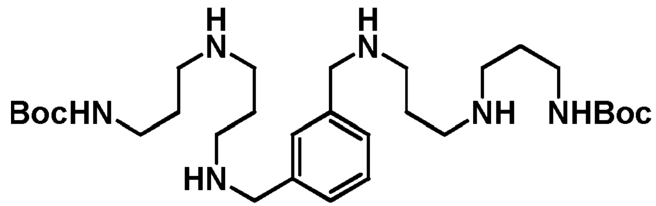 | + | | |
| CZ-32 | 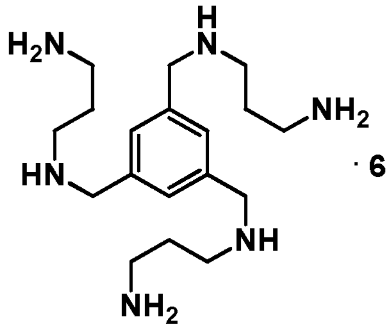 · 6 HCl | ++ | ++ | |
| CZ-33 | 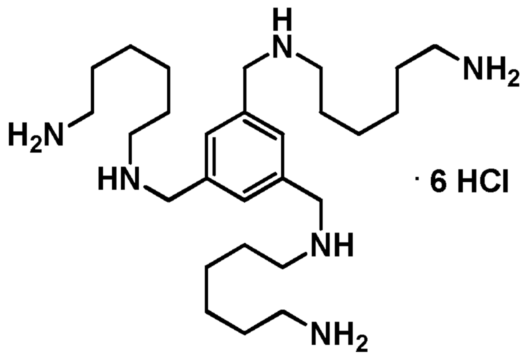 · 6 HCl | ++ | + | |

(continued)

| CZ No. | Compound | MIC (MRSA) | MIC (P. *aeruginosa)* | MIC (A. *bumannii)* |
|---|---|---|---|---|
| CZ-40 | 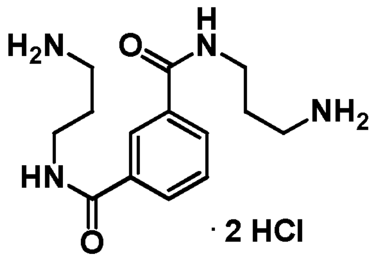 · 2 HCl | + | + | |
| CZ-41 | 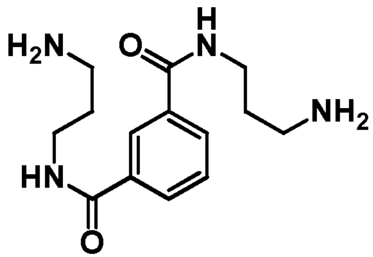 | + | | |
| CZ-42 | 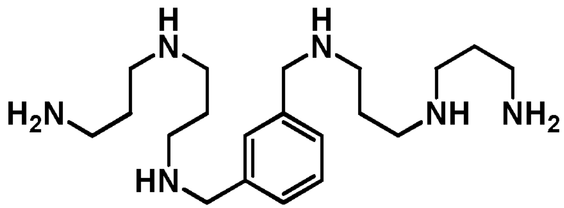 | ++ | ++ | |

(continued)

| CZ No. | Compound | MIC (MRSA) | MIC (P. *aeruginosa)* | MIC (A. *bumannii)* |
|---|---|---|---|---|
| CZ-43 | 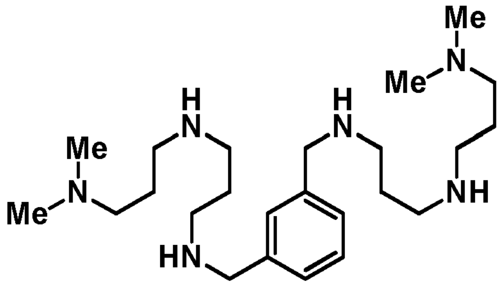 · 6 HCl | + | + | |
| CZ-46 | 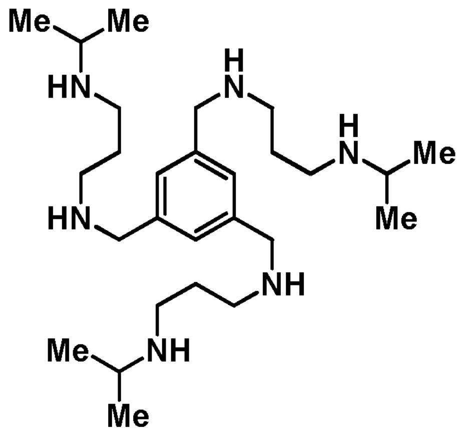 | | | |

(continued)

| CZ No. | Compound | MIC (MRSA) | MIC (P. *aeruginosa)* | MIC (A. *bumannii)* |
|---|---|---|---|---|
| CZ-51 | 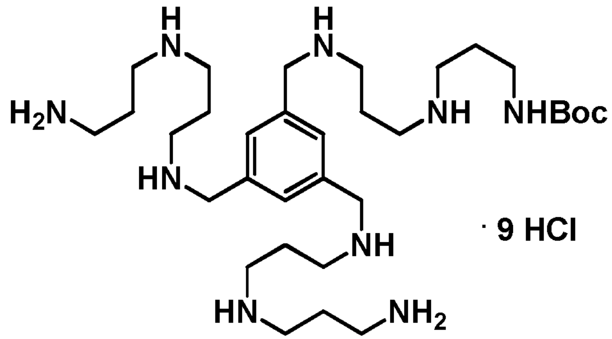 · 9 HCl mixture of mono / di / tri-Boc | +++ | +++ | |
| CZ-52 | 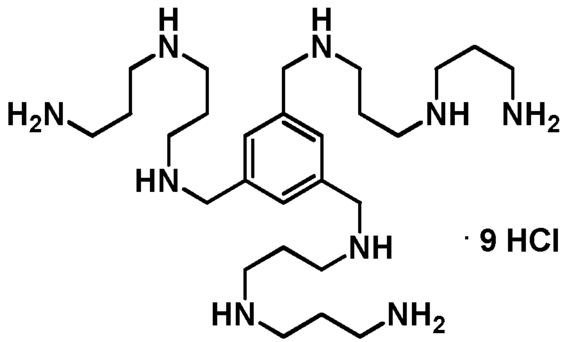 · 9 HCl | +++ | +++ | + |
| CZ-53 | 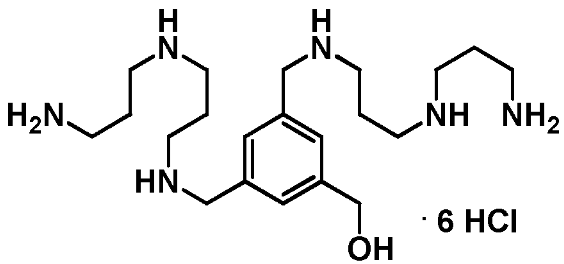 · 6 HCl | +++ | ++ to +++ | |

(continued)

| CZ No. | Compound | MIC (MRSA) | MIC (P. *aeruginosa)* | MIC (A. *bumannii)* |
|---|---|---|---|---|
| CZ-54 | 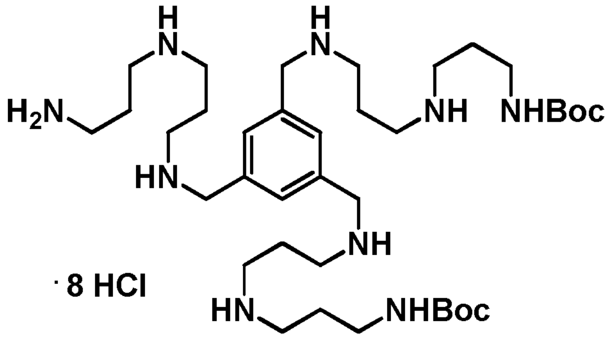 · 8 HCl | +++ | +++ | |
| CZ-55 | 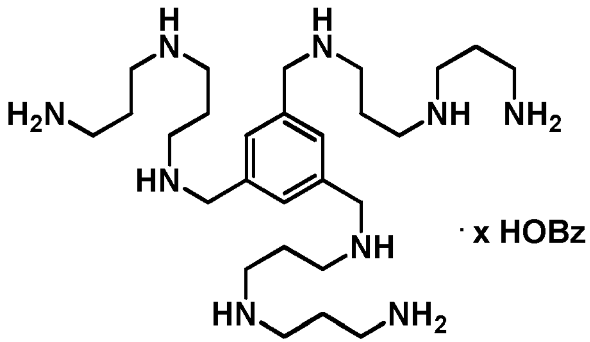 · x HOBz | +++ | +++ | + |
| CZ-56 | 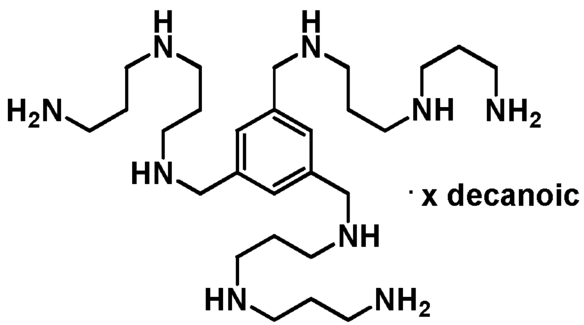 · x decanoic acid | | | |

(continued)

| CZ No. | Compound | MIC (MRSA) | MIC (P. *aeruginosa)* | MIC (A. *bumannii)* |
|---|---|---|---|---|
| CZ-57 | 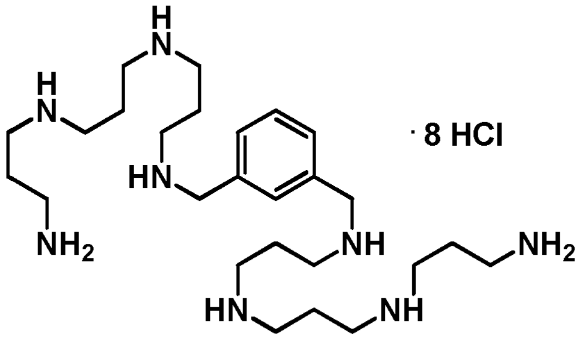 · 8 HCl | +++ | + | |
| CZ-58 | 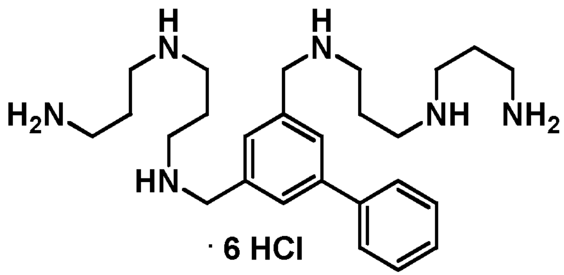 · 6 HCl | +++ | ++ to +++ | ++ |
| CZ-60 | 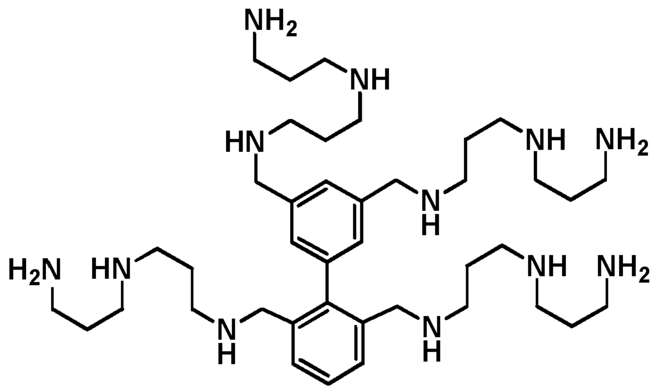 | +++ | + | + |

(continued)

| CZ No. | Compound | MIC (MRSA) | MIC (P. *aeruginosa)* | MIC (A. *bumannii)* |
|---|---|---|---|---|
| CZ-61 | 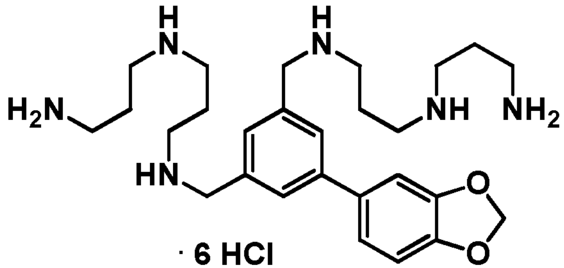 · 6 HCl | +++ | +++ | +++ |
| CZ-62 | 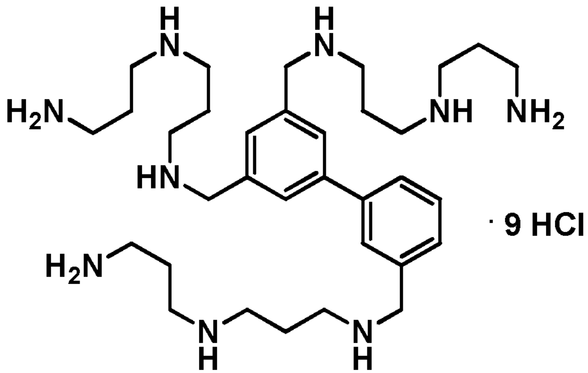 · 9 HCl | +++ | +++ | +++ |
| CZ-63 | 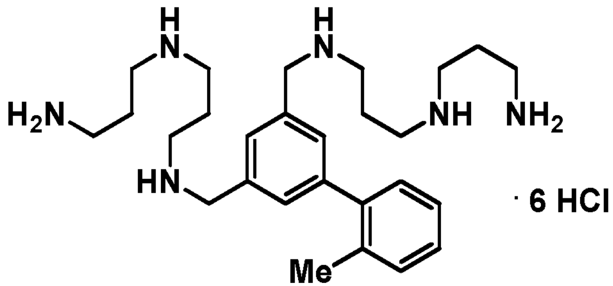 · 6 HCl | +++ | +++ | +++ |

(continued)

| CZ No. | Compound | MIC (MRSA) | MIC (P. *aeruginosa)* | MIC (A. *bumannii)* |
|---|---|---|---|---|
| CZ-64 | 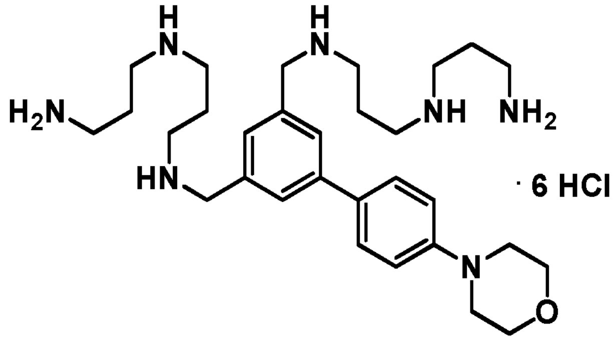 | | | |
| CZ-65 | 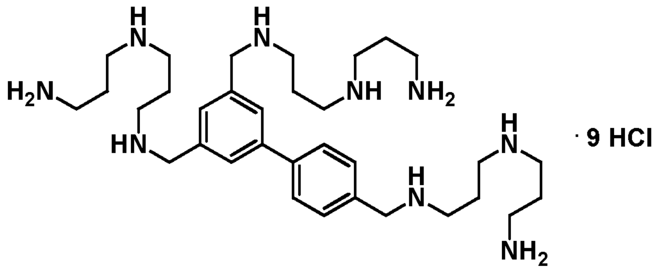 | +++ | +++ | +++ |
| CZ-66 | 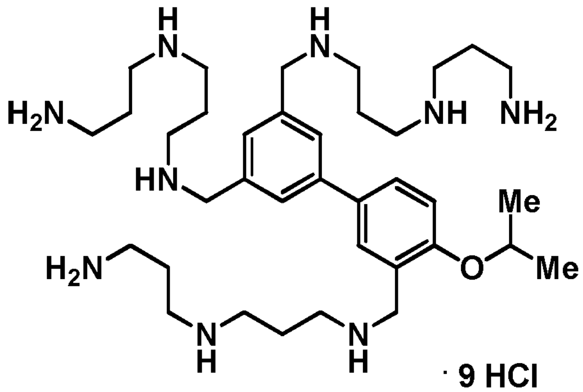 | +++ | +++ | |

(continued)

| CZ No. | Compound | MIC (MRSA) | MIC (P. *aeruginosa)* | MIC (A. *bumannii)* |
|---|---|---|---|---|
| CZ-67 | 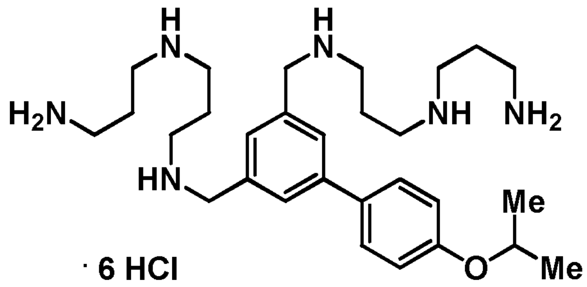 · 6 HCl | | | |
| CZ-68 | 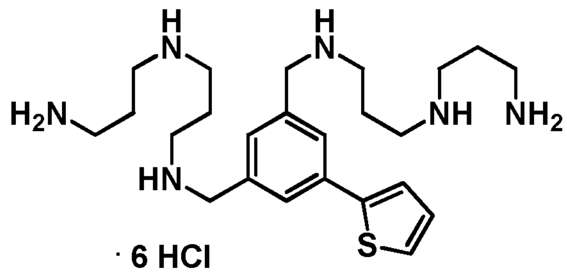 · 6 HCl | | | |
| CZ-69 | 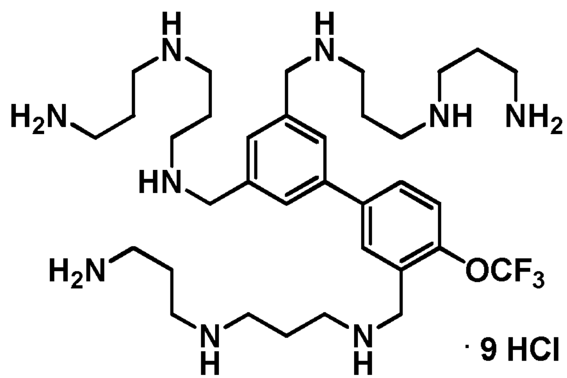 · 9 HCl | +++ | +++ | |

(continued)

| CZ No. | Compound | MIC (MRSA) | MIC (P. *aeruginosa)* | MIC (A. *bumannii)* |
|---|---|---|---|---|
| CZ-70 | 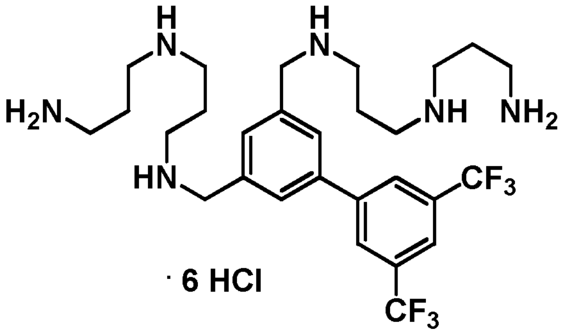 · 6 HCl | +++ | +++ | |
| CZ-71 | 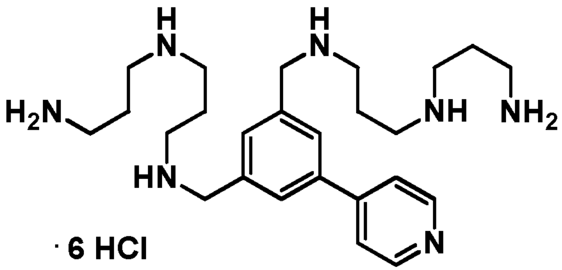 · 6 HCl | +++ | + | |
| CZ-72 | 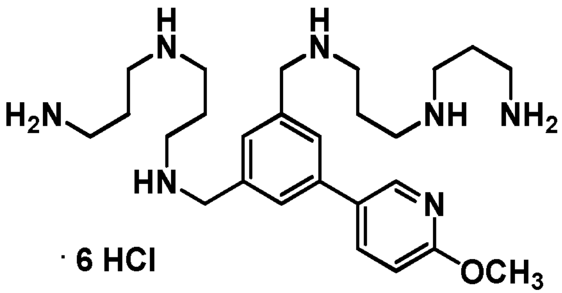 · 6 HCl | +++ | +++ | |

(continued)

| CZ No. | Compound | MIC (MRSA) | MIC (P. *aeruginosa)* | MIC (A. *bumannii)* |
|---|---|---|---|---|
| CZ-73 | 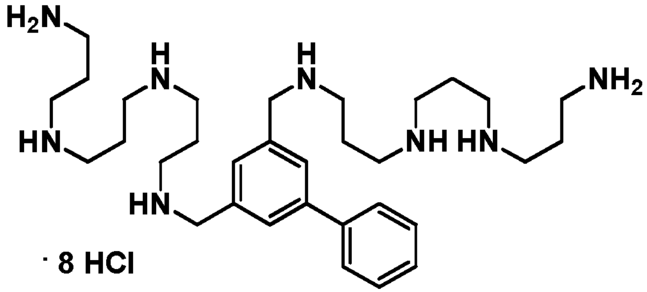 · 8 HCl | +++ | +++ | ++ |
| CZ-74 | 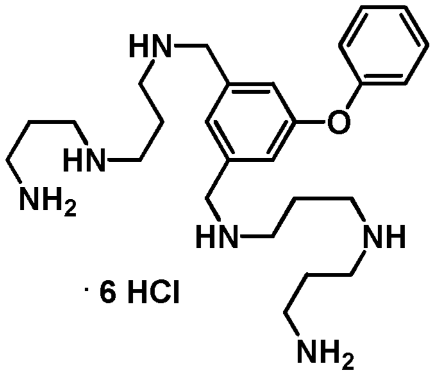 · 6 HCl | +++ | +++ | |
| CZ-75 | 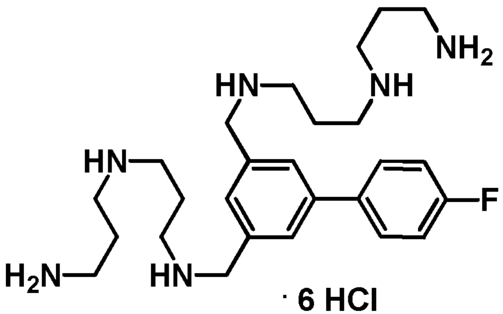 · 6 HCl | +++ | +++ | |

(continued)

| CZ No. | Compound | MIC (MRSA) | MIC (P. *aeruginosa)* | MIC (A. *bumannii)* |
|---|---|---|---|---|
| CZ-76 | 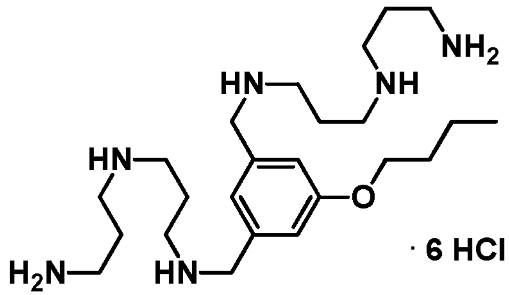 · 6 HCl | +++ | ++ | |
| CZ-77 | 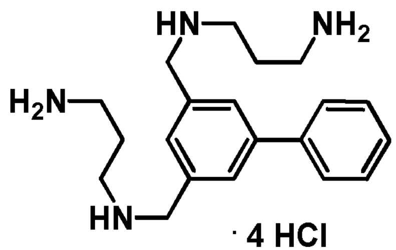 · 4 HCl | +++ | +++ | |
| CZ-81 | 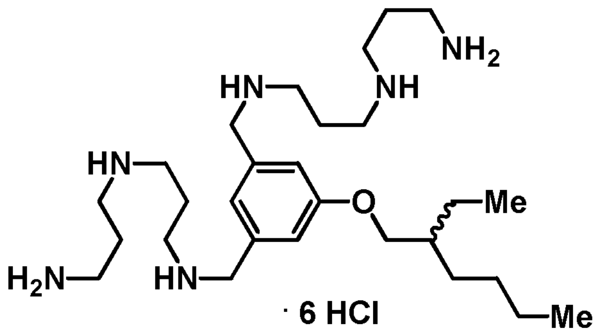 · 6 HCl | +++ | +++ | +++ |

(continued)

| CZ No. | Compound | MIC (MRSA) | MIC (P. *aeruginosa)* | MIC (A. *bumannii)* |
|---|---|---|---|---|
| CZ-83 | 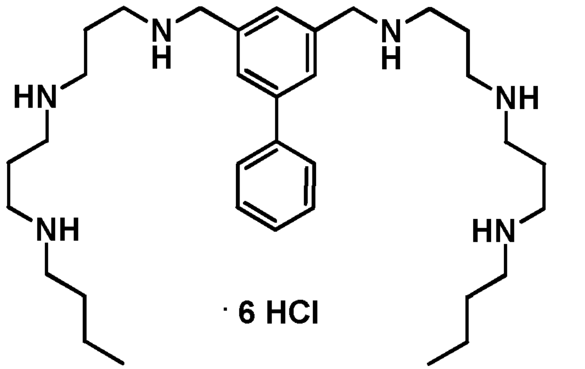 · 6 HCl | +++ | +++ | +++ |
| CZ-84 | 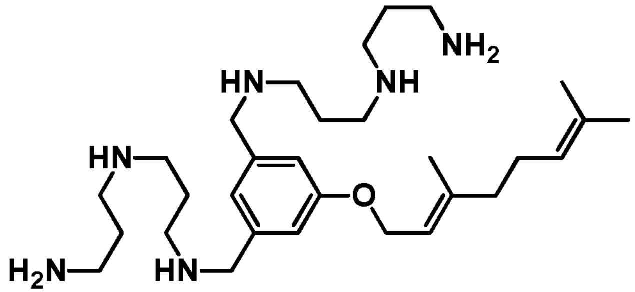 | | | |
| CZ-86 | 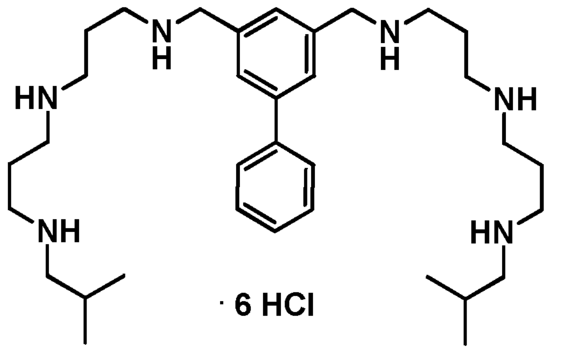 · 6 HCl | +++ | ++ to +++ | +++ |

(continued)

| CZ No. | Compound | MIC (MRSA) | MIC (P. *aeruginosa)* | MIC (A. *bumannii)* |
|---|---|---|---|---|
| CZ-86 Free Base | 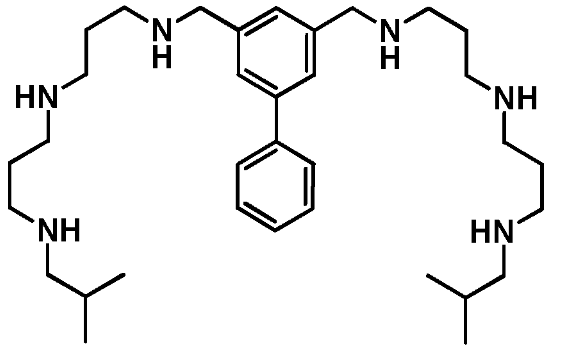 | | | |
| CZ-87 | 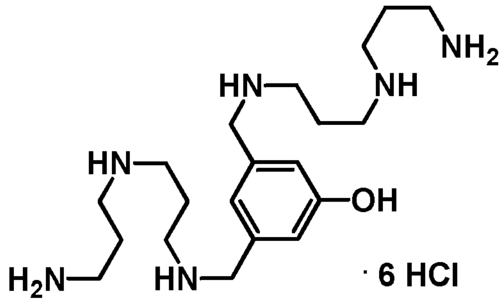 · 6 HCl | +++ | +++ | |
| CZ-88 | 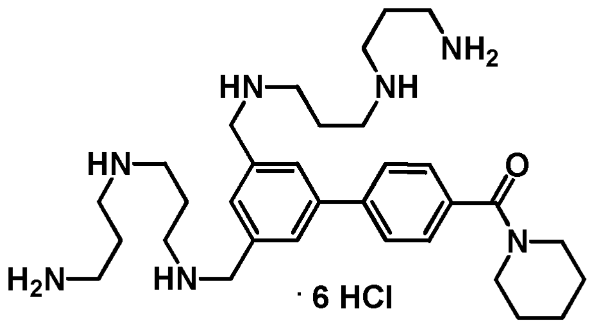 · 6 HCl | +++ | +++ | |

(continued)

| CZ No. | Compound | MIC (MRSA) | MIC (P. *aeruginosa)* | MIC (A. *bumannii)* |
|---|---|---|---|---|
| CZ-89 | 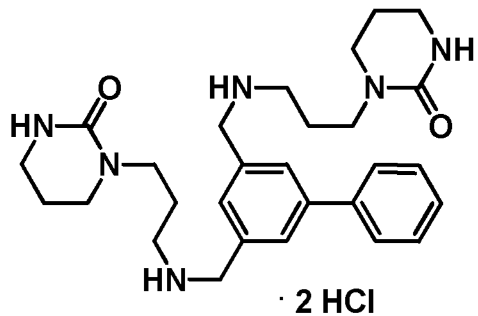 · 2 HCl | +++ | +++ | |
| CZ-90 | 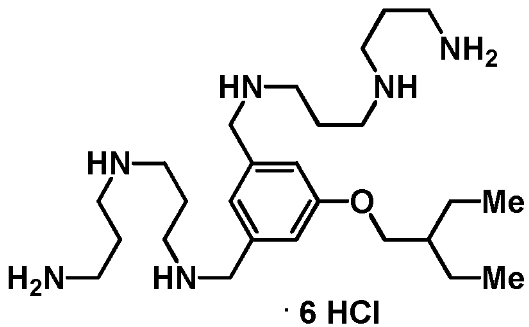 · 6 HCl | +++ | +++ | |

(continued)

| CZ No. | Compound | MIC (MRSA) | MIC (P. *aeruginosa)* | MIC (A. *bumannii)* |
|---|---|---|---|---|
| CZ-91 | 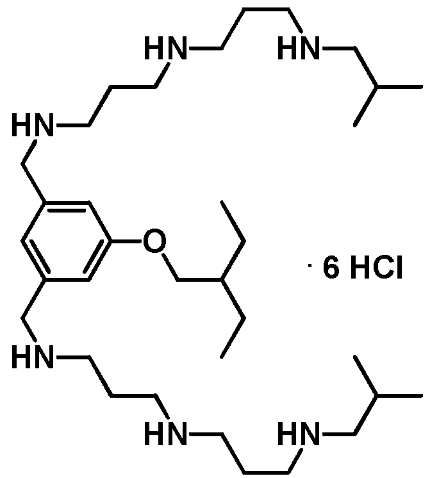 · 6 HCl | +++ | +++ | |
| CZ-92 | 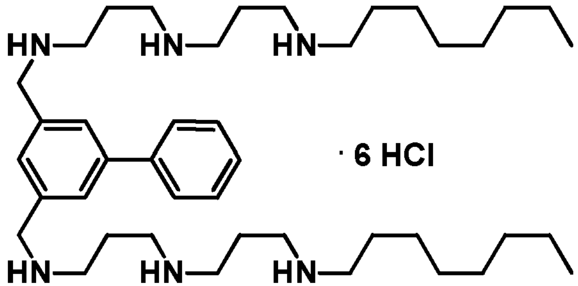 · 6 HCl | +++ | +++ | |
| CZ-93 | 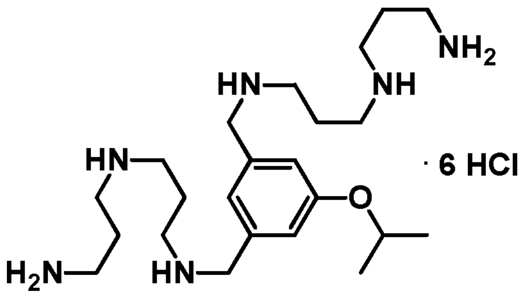 · 6 HCl | +++ | +++ | |

(continued)

| CZ No. | Compound | MIC (MRSA) | MIC (P. *aeruginosa)* | MIC (A. *bumannii)* |
|---|---|---|---|---|
| CZ-94 | 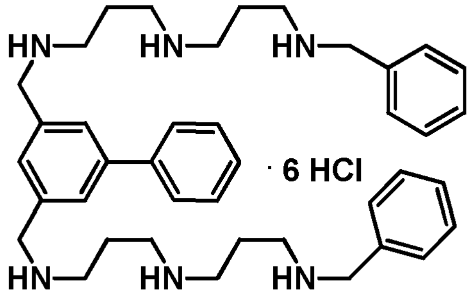 · 6 HCl | +++ | +++ | |
| CZ-95 | 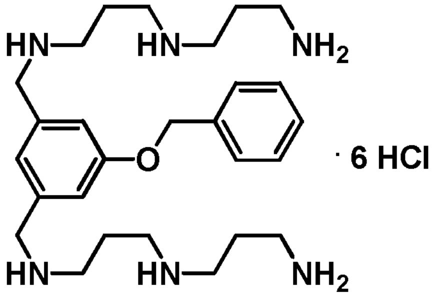 · 6 HCl | +++ | +++ | |
| CZ-96 | 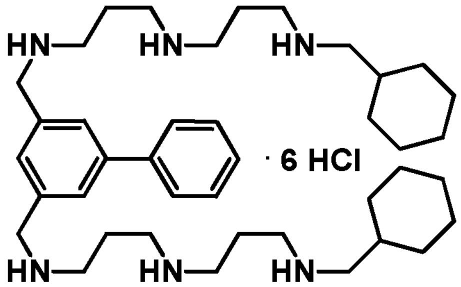 · 6 HCl | +++ | +++ | |

(continued)

| CZ No. | Compound | MIC (MRSA) | MIC (P. *aeruginosa)* | MIC (A. *bumannii)* |
|---|---|---|---|---|
| CZ-97 | 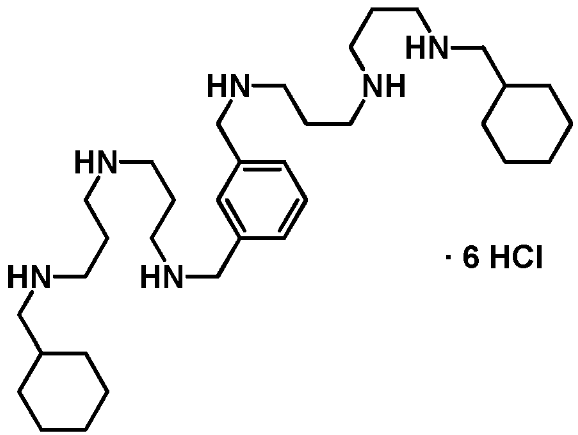 · 6 HCl | +++ | +++ | |
| CZ-98 | 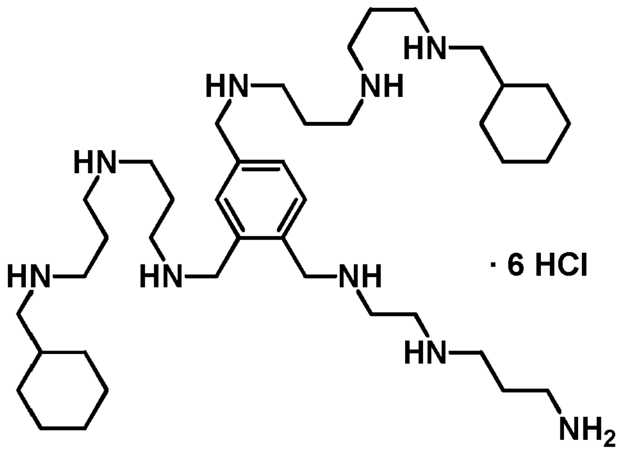 · 6 HCl | | | |

(continued)

| CZ No. | Compound | MIC (MRSA) | MIC (P. *aeruginosa)* | MIC (A. *bumannii)* |
|---|---|---|---|---|
| CZ-99 | 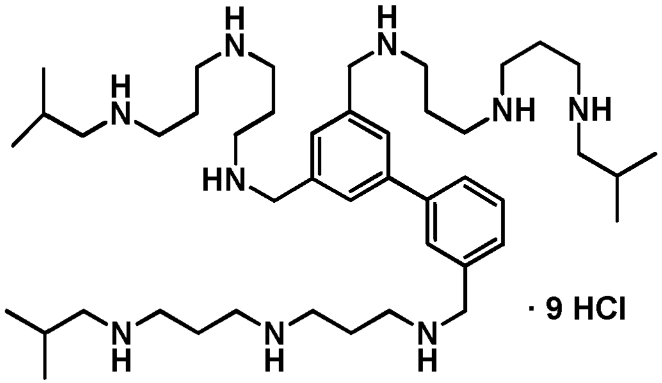 · 9 HCl | +++ | ++ to +++ | |
| CZ-100 | 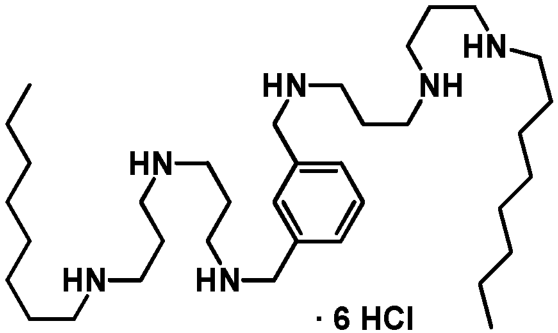 · 6 HCl | +++ | +++ | +++ |
| CZ-101 | 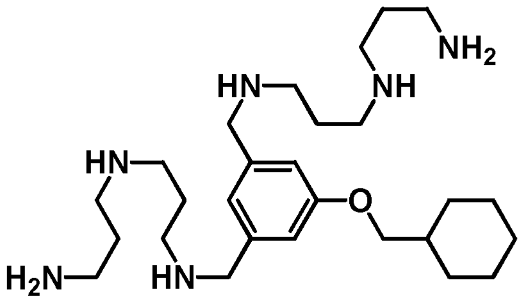 · 6 HCl | +++ | +++ | |

(continued)

| CZ No. | Compound | MIC (MRSA) | MIC (P. *aeruginosa)* | MIC (A. *bumannii)* |
|---|---|---|---|---|
| CZ-102 | 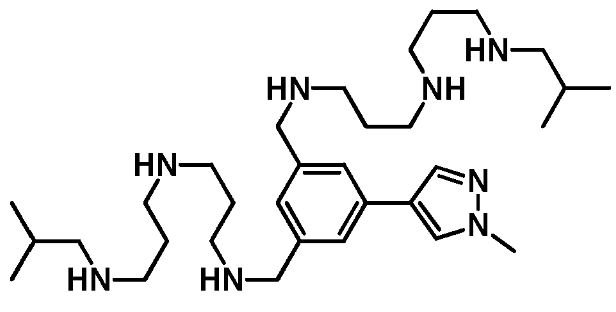 · 6 HCl | +++ | ++ | |
| CZ-103 | 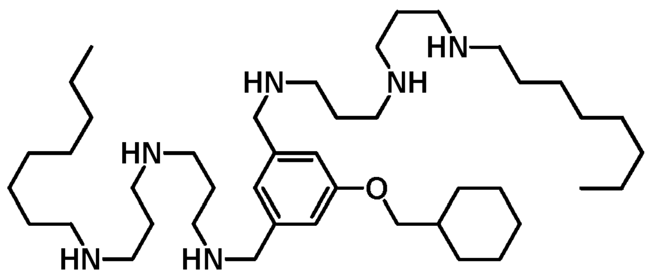 · 6 HCl | +++ | +++ | +++ |
| CZ-104 | 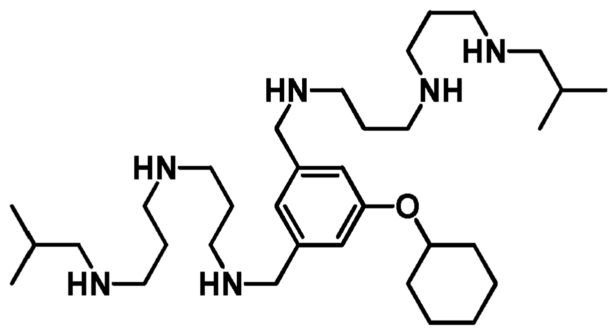 | +++ | ++ to +++ | |

(continued)

| CZ No. | Compound | MIC (MRSA) | MIC (P. *aeruginosa)* | MIC (A. *bumannii)* |
|---|---|---|---|---|
| CZ-105 | 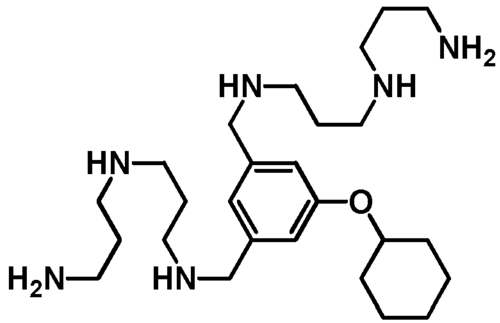 · 6 HCl | +++ | ++ | |
| CZ-106 | 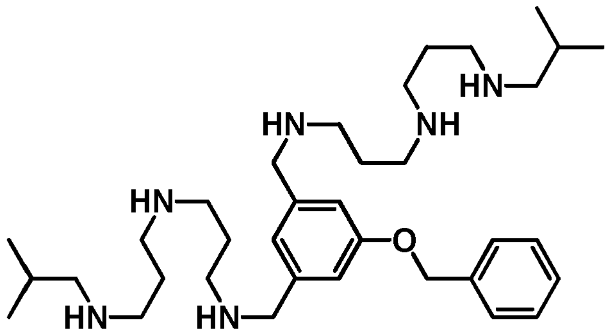 · 6 HCl | +++ | ++ | |

(continued)

| CZ No. | Compound | MIC (MRSA) | MIC (P. *aeruginosa)* | MIC (A. *bumannii)* |
|---|---|---|---|---|
| CZ-107 | 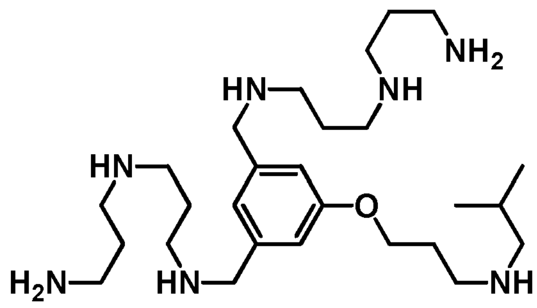 · 7 HCl | +++ | ++ | |
| CZ-108 | 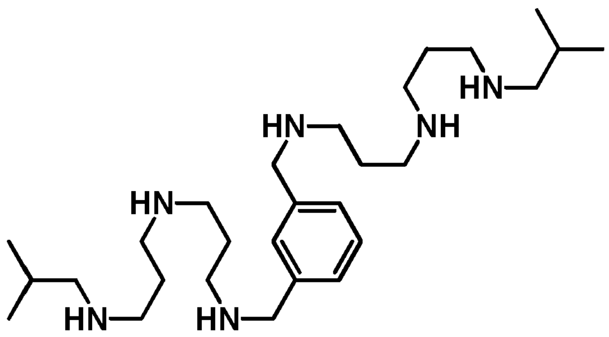 · 6 HCl | +++ | +++ | |

(continued)

| CZ No. | Compound | MIC (MRSA) | MIC (P. *aeruginosa)* | MIC (A. *bumannii)* |
|---|---|---|---|---|
| CZ-109 | 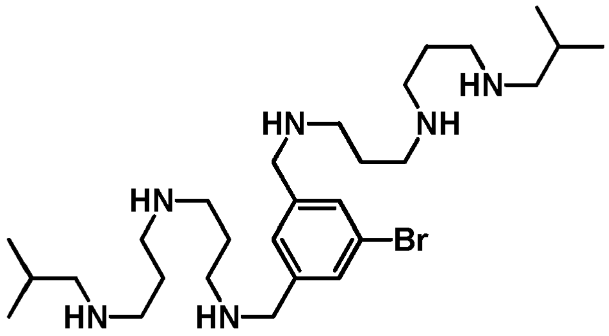 · 6 HCl | +++ | +++ | |
| CZ-110 | 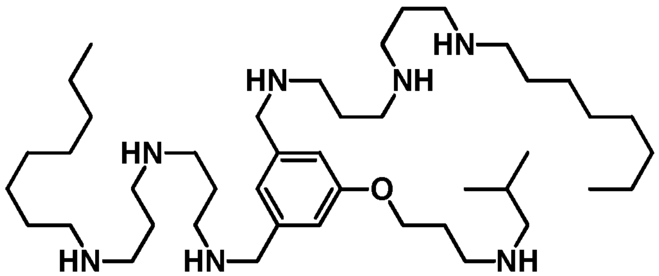 · 6 HCl | +++ | +++ | +++ |
| CZ-111 | 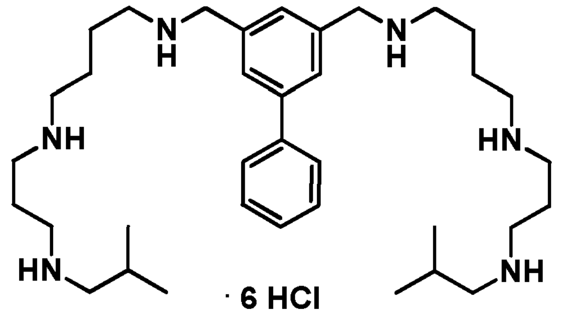 · 6 HCl | +++ | +++ | |

(continued)

| CZ No. | Compound | MIC (MRSA) | MIC (P. *aeruginosa)* | MIC (A. *bumannii)* |
|---|---|---|---|---|
| CZ-112 | 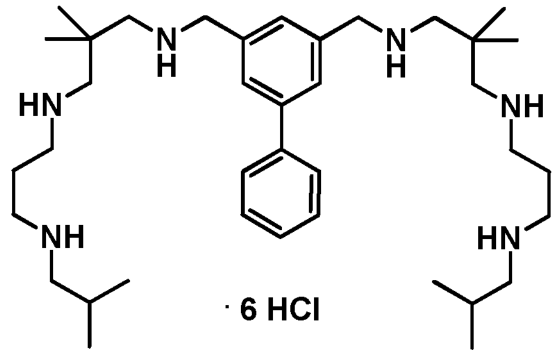 · 6 HCl | +++ | +++ | |
| CZ-113 | 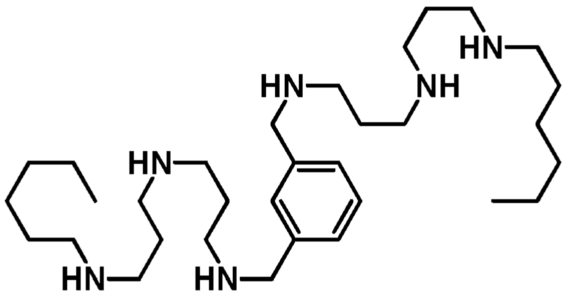 · 6 HCl | +++ | +++ | |
| CZ-114 | 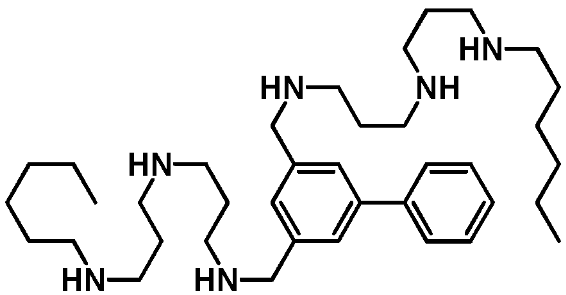 · 6 HCl | +++ | +++ | |

(continued)

| CZ No. | Compound | MIC (MRSA) | MIC (P. *aeruginosa)* | MIC (A. *bumannii)* |
|---|---|---|---|---|
| CZ-115 | 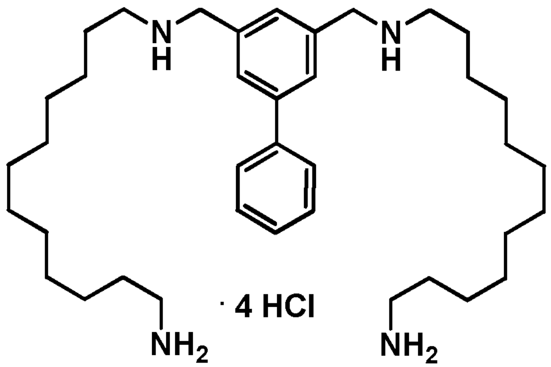 | +++ | +++ | |
| CZ-116 | 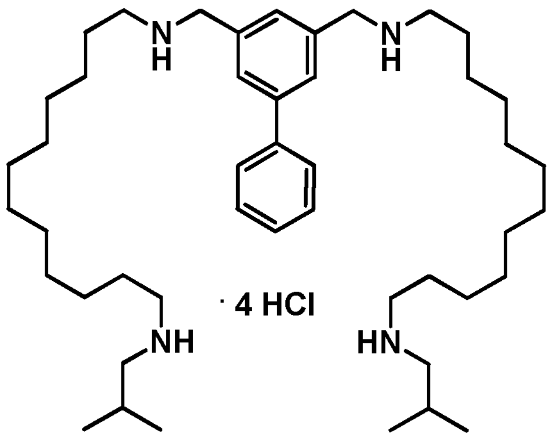 | +++ | +++ | |

(continued)

| CZ No. | Compound | MIC (MRSA) | MIC (P. *aeruginosa)* | MIC (A. *bumannii)* |
|---|---|---|---|---|
| CZ-117 | 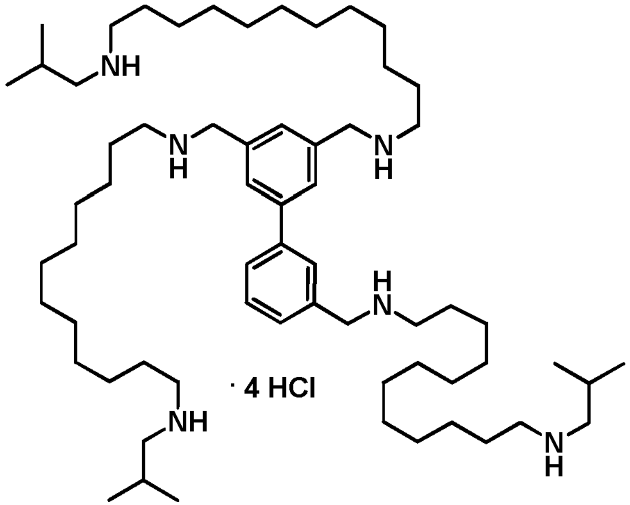 · 4 HCl | +++ | +++ | |
| CZ-118 | 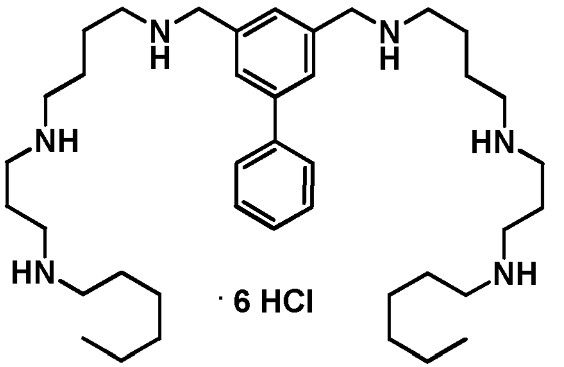 · 6 HCl | +++ | +++ | |

(continued)

| CZ No. | Compound | MIC (MRSA) | MIC (P. *aeruginosa)* | MIC (A. *bumannii)* |
|---|---|---|---|---|
| CZ-119 | 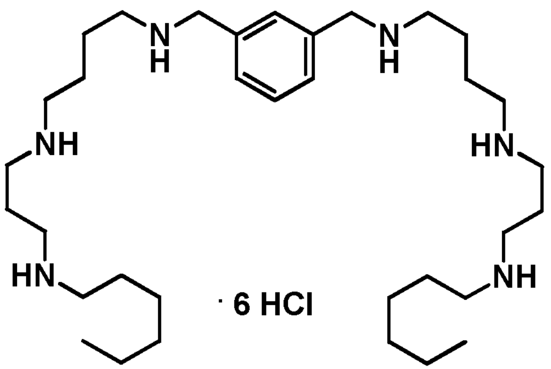 · 6 HCl | +++ | +++ | |

+ = MIC >100 μM.
++ = 100 μM ≥ MIC ≥ 50 μM. MIC results of ">n μM" or "<n μM" will also be designated as ++ if 100 ≥ n ≥ 50.
+++ = 50 μM > MIC. MIC results of ">n μM" or "<n μM" will also be designated as +++ if n < 50.

**Example 9**

***Biofilm Dispersion***

The compounds of the present invention such as CZ 25, CS-58 and CS-52 have excellent dispersal characteristics. To test for dispersion, biofilms of each isolate were grown on the surface of commercially pure titanium (Ti) coupons (1/2" diameter × 1/8" height) in a CDC biofilm reactor. The growth conditions for this reactor were the same as those for the membrane biofilm reactor. After biofilms were grown for 48 hours, each Ti coupon was aseptically removed and placed into 2 mL of cation adjusted Mueller Hinton broth (CAMHB) for 2 hours. The CAMHB had a final concentration of 0.25% (2.5 mg/mL) CZ-25 or CZ-52. Each compound was tested n=3 times.

Following the 2-hour exposure, each coupon was fixed in 0.25% glutaraldehyde for 24 hours, dehydrated using ascending concentrations of ethanol (70%, 95%, 100%) with 3x 10 min. changes and desiccated. One side of each coupon was carbon coated and imaged using a JEOL JSM-6610 SEM to directly observe the surface of the coupons and determine the ability of CZ compounds to disperse biofilms compared to positive controls that had no treatment. When compared to untreated controls, data have indicated that CZ-25, CZ-52 and CZ-58 have the ability to disperse biofilms from the surface of a material (FIG. 17) such that a monolayer of cells or reduced communities remain.

FIG. 17 provides SEM images of Ti coupons with MRSA biofilms exposed to CZ-25 (left), CZ-52 (middle) or water only (right). CZ-25 and CZ-52 demonstrated the ability to disperse the majority of biofilms such that a monolayer of cells remained on the surface of the Ti. Those samples treated with water only had biofilm communities that remained in all areas of the coupons.

**Example 10**

*Preparation of $N^1$,$N^{1'}$-(1,3-phenylenebis(methylene))bis($N^3$-(3-aminopropyl)propane-1,3-diamine), hydrochloride salt[Compound VII-2]*

The polyamine compound **(VII-2)** may be synthesized as shown in Scheme 7. Norspermidine was reacted with di-t-butyl dicarbonate compound to provide N-Boc protected norspermidine using the following procedure: To a solution of norspermidine (27.2 g, 207.7 mmol, 3.0 equiv.) in THF solvent (1000 mL) at 0°C was added di-*t*-butyl dicarbonate (18.1 g, 83.1 mmol, 1 equiv.) dropwise over a period of two hours. During the period of addition, the solution went from clear to a cloudy white. Following addition, the reaction mixture was allowed to stir at room temperature for a period of 12 hours. The THF solvent was evaporated, and the residue was dissolved in $CH_2Cl_2$ (250 mL) and washed with water (250 mL). The resultant aqueous layer was washed with $CH_2Cl_2$ (4 × 100 mL). The organic layer was dried with $Na_2SO_4$ and evaporated to afford the Boc-protected amine as a clear viscous liquid.

## Scheme 7

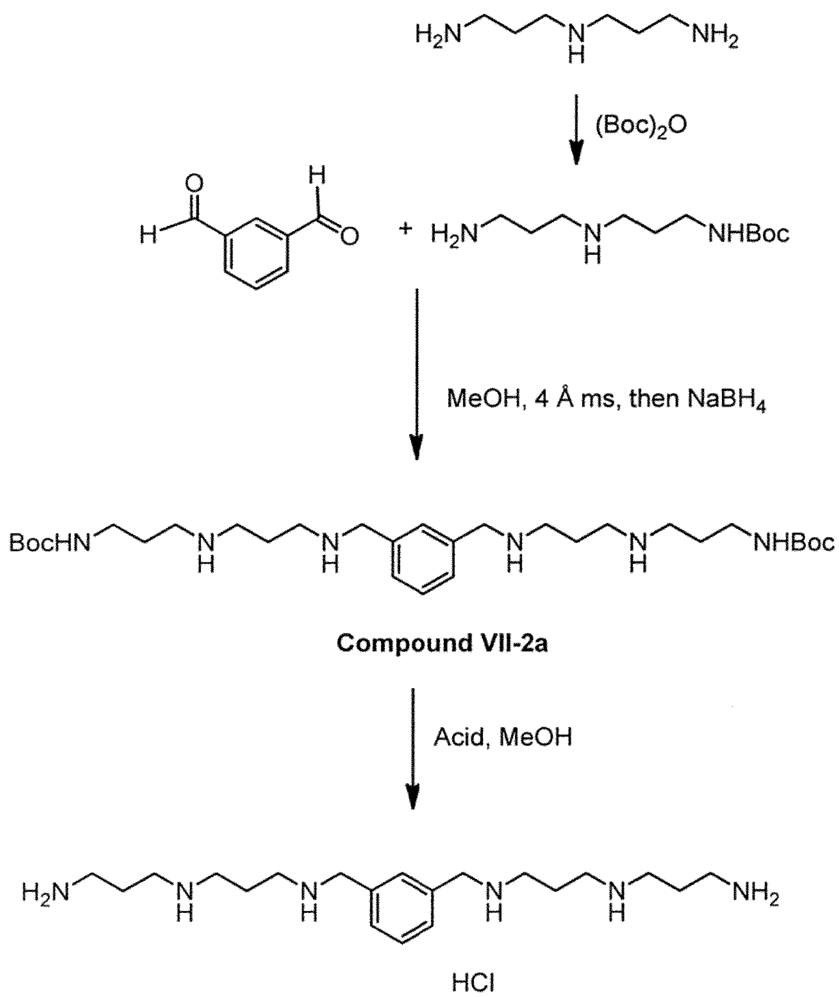


Compound VII-2a

HCl

Compound VII-2

Benzene-1,3-dicarboxyaldehyde was reacted with at least two equivalents of the N-Boc protected norspermidine in the presence of molecular sieve and methanol solvent to provide a corresponding polyamide product, which was then reduced by sodium borohydride ($NaBH_4$) to produce the N-Boc protected polyamine compound **VII-2a** using following procedure: To a stirring solution of 4 Å molecular sieve (100 mg) in MeOH solvent (25 mL) was added benzene-1,3-dicarboxyaldehyde (0.78 g, 5.87 mmol, 1 equiv.) followed by the Boc-protected amine (2.71 g, 11.74 mmol, 2 equiv.). The solution was stirred for 12 hours at room temperature. The newly formed imine was quenched at room temperature by the addition of $NaBH_4$ (0.89 g, 23.48 mmol, 4 equiv.) after which the solution was stirred for an additional one hour. The solution was filtered through a pad of celite, and the solvent evaporated *in vacuo* to afford brown oil. The crude mixture was dissolved in EtOAc (100 mL) and washed with 10% NaOH (100 mL): The resulting NaOH was further washed with EtOAc (50 mL $\times$ 1). The organic layer was dried with $Na_2SO_4$ and concentrated under reduced pressure to provide a crude Boc-protected product.

The Boc-protecting group on the terminal amine of the polyamine compound **VII-2a** was deprotected by subjecting compound **VII-2a** to acid hydrolysis to produce the polyamine compound (**VII-2**) using the following procedure: The crude Boc-protected product was added to a stirring solution of HCl in MeOH (100 mL) and left for one hours. The solvent was evaporated *in vacuo* to yield a white to off-white solid which was recrystallized from MeOH to provide the polyamine compound (**VII-2**) as white solid to off-white solid (30% yield). $^1H$ NMR (500 MHz, $D_2O$): 7.60 (s, 4H), 4.33 (s, 4H), 3.26-3.18 (m, 12H), 3.12 (t, *J*=8Hz, 4H), 2.21-2.08 (m, 8H).

**Example 11**

*Preparation of $N^1$,$N^{1'}$-(((1, 3-phenylenebis(methylene))bis(azanediyl))bis(propane-3, 1-diyl))bis($N^3$,$N^3$-dimethylpropane-1, 3-diamine), hydrochloride salt [Compound VII-2C]*

The polyamine compound **(VII-2C)** may be synthesized as shown in Scheme 8. Benzene-1,3-dicarboxyaldehyde was reacted with at least two equivalents of N, N-dimethylnorspermidine in the presence of molecular sieve and methanol solvent to provide a corresponding polyamide product, which was then reduced by sodium borohydride ($NaBH_4$) to produce the polyamine compound **VII-2B** using the following procedure: To a stirring solution of 4 Å molecular sieve (100 mg) in MeOH solvent (25 mL) was added benzene-1,3-dicarboxyaldehyde (0.78 g, 5.87 mmol, 1 equiv.) followed by N, N-dimethylnorspermidine (2.71 g, 11.74 mmol, 2 equiv). The solution was stirred for 12 hours at room temperature. The newly formed imine was quenched at room temperature by the addition of $NaBH_4$ (0.89 g, 23.48 mmol, 4 equiv.) after which the solution was stirred for an additional one hour. The solution was filtered through a pad of celite, and the solvent evaporated *in vacuo* to afford brown oil. The crude mixture was dissolved in EtOAc (100 mL) and washed with 10% NaOH (100 mL). The resulting NaOH solution was further washed with EtOAc (50 mL × 1). The organic layer was dried with $Na_2SO_4$ and concentrated under reduced pressure to provide the polyamine compound **VII-2B.**

**Scheme 8**

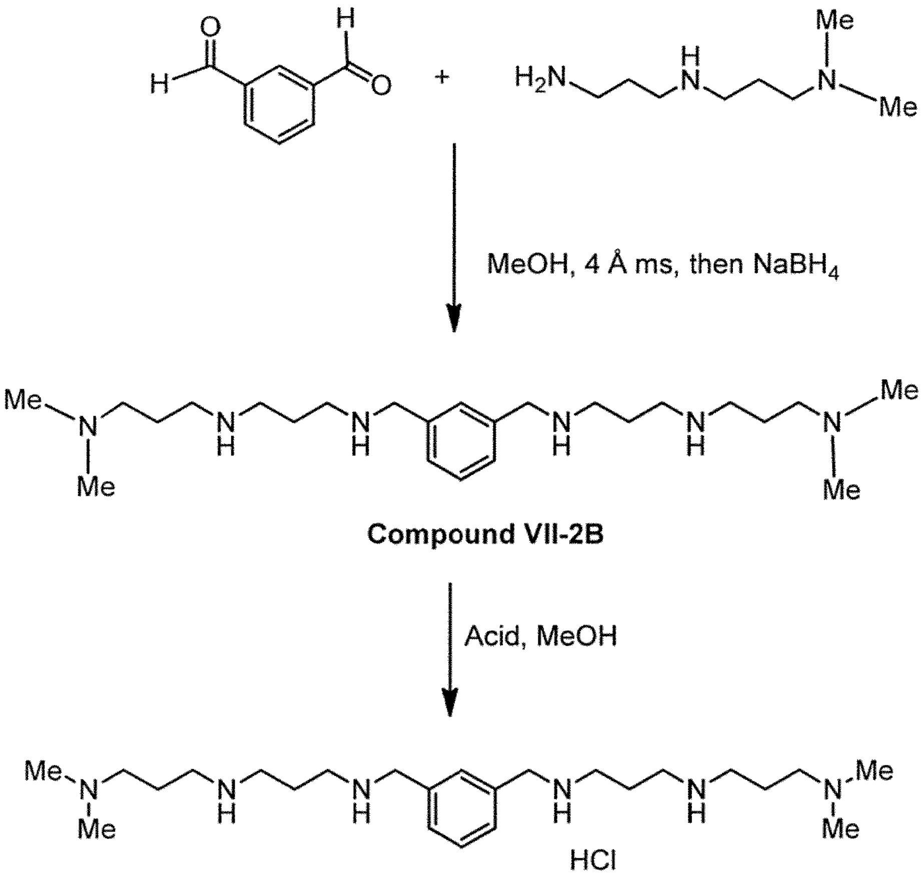


**Compound VII-2B**

**Compound VII-2C**

The crude the polyamine compound **VII-2B** was added to a stirring solution of HCl in MeOH solvent (100 mL) and left for one hour. The solvent was evaporated *in vacuo* to yield a white to off-white solid, which was recrystallized from MeOH to afford the polyamine compound **VII-2C** at about 30% yield as white solid to off-white solid. $^1H$ NMR (500 MHz, $D_2O$): 7.59 (s, 4H), 4.24 (s, 4H), 3.29-3.15 (m, 16H), 2.91 (s, 12H), 2.21-2.15 (m, 8H).

**Example 12**

*Preparation of N-benzyl-1,12-dodecyldiamine (V-1)*

The polyamine compound ***(V-1)*** may be synthesized as shown in Scheme 9 from 1,12-dodecyldiamine, either via synthetic route **[A]** or **[B].**

**Scheme 9**

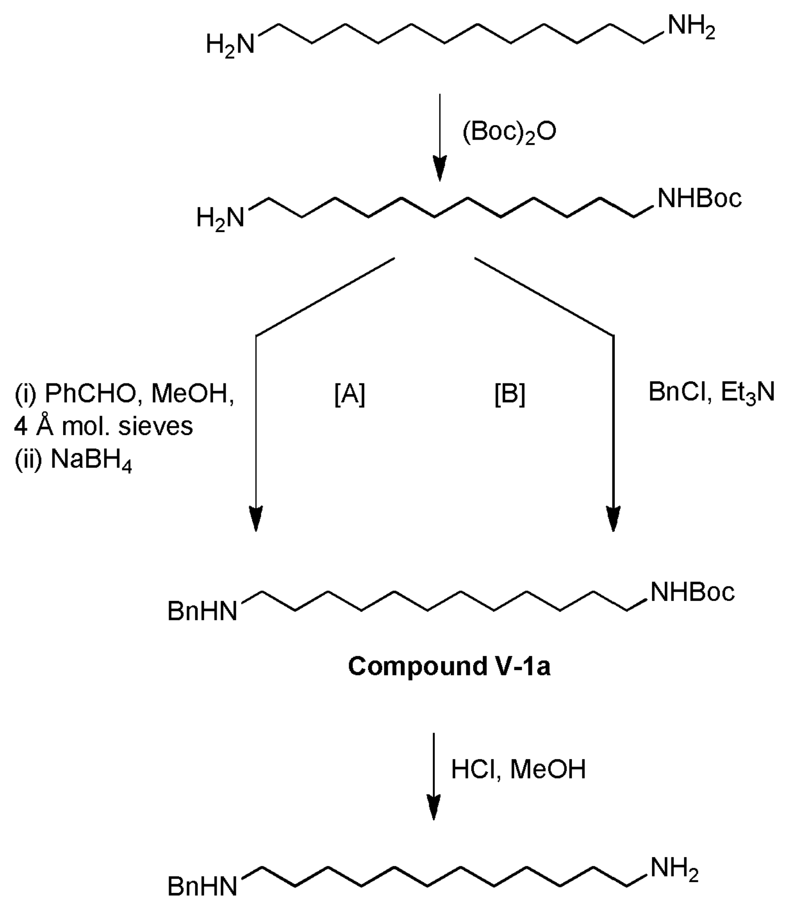


**Compound V-1a**

**Compound V-1**

In the synthetic route **[A],** N-Boc protected-1,12-dodecyldiamine was reacted with benzyl chloride in the presence of base and solvent to provide a corresponding polyamide compound **V-1a.** In the synthetic route **[B],** N-Boc protected-1,12-dodecyldiamine, prepared as described in Example 10 as following: To a solution of amine (27.2 g, 207.7 mmol, 3.0 equiv.) in THF solvent (1000 mL) at 0°C was added di-tert-butyl dicarbonate (18.1 g, 83.1 mmol, 1 equiv.) dropwise over a period of two hours. During the period of addition, the solution went from clear to a cloudy white. Following addition, the reaction mixture was allowed to stir at room temperature for a period of 12 hours. The THF solvent was evaporated, and the residue was dissolved in $CH_2Cl_2$ (250 mL) and washed with water (250 mL). The resultant aqueous layer was washed with $CH_2Cl_2$ (4 × 100 mL). The organic layer was dried with $Na_2SO_4$ and evaporated to afford the N-Boc protected-1,12-dodecyldiamine amine as a clear viscous liquid.

The N-Boc protected-1,12-dodecyldiamine was reacted with benzaldehyde in the presence of molecular sieve and methanol solvent to provide a corresponding polyamide product, which was then reduced by sodium borohydride ($NaBH_4$) to produce the N-Boc protected polyamine compound **V-1a** using the following procedure: To a stirring solution of 4 Å molecular sieve (100 mg) in MeOH solvent (25 mL) was added the benzaldehyde (0.78 g, 5.87 mmol, 1 equiv.) followed by the N-Boc protected- 1, 12-dodecyldiamine (1.36 g, 5.87 mmol, 1 equiv.). The solution was stirred for 12 hours at room temperature. The newly formed imine was quenched at room temperature by the addition of $NaBH_4$ (0.89 g, 23.48 mmol, 4 equiv.) after which the solution was stirred for an additional one hour. The solution was filtered through a pad of celite, and the solvent evaporated *in vacuo* to afford brown oil. The crude mixture was dissolved in EtOAc (100

mL) and washed with 10% NaOH (100 mL). The resulting NaOH solution was further washed with EtOAc (50 mL x 1). The organic layer was dried with $Na_2SO_4$ and concentrated under reduced pressure to provide the N-Boc protected polyamine compound **V-1a.**

The Boc protecting group on the terminal amine of the polyamine compound **V-Ia** was deprotected by treating compound **V-Ia** with acid to produce the polyamine compound (**V-1**) using following procedure: The crude N-Boc protected polyamine compound **V-Ia** was added to a stirring solution of HCl in MeOH solvent (100 mL) and left for one hour. The solvent was evaporated in vacuo to yield a white to off-white solid, which was recrystallized from MeOH to afford the polyamine compound (**V-1**) at about 30% yield as a white solid to off-white solid. $^1H$ NMR (500 MHz, $D_2O$): 7.42 (s, 5H), 4.15 (s, 2H), 3.26 (p, *J* = 1.5 Hz, 2H), 2.96-2.89 (m, 4H), 1.63-1.56 (m, 4H), 1.28-1.21 (m, 14H).

**Example 13**

*Preparation of 1,3, 5-tris-[N-(3-aminopropyl)-methylamine]-benzene (VI-1)*

The polyamine compound **(VI-1)** may be synthesized as shown in Scheme 10.

**Scheme 10**

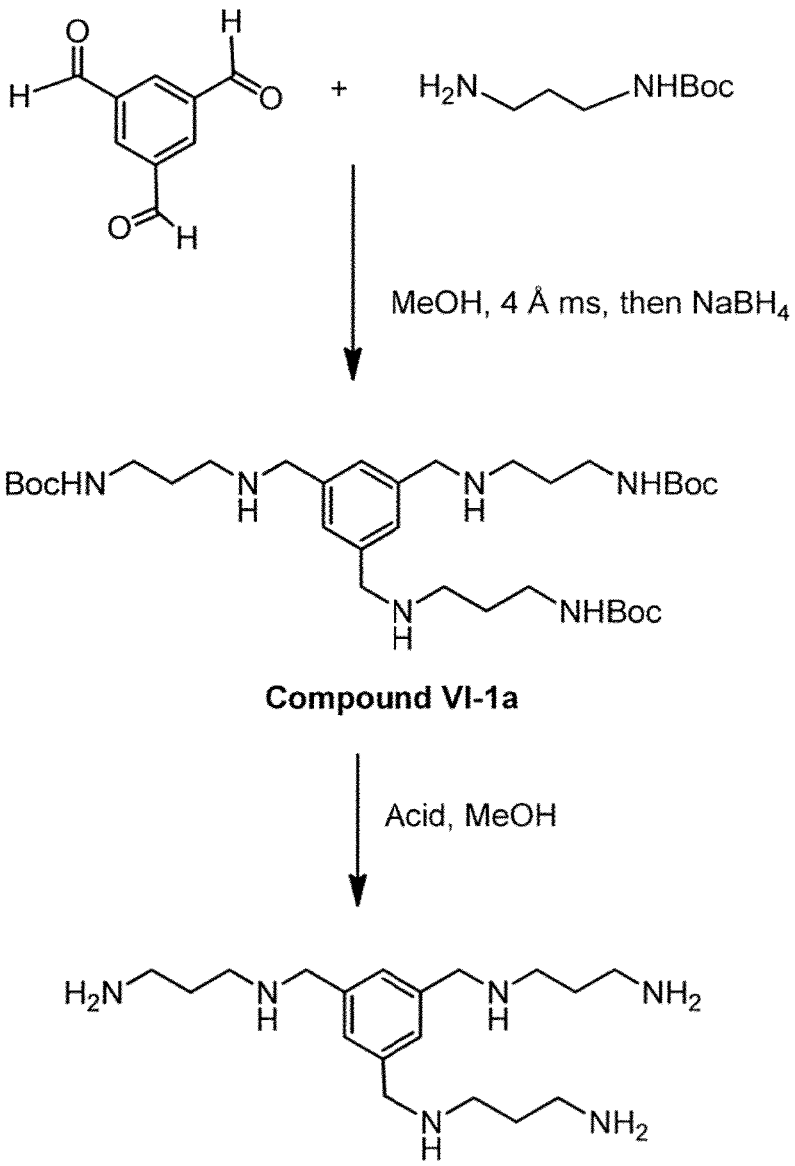


**Compound VI-1a**

**Compound VI-1**

1,3-Diaminopropane was reacted with di-t-butyldicarbonate compound to provide N-Boc-1,3-diaminopropane using following procedure: To a solution of amine (27.2 g, 207.7 mmol, 3.0 equiv.) in THF solvent (1000 mL) at 0°C was added di-tert-butyl dicarbonate (18.1 g, 83.1 mmol, 1 equiv.) dropwise over a period of two hours. During the period of addition, the solution went from clear to a cloudy white. Following addition, the reaction mixture was allowed to stir at room temperature for a period of 12 hours. The THF solvent was evaporated, and the residue was dissolved in $CH_2Cl_2$ (250 mL) and washed with water (250 mL). The resultant aqueous layer was backwashed with $CH_2Cl_2$ (4 × 100 mL). The organic layer was dried with $Na_2SO_4$ and evaporated to afford the N-Boc-1,3-diaminopropane as a clear viscous liquid.

Benzene-1,3,5-tricarboxyaldehyde was reacted with at least three equivalents of three equivalents of the N-Boc protected 1,3-diaminopropane in the presence of molecular sieve and methanol solvent to provide a corresponding polyamide product, which was then reduced by sodium borohydride ($NaBH_4$) to produce the N-Boc protected polyamine compound **VI-1a** using following procedure: To a stirring solution of 4 Å molecular seive (100 mg) in MeOH solvent (20 mL) was added benzene-1,3,5-tricarboxyaldehyde (64 mg, 0.39 mmol, 1 equiv.) followed by N -Boc protected 1,3-diaminopropane (222 mg, 1.19 mmol, 3 equiv.). The solution was stirred for 12 hours at room temperature. The newly formed imine was quenched at room temperature by the addition of $NaBH_4$ (90 mg, 2.38 mmol, 6 equiv.) after which the solution was stirred for an additional one hour. The solution was filtered through a pad of celite, and the solvent evaporated *in vacuo* to afford brown oil. The crude mixture was dissolved in EtOAc (100 mL) and washed with 10% NaOH (100 mL). The resulting NaOH was further backwashed with EtOAc (50 mL × 1). The organic layer was dried with $Na_2SO_4$ and concentrated under reduced pressure to provide the N-Boc protected polyamine compound **VI-1a.**

The Boc protecting group on the terminal amine of the polyamine compound **VI-1a** was deprotected by treating compound **VI-1a** with acid to produce the polyamine compound (VI-1) using following procedure: The crude polyamine compound **VI-1a** was added to a stirring solution of HCl in MeOH solvent (100 mL) and left for one hour. The solvent was evaporated in vacuo to yield a white to off-white solid, which was recrystallized from MeOH solvent to afford the polyamine compound **VI-1** at about 30% yield as white solid to off-white solid.

**Example 14**

*Preparation of $N^1$,$N^{1'}$,$N^{1''}$-(benzene-1,3,5-triyltris(methylene))tris($N^3$-isopropylpropane-1,3-diamine) (HCl) (VII-4C)*

The polyamine compound (**VII-4C**) may be synthesized as shown in Scheme 11.

**Scheme 11**

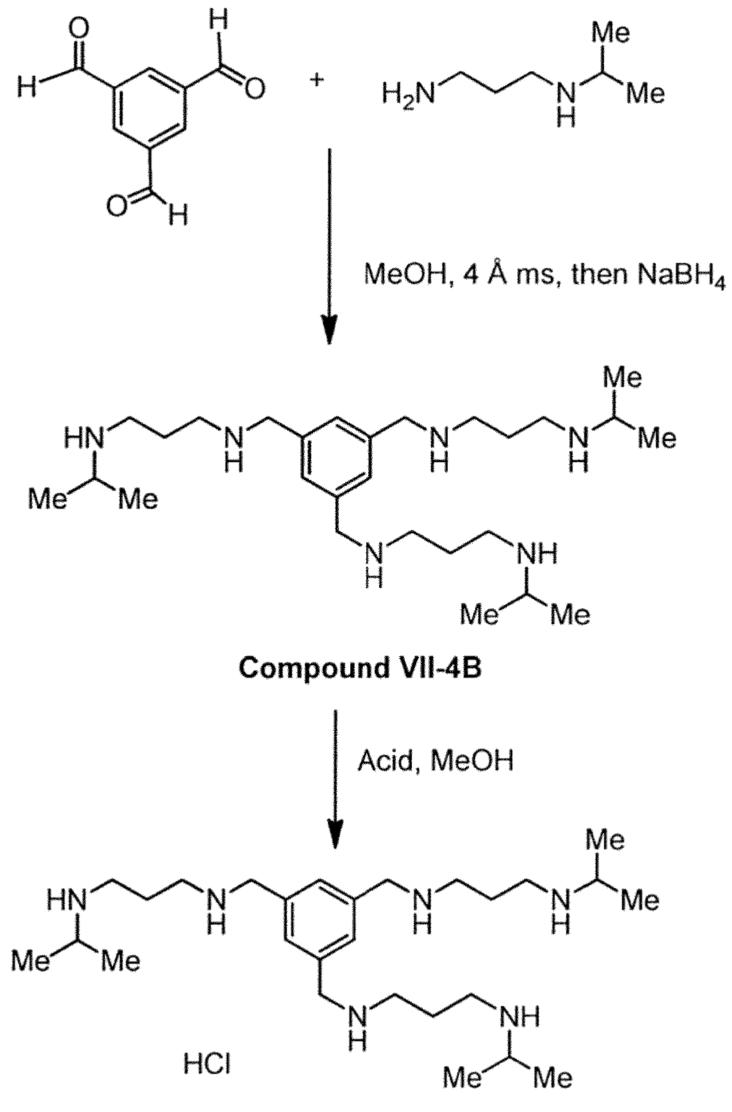


**Compound VII-4B**

**Compound VII-4C**

Benzene-1,3,5-tricarboxyaldehyde was reacted with at least three equivalents of N,N-dimethylnorspermidine in the presence of molecular sieve and methanol solvent to provide a corresponding polyamide product, which was then reduced by sodium borohydride ($NaBH_4$) to produce the N-Boc protected polyamine compound **VII-4B** using the following procedure: To a stirring solution of 4 Å molecular sieve (100 mg) in MeOH solvent (20 mL) was added benzene-1,3,5-tricarboxyaldehyde (64 mg, 0.39 mmol, 1 equiv.) followed by N, N-dimethylnorspermidine (222 mg, 1.19 mmol, 3 equiv.). The solution was stirred for 12 hours at room temperature. The newly formed imine was quenched at room temperature by the addition of $NaBH_4$ (90 mg, 2.38 mmol, 6 equiv.) after which the solution was stirred for an additional one hour. The solution was filtered through a pad of celite, and the solvent evaporated *in vacuo* to afford brown oil. The crude mixture was dissolved in EtOAc (100 mL) and washed with 10% NaOH (100 mL). The resulting NaOH was further backwashed with EtOAc (50 mL $\times$ 1). The organic layer was dried with $Na_2SO_4$ and concentrated under reduced pressure to provide the crude N-Boc protected polyamine compound **VII-4B.**

The polyamine compound **VII-4B** was added to a stirring solution of HCl in MeOH solvent (100 mL) and left for one hour. The solvent was evaporated *in vacuo* to afford the polyamine compound **VII-4C** as viscous liquid at about 30% yield. $^1$H NMR (500 MHz, $D_2O$): 7.68 (s, 3H), 4.35 (s, 6H), 3.41 (p, *J* = 6.5 Hz, 3H), 3.23 (t, *J* = 7.5 Hz, 6H), 3.14-3.07 (m, 6H), 2.14-2.11 (m, 6H), 1.30 (d, *J* = 6.5 Hz, 18H).

**Example 15**

*Preparation of 1,3-bis-[N-(N'-3-aminopropyl)propyl-3-amine)-methylamine]-benzene (VII-2)*

The polyamine compound **(VII-2)** may be synthesized as shown in Scheme 12. N-(3-Aminopropyl)propane-1,3-diamine (i.e., norspermidine) was reacted with di-*t*-butyldicarbonate compound to provide a N-Boc protected norspermidine compound. Benzene-1,3,5-tricarboxyaldehyde was reacted with at least three equivalents of three equivalents of the N-Boc protected norspermidine compound in the presence of molecular sieve and methanol solvent to provide a corresponding polyamide product, which was then reduced by sodium borohydride ($NaBH_4$) to produce the N-Boc protected polyamine compound **VII-2a.** using the following procedure: To a stirring solution of 4 Å molecular sieve (100 mg) in MeOH solvent (20 mL) was added benzene-1,3,5-tricarboxyaldehyde (64 mg, 0.39 mmol, 1 equiv.) followed by the N-Boc protected norspermidine compound (222 mg, 1.19 mmol, 3 equiv.). The solution was stirred for 12 hours at room temperature. The newly formed imine was quenched at room temperature by the addition of $NaBH_4$ (90 mg, 2.38 mmol, 6 equiv.) after which the solution was stirred for an additional one hour. The solution was filtered through a pad of celite, and the solvent evaporated *in vacuo* to afford brown oil. The crude mixture was dissolved in EtOAc (100 mL) and washed with 10% NaOH (100 mL). The resulting NaOH was further backwashed with EtOAc (50 mL × 1). The organic layer was dried with $Na_2SO_4$ and concentrated under reduced pressure to provide the crude N-Boc protected polyamine compound **VII-2a.**

**Scheme 12**

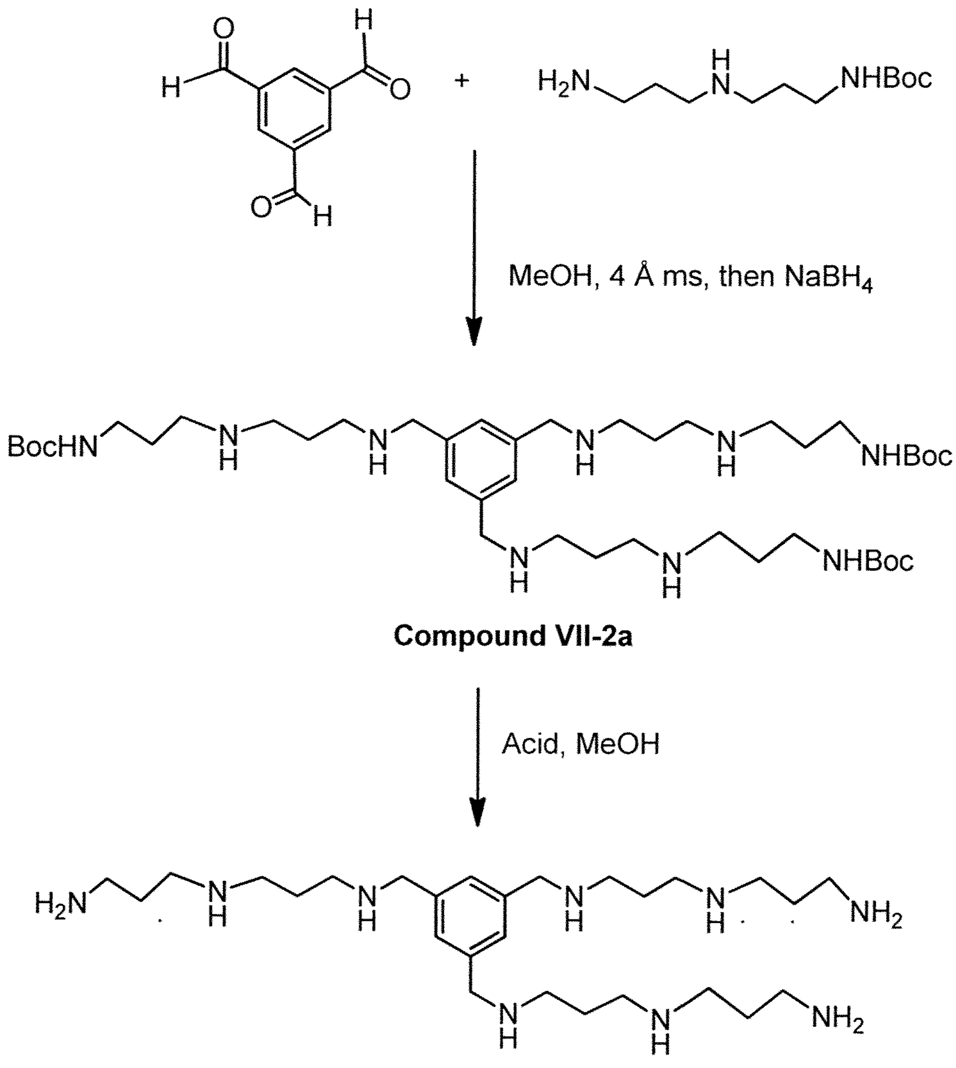


Compound VII-2a

**Compound VII-2**

The Boc protecting group on the terminal amine of the polyamine compound **VII-2a** was deprotected by treating compound **VII-2a** with acid to produce the polyamine compound **(VII-2)** using following procedure: The crude polyamine compound **VII-2a** was added to a stirring solution of HCl in MeOH solvent (100 mL) and left for one hour. The solvent was evaporated *in vacuo* to yield a white to off-white solid, which was recrystallized from MeOH to afford the polyamine compound **VII-2** as white solid to off-white solid at 30% yield.

**Example 16**

*Preparation of 1,3-bis-[N-3-aminopropyl]benzenediamide (VIII-3)*

The polyamine compound (**VIII-3**) may be synthesized as shown in Scheme 13.

**Scheme 13**

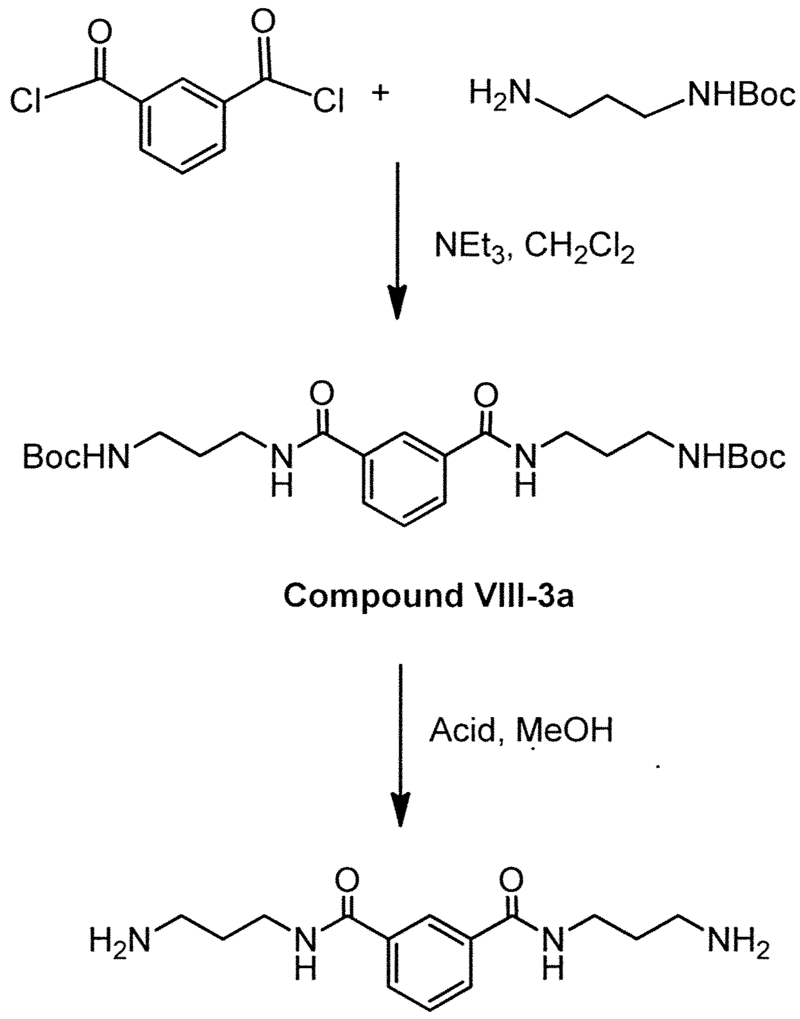


**Compound VIII-3a**

**Compound VIII-3**

Benzene-1,3-dicarbonylchloride was reacted with at least two equivalents of the N-Boc protected 1,3-diaminopropane (prepared as described in Example 4) in the presence of triethylamine ($NEt_3$) and dichloromethane solvent to provide a corresponding N-Boc protected polyamide compound **VIII-3a.** The Boc protecting group on the terminal amine of the polyamine compound **VIII-3a** was deprotected by treating compound **VIII-3a** with acid, such as acetyl chloride, in methanol to produce the polyamine compound **(VIII-3).**

**Example 17**

*General method for preparation of dialdehyde aryl starting material*

An aldehyde precursor to the polyamine compound may be produced by the palladium coupling method of General Synthetic Scheme 14.

**General Synthetic Scheme 14**

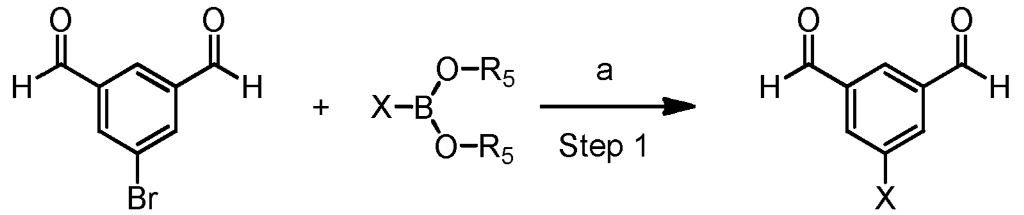


X = aryl, alkyl, vinyl, heteroaryl
Reagents: (a) 5:1 DME/$H_2O$, $Pd(PPh_3)_4$, $K_2CO_3$

Specific examples of this method are set forth in the following Examples, such as Example 68.

**Example 18**

*Preparation of $N^1$,$N^{1'}$-(1,3-phenylenebis(methylene))bis(N3-(3-ammoniopropyl)propane-1,3-diaminium) chloride* (CZ-25)

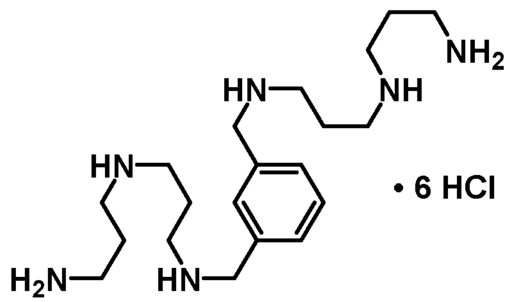

Step 1: *Di-tert-butyl (((((1,3-phenylenebis(methylene))bis(azanediyl))bis(propane-3,1-diyl))bis(azanediyl))bis(propane-3,1-diyl))dicarbamate.* Isophthalaldehyde (1.07 g, 7.99 mmol) and MeOH (50 mL) were added to a round-bottom flask. To the solution was added tert-butyl (3-((3-aminopropyl)amino)propyl)carbamate (3.69 g, 16.0 mmol) and the reaction mixture was stirred for 24 h. Sodium borohydride (1.21 g, 32.0 mmol) was added portionwise and stirred for 1 h. The reaction mixture was concentrated under reduced pressure, and aq. NaOH (10%, 150 mL) and EtOAc (150 mL) were added. The layers were separated, and the aqueous layer was extracted with EtOAc (150 mL). The organic layers were combined, dried over $Na_2SO_4$, filtered, and concentrated under reduced pressure to afford the desired product as a clear oil. The intermediate was carried forward without further purification.

Step 2: *$N^1$,$N^{1'}$-(1,3-Phenylenebis(methylene))bis($N^3$-(3-aminopropyljpropane-1,3-diamine):* To the crude di-*tert*-butyl (((((1,3-phenylenebis(methylene))bis(azanediyl))-bis(propane-3,1-diyl))bis(azanediyl))bis(propane-3,1-diyl))dicarbamate from Step 1 was added methanolic HCl (150 mL, 1.0 M). The reaction mixture was stirred for 2 h and concentrated under reduced pressure. The solid was collected by vacuum filtration, and it was washed with $Et_2O$ (30 mL) and hot MeOH (30 mL) to afford the desired product (1.1 g, 56%) as a white solid. $^1H$ NMR (500 MHz, $D_2O$) δ ppm 7.62 (s, 4H), 4.36 (s, 4H), 3.28-3.21 (m, 12H), 3.15 (t, $J$ = 8.0 Hz, 4H), 2.23-2.11 (m, 8H). $^{13}C$ NMR (125 MHz, $D_2O$) 131.4, 131.3, 131.1, 130.2, 50.8, 44.7, 44.6, 44.1, 36.5, 23.6, 22.6. LRMS $[M+H]^+$ 365.3.

**Example 19**

*Preparation of $N^1$-benzylbutane-1,4-diaminium chloride* (CZ-3)

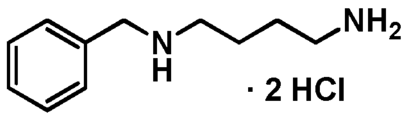

Example 19 was prepared in a similar fashion to Example 18 (CZ-25) from benzaldehyde and tert-butyl (4-aminobutyl)carbamate. $^1$H NMR (500 MHz, $D_2O$) δ 7.52 (s, 5H), 4.26 (s, 2H), 3.15 (t, *J*= 8 Hz, 2H), 3.07 (t, *J*= 7.5 Hz, 2H), 1.86-1.74 (m, 4H). $^{13}$C NMR (125 MHz, $D_2O$) 130.6, 129.9, 129.7, 129.2, 51.0, 46.3, 38.8, 23.9, 22.7. LRMS $[M+H]^+$ 179.2.

**Example 20**

*Preparation of $N^1$-benzylpropane-1,3-diamine* (CZ-4)

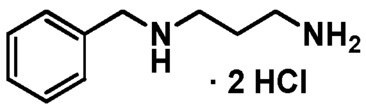

Example 20 was prepared in a similar fashion to Example 18 (CZ-25) from benzaldehyde and tert-butyl (3-aminopropyl)carbamate. $^1$H NMR (500 MHz, $D_2O$) δ 7.49 (quint, *J* = 3 Hz, 5H), 4.26 (s, 2H), 3.17 (t, J = 8 Hz, 2H), 3.07 (t, J = 8 Hz, 2H), 2.09 (quint, *J* = 7.5 Hz, 2H). $^{13}$C NMR (125 MHz, $D_2O$) δ 130.6, 129.9, 129.8, 129.4, 51.4, 44.1, 36.7, 23.9. LRMS $[M+H]^+$ 291.3.

**Example 21**

*Preparation of $N^1$-benzyldodecane-1,12-diamine, hydrochloride salt* (CZ-5)

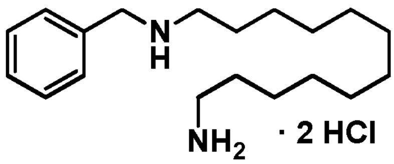

Example 21 was prepared in a similar fashion to Example 18 (CZ-25) from benzaldehyde and *tert*-butyl (12-aminododecyl)carbamate.

**Example 22**

*Preparation of $N^1$-(3-(benzylamino)propyl)-$N^3$,$N^3$-dimethylpropane-1,3-diamine, hydrochloride salt* (CZ-6)

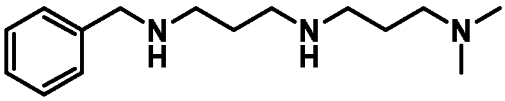

• 3 HCl

Example 22 was prepared in a similar fashion to Example 18 (CZ-25) from benzaldehyde and *N*[1]-(3-aminopropyl)-$N^3$,$N^3$-dimethylpropane-1,3-diamine. $^1$H NMR (500 MHz, $D_2O$) δ 7.41 (s, 5H), 4.18 (s, 2H), 3.20-3.08 (m, 8 H), 2.83 (s, 6H), 2.15-2.03 (m, 4H). $^{13}$C NMR (125 MHz, $D_2O$) 131.0, 130.5, 130.4, 129.9, 54.8, 51.8, 45.3, 45.1, 44.5, 43.5, 23.2, 21.8.

**Example 23**

*Preparation of $N^1$-(3-aminopropyl)-$N^3$-benzylpropane-1,3-diamine, hydrochloride salt* (CZ-7)

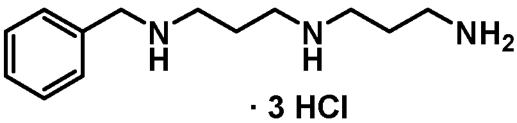

· 3 HCl

Example 23 was prepared in a similar fashion to Example 18 (CZ-25) from benzaldehyde and *tert*-butyl (3-((3-aminopropyl)amino)propyl)carbamate. $^1$H NMR (500 MHz, $D_2O$) δ 7.49 (s, 5H), 4.25 (s, 2H), 3.20-3.15 (m, 6H), 3.11 (t, J = 8 Hz, 2H), 2.17-2.07 (m, 4H). $^{13}$C NMR (125 MHz, $D_2O$) δ 130.3, 129.8, 129.7, 129.2, 51.2, 44.6, 44.6, 43.8, 36.5, 23.7, 22.6. LRMS $[M+H]^+$ 222.2.

**Example 24**

*Preparation of $N^1$-benzylhexane-1,6-diamine, hydrochloride salt* (CZ-9)

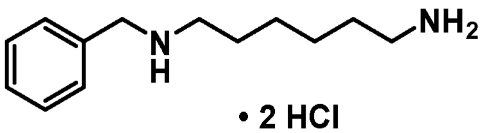

• 2 HCl

Example 24 was prepared in a similar fashion to Example 18 (CZ-25) from benzaldehyde and *tert*-butyl (6-aminohexyl)carbamate.

**Example 25**

*Preparation of $N^1$-Benzyl-N,$N^{12}$,$N^{12}$-trimethyldodecane-1,12-diamine, hydrochloride salt* (CZ-10)

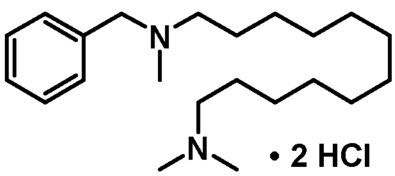

• 2 HCl

Example 25 was prepared in a similar fashion to Example 18 (CZ-25) from benzaldehyde and $N^1$,$N^1$,$N^{12}$-trimethyldodecane-1,12-diamine.

**Example 26**

*Preparation of $N^1$-benzyl-$N^1$,$N^{12}$,$N^{12}$-trimethyldodecane-1,12-diamine, hydrochloride salt* (CZ-11)

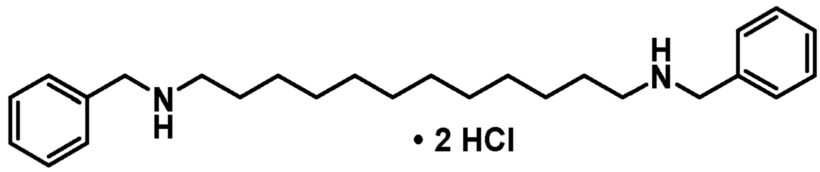

Example 26 was prepared in a similar fashion to Example 18 (CZ-25) from benzaldehyde and dodecane-1,12-diamine. $^1$H NMR (500 MHz, $D_2O$ + $CD_3OD$) δ 7.53 (s, 10H), 4.24 (s, 4H), 3.04 (t, J = 8.5 Hz, 4H), 1.77-1.70 (m, 4H), 1.42-1.28 (m, 16 H). $^{13}$C NMR (125 MHz, $D_2O$ + $CD_3OD$) δ 131.0, 129.8, 129.6, 129.3, 50.9, 47.0, 29.2, 29.1, 28.7, 26.2, 25.7.

**Example 27**

*Preparation of $N^1$-benzyl-$N^1$,$N^{12}$,$N^{12}$-trimethyldodecane-1,12-diamine, hydrochloride salt* (CZ-12)

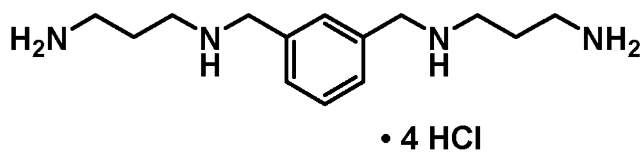

Example 27 was prepared in a similar fashion to Example 18 (CZ-25) from isophthalaldehyde and tert-butyl (3-aminopropyl)carbamate.

**Example** 28

*Preparation of $N^1$-(3-methoxybenzyl)dodecane-1,12-diamine, hydrochloride salt* (CZ-13)

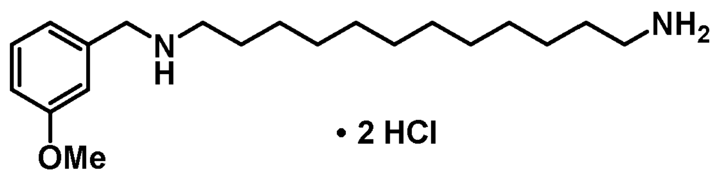

Example 28 was prepared in a similar fashion to Example 18 (CZ-25) from 3-methoxybenzaldehyde and *tert*-butyl (12-aminododecyl)carbamate. $^1$H NMR (500 MHz, $D_2O$) δ 7.40 (t, *J*= 8.0 Hz, 1H), 7.06 (s, 1H), 7.05 (s, 1H), 7.03 (s, 1H), 4.16 (s, 2H), 3.82 (s, 3H), 3.00-2.93 (m, 4H). 1.66-1.58 (m, 4H), 1.32-1.22 (m, 16H). $^{13}$C NMR (125 MHz, $D_2O$) δ 158.4, 131.5, 129.8, 121.6, 114.5, 114.3, 54.7, 49.8, 46.1, 38.7, 27.8, 27.7, 27.4, 25.9, 24.8, 24.5.

**Example 29**

*Preparation of $N^1$-(3-aminopropyl)-$N^3$-(3-(benzylamino)propyl)propane-1,3-diamine, hydrochloride salt* (CZ-16)

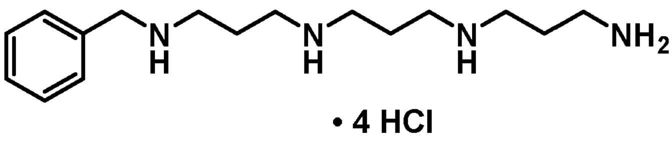

Example 29 was prepared in a similar fashion to Example 18 (CZ-25) from benzaldehyde and tert-butyl (3-((3-((3-aminopropyl)amino)propyl)amino)propyl)carbamate. $^1$H NMR (500 MHz, $D_2O$) δ 7.49 (s, 5H), 4.27 (s, 2H), 3.51-3.49 (m, 6H), 3.27-3.14 (m, 6H), 3.12 (t, $J$ = 8.0 Hz, 2H), 2.21-2.10 (m, 4H). $^{13}$C NMR (125 MHz, $D_2O$) δ 130.5, 130.0, 130.0, 129.5, 51.4, 45.3, 45.2, 43.9, 43.2, 36.6, 27.8, 23.9, 22.8.

**Example 30**

*Preparation of N$^1$,N$^1$-((2,4,6-trimethyl-1,3-phenylene)bis(methylene))bis(propane-1,3-diaminium) chloride* (CZ-19)

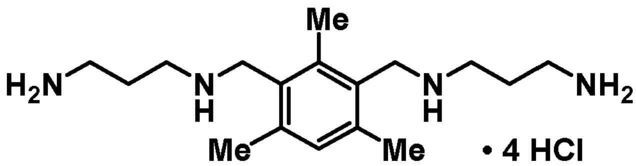

Example 30 was prepared in a similar fashion to Example 18 (CZ-25) from 2,4-bis(bromomethyl)-1,3,5-trimethylbenzene *tert*-butyl (3-aminopropyl)carbamate.

**Example 31**

*Preparation of N$^1$,N$^{1'}$-(1,3-phenylenebis(methylene))bis(dodecane-1,12-diamine), hydrochloride salt* (CZ-21)

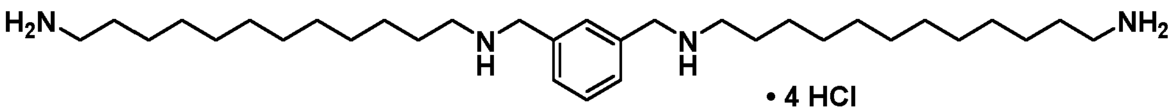

Example 31 was prepared in a similar fashion to Example 18 (CZ-25) from isophthalaldehyde and *tert*-butyl (12-aminododecyl)carbamate. $^1$H NMR (500 MHz, $D_2O$) δ 7.65 (s, 3H), 4.36 (s, 4H), 3.11 (t, $J$= 8.0 Hz, 4H), 3.06 (t, $J$= 7.5 Hz, 4H), 1.77-1.68 (m, 8H), 1.47-1.33 (m, 32H). $^{13}$C NMR (125 MHz, $D_2O$) δ 130.4, 129.9, 129.7, 128.9, 49.0, 45.6, 38.2, 27.3, 27.3, 27.2, 27.1, 26.9, 26.8, 25.4, 24.4, 24.3, 24.0. LRMS $[M+H]^+$ 503.5.

**Example 32**

*Preparation of (3-(((3-((3-aminopropyl)amino)propyl)amino)methyl)phenyl)methanol, hydrochloride salt* (CZ-22)

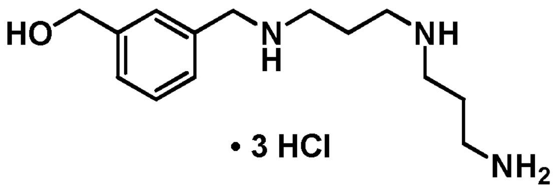

Example 32 was prepared in a similar fashion to Example 18 (CZ-25) from isophthalaldehyde and $N^1$,$N^1$-dimethylpropane-1,3-diamine.

**Example 33**

*Preparation of $N^1$,$N^{1'}$-(1,3-phenylenebis(methylene))bis(hexane-1,6-diamine), hydrochloride salt* (CZ-26)

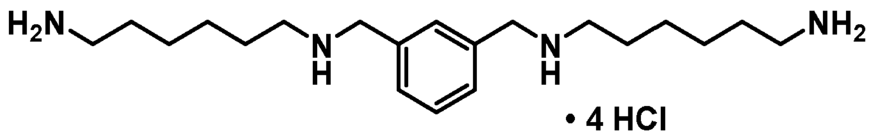

Example 33 was prepared in a similar fashion to Example 18 (CZ-25) from isophthalaldehyde and *tert*-butyl (6-aminohexyl)carbamate.

**Example 34**

*Preparation of di-tert-butyl (((((1,3-phenylenebis(methylene))bis(azanediyl))bis(propane-3,1-diyl))bis(azanediyl))bis(propane-3,1-diyl))dicarbamate* (CZ-27)

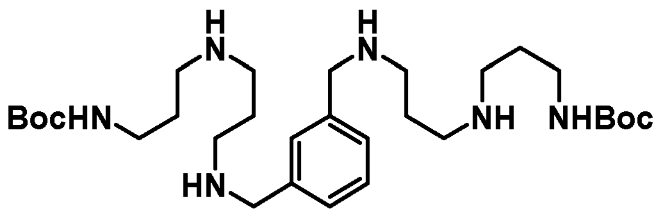

Example 34 was prepared in a similar fashion to Example Example 18 (CZ-25) from benzaldehyde and *tert*-butyl (3-((3-aminopropyl)amino)propyl)carbamate.

**Example 35**

*Preparation of $N^1$,$N^{1'}$,$N^{1''}$-(benzene-1,3,5-triyltris(methylene))tris(propane-1,3-diamine) hydrochloride salt* (CZ-32)

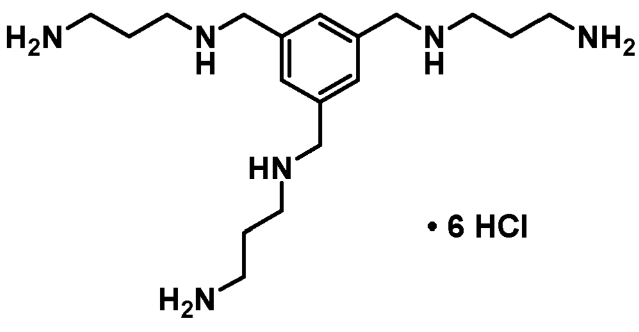

Example 35 was prepared in a similar fashion to Example 18 (CZ-25) from benzene-1,3,5-tricarbaldehyde and *tert*-butyl (3-aminopropyl)carbamate.

**Example 36**

*Preparation of $N^1$,$N^{1'}$,$N^{1''}$-(benzene-1,3,5-triyltris(methylene))tris(hexane-1,6-diamine), hydrochloride salt* (CZ-33)

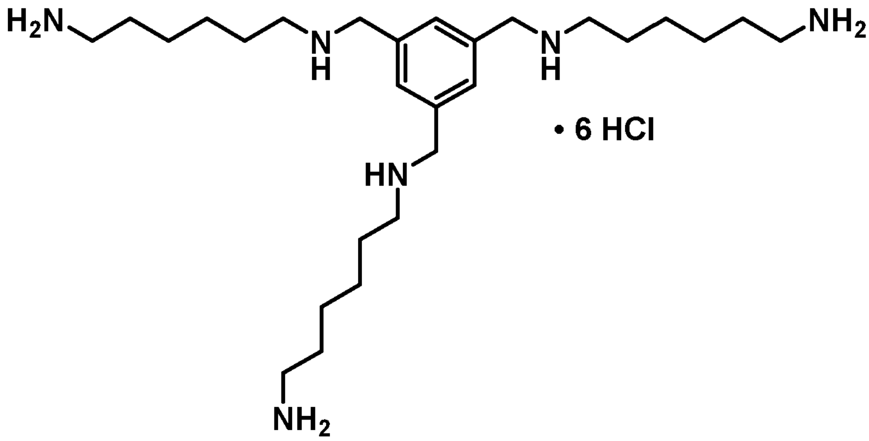

Example 36 was prepared in a similar fashion to Example 18 (CZ-25) from benzene-1,3,5-tricarbaldehyde and *tert*-butyl (6-aminohexyl)carbamate.

**Example 37**

*Preparation of $N^1$,$N^{1'}$-(((1,3-phenylenebis(methylene))bis(azanediyl))bis(propane-3,1-diyl))bis($N^3$,$N^3$-dimethylpropane-1,3-diamine), hydrochloride salt* (CZ-43)

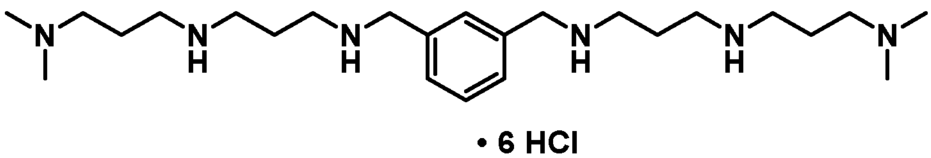

Example 37 was prepared in a similar fashion to Example 18 (CZ-25) from isophthalaldehyde and *N*[1]-(3-aminopropyl)-$N^3$,$N^3$-dimethylpropane-1,3-diamine. $^1$H NMR (500 MHz, $D_2O$) $\delta$ 7.57 (s, 4H), 4.31 (s, 4H), 3.28-3.14 (m, 16H), 2.91 (s, 12H), 2.20-2.10 (m, 8H). $^{13}$C NMR (125 MHz, $D_2O$) $\delta$ 132.1, 131.9, 131.8, 130.9, 54.8, 51.5,45.3,45.1, 44.8, 43.5, 23.3, 21.9.

**Example 38**

*Preparation of $N^1$,$N^{1'}$,$N^{1''}$-(benzene-1,3,5-triyltris(methylene))tris($N^3$,$N^3$-dimethylpropane-1,3-diamine), hydrochloride salt* (CZ-46)

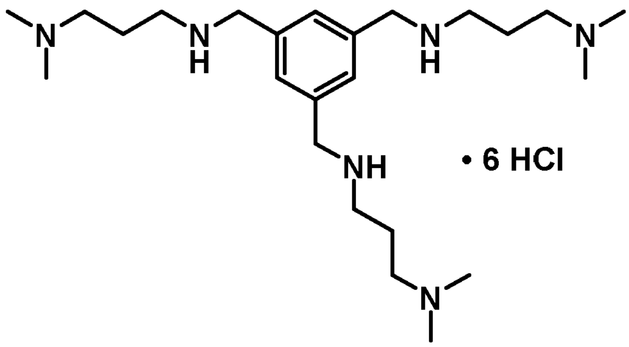

Example 38 was prepared in a similar fashion to Example 18 (CZ-25) from benzene-1,3,5-tricarbaldehyde and $N^1$,$N^1$-dimethylpropane-1,3-diamine.

**Example 39**

*Preparation of (3, 5-bis(((3-((3-aminopropyl)amino)propyl)amino)methyl)phenyl)-methanol, hydrochloride salt* (CZ-53)

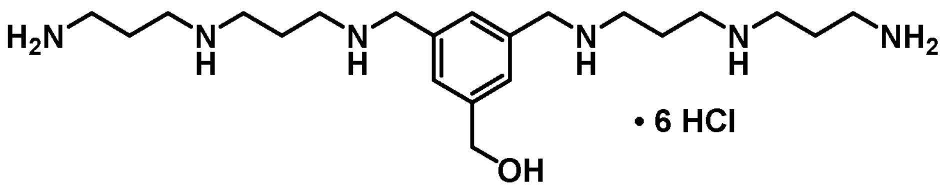

Example 3 was prepared in a similar fashion to Example 18 (CZ-25) from 5-(hydroxymethyl)isophthalaldehyde and *tert*-butyl (3-((3-aminopropyl)amino)propyl)carbamate. $^1$H NMR (500 MHz, $D_2O$) δ 7.57 (s, 2H), 7.55 (s, 1H), 4.72 (s, 2H), 4.35 (s, 4H), 3.26-3.19 (m, 12H), 3.13 (t, *J* = 7.5 Hz, 4H), 2.21-2.09 (m, 8H). $^{13}$C NMR (125 MHz, $D_2O$) δ 142.8, 132.0, 130.7, 130.0, 63.2, 51.0, 44.9, 44.8, 44.4, 36.7, 23.9, 22.9.

**Example 40**

*Preparation of tri-tert-butyl-(((((benzene-1,3,5-triyltris(methylene))tris(azanediyl))tris-(propane-3,1-diyl))tris(azanediyl))tris(propane-3,1-diyl))tricarbamate, hydrochloride salt* (CZ-54)

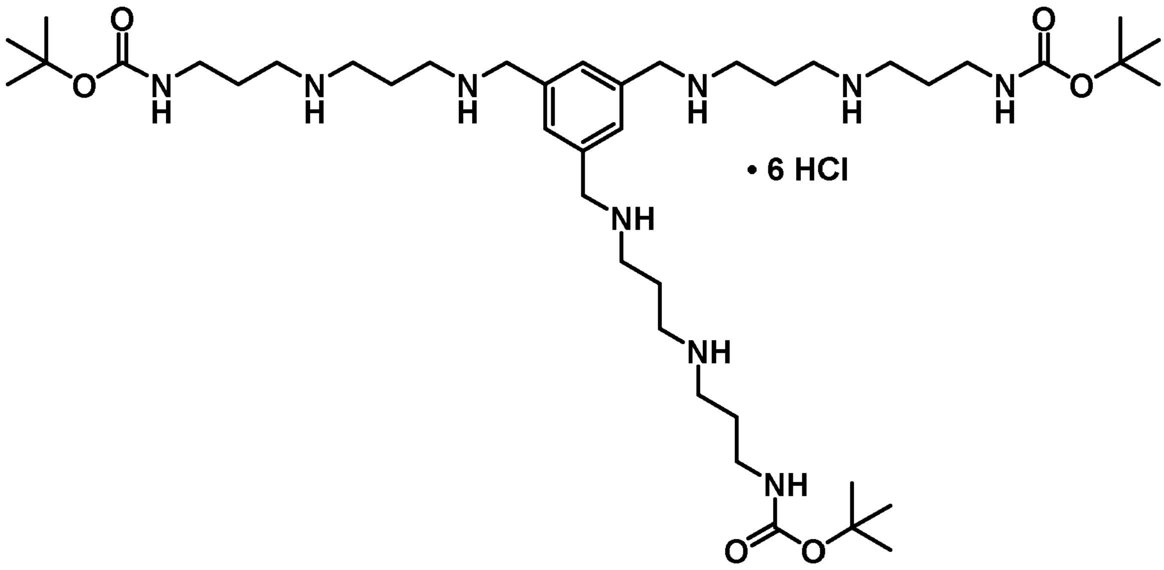

Example 40 was prepared in a similar fashion to Example 18 (CZ-25) from benzene-1,3,5-tricarbaldehyde and tert-butyl (3-((3-aminopropyl)amino)propyl)carbamate.

**Example 41**

*Preparation of $N^1$,$N^{1'}$-(1,3-phenylenebis(methylene))bis($N^3$-(3-((3-aminopropyl)amino)propyl)propane-1,3-diamine), hydrochloride salt* (CZ-57)

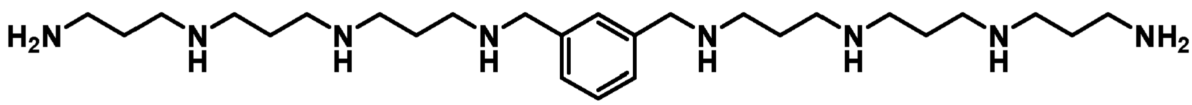

• 8 HCl

Example 41 was prepared in a similar fashion to Example 18 (CZ-25) from isophthalaldehyde and *tert*-butyl (3-((3-((3-aminopropyl)amino)propyl)amino)propyl)-carbamate.

**Example 42**

*Preparation of 3,3'-(isophthaloylbis(azanediyl))bis(propan-1-aminium) chloride* (CZ-40)

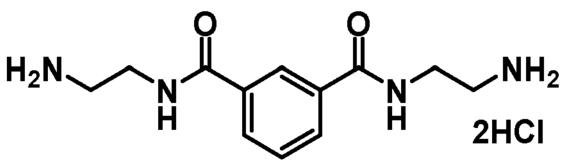

Step 1: *tert-Butyl(5-(3-((3-((tert-butoxycarbonyl)amino)propyl)carbamoyl)phenyl)-5-oxopentyl)carbamate.* Isophthaloyl chloride (344 mg, 1.70 mmol), triethylamine (343 mg, 3.41 mmol), and $CH_2Cl_2$ (10 mL) were added to a round-bottom flask. To the solution was added *tert*-butyl (3-aminopropyl)carbamate (593 mg, 3.41 mmol), and the reaction mixture was stirred for 16 h. The mixture was washed with aq. NaOH (10%, 50 mL) and the layers separated. The aqueous layer was extracted with $CH_2Cl_2$ (2 x 50 mL) and organic layers were combined, dried over $Na_2SO_4$, filtered, and concentrated under reduced pressure. Purification by column chromatography (hexanes/EtOAc) afforded the desired product, which was used without further purification.

Step 2: *3,3'-(Isophthaloylbis(azanediyl))bis(propan-1-aminium) chloride.* To the crude

*tert*-butyl(5-(3-((3-((*tert*-butoxycarbonyl)amino)propyl)carbamoyl)phenyl)-5-oxopentyl)carbamate from Step 1 was added methanolic HCl (150 mL, 1.0 M). The reaction mixture was stirred for 2 h and concentrated under reduced pressure. The solid was collected by vacuum filtration and washed with $Et_2O$ (30 mL) and hot MeOH (30 mL) to afford the desired product (0.388 g, 56%) as a white solid.

**Example 43**

*Preparation ofN$^1$,N$^{1'}$,N$^{1''}$-(benzene-1,3,5-triyltris(methylene))tris(N$^3$-(3-aminopropyl)propane-1,3-diamine), hydrochloride salt* (CZ-52)

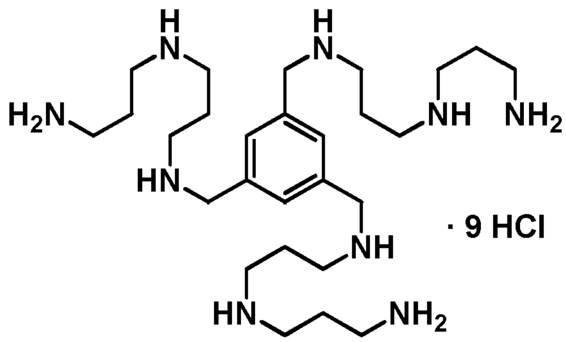

Step 1: *Tri-tert-butyl (((((benzene-1,3,5-triyltris(methylene))tris(azanediyl))-tris(propane-3,1-diyl))tris(azanediyl))tris(propane-3,1-diyl))tricarbamate.* Benzene-1,3,5-tricarbaldehyde (1.80 g, 11.04 mmol) and MeOH (50 mL) were added to a round-bottom flask. To the solution was added trimethyl orthoformate (165.6 g, 17.6 mmol) and *tert*-butyl (3-((3-aminopropyl)amino)propyl)carbamate (7.66 g, 33.1 mmol) and the reaction mixture was stirred for 24 h. Sodium borohydride (1.67 g, 44.2 mmol) was added portionwise and stirred for 1 h. The reaction mixture was concentrated under reduced pressure and aq. NaOH (10%, 150 mL) and EtOAc (150 mL) were added. The layers were separated and the aqueous layer was extracted with EtOAc (150 mL). The organic layers combined, dried over $Na_2SO_4$, filtered, and concentrated under reduced pressure to afford the desired product as a clear oil. The intermediate was carried forward without further purification.

Step 2: *N$^1$,N$^{1'}$,N$^{1''}$-(Benzene-1,3,5-triyltris(methylene))tris(N$^3$-(3-aminopropyl)propane-1,3-diamine), hydrochloride salt.* To the crude tri-tert-butyl (((((benzene-1,3,5 5-triyltris(methylene))tris(azanediyl))tris(propane-3,1-diyl))tris(azanediyl))tris(propane-3,1-diyl))tricarbamate from Step 1 was added methanolic HCl (150 mL, 1.0 M). The reaction mixture was stirred for 2 h and concentrated under reduced pressure. The solid was collected by vacuum filtration and washed with $Et_2O$ (30 mL) and hot MeOH (30 mL) to afford the desired product (5.6 g, 61%) as a white solid. LRMS $[M+H]^+$ 508.5.

**Example 44**

*Preparation of tert-butyl (3-((3-((3, 5-bis(((3-((3-aminopropyl)amino)propyl)amino)methyl)-benzyl)amino)propyl)amino)propyl)carbamate* (CZ-51)

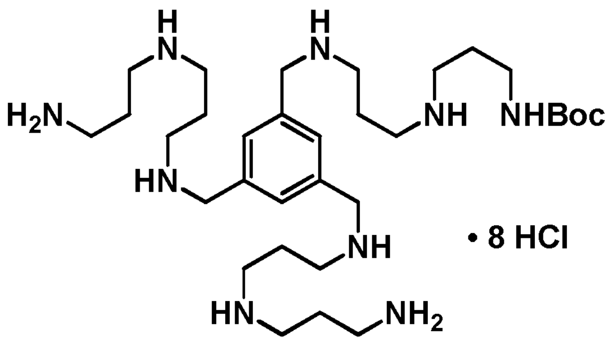

Example 44 was prepared in a similar fashion to Example 43 (CZ-52) from benzene-1,3,5-tricarbaldehyde and *tert*-butyl (3-((3-aminopropyl)amino)propyl)carbamate.

**Example 45**

*Preparation of N1,N1'-([1,1'-biphenyl]-3,5-diylbis(methylene))bis(N3-(3-ammoniopropyl)propane-1,3-diaminium) chloride* (CZ-58)

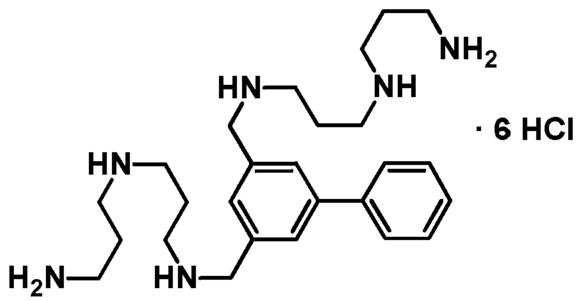

Step 1: *[1,1'-Biphenyl]-3,5-dicarbaldehyde.* A solution of 5-bromoisophthaldehyde (40.0 g, 187.8 mmol) (Med. Chem. Commun., 2012, 3, 763-770), phenylboronic acid (22.9 g, 187.8 mmol) and potassium carbonate (64.8 g, 469.5 mmol) in DME/$H_2O$ (5:1, 600 mL) was purged with $N_2$ for 5 min. Tetrakispalladium triphenylphosphine (1.1 g, 0.9 mmol) was added, and the reaction mixture was heated to 90 °C and stirred for 16 h. The reaction mixture was cooled to rt, filtered through a pad of Celite diatomaceous earth, and the solvent evaporated under reduced pressure. Purification by column chromatography (10% EtOAc/hexanes) afforded the desired product (74%, 29.1 g) as an off-white solid. $^1H$ NMR (300 MHz, $CDCl_3$) δ ppm 10.18 (s, 2H), 8.37 (d, *J*= 1.2 Hz, 2H), 8.35 (t, *J*= 1.5 Hz, 1H), 7.70-7.66 (m, 2H), 7.55-7.43 (m, 3H).

Step 2: *Di-tert-butyl (((((([1,1'-biphenyl]-3,5-diylbis(methylene))bis(azanediyl))bis-(propane-3,1-diyl))bis(azanediyl))bis(propane-3,1-diyl))dicarhamate.* [1,1'-Biphenyl]-3,5-dicarbaldehyde (3.62 g, 17.24 mmol) and MeOH (100 mL) were added to a round-bottom flask. To the solution was added *tert*-butyl (3-((3-aminopropyl)amino)propyl)carbamate (7.96 g, 34.4 mmol), and the reaction mixture was stirred for 24 h. Sodium borohydride (2.62 g, 69.0 mmol) was added, and the mixture was stirred for 1 h. The solvent was removed under reduced pressure to afford a white solid. Aqueous NaOH (10%, 200 mL) was added, and the solution was extracted with EtOAc (200 mL). The organic layer was separated, and the aqueous layer was extracted with EtOAc (200 mL). The organic layers were combined, dried over $Na_2SO_4$, filtered, and concentrated under reduced pressure to afford the desired product as a clear oil that was used without further purification.

Step 3: *N1,N1'-([1,1'-biphenyl]-3,5-diylbis(methylene))bis(N3-(3-ammoniopropyl)propane-1,3-diaminium) chloride.* To the crude *N*1,*N*1'-([1,1'-biphenyl]-3,5-diylbis(methylene))bis(*N*3-(3-ammoniopropyl)propane-1,3-diaminium) chloride from Step 2 was added methanolic HCl (150 mL, 1.0 M). The reaction mixture was stirred for 2 h and concentrated under reduced pressure. The solid was collected by vacuum filtration and washed with $Et_2O$ (30 mL) and hot MeOH (30 mL) to afford the desired product (6.8 g, 60%) as a white solid. $^1H$ NMR ($D_2O$, 500 MHz) δ ppm 7.85 (s, 2H), 7.74 (d, *J*= 7.5 Hz, 2H), 7.59-7.56 (m, 3H), 7.50-7.49 (m, 1H), 4.38 (s, 4H), 3.29 (t, *J* =8.0 Hz, 4H), 3.23 (q, *J*= 5.0 Hz, 8H), 3.14 (t, *J*= 8.0 Hz, 4H), 2.25-2.19 (m, 4H), 2.17-2.11 (m, 4H). $^{13}C$ NMR (125 MHz, $D_2O$) 142.3, 138.8, 132.1, 130.0, 129.5, 129.2, 128.4, 127.0, 50.8, 44.7, 44.6, 44.2, 36.5,23.7,22.7. LRMS $[M+H]^+$ 441.4.

**Example 46**

*Preparation of N1,N1'-((5-(benzo[d][1,3]dioxol-5-yl)-1,3-phenylene)bis(methylene))bis(N3-(3-ammoniopropyl)propane-1,3-diaminium) chloride* (CZ-61)

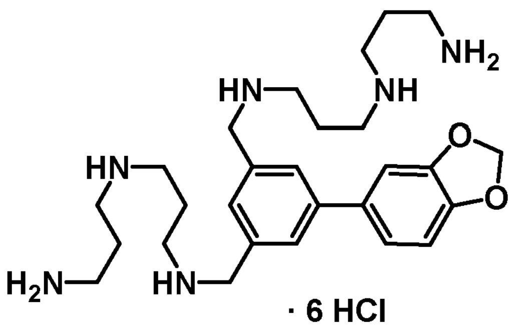

Example 46 was prepared in a similar fashion to Example 45 (CZ-58) from 5-bromoisophthaldehyde, benzo[*d*][1,3]dioxol-5-ylboronic acid, and *tert*-butyl (3-((3-aminopropyl)amino)propyl)carbamate. *N*1,*N*1'-((5-(benzo[d][1,3]dioxol-5-yl)-1,3-phenylene)bis(methylene))bis(*N*3-(3-aminopropyl)propane-1,3-diamine), hydrochloride salt: $^1$H NMR (500 MHz, $D_2O$) δ 7.72 (s, 2H), 7.49 (s, 1H), 7.15 (s, 2H), 6.93 (d, J = 8.5 Hz, 1H), 5.95 (s, 2H), 4.30 (s, 4H), 3.21-3.12 (m, 8H), 3.05 (t, J = 8.0 Hz, 4H), 2.17-2.02 (m, 8H). $^{13}$C NMR (125 MHz, $D_2O$) δ 148.1, 147.6, 142.3, 133.3, 132.2, 130.8, 129.7, 129.6, 129.4, 121.1, 109.0, 107.5, 101.6, 51.0, 44.8, 44.8, 44.4, 36.6, 23.9, 22.8.

**Example 47**

*Preparation of N1,N1',N1"-([1,1'-biphenyl]-3,3',5-triyltris(methylene))tris(N3-(3-aminopropyl)propane-1,3-diamine), hydrochloride salt* (CZ-62)

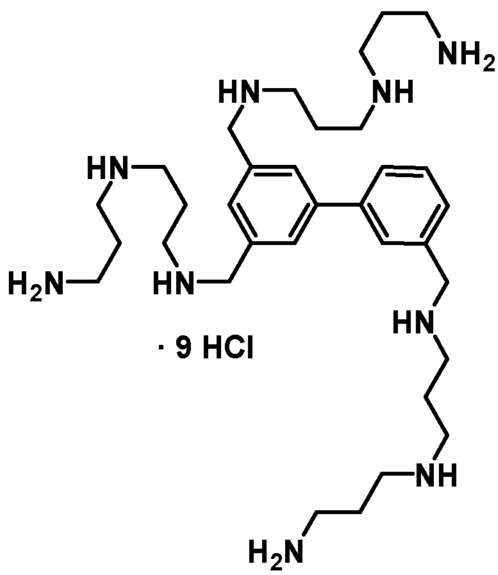

Example 47 was prepared in a similar fashion to Example 45 (CZ-58) from 5-bromoisophthaldehyde, (3-formylphenyl)boronic acid and *tert*-butyl (3-((3-aminopropyl)amino)propyl)carbamate. $^1$H NMR (400 MHz, $D_2O$) δ 7.96 (s, 2H), 7.93-7.85 (m, 2H), 7.71-7.61 (m, *J* = 8.5 Hz, 3H), 4.47 (s, 4H), 4.44 (s, 2H), 3.36-3.23 (m, 18H), 3.17 (t, *J*= 8.0 Hz, 6H), 2.30-2.22 (m, 6H), 2.20-2.13 (m, 6H). LRMS $[M+H]^+$ 584.5.

**Example 48**

*Preparation of N1,N1'-((2'-methyl-[1,1'-biphenyl]-3,5-diyl)bis(methylene))bis(N3-(3-aminopropyl)propane-1,3-diamine), hydrochloride salt* (CZ-63)

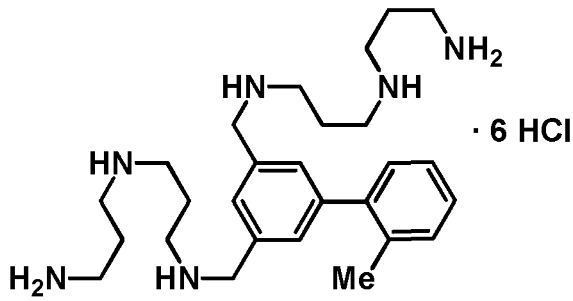

Example 48 was prepared in a similar fashion to Example 45 (CZ-58) from 5-bromoisophthaldehyde, *o*-tolylboronic acid and *tert*-butyl (3-((3-aminopropyl)amino)propyl)carbamate. $^1H$ NMR (500 MHz, $D_2O$) $\delta$ 7.59 (s, 1H), 7.56(d, J = 1.0 Hz, 2H), 7.41-7.40 (m, 2H), 7.38-7.35 (m, 1H), 7.32-7.31 (m, 1H), 4.38 (s, 4H), 3.25 (t, J =7.5 Hz, 4H), 3.20-3.18 (m, 8H), 3.11 (t, J = 8.0 Hz, 4H), 2.25 (m, 3H), 2.20-2.15 (m, 4H), 2.14-2.07 (m, 4H)). $^{13}C$ NMR (125 MHz, $D_2O$) $\delta$ 143.3, 139.9, 135.6, 131.8, 131.7, 131.5, 131.0, 130.5, 129.9, 129.7, 128.2, 126.1, 50.8, 44.7, 44.6, 44.2, 36.6, 23.7, 22.7. LRMS $[M+H]^+$ 455.4.

**Example 49**

*Preparation ofN$^1$,N$^{1'}$-((4'-morpholino-[1,1'-biphenyl]-3,5-diyl)bis(methylene))bis(N3-(3-aminopropyl)propane-1,3-diamine), hydrochloride salt* (CZ-64)

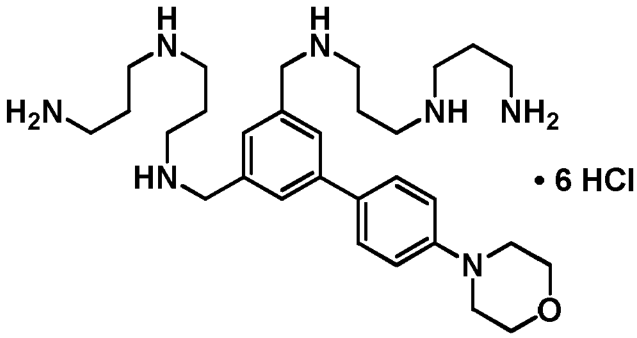

Example 49 was prepared in a similar fashion to Example 45 (CZ-58) from 5-bromoisophthaldehyde, (4-morpholinophenyl)boronic acid and *tert-butyl* (3-((3-aminopropyl)amino)propyl)carbamate.

**Example 50**

*Preparation of N$^1$,N$^{1'}$,N$^{1''}$-([1,1''-biphenyl]-3,4',5-triyltris(methylene))tris(N$^3$-(3-aminopropyl)propane-1,3-diamine), hydrochloride salt* (CZ-65)

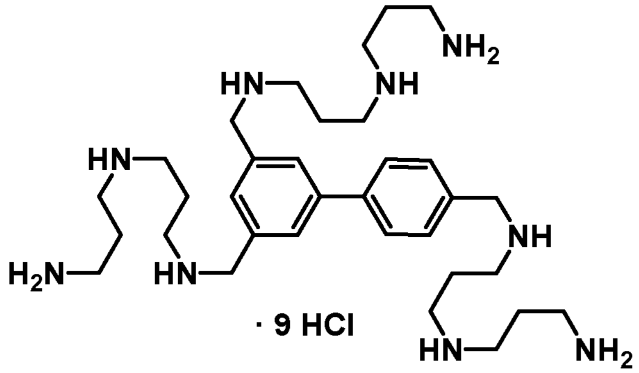

Example 50 was prepared in a similar fashion to Example 45 (CZ-58) from 5-bromoisophthaldehyde, (4-formylphenyl)boronic acid and *tert*-butyl (3-((3-aminopropyl)amino)propyl)carbamate. $^1$H NMR (500 MHz, $D_2O$) δ 7.92 (s, 2H), 7.84 (d, J = 8.5 Hz, 2H), 7.65 (d, J = 8.5 Hz, 3H), 4.43 (s, 4H), 4.37 (s, 2H), 3.31-3.28 (m, 6H), 3.26-3.20 (m, 12H), 3.14 (t, J = 8.0 Hz, 6H), 2.25-2.17 (m, 6H), 2.16-2.10 (m, 6H). $^{13}$C NMR (125 MHz, $D_2O$) δ 141.6, 140.1, 132.2, 130.6, 130.4, 129.7, 127.8, 50.8, 44.7, 44.6, 44.2, 36.5, 23.7, 22.7, 22.6. LRMS $[M+H]^+$ 584.5.

## Example 51

*Preparation of $N^1$,$N^{1'}$$N^{1''}$-((4'-isopropoxy-[1,1'-biphenyl]-3,3',5-triyl)tris(methylene))-tris($N^3$-(3-aminopropyl)propane-1, 3-diamine), hydrochloride salt* (CZ-66)

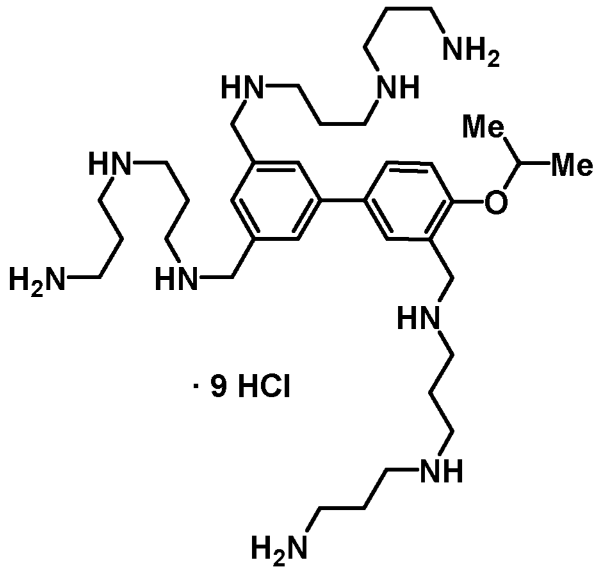

Example 51 was prepared in a similar fashion to Example 45 (CZ-58) from 5-bromoisophthaldehyde, (3-formyl-4-isopropoxyphenyl)boronic acid and *tert*-butyl (3-((3-aminopropyl)amino)propyl)carbamate. $^1$H NMR (500 MHz, $D_2O$) δ 7.93 (s, 2H), 7.88-7.82 (m, 2H), 7.65 (s, 1H), 7.35 (d, J = 9.0 Hz, 1H), 4.94-4.87 (m, 1H), 4.47 (s, 4H), 4.43 (s, 2H), 3.39-3.28 (m, 18H), 3.22 (t, J = 8.0 Hz, 6H), 2.33-2.18 (m, 12H), 1.48 (d, J = 6.5 Hz, 6H). $^{13}$C NMR (125 MHz, D2O) δ 158.9, 143.9, 134.8, 133.8, 132.9, 132.6, 132.3, 131.8, 122.7, 117.0, 84.4, 74.5, 53.5, 49.6, 47.3, 47.3, 47.0, 46.9, 46.7, 39.2, 26.3, 25.3, 25.2, 23.9. LRMS $[M+H]^+$ 642.5.

**Example 52**

*Preparation of $N^1$,$N^{1'}$-((4'-isopropoxy-[1,1"-biphenyl]-3,5-diyl)bis(methylene))bis($N^3$-(3-aminopropyl)propane-1,3-diamine), hydrochloride salt* (CZ-67)

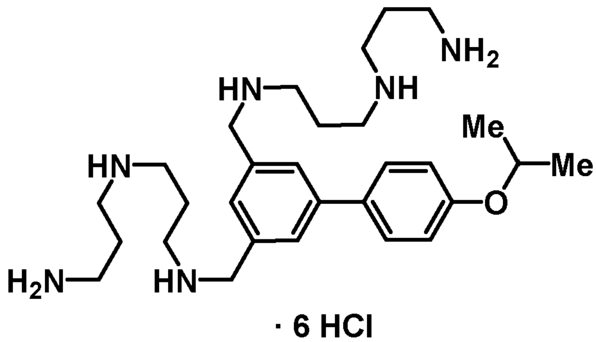

Example 52 was prepared in a similar fashion to Example 45 (CZ-58) from 5-bromoisophthaldehyde, (4-isopropoxyphenyl)boronic acid, and tert-butyl (3-((3-aminopropyl)amino)propyl)carbamate. $^1$H NMR (500 MHz, $D_2O$) δ 7.81 (s, 2H), 7.68 (d, *J* = 8.5 Hz, 2H), 7.54 (s, 1H), 7.12 (d, *J* = 8.5 Hz, 2H), 4.78-4.72 (m, 1H), 4.37 (s, 4H), 3.28 (t, *J* = 8.0 Hz, 4H), 3.24-3.19 (m, 8H), 3.13 (t, *J*= 8.0 Hz, 4H), 2.24-2.19 (m, 4H), 2.17-2.09 (m, 4H), 1.35 (d, *J*= 6.0 Hz, 6H). $^{13}$C NMR (125 MHz, $D_2O$) δ 157.0, 141.8, 132.0, 131.8, 129.4, 129.0, 128.3, 116.8, 71.6, 50.8, 44.6, 44.6, 44.1, 36.4, 23.6, 22.6, 21.0.

**Example 53**

*Preparation of $N^1$,$N^{1'}$-((5-(thiophen-2-yl]-1,3-phenylene)bis(methylene))bis($N^3$-(3-aminopropyl)propane-1,3-diamine), hydrochloride salt* (CZ-68)

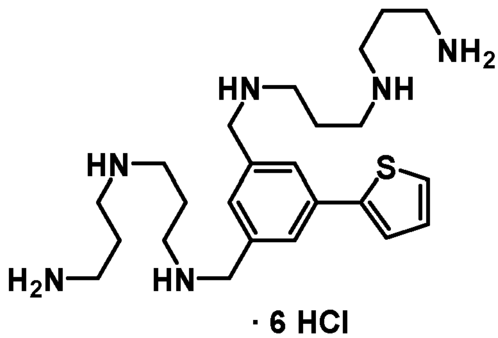

Example 53 was prepared in a similar fashion to Example 45 (CZ-58) from 5-bromoisophthaldehyde, (4-(thiophen-2-yl)phenyl)boronic acid and *tert-butyl* (3-((3-aminopropyl)amino)propyl)carbamate. $^1$H NMR (500 MHz, $D_2O$) δ 7.90 (s, 2H), 7.83 (t, J = 1.0 Hz, 1H), 7.63-7.61 (m, 1H), 7.60-7.57 (m, 1H), 7.53 (s, 1H), 4.38 (s, 4H), 3.27 (t, J = 8.0 Hz, 4H), 3.23-3.19 (m, 8H), 3.13 (t, J = 8.0 Hz, 4H), 2.23-2.17 (m, 4H), 2.15-2.09 (m, 4H). $^{13}$C NMR (125 MHz, $D_2O$) δ 139.9, 137.1, 132.2, 129.6, 128.7, 127.7, 125.9, 122.3, 50.8, 44.7, 44.6, 44.1, 36.5, 23.7, 22.6. LRMS $[M+H]^+$ 447.3.

**Example 54**

*Preparation of $N^1$,$N^{1'}$,;$N^{1''}$-((4'-(trifluoromethyl)-[1,1'-biphenyl]-3,3',5-triyl)tris(methylene))tris(N3-(3-aminopropyl)propane-1,3-diamine), hydrochloride salt* (CZ-69)

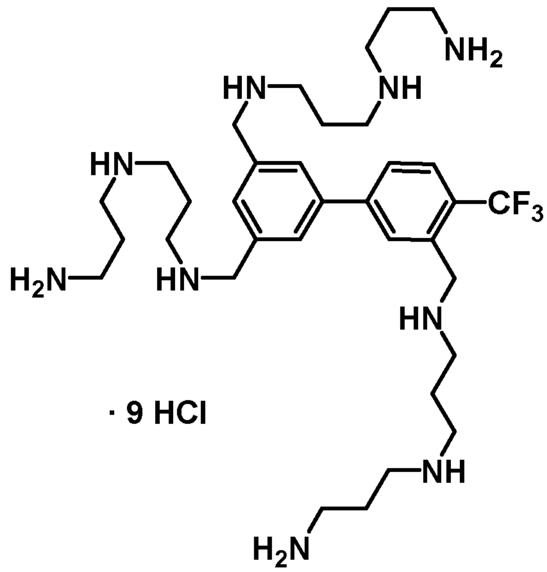

Example 54 was prepared in a similar fashion to Example 45 (CZ-58) from 5-bromoisophthaldehyde, (3-formyl-4-(trifluoromethyl)phenyl)boronic acid and *tert*-butyl (3-((3-aminopropyl)amino)propyl)carbamate. $^{1}H$ NMR (500 MHz, $D_2O$) δ 7.93 (s, 2H), 7.87 (s, 2H), 7.61 (s, 2H), 4.45 (s, 2H), 4.38 (s, 4H), 3.27-3.14 (m, 16H), 3.10-06 (m, 8H), 2.25-2.05 (m, 12H).

**Example 55**

*Preparation of N$^1$,N$^{1''}$-((3',5'-bis(trifluoromethyl)-[1,1'-biphenyl]-3,5-diyl)bis(methylene))bis(N$^3$-(3-aminopropyl)propane-1,3-diamine), hydrochloride salt* (CZ-70)

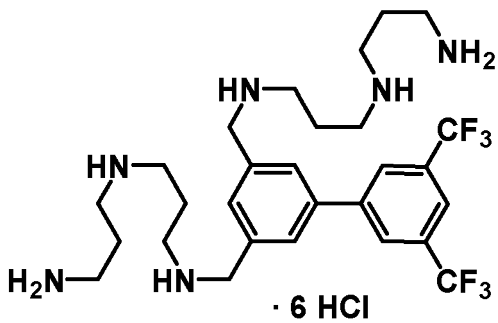

Example 55 was prepared in a similar fashion to Example 45 (CZ-58) from 5-bromoisophthaldehyde, (3,5-bis(trifluoromethyl)phenyl)boronic acid and *tert*-butyl (3-((3-aminopropyl)amino)propyl)carbamate. $^{1}H$ NMR (500 MHz, $D_2O$) δ 8.20 (s, 2H), 8.07 (s, 1H), 7.91 (s, 2H), 7.69 (s, 1H), 4.41 (s, 4H), 3.30 (t, J = 7.5 Hz, 4H), 3.26-3.21(m, 8H), 3.14 (t, J = 8.0 Hz, 4H), 2.23-2.19 (m, 4H), 2.17-2.10 (m, 4H). $^{13}C$ NMR (125 MHz, $D_2O$) δ 140.6, 139.7, 132.4, 131.5, 131.2, 129.9, 127.5, 124.4, 122.2, 50.7, 44.7, 44.6, 44.3, 36.5, 23.7, 22.6. LRMS $[M+H]^+$ 577.3.

**Example 56**

*Preparation of N$^1$,N$^{1'}$-((5-(pyridin-4-yl)-1,3-phenylene)bis(methylene))bis(N$^3$-(3-aminopropyl)propane-1,3-diamine), hydrochloride salt* (CZ-71)

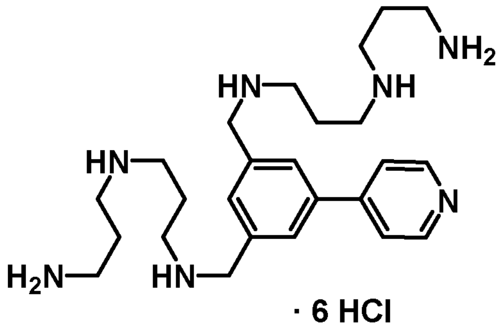

· 6 HCl

Example 56 was prepared in a similar fashion to Example 45 (CZ-58) from 5-bromoisophthaldehyde, pyridin-4-ylboronic acid and *tert*-butyl (3-((3-aminopropyl)amino)propyl)carbamate. $^1$H NMR (500 MHz, $D_2O$) $\delta$ 8.87 (d, J = 6.0 Hz, 2H), 8.39 (d, J = 6.5 Hz, 2H), 8.15 (s, 2H), 7.88 (s, 1H), 4.47 (s, 4H), 3.30 (t, J = 8.0 Hz, 4H), 3.24-3.19(m, 8H), 3.12 (t, J = 8.0 Hz, 4H), 2.24-2.19 (m, 4H), 2.15-2.08 (m, 4H). $^{13}$C NMR (125 MHz, $D_2O$) $\delta$ 156.6, 141.6, 136.8, 134.4, 133.2, 131.0, 125.2, 50.8, 44.9, 44.8, 44.6, 36.7,23.9,22.9. LRMS $[M+H]^+$442.4.

**Example 57**

*Preparation of $N^1$,$N^{1'}$-((5-(6-methoxypyridin-3-yl)-1,3-phenylene)bis(methylene))bis($N^3$-(3-aminopropyl)propane-1,3-diamine), hydrochloride salt* (CZ-72)

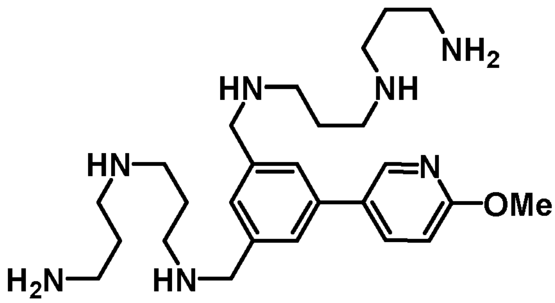

· 6 HCl

Example 57 was prepared in a similar fashion to Example 45 (CZ-58) from 5-bromoisophthaldehyde, (6-methoxypyridin-3-yl)boronic acid and *tert*-butyl (3-((3-aminopropyl)amino)propyl)carbamate. LRMS $[M+H]^+$ 472.4.

**Example 58**

*Preparation of $N^1$,$N^{1'}$-([1,1'-biphenyl]-3,5-diylbis(methylene))bis($N^3$-(3-((3-aminopropyl)amino)propyl)propane-1,3-diamine), hydrochloride salt* (CZ-73)

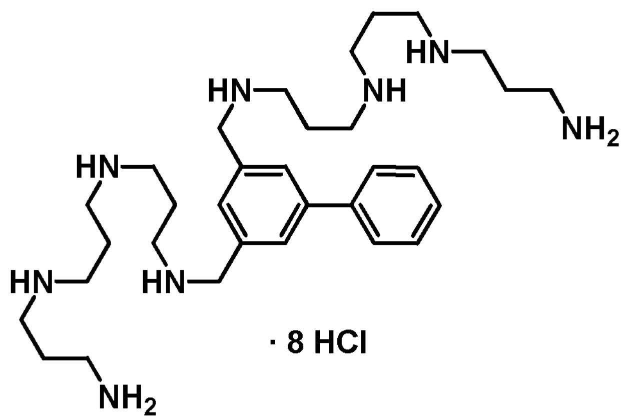

Example 58 was prepared in a similar fashion to Example 45 (CZ-58) from 5-bromoisophthaldehyde, phenylboronic acid and *tert*-butyl (3-((3-((3-aminopropyl)amino)propyl)amino)propyl)carbamate.

**Example 59**

*Preparation of N[1],N[1']-((5-phenoxy-1,3-phenylene)bis(methylene))bis(N[3]-(3-aminopropyl)propane-1,3-diamine), hydrochloride salt* (CZ-74)

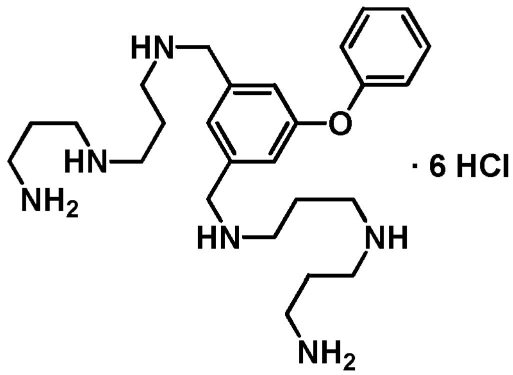

Step 1: *Dimethyl 5-phenoxyisophthalate.* 5-Hydroxyisophthalate (210 mg, 1.00 mmol), phenylboronic acid (0.244 g, 2.0 mmol), copper(II) acetate (0.182 mg, 1.0 mmol) and triethylamine (0.303 mg, 3.0 mmol) in $CH_2Cl_2$ (20 mL) was stirred at rt in the open air for 38 h. Additional phenylboronic acid (0.061 mg, 0.50 mmol) was added, and the reaction mixture was stirred at rt in the open air. After 24 h, additional phenylboronic acid (0.061 g, 0.50 mmol) and copper acetate (0.020 g, 0.11 mmol) were added to the reaction mixture, and the mixture was stirred for 6 h. The mixture was diluted with $CHCl_2$ (20 mL) and aqueous HCl (10 mL). The organic layer was separated, washed with aq HCl, aq $NaHCO_3$, dried over $MgSO_4$, and concentrated under reduced pressure. Purification by column chromatography (10% EtOAc/hexanes) afforded the desired product (0.200 g 70 %) as a white solid. $^1H$ NMR (300 MHz, $CDCl_3$) $\delta$ ppm 8.42-8.41 (m, 1H), 7.85 (d, *J*= 1.5 Hz, 2H), 7.42-7.36 (m, 2H), 7.21-7.16 (m, 1H), 7.05-7.01 (m, 2H), 3.93 (s, 6H).

Step 2: *5-Phenoxyisophthalaldehyde.* To a solution of dimethyl 5-phenoxyisophthalate (0.143 g, 0.50 mmol) in toluene (12 mL) was added drop-wise a solution of Red-Al (60% in toluene, 0.673 mL, 2.0 mmol) and 1-methylpiperazine (0.243 mL, 2.2 mmol) in THF at 5 °C, and the resulting mixture was stirred for 2 h. The reaction was quenched with $H_2O$ (4 mL), and then extracted with EtOAc (20 mL). The organic layer was washed with $H_2O$ (10 mL), dried over anhydrous $Na_2SO_4$, and then concentrated under reduced pressure. Purification by flash column chromatography (10% EtOAc/hexanes) afforded the desired product (0.094 g, 83 %) as a white solid. $^1H$ NMR (300 MHz, $CDCl_3$) $\delta$ ppm 10.04 (s, 2 H), 8.08 (s, 1 H), 7.72 (t, J = 1.5 Hz, 2H), 7.46-7.40 (m, 2H), 7.28-7.24 (m, 1H), 7.09-7.06 (m, 2H).

Step 3: *Di-tert-butyl ((((((5-phenoxy-1,3-phenylene)bis(methylene))-bis(azanediyl))bis (propane-3,1-diyl))bis(azanediyl)) bis (propane-3,1-diyl))dicarbamate.* To a solution of 5-phenoxyisophthalaldehyde (0.060 g, 0.27 mmol) and 3 Å mol. sieves in MeOH, was added *N*1-(3-aminopropyl)-*N*3-isobutylpropane-1,3-diamine (0.122 g, 0.53 mmol). The resultant mixture was stirred for 18 h. Sodium borohydride (0.020 g, 0.53 mmol) was added portionwise, and the

reaction mixture was stirred for 1 h. The MeOH was evaporated, and the white solid was taken up in EtOAc (10 mL) and washed with aq. NaOH (10%, 4 mL). The aqueous layer was back-extracted with EtOAc (10 mL). The organic layers were combined, dried over $Na_2SO_4$, filtered and evaporated to afford clear oil. Methanolic HCl (30 mL, 1.0M) was added to the resultant oil, and the reaction mixture was stirred for 1 h. Evaporation and collection by vacuum filtration afforded a white solid, which was washed hot MeOH (25 mL) to give the desired product (0.064 g, 52%) as a white solid. $^1$H NMR (500 MHz, $D_2O$) $\delta$ 7.50 (t, *J* = 7.5 Hz, 2H), 7.36 (S, 1H), 7.30 (d, *J* = 7.5 Hz, 1H), 7.24 (s, 2H), 7.17 (d, *J*= 8.0 Hz, 2H), 4.29 (s, 4H), 3.25-3.18 (m, 12H), 3.14 (t, *J*= 8.0 Hz, 4H), 2.21-2.10 (m, 8H). $^{13}$C NMR (125 MHz, $D_2O$) $\delta$ 158.2, 155.6, 133.3, 130.3, 124.8, 120.5, 119.7, 119.3, 50.5, 44.7, 44.6, 44.2, 36.5, 23.7, 22.6. LRMS $[M+H]^+$ 457.4.

**Example 60**

*Preparation of N¹,N¹”-((4’-fluoro-[1,1’-biphenyl]-3, 5-diyl)bis(methylene))bis(N3-(3-aminopropyljpropane-1,3-diamine), hydrochloride salt* (CZ-75)

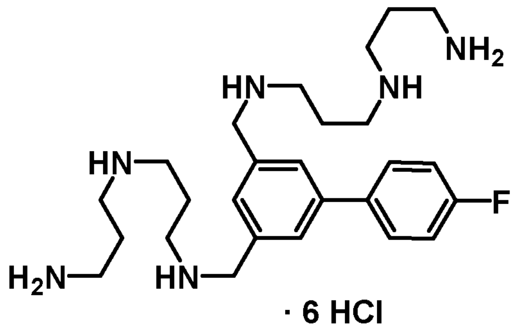

Example 60 was prepared in a similar fashion to Example 45 (CZ-58) from 5-bromoisophthaldehyde, (4-fluorophenyl)boronic acid and *tert*-butyl (3-((3-aminopropyl)amino)propyl)carbamate. $^1$H NMR (500 MHz, $D_2O$) $\delta$ 7.80 (s, 2H), 7.71-7.68 (m, 2H), 7.56 (s, 1H), 7.25 (t, J = 9 Hz, 2H), 4.37 (s, 4H), 3.26 (t, J = 8 Hz, 4H), 3.19 (q, J = 8 Hz, 8H), 3.11 (t, J = 7.5 Hz, 4H), 2.22-2.07 (m, 8H). $^{13}$C NMR (125 MHz, $D_2O$) $\delta$ 162.9 (d, 244 Hz), 135.3(d, 3 Hz), 132.3, 130.1, 129.7, 129.1 (d, 8 Hz), 116.1 (d, 21 Hz), 51.1, 44.9, 44.8, 44.4, 36.7, 23.9, 22.9. LRMS $[M+H]^+$ 459.4.

**Example 61**

*Preparation of N¹,N¹’-([1,1’-biphenyl]-3,5-diylbis(methylene))bis(propane-1,3-diamine), hydrochloride salt* (CZ-77)

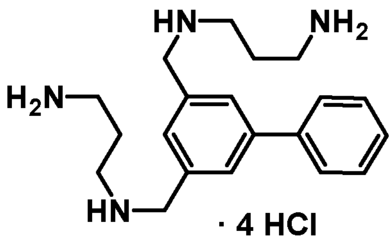

Example 61 was prepared in a similar fashion to Example 45 (CZ-58) from 5-bromoisophthaldehyde, phenylboronic acid and tert-butyl (3-aminopropyl)carbamate. *N¹,N¹’*-([1,1’-biphenyl]-3,5-diylbis(methylene))bis(propane-1,3-diamine). $^1$H NMR (300 MHz, $D_2O$) $\delta$ 7.82 (s, 2H), 7.71-7.64 (m, 2H), 7.54-7.41 (m, 4H), 4.34 (s, 4H), 3.22 (t, *J*= 7.8 Hz, 4H), 3.08 (t, *J*= 7.5 Hz, 4H), 2.11 (quint, *J*= 7.5 Hz, 4H).

**Example 62**

*Preparation ofN$^1$,N$^{1'}$-((5-butoxy-1,3-phenylene)bis(methylene))bis(N3-(3-aminopropyl)propane-1,3-diamine), hydrochloride salt* (CZ-76)

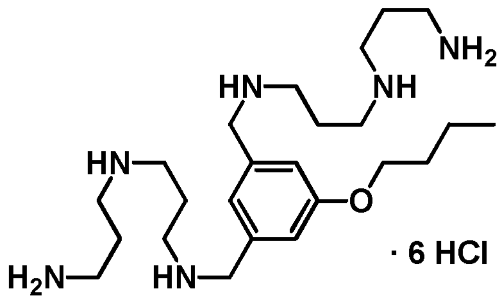

Step 1: *Dimethyl 5-butoxyisophthalate.* Dimethyl 5-hydroxyisophthalate (0.40 g, 1.90 mmol), cesium carbonate (1.30 g, 3.80 mmol) and $CH_3CN$ (12 mL) were added to a round-bottom flask and stirred for 15-30 min. Iodobutane (0.42 g, 2.28 mmol) was added,and the reaction was stirred for 16 h. The solvent was concentrated under reduced pressure, and the reaction mixture was partitioned between EtOAc (50 mL) and $H_2O$ (50 mL). The organic layer was separated and the aqueous layer extracted with EtOAc (50 mL). The combined organics were dried over $Na_2SO_4$, filtered, and concentrated under reduced pressure to afford the desired product which was used without further purification.

Step 2: *(5-Butoxy-1,3-phenylene)dimethanol.* To a solution of dimethyl 5-butoxyisophthalate (0.51 g, 1.90 mmol) in THF (16 mL) was slowly added $LiAlH_4$ (0.40 g, 10.5 mmol). The reaction mixture was stirred for 8 h. The reaction was quenched by the addition of 2N aq. HCl (5 mL). The mixture was extracted with $Et_2O$ (2 x 25 mL) and EtOAc (2 x 25 mL), the organics layers combined, dried over $Na_2SO_4$, filtered, and concentrated under reduced pressure to afford the desired product (0.24 g) as an oil, which was used without further purification.

Step 3: *5-Butoxyisophthalaldehyde.* (5-Butoxy-1,3-phenylene)dimethanol (0.24 g, 1.15 mmol) and $CH_2Cl_2$ (10 mL) were added to a round-bottom flask. To the solution was added PCC (0.74 g, 3.45 mmol), and the reaction was stirred at rt for 16 h. The reaction mixture was concentrated under reduced pressure. Purification by column chromatography (90% hexanes/EtOAc) afforded the desired product (0.18 g, 44%, 3 steps) as a white semi-solid. $^1H$ NMR (300 MHz, $CDCl_3$) $\delta$ ppm 9.99 (s, 2H), 7.89 (s, 1H), 7.58 (s, 2H), 4.03 (t, *J* = 6.6 Hz, 2H), 1.77 (quint, *J* = 6.6 Hz, 2H), 1.47 (sext, *J* = 7.2 Hz, 2H), 0.94 (t, *J*= 7.5 Hz, 3H). $^{13}C$ NMR (75 MHz, $CDCl_3$) $\delta$ ppm 191.3, 160.6, 138.6, 124.2, 120.1, 68.9, 31.3, 19.4, 14.1.

Step 4: *Di-tert-butyl((((((5-butoxy-1,3-phenylene)bis(methylene))bis(azanediyl))-bis(propane-3,1-diyl))bis(azanediyl))bis(propane-3,1-diyl))dicarbamate. 5*-Butoxyisophthalaldehyde (0.80 g, 0.85 mmol) and MeOH (15 mL) were added to a round-bottom flask. To the solution was added tert-butyl (3-((3-aminopropyl)amino)propyl)-carbamate (0.39 g, 1.70 mmol), and the reaction mixture was stirred at rt for 24 h. Sodium borohydride (0.13 g, 3.40 mmol) was added and the reaction mixture stirred for 1 h. The reaction mixture was concentrated under reduced pressure and aq. NaOH (10%, 50 mL) and EtOAc (50 mL) were added. The layers were separated and the aqueous layer was extracted with EtOAc (50 mL). The organic layers combined, dried over $Na_2SO_4$, filtered, and concentrated under reduced pressure to afford the desired product as a clear oil which was used without further purification.

Step 5: *N$^1$,N$^{1'}$-((5-Butoxy-1,3-phenylene)bis(methylene))bis(N3-(3-aminopropyl)propane-1,3-diamine), hydrochloride salt.* To the crude di-*tert*-butyl ((((((5-butoxy-1,3-phenylene)bis(methylene))bis(azanediyl))bis(propane-3,1-diyl))bis(azanediyl))-bis(propane-3,1-diyl))dicarbamate from Step 4 was added methanolic HCl (50 mL, 1.0 M). The reaction mixture was stirred for 2 h and concentrated under reduced pressure. The solid was collected by vacuum filtration and washed with $Et_2O$ (10 mL) and hot MeOH (10 mL) to afford the desired product (0.27 g, 48%) as a white solid. $^1H$ NMR (500 MHz, $D_2O$) $\delta$ ppm 7.20 (s, 3H), 4.30 (s, 4H), 4.15 (t, *J*= 6.5 Hz, 2H), 3.26-3.20 (m, 12H), 3.14 (t, *J*= 7.5 Hz, 4H), 2.22-2.10 (m, 8H), 1.79 (quint, *J*= 6.5 Hz, 2H), 1.48 (sext, *J*= 7.5 Hz, 2H), 0.96 (t, J = 7 Hz, 3H). $^{13}C$ NMR (125 MHz, $D_2O$) 159.2, 132.9, 123.5, 117.3, 68.8, 50.8, 44.7, 44.6, 44.1, 36.5, 30.3, 23.7, 22.6, 18.5, 13.0. LRMS $[M+H]^+$ 437.4.

**Example 63**

*Preparation of $N^1$,$N^{1'}$-((5-((2-ethylhexyl)oxy)-1,3-phenylene)bis(methylene))bis($N^3$-(3-aminopropyl)propane-1,3-diamine), hydrochloride salt* (CZ-81)

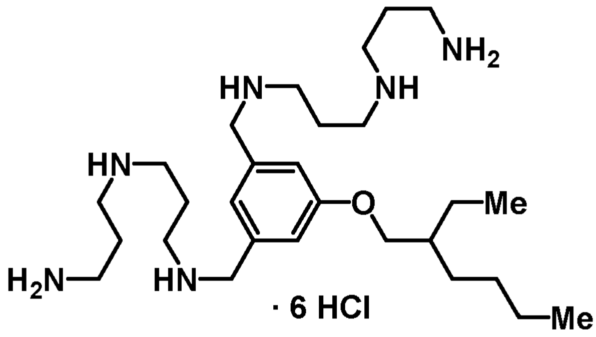

Example 63 was prepared in a similar fashion to Example 62 (CZ-76) from dimethyl 5-butoxyisophthalate, 3-(bromomethyl)heptane and *tert*-butyl (3-((3-aminopropyl)amino)propyl)carbamate. LRMS $[M+H]^+$493.5.

**Example 64**

*Preparation of $N^1$,$N^{1'}$-((5-(2-ethylbutoxy)-1,3-phenylene)bis(methylene))bis($N^3$-(3-aminopropyl)propane-1,3-diamine), hydrochloride salt* (CZ-90)

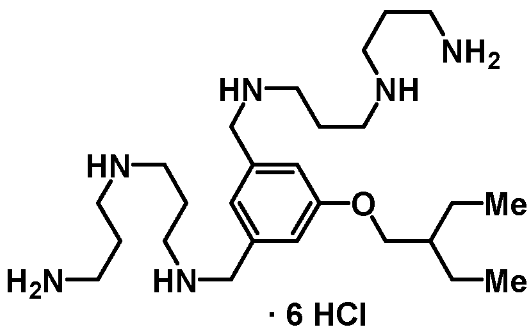

Example 64 was prepared in a similar fashion to Example 62 (CZ-76) from dimethyl 5-butoxyisophthalate, 3-(bromomethyl)pentane and *tert*-butyl (3-((3-aminopropyl)amino)propyl)carbamate. $^1$H NMR (500 MHz, $D_2O$) δ 7.23 (s, 2H), 7.22 (s, 1H), 4.32 (s, 4H), 4.06 (t, *J*= 7.0 Hz, 2H), 3.27-3.18 (m, 12H), 3.15 (t, *J*= 8.0 Hz, 4H), 2.24-2.12 (m, 8H), 1.72 (m , *J* = 6.0 Hz, 1H), 1.52-1.44 (m, 4H), 0.94-0.91 (m, 6H). $^{13}$C NMR (125 MHz, $D_2O$) δ 159.6, 132.9, 123.5, 117.5, 71.43, 50.8, 44.7, 44.6, 44.1, 40.1, 36.5, 23.7, 22.6, 10.3. LRMS $[M+H]^+$ 465.4.

**Example 65**

*Preparation of $N^1$,$N^{1'}$-((5-(Benzyloxy)-1,3-phenylene)bis(methylene))bis($N^3$-(3-aminopropyl)propane-1,3-diamine), hydrochloride salt* (CZ-95)

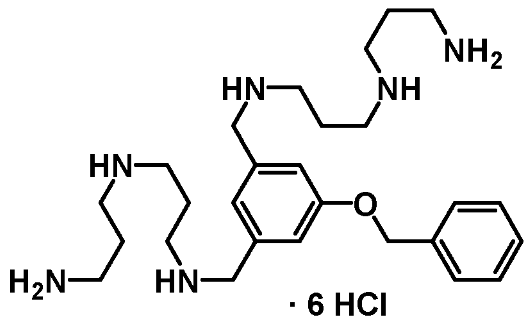

Example 65 was prepared in a similar fashion to Example 62 (CZ-76) from dimethyl 5-butoxyisophthalate, benzyl bromide and *tert*-butyl (3-((3-aminopropyl)amino)propyl)carbamate. $^{1}H$ NMR (500 MHz, $D_2O$) δ 7.52 (d, J = 7.0 Hz, 2H), 7.49-7.41 (m, 3H), 7.23 (s, 2H), 7.20 (s, 1H), 5.23 (s, 2H), 4.29 (s, 4H), 3.22-3.15 (m, 12H), 3.13 (t, J = 8.0 Hz, 4H), 2.20-2.09 (m, 8H). $^{13}C$ NMR (125 MHz, $D_2O$) δ 158.7, 136.0, 133.0, 132.5, 128.8, 128.5, 128.0, 123.9, 117.7, 70.4, 50.7, 44.7, 44.6, 44.3, 44.0, 36.5, 23.7, 22.6. LRMS $[M+H]^+$ 471.4.

**Example 66**

*Preparation of $N^1$,$N^{1'}$-((5-(cyclohexylmethoxy)-1,3-phenylene)bis(methylene))bis($N^3$-(3-aminopropyl)propane-1,3-diamine), hydrochloride salt* (CZ-101)

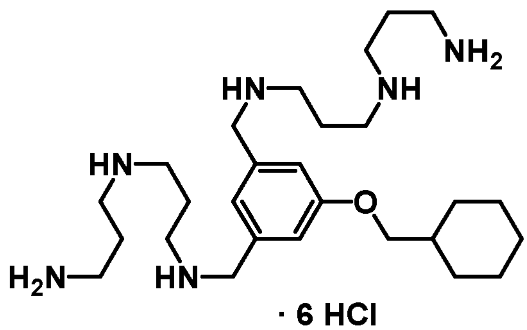

Example 66 was prepared in a similar fashion to Example 62 (CZ-76) from dimethyl 5-butoxyisophthalate, (bromomethyl)cyclohexane and *tert*-butyl (3-((3-aminopropyl)amino)propyl)carbamate. $^{1}H$ NMR (500 MHz, $D_2O$) δ 7.16 (d, J = 7.5 Hz, 3H), 4.27 (s, 4H), 3.93 (t, J = 6.0 Hz, 2H), 3.23-3.09 (m, 16H), 2.19-2.07 (m, 8H), 1.83-1.66 (m, 6H), 1.31-1.17 (m, 3H), 1.10-1.05 (m, 2H). $^{13}C$ NMR (125 MHz, $D_2O$) δ 131.4, 131.2, 131.1, 130.2, 50.8, 47.9, 44.6, 44.6, 44.2, 44.1, 30.9, 28.1, 28.1, 25.6, 25.4, 22.6, 21.9, 13.3.

**Example 67**

*Preparation of $N^1$,$N^{1'}$-([1,1''-biphenyl]-3,5-diylbis(methylene))bis($N^3$-(3-(butylamino)propyl)propane-1,3-diamine), hydrochloride salt* (CZ-83)

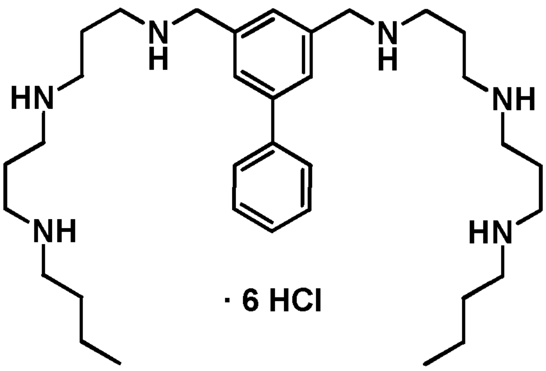

Step 1: *[1,1'-Biphenyl]-3,5-dicarbaldehyde.* A solution of 5-bromoisophthaldehyde (40.0 g, 187.8 mmol), phenylboronic acid (22.9 g, 187.8) and potassium carbonate (64.8 g, 469.5 mmol) in DME/$H_2O$ (5:1 600 mL) was purged with $N_2$ for 5 min. Tetrakispalladium triphenylphosphine (1.1 g mg, 0.9 mmol) was added and the reaction mixture was heated to 90 °C and stirred for 16 h. The reaction mixture was cooled, filtered through a pad of celite and the solvent evaporated under reduced pressure. Purification by column chromatography (10% EtOAc/hexanes) afforded the desired product (74%, 29.1 g) as a tan solid. $^1$H NMR (300 MHz, $CDCl_3$) δ ppm 10.18 (s, 2H), 8.37 (d, *J* = 1.2 Hz, 2H), 8.35 (t, *J*= 1.5 Hz, 1H), 7.70-7.66 (m, 2H), 7.55-7.43 (m, 3H).

Step 2: *$N^1$,$N^{1'}$-([1,1'-biphenyl]-3,5-diylbis(methylene))bis($N^3$-(3-(butylamino)propyl)propane-1,3-diamine).* [1,1'-Biphenyl]-3,5-dicarbaldehyde (0.66 g, 3.15 mmol) and MeOH (50 mL) were added to a round-bottom flask. To the solution was added *$N^1$*-(3-aminopropyl)-$N^3$-butylpropane-1,3-diamine (1.18 g, 6.31 mmol) and the reaction mixture was stirred at rt for 24 h. Sodium borohydride (0.48 g, 12.62 mmol) was added and the reaction mixture stirred for 1 h. The reaction mixture was concentrated under reduced pressure to afford a white solid. Aq. NaOH (10%, 100 mL) and EtOAc (100 mL) were added and the reaction mixture stirred for 1 h. The layers were separated and the aqueous layer was extracted with EtOAc (100 mL). The organic layers were combined, dried over $Na_2SO_4$, filtered, and concentrated under reduced pressure to afford a clear oil which was used without further purification.

Step 3: *$N^1$,$N^{1'}$-([1,1'-biphenyl]-3,5-diylbis(methylene))bis($N^3$-(3-(butylamino)propyl)propane-1,3-diamine) hydrochloride salt.* To the crude *$N^1$,$N^{1'}$*-([1,1'-biphenyl]-3,5-diylbis(methylene))bis(*$N^3$*-(3-(butylamino)propyl)propane-1,3-diamine) from step 2 was subjected to acidification with methanolic HCl (100 mL, 1.0M). The reaction mixture stirred at rt for 2 h. The reaction mixture was concentrated under reduced pressure and the solid was collected by vacuum filtration and washed with $Et_2O$ (50 mL) and hot MeOH (50 mL) to afford the desired product (1.24 g, 51%) as a white solid. $^1$H NMR (500 MHz, $D_2O$) δ 7.83 (s, 2H), 7.71 (d, J = 7.5 Hz, 2H), 7.55-7.52(m, 3H), 7.49-7.45 (m, 1H), 4.35 (s, 4H), 3.26 (t, J = 7.5 Hz, 4H), 3.21-3.16 (m, 8H), 3.14 (t, J = 8.0 Hz, 4H), 3.05 (t, J = 8.0 Hz, 4H), 2.21-2.10 (m, 8H), 1.65 (quint, J = 7.0 Hz, 4H), 1.37 (sext, J = 8.0 Hz, 4H), 0.91 (t, J = 7.0 Hz, 6H). $^{13}$C NMR (125 MHz, $D_2O$) δ 142.5, 138.9, 132.3, 130.3, 129.8, 129.5, 128.7, 127.3, 51.0, 47.8, 44.9, 44.5, 44.4, 27.7, 22.9, 22.9, 19.3, 13.0. LRMS $[M+H]^+$ 553.5.

**Example** 68

*Preparation of $N^1$,$N^{1'}$-([1,1'-biphenyl]-3,5-diylbis(methylene))bis($N^3$-(3-(isobutylamino)propyl)propane-1,3-diamine), hydrochloride salt* (CZ-86)

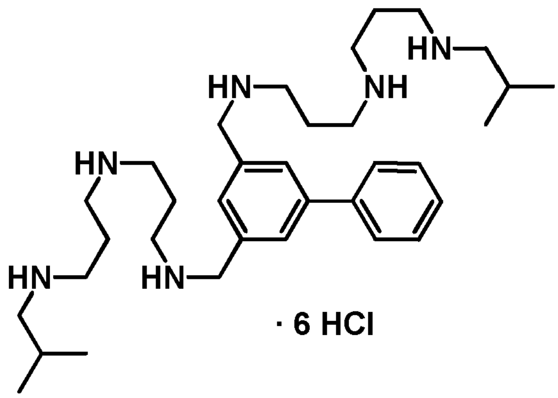

**Method 1**

Step 1: *[1,1'-Biphenyl]-3,5-dicarbaldehyde.* A solution of 5-bromoisophthaldehyde (40.0 g, 187.8 mmol), phenylboronic acid (22.9 g, 187.8) and potassium carbonate (64.8 g, 469.5 mmol) in DME/$H_2O$ (5:1 600 mL) was purged with $N_2$ for 5 min. Tetrakispalladium triphenylphosphine (1.1 g mg, 0.9 mmol) was added and the reaction mixture was heated to 90 °C and stirred for 16 h. The reaction mixture was cooled, filtered through a pad of celite and the solvent evaporated under reduced pressure. Purification by column chromatography (10% EtOAc/hexanes) afforded the desired product (74%, 29.1 g) as a tan solid. $^1$H NMR (300 MHz, $CDCl_3$) δ ppm 10.18 (s, 2H), 8.37 (d, *J* = 1.2 Hz, 2H), 8.35 (t, *J*= 1.5 Hz, 1H), 7.70-7.66 (m, 2H), 7.55-7.43 (m, 3H).

Step 2: *Di-tert-butyl ((((([1,1'-biphenyl]-3,5-diylbis(methylene))bis(azanediyl))-bis(propane-3,1-diyl))bis(azanediyl))bis(propane-3,1-diyl))dicarbamate.* [1,1'-Biphenyl]-3,5-dicarbaldehyde (3.62 g, 17.2 mmol, *1*) and MeOH (100 mL) were added to a round-bottom flask. To the solution was added *tert*-butyl (3-((3-aminopropyl)amino)propyl)-carbamate (7.96 g, 34.4 mmol), and the reaction mixture was stirred at rt for 24 h. Sodium borohydride (2.62 g, 69.0 mmol) was added and the reaction mixture stirred for 1 h. The reaction mixture was concentrated under reduced pressure to afford a white solid. Aqueous NaOH (10%, 200 mL) and EtOAc (200 mL) were added, and the reaction mixture was stirred for 1 h. The layers were separated, and the aqueous layer was extracted with EtOAc (200 mL). The organic layers were combined, dried over $Na_2SO_4$, filtered, and concentrated under reduced pressure to afford a clear oil that was used without further purification.

Step 3: *$N^1$,$N^{1'}$-([1,1'-biphenyl]-3,5-diylbis(methylene))bis($N^3$-(3-aminopropyl)propane-1,3-diamine), hydrochloride salt.* To the crude di-*tert*-butyl ((((([1,1'-biphenyl] -3,5 -diylbis(methylene))bis(azanediyl))bis(propane-3,1-diyl))bis(azanediyl))-bis(propane-3,1-diyl))dicarbamate from Step 2 was subjected to acidification with methanolic HCl (200 mL, 1.0M). The reaction mixture stirred at rt for 2 h. The reaction mixture was concentrated under reduced pressure, and the solid was collected by vacuum filtration and washed with $Et_2O$ (50 mL) and hot MeOH (50 mL) to afford the desired product (6.8 g, 60%) as a white solid. $^1$H NMR (500 MHz, $D_2O$) δ 7.85 (s, 2H), 7.74 (d, J = 7.5 Hz, 2H), 7.59-7.56 (m, 3H), 7.50-7.49 (m, 1H), 4.38 (s, 4H), 3.29 (t, J = 8.0 Hz, 4H), 3.23 (q, J = 5.0 Hz, 8H), 3.14 (t, J = 8.0 Hz, 4H), 2.25-2.19 (m, 4H), 2.17-2.11 (m, 4H). LRMS $[M+H]^+$ 441.4.

Step 4: *$N^1$,$N^{1'}$-([1,1'-Biphenyl]-3,5-diylbis(methylene))bis($N^3$-(3-(isobutylamino)propyl)propane-1,3-diamine).* To *$N^1$*,*$N^{1'}$*-([1,1'-biphenyl]-3,5-diylbis(methylene))bis(*$N^3$*-(3-aminopropyl)propane-1,3-diamine), hydrochloride salt was added aq. NaOH (10%, 100 mL) and 75% $CHCl_3$/*i*-propanol (100 mL). The layers were separate and the aqueous layer was extracted with 75% $CHCl_3$/*i*-propanol (4 x 100 mL). The organic layers were combined dried over $Na_2SO_4$, filtered, and concentrated under reduced pressure to afford a clear oil which was used without further purification.

To a round-bottom flask was added the crude *$N^1$*,*$N^{1'}$*-([1,1'-biphenyl]-3,5-diylbis(methylene))bis($N^3$-(3-aminopropyl)propane-1,3-diamine) (3.5 g, 8.0 mmol) and MeOH (50 mL). To the solution was added isobutyraldehyde (1.15 g, 15.9 mmol) and the reaction mixture was stirred at rt for 24 h. Sodium borohydride (1.2 g, 31.9 mmol) was added and the reaction mixture was stirred for 1 h. The reaction mixture was concentrated under reduced pressure to afford a white solid. Aq. NaOH (10%, 200 mL) and EtOAc (200 mL) were added, and the mixture was stirred for 1 h. The layers were separated and the aqueous layer was extracted with EtOAc (200 mL). The combined organics were dried over $Na_2SO_4$, filtered, and concentrated under reduced pressure to afford the desired product as a clear oil which was used without further purification.

Step 5: *$N^1$,$N^{1'}$-([1,1'-Biphenyl]-3,5-diylbis(methylene))bis($N^3$-(3-(isobutylamino)propyl)propane-1, 3-diamine), hydrochloride salt.* To the crude *$N^1$*,*$N^{1'}$*-([1,1'-biphenyl]-3,5-diylbis(methylene))bis($N^3$-(3-(isobutylamino)propyl)propane-1,3-diamine) was added methanolic HCl (200 mL, 1.0M). The reaction mixture was stirred at rt for 1 h. The reaction mixture was concentrated under reduced pressure, and the solid was collected by vacuum filtration and washed with $Et_2O$ (50 mL) and hot MeOH (50 mL) to afford the desired product as a white solid (3.07 g, 50%). $^1$H NMR (500 MHz, $D_2O$) δ 7.87 (s, 2H), 7.75 (d, *J*= 7.5, 2H), 7.59-7.48 (m, 4H), 4.39 (s, 4H), 3.26 (t, *J*= 8.0 Hz, 4H), 3.21-3.12 (m, 12H), 2.92 (d, *J*= 7.0 Hz, 4H), 2.21-2.10 (m, 8H), 2.01 (sept, *J*= 7.0 Hz, 2H), 0.99 (d, *J* = 7.0 Hz, 12H). $^{13}$C NMR (125 MHz, $D_2O$) δ 142.8, 139.1, 132.4, 130.1, 129.8, 129.5, 128.7, 127.3, 55.1, 51.1, 44.9, 44.8, 44.4, 25.8, 22.8, 22.7, 19.2. LRMS $[M+H]^+$ 553.5.

**Method 1I**

Step 1: *[1,1'-Biphenyl]-3,5-dicarbaldehyde.* A solution of 5-bromoisophthaldehyde (40.0 g, 187.8 mmol), phenylboronic acid (22.9 g, 187.8) and potassium carbonate (64.8 g, 469.5 mmol) in DME/$H_2O$ (5:1, 600 mL) was purged with $N_2$ for 5 min. Tetrakispalladium triphenylphosphine (1.1 g mg, 0.9 mmol) was added and the reaction mixture was heated to 90 °C and stirred for 16 h. The reaction mixture was cooled, filtered through a pad of Celite diatomaceous earth, and the solvent was evaporated under reduced pressure. Purification by column chromatography (10% EtOAc/hexanes) afforded the desired product (74%, 29.1 g) as a tan solid. $^1$H NMR (300 MHz, $CDCl_3$) δ ppm 10.18 (s, 2H), 8.37 (d, *J*= 1.2 Hz, 2H), 8.35 (t, *J*= 1.5 Hz, 1H), 7.70-7.66 (m, 2H), 7.55-7.43 (m, 3H).

Step 2: *$N^1$,$N^{1'}$-([1,1'-Biphenyl]-3,5-diylbis(methylene))bis($N^3$-(3-(isobutylamino)propyl)propane-1,3-diamine).* [1,1'-Biphenyl]-3,5-dicarbaldehyde (3.03 g, 14.4 mmol) and MeOH (60 mL) were added to a round-bottom flask. To the solution was added *$N^1$*-(3-aminopropyl)-*$N^3$*-isobutylpropane-1,3-diamine (5.40 g, 28.8 mmol), and the reaction mixture was stirred at rt for 24 h. Sodium borohydride (0.55 g, 14.4 mmol) was added, and the reaction mixture was stirred for 2 h. The reaction mixture was concentrated under reduced pressure to afford a white solid. Aqueous NaOH (10%, 50 mL) was added, and the reaction mixture stirred for 30 min. The solution was extracted with $CHCl_3$ (3 x 75 mL). The organic layers were combined, dried over $Na_2SO_4$, filtered, and concentrated under reduced pressure to afford a clear oil which was used without further purification.

Step 3: *$N^1$*,*$N^{1'}$*-([1,1'-Biphenyl]-3,5-diylbis(methylene))bis(*$N^3$*-(3-(isobutylamino)propyl)propane-1,3-diamine) hydrochloride salt: To the crude *$N^1$*,*$N^{1'}$*-([1,1'-biphenyl]-3,5-diylbis(methylene))bis(*$N^3$*-(3-(isobutylamino)propyl)propane-1,3-diamine) from step 2 was added methanolic HCl (75 mL, 2.0M). The resulting white solid was collected by vacuum filtration and washed with minimal MeOH and $Et_2O$ and stored under high vacuum to afford the desired product (9.3 g, 89%) as a white solid.

**Example 69**

*Preparation of 3, 5-bis(((3-((3-aminopropyl)amino)propyl)amino)methyl)phenol, hydrochloride salt* (CZ-87)

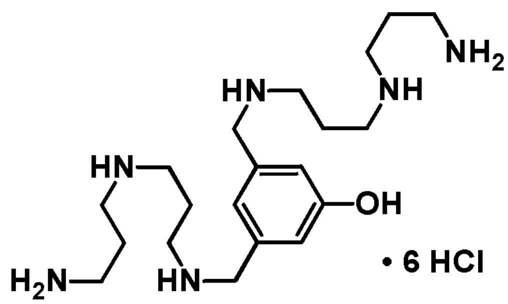

Example 69 was prepared in a similar fashion to Example 62 (CZ-76) from dimethyl 5-butoxyisophthalate and *tert*-butyl (3-((3-aminopropyl)amino)propyl)carbamate.

**Example 70**

*Preparation of (3',5 '-Bis(((3-((3-aminopropyl)amino)propyl)amino)methyl)-[1,1'-biphenyl]-4-yl)(piperidin-1-yl)methanone, hydrochloride salt* (CZ-88)

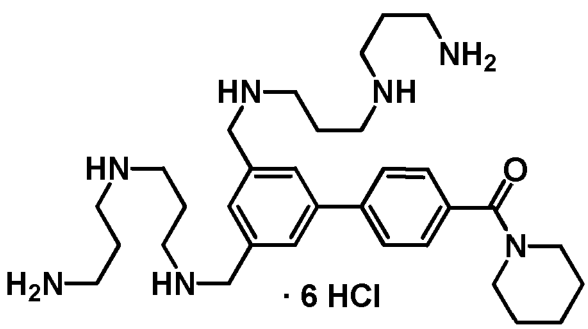

Example 70 was prepared in a similar fashion to Example 68 (CZ-86) from 5-bromoisophthaldehyde, piperidin-1-yl(4-(4,4,5,5-tetramethyl-1,3,2-dioxaborolan-2-yl)-phenyl)methanone and *tert*-butyl (3-((3-aminopropyl)amino)propyl)carbamate. $^1$H NMR (300 MHz, $D_2O$) δ 7.87 (s, 2H), 7.78 (d, J = 8.4 Hz, 2H), 7.58 (s, 1H), 7.54-7.49 (m, 2H), 4.37 (s, 4H), 3.68 (bs, 2H), 3.42-3.38 (m, 2H), 3.26-3.13 (m, 12H), 3.07 (t, J = 7.8 Hz, 4H), 2.20-2.01 (m, 8H), 1.71-1.64 (m, 4H), 1.58-1.49 (m, 4H). LRMS $[M+H]^+$ 552.4.

**Example 71**

*Preparation of 1,11'-biphenyl]-3,5diylbis(methylene))bis(azanediyl))bis(propane-3,1-diyl))bis(tetrahydropyrimidin-2(1H)-one), hydrochloride salt* (CZ-89)

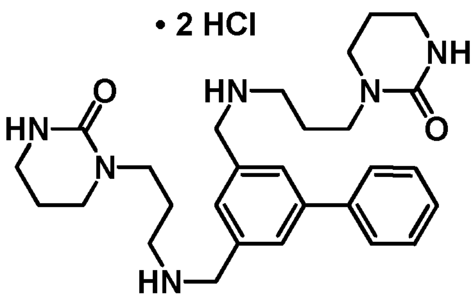

Example 71 was prepared in a similar fashion to Example 68 (CZ-86) from 5-bromoisophthaldehyde, phenyl boronic acid, 1-(3-aminopropyl)tetrahydropyrimidin-2(1H)-one and tert-butyl (3-((3-aminopropyl)amino)propyl)carbamate. $^1H$ NMR (300 MHz, $D_2O$) δ 7.80 (d, J = 6.5 Hz, 2H), 7.71-7.68 (m, 2H), 7.56-7.45 (m, 4H), 4.30 (s, 4H), 3.34 (t, J = 6.6 Hz, 4H), 3.21 (t, J = 6.0 Hz, 4H), 3.11 (t, J = 6.0 Hz, 4H), 3.04 (t, J = 7.2 Hz, 4H), 1.97-1.88 (m, 4H), 1.78 (p, J = 6.0 Hz, 4H). LRMS $[M+H]^+$ 493.4.

**Example 72**

*Preparation of $N^1$,$N^{1'}$-([1,1'-biphenyl]-3,5-diylbis(methylene))bis($N^3$-(3-(octylamino)propyl)-propane-1,3-diamine), hydrochloride salt* (CZ-92)

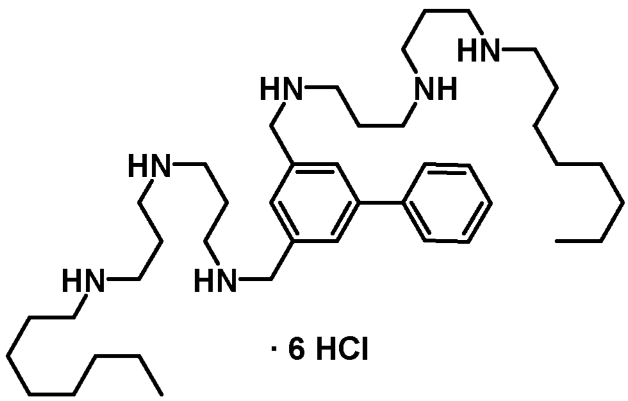

Example 72 was prepared in a similar fashion to Example 68 (CZ-86) from 5-bromoisophthaldehyde, phenylboronic acid, *tert*-butyl (3-((3-aminopropyl)amino)propyl)-carbamate, and octanal. $^1H$ NMR (500 MHz, $D_2O$) δ 7.88 (s, 2H), 7.75 (d, J = 8.0 Hz, 2H), 7.60-7.56(m, 3H), 7.53-7.50 (m, 1H), 4.40 (s, 4H), 3.26 (t, J = 8.0 Hz, 4H), 3.21-3.16 (m, 8H), 3.14 (t, J = 8.0 Hz, 4H), 3.06 (t, J = 8.0 Hz, 4H), 2.21-2.09 (m, 4H), 1.69-1.65 (m, 4H), 1.38-1.28 (m, 20H), 0.86 (t, J = 6.0 Hz, 4H). $^{13}C$ NMR (125 MHz, $D_2O$) δ 142.5, 138.8, 132.2, 129.9, 129.5, 129.3, 128.5, 127.0, 50.8, 47.8, 44.6, 44.1, 30.9, 28.1, 28.0, 25.6, 25.4, 22.7, 22.6, 21.9, 13.3. LRMS $[M+H]/2^+$ 333.3.

**Example 73**

*Preparation of $N^1$,$N^{1'}$-([1,1"-biphenyl]-3,5-diylbis(methylene))bis($N^3$-(3-(benzylamino)propyl)propane-1,3-diamine), hydrochloride salt* (CZ-94)

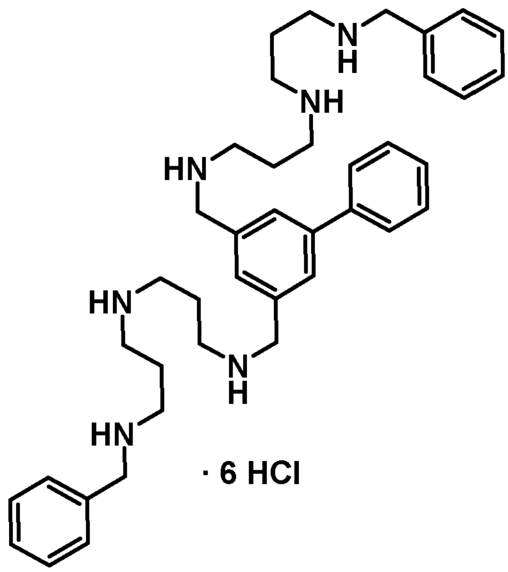

Example 73 was prepared in a similar fashion to Example 68 (CZ-86) from 5-bromoisophthaldehyde, phenylboronic acid, *tert*-butyl (3-((3-aminopropyl)amino)propyl)-carbamate, and benzaldehyde. $^1$H NMR (300 MHz, $D_2O$) δ 7.84 (s, 2H), 7.74-7.70 (m, 2H), 7.58-7.50 (m, 4H), 7.47 (s, 10H), 4.37 (s, 4H), 4.24 (s, 4H), 3.26-3.11 (m, 16H), 2.20-2.05 (m, 8H).

**Example 74**

*Preparation of $N^1$,$N^{1'}$-([1,1''-biphenyl]-3,5-diylbis(methylene))bis($N^3$-(3-((cyclohexylmethyl)amino)propyl)propane-1,3-diamine), hydrochloride salt* (CZ-96)

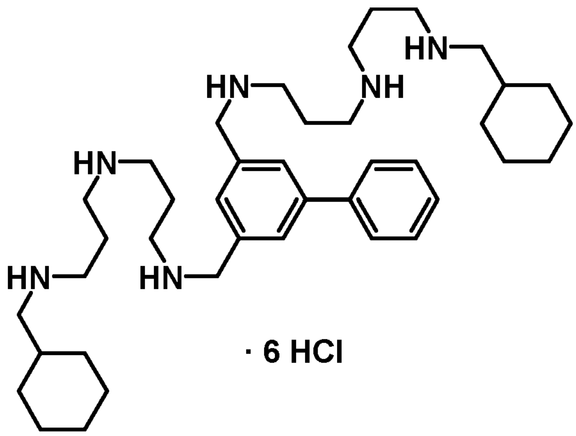

Example 74 was prepared in a similar fashion to Example 68 (CZ-86) from 5-bromoisophthaldehyde, phenylboronic acid and *N*$^1$-(3-aminopropyl)-N$^3$-(cyclohexylmethyl)propane-1,3-diamine. $^1$H NMR (500 MHz, $D_2O$) δ 7.87 (s, 2H), 7.75 (d, J = 7.5 Hz, 2H), 7.59-7.56(m, 3H), 7.51-7.49 (m, 1H), 4.40 (s, 4H), 3.28 (t, J = 8.0 Hz, 4H), 3.24-3.18 (m, 8H), 3.15 (t, J = 8.0 Hz, 4H), 2.93 (d, J = 7.0 Hz, 4H), 2.24-2.12 (m, 8H), 1.74-1.60 (m, 12H), 1.30-1.14 (m, 6H), 1.05-0.98 (m, 4H). $^{13}$C NMR (125 MHz, $D_2O$) δ 142.4, 138.8, 132.1, 130.0, 129.6, 129.3, 128.5, 127.0, 53.7, 50.8, 44.7, 44.6, 44.2, 34.5, 29.7, 25.4, 24.9, 22.6, 22.5. LRMS $[M+H]^+$ 633.5.

**Example 75**

*Preparation of $N^1$,$N^{1'}$-((5-(1-methyl-1H-pyrazol-4-yl)-1,3-phenylene)bis(methylene))bis(N3-(3-(isobutylamino)propyl)propane-1,3-diamine), hydrochloride salt* (CZ-102)

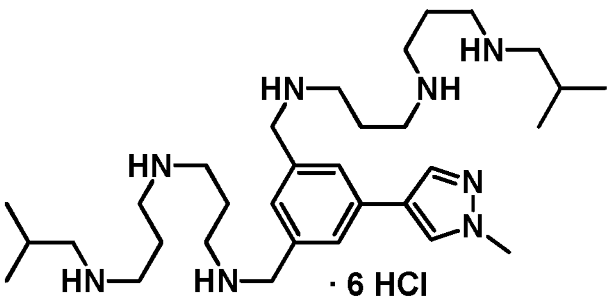

Example 75 was prepared in a similar fashion to Example 68 (CZ-86) from 5-bromoisophthaldehyde, (1-methyl-1H-pyrazol-4-yl)boronic acid and *tert-butyl* (3-((3-aminopropyl)amino)propyl)carbamate. $^1H$ NMR (300 MHz, $D_2O$) δ 7.75-7.69 (m, 4H), 6.60 (d, J = 2.1 Hz, 2H), 4.38 (s, 4H), 3.92 (s, 3H), 3.27-3.09 (m, 16H), 2.89 (d, J = 7.2 Hz, 4H), 2.21-2.06 (m, 8H), 2.03-1.94 (m, 2H), 0.97 (d, J = 6.9 Hz, 12H). $^1H$ NMR (300 MHz, $D_2O$) δ 7.75-7.69 (m, 4H), 6.60 (d, J = 2.1 Hz, 2H), 4.38 (s, 4H), 3.92 (s, 3H), 3.27-3.09 (m, 16H), 2.89 (d, J = 7.2 Hz, 4H), 2.21-2.06 (m, 8H), 2.03-1.94 (m, 2H), 0.97 (d, J = 6.9 Hz, 12H).

**Example 76**

*Preparation of $N^1$,$N^{1'}$-([1,1'-biphenyl]-3,5-diylbis(methylene))bis($N^4$-(3-(isobutylamino)propyl)butane-1,4-diamine), hydrochloride salt* (CZ-111)

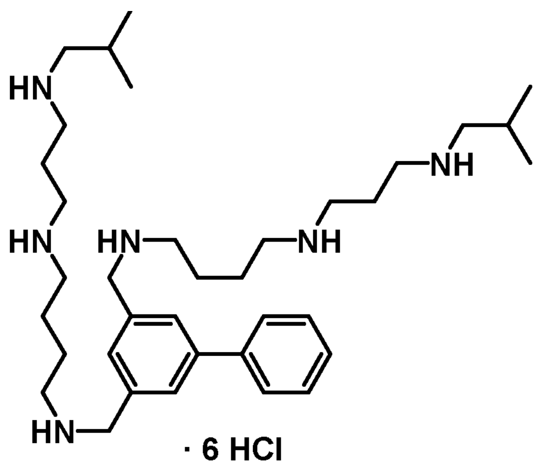

Example 76 was prepared in a similar fashion to Example 76 (CZ-86) from 5-bromoisophthaldehyde, phenylboronic acid and *$N^1$*-(3-aminopropyl)-*$N^3$*-isobutylpropane-1,3-diamine. $^1H$ NMR (500 MHz, $D_2O$) δ 7.86 (s, 2H), 7.75 (d, J = 7.5 Hz, 2H), 7.58 (t, J = 8.0 Hz, 3H), 7.50 (t, J = 7.5 Hz, 1H), 4.37 (s, 4H), 3.20-3.11 (m, 16H), 2.92 (d, J = 7.5 Hz, 4H), 2.16-2.10 (m, 4H), 2.06-1.97 (m, 2H), 1.88-1.80 (m, 8H), 0.99 (d, J = 6.5 Hz, 12H). $^{13}C$ NMR (125 MHz, $D_2O$) δ 142.7, 139.1, 132.6, 130.1, 129.7, 129.5, 128.7, 127.7, 55.1, 50.9, 47.2, 46.7, 44.5, 44.7, 25.8, 23.0, 22.9, 22.7, 19.2. LRMS $[M+H]^+$ 581.5.

**Example 77**

*Preparation of $N^1$,$N^{1'}$-([1,1'-biphenyl]-3,5-diylbis(methylene))bis($N^3$-(3-(isobutylamino)propyl)-2,2-dimethylpropane-1,3-diamine), hydrochloride salt* (CZ-112)

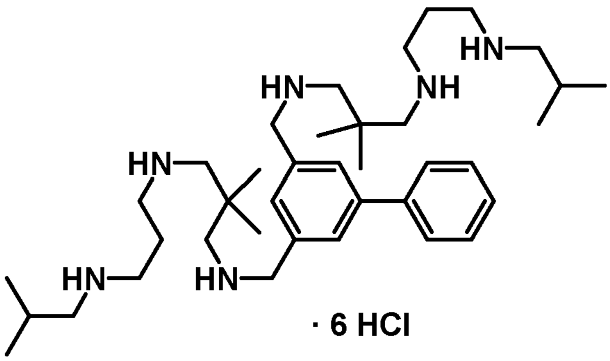

Example 77 was prepared in a similar fashion to Example 68 (CZ-86) from 5-bromoisophthaldehyde, phenylboronic acid and $N^1$-(3-(isobutylamino)propyl)-2,2-dimethylpropane-1,3-diamine. NMR (500 MHz, $D_2O$) δ 7.89 (s, 2H), 7.71 (d, J = 7.5 Hz, 2H), 7.62 (s, 1H), 7.52 (t, J = 7.5 Hz, 2H), 7.44 (t, J = 7.5 Hz, 1H), 4.39 (s, 4H), 3.19-3.01 (m, 16H), 2.89 (d, J = 7.5 Hz, 4H), 2.21-2.15 (m, 4H), 2.03-1.96 (m, 2H), 1.18 (s, 12H), 0.97 (d, J = 6.5 Hz, 12H). $^{13}C$ NMR (125 MHz, $D_2O$) δ 142.2, 138.8, 131.7, 131.4, 130.2, 129.5, 128.7, 127.2, 55.6, 55.1, 4.9, 52.1, 46.3, 45.0, 33.2, 25.8, 22.5, 22.4, 21.9, 19.3. LRMS $[M+H]^+$ 609.5.

**Example 78**

*Preparation of $N^1$,$N^{1'}$-([1,1'-biphenyl]-3,5-diylbis(methylene))bis($N^3$-(3-(hexylamino)propyl)propane-1,3-diamine), hydrochloride salt* (CZ-114)

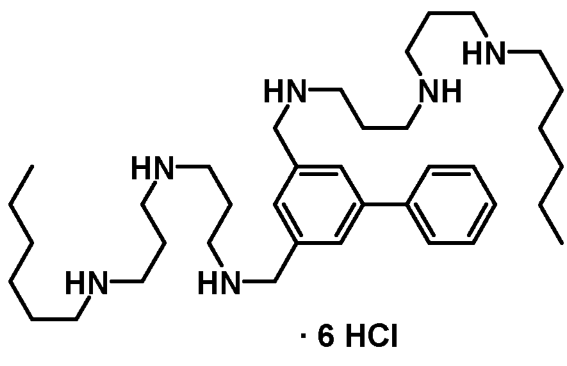

Example 78 was prepared in a similar fashion to Example 68 (CZ-86) from 5-bromoisophthaldehyde, phenylboronic acid and $N^1$-(3-aminopropyl)-$N^3$-hexylpropane-1,3-diamine. $^1H$ NMR (500 MHz, $D_2O$) δ 7.83 (s, 2H), 7.71 (d, J = 7.5 Hz, 2H), 7.55-7.53 (m, 3H), 7.47 (t, J = 6.5 Hz, 1H), 4.34 (s, 4H), 3.25 (t, J =8 Hz, 4H), 3.19 (q, J = 8 Hz, 8H), 3.13 (t, J = 8 Hz, 4H), 3.04 (t, J = 8.5 Hz, 4H), 2.22-2.09 (m, 8H), 1.66 (p, J = 7.5 Hz, 4H), 1.36-1.31 (m, 4H), 1.28-1.27 (m, 8H), 0.85 (t, J = 7 Hz, 6H). $^{13}C$ NMR (125 MHz, $D_2O$) δ 142.5, 139.0, 132.3, 130.3, 129.8, 129.5, 128.7, 127.3, 51.0, 48.1, 44.9, 44.4, 44.4, 30.6, 25.6, 25.5, 22.9, 22.8, 21.9, 13.5. LRMS $[M+H]^+$ 609.5.

**Example 79**

*Preparation of $N^1$,$N^{1'}$-([1,1'-biphenyl]-3,5-diylbis(methylene))bis(dodecane-1,12-diamine), hydrochloride salt* (CZ-115)

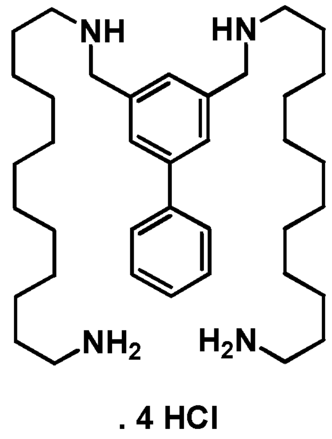

Example 79 was prepared in a similar fashion to Example 68 (CZ-86) from 5-bromoisophthaldehyde, phenylboronic acid and tert-butyl (12-aminododecyl)carbamate. $^1$H NMR (500 MHz, $D_2O$) δ 7.92 (s, 2H), 7.80 (d, J = 8.5 Hz, 2H), 7.61-7.64 (m, 3H), 7.57-7.54 (m, 1H), 4.41 (s, 4H), 3.10 (t, J =7.5 Hz, 4H), 3.03 (t, J = 7.5 Hz, 4H), 1.77-1.66 (m, 8H), 1.39-1.36 (m, 12H), 1.35-1.28 (m, 22H). $^{13}$C NMR (125 MHz, $D_2O$) δ 142.5, 139.0, 132.3, 130.3, 129.8, 129.5, 128.7, 127.3, 51.0, 48.1, 44.9, 44.4, 44.4, 30.6, 25.6, 25.5, 22.9, 22.8, 21.9, 13.5. LRMS $[M+H]^+$ 579.5.

**Example 80**

*Preparation of $N^1$,$N^{1'}$-([1,1'-biphenyl]-3,5-diylbis(methylene))bis($N^{12}$-isobutyldodecane-1,12-diamine), hydrochloride salt* (CZ-116)

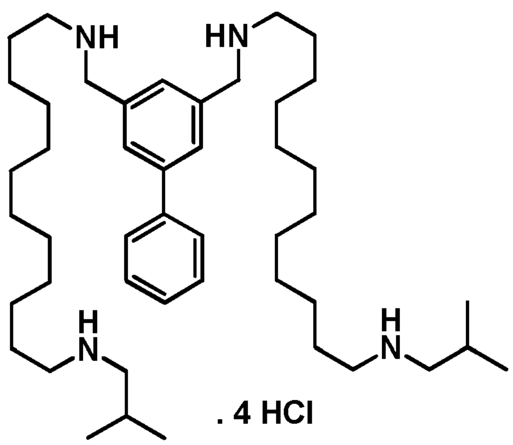

Example 80 was prepared in a similar fashion to Example 68 (CZ-86) from 5-bromoisophthaldehyde, phenylboronic acid and *N*$^1$-sobutyldodecane-1,12-diamine. $^1$H NMR (500 MHz, $D_2O$ + $CD_3OD$) δ 7.90 (s, 2H), 7.76 (d, J = 7.5 Hz, 2H), 7.60-7.56 (m, 3H), 7.52 (t, J = 7.5 Hz, 1H), 4.38 (s, 4H), 3.04 (t, J =8.0 Hz, 4H), 3.01 (t, J = 8.5 Hz, 4H), 2.88 (d, J = 7.5, 4H), 2.06-1.98 (m, 2H), 1.74-1.65 (m, 8H), 1.33-1.30 (m, 12H), 1.28-1.23 (m, 20H), 1.01 (d, J = 7.5, 12H). $^{13}$C NMR (125 MHz, $D_2O$ + $CD_3OD$) δ 142.5, 138.8, 132.4, 130.1, 129.5, 129.3, 128.5, 127.0, 51.1, 46.6, 39.5, 28.6, 28.5, 28.4, 28.2, 28.0, 26.7, 25.6, 25.6, 25.2. LRMS $[M+H]^+$ 691.6.

**Example 81**

*Preparation of $N^1$,$N^{1'}$,$N^{1''}$-([1,1'-biphenyl]-3,3'5-triyltris(methylene))tris($N^{12}$-isobutyldodecane-1,12-diamine)* (CZ-117)

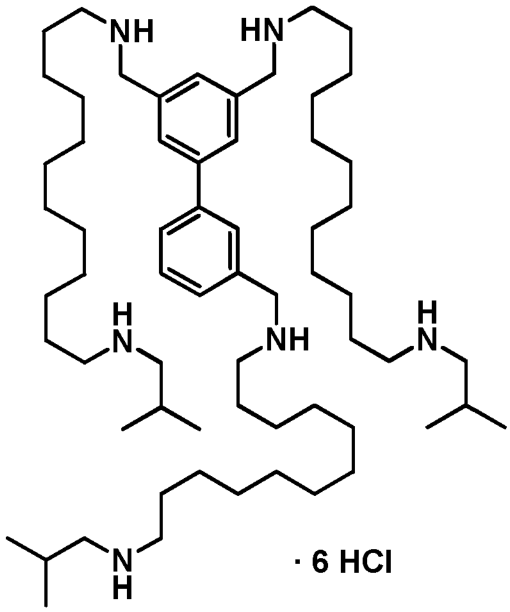

Example 81 was prepared in a similar fashion to Example 68 (CZ-86) from 5-bromoisophthaldehyde, 3-formylphenylboronic acid and $N^1$-isobutyldodecane-1,12-diamine. $^1$H NMR (500 MHz, $D_2O$) δ 7.97 (s, 2H), 7.89 (d, J = 8.0 Hz, 2H), 7.69-7.66 (m, 3H), 4.43 (s, 4H), 4.37 (s, 2H), 3.14-3.05 (m, 12H), 2.94 (d, J = 7.0, 6H), 2.10-2.01 (m, 3H), 1.78-1.71 (m, 12H), 1.39-1.26 (m, 48H), 1.50 (d, J = 6.5, 18H). 13C NMR (125 MHz, $D_2O$) δ 144.1, 142.5, 135.1, 133.4, 133.2, 133.1, 132.5, 132.1, 130.3, 130.3, 57.0, 52.9, 52.7, 50.6, 49.4, 49.3, 31.2, 31.1, 31.1, 31.0, 30.6, 28.3, 28.3, 28.2, 28.0, 27.8, 21.7. LRMS $[M+H]^+$ 960.0.

**Example 82**

*Preparation of $N^1$,$N^{1'}$-([1,1'-biphenyl]-3,5-diylbis(methylene))bis($N^4$-(3-(hexylamino)propyl)butane-1,4-diamine), hydrochloride salt* (CZ-118)

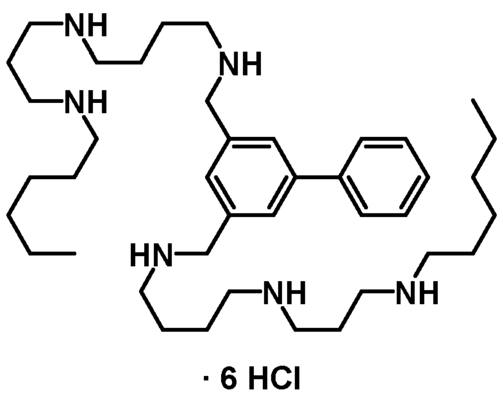

Example 82 was prepared in a similar fashion to Example 68 (CZ-86) from 5-bromoisophthaldehyde, phenylboronic acid and $N^1$-(3-aminopropyl)-$N^3$-hexylpropane-1,3-diamine. $^1$H NMR (500 MHz, $D_2O$) δ 7.87 (s, 2H), 7.76 (d, J = 7.5 Hz, 2H), 7.58 (t, J = 8.0 Hz, 3H), 7.51 (t, J = 7.5 Hz, 1H), 4.37 (s, 4H), 3.21-3.14 (m, 12H), 3.07 (t, J = 7.0 Hz, 4H),

2.16-2.09 (m, 4H), 1.90-1.77 (m, 10H), 1.69 (quint, J = 7 Hz, 4H), 1.39-1.37 (m, 4H), 1.32-1.31 (m, 10H), 0.86 (t, J = 7 Hz, 6H). $^{13}C$ NMR (125 MHz, $D_2O$) δ 142.6, 139.1, 132.6, 130.2, 129.7, 129.5, 128.7, 127.3, 50.9, 48.1, 47.2, 46.7, 44.7, 44.5, 30.6, 25.6, 25.5, 23.0, 23.0, 22.8, 21.9, 13.4. LRMS $[M+H]^+$ 637.6.

**Example 83**

*Preparation of N¹,N¹'-((5-(2-ethylbutoxy)-1,3-phenylene)bis(methylene))bis(N³-(3-(isobutylammonio)propyl)propane-1,3-diaminium) chloride* (CZ-91)

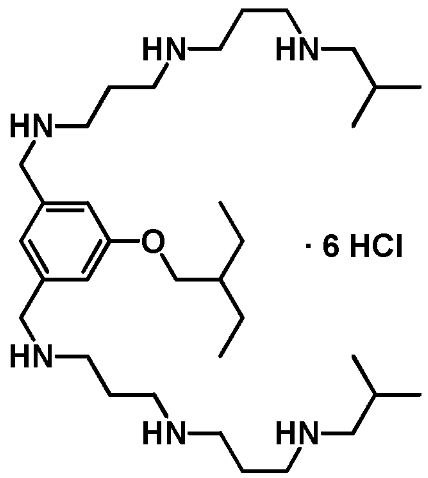

Step 1: *Dimethyl 5-(2-ethylbutoxy)isophthalate.* Dimethyl 5-hydroxyisophthalate (0.44 g, 2.10 mmol) cesium carbonate (1.37 g, 4.20 mmol) and $CH_3CN$ (20 mL) were added to a round-bottom flask and stirred for 15-30 min. 3-(Bromomethyl)pentane (0.42 g, 2.52 mmol) was added and the reaction was stirred for 16 h. The solvent was removed under reduced pressure and the reaction mixture was partitioned between EtOAc (50 mL) and $H_2O$ (50 mL). The layers were separated and the aqueous layer was extracted with EtOAc (50 mL). The combined organics were dried over $Na_2SO_4$, filtered, and concentrated under reduced pressure to afford the desired product which was used without further purification.

Step 2: *(5-(2-Ethylbutoxy)-1,3-phenylene)dimethanol.* To a solution of dimethyl 5-(2-ethylbutoxy)isophthalate (0.62 g, 2.10 mmol) in THF (20 mL) was added $LiAlH_4$ (0.24 g, 6.32 mmol). The reaction mixture was stirred for 8 h and subsequently quenched with 2N HCl (10 mL) and extracted with $Et_2O$ (2 x 25 mL) and EtOAc (2 x 25 mL). The combined organics were dried over $Na_2SO_4$, filtered, and concentrated under reduced pressure to afford the desired product which was used without further purification.

Step 3: *5-(2-Ethylbutoxy)isophthalaldehyde.* (5-(2-Ethylbutoxy)-1,3-phenylene)dimethanol (0.50 g, 2.10 mmol) and $CH_2Cl_2$ (10 mL) were added to a round-bottom flask. To this solution was added PCC (1.35 g, 6.30 mmol), and the reaction was stirred for 16 h. The reaction mixture was concentrated under reduced pressure. Purification by column chromatography (90% hexanes/EtOAc) afforded the desired product (0.13 g, 25%, 3 steps) as a white semi-solid. $^1H$ NMR (500 MHz, $CDCl_3$) δ ppm 10.01 (s, 2H), 7.90 (s, 1H), 7.62 (s, 2H), 3.94 (d, *J*= 5.5 Hz, 2H), 1.68 (hept, *J*= 6.5 Hz, 1H), 1.46 (dec, *J*= 6.5 Hz, 4H), 0.91 (t, J = 7.5 Hz, 6H). $^{13}C$ NMR (125 MHz, $CDCl_3$) δ ppm 191.1, 160.7, 138.5, 124.1, 120.0, 71.1, 40.9, 23.5, 11.3.

Step 4: *N¹,N¹'-((5-(2-Ethylbutoxy)-1,3-phenylene)bis(methylene))bis(N³-(3-(isobutylamino)propyl) propane-1,3-diamine).* 5-(2-Ethylbutoxy)isophthalaldehyde (0.20 g, 0.88 mmol) and MeOH (15 mL) were added to a round-bottom flask. To the solution was added *N*¹-(3-aminopropyl)-*N*³-isobutylpropane-1,3-diamine (0.33 g, 1.76 mmol) and the reaction mixture was stirred for 24 h. Sodium borohydride (0.13 g, 3.52 mmol) was added and the reaction mixture stirred for 1 h. The reaction mixture was concentrated under reduced pressure to afford a white solid. Aqueous NaOH (10%, 50 mL) and EtOAc (50 mL) were added and the reaction mixture stirred for 1 h. The layers were separated and the aqueous layer was extracted with EtOAc (50 mL). The organic layers were combined, dried over $Na_2SO_4$, filtered, and concentrated under reduced pressure to afford the desired product a clear oil.

Step 5: *N¹,N¹'-((5-(2-Ethylbutoxy)-1,3-phenylene)bis(methylene))bis(N³-(3-(isobutylammonio)propyl)propane-1,3-diaminium) chloride.* To *N*¹,*N*¹'-((5-(2-ethylbutoxy)-1,3-phenylene)bis(methylene))bis(*N*³-(3-(isobutylamino)propyl) propane-1,3-diamine) from step 4 was which was added methanolic HCl (50 mL, 1.0M). The reaction mixture was stirred

for 1 h, the reaction mixture was concentrated under reduced pressure and the solid collected by vacuum filtration. The solid was washed with $Et_2O$ (10 mL) and hot MeOH (10 mL) to afford the desired product (0.42 g, 59%) as a white solid. $^1H$ NMR (500 MHz, $D_2O$) δ 7.20 (s, 2H), 7.18 (s, 1H), 4.29 (s, 4H), 4.05 (d, J = 6.0 Hz, 2H), 3.25-3.15 (m, 16H), 2.94 (d, J = 7.0 Hz, 2H), 2.21-2.12 (m, 8H), 2.07-1.99 (m, 2H), 1.72 (p, J = 6.5 Hz, 1H), 1.50-1.43 (m, 4H), 1.00 (d, J = 7.0 Hz, 12H), 0.92 (t, J = 6.0 Hz, 6H). $^{13}C$ NMR (125 MHz, $D_2O$) δ 159.6, 132.9, 123.4, 117.4, 71.4, 54.8, 50.8, 44.7, 44.6, 44.6, 44.1, 40.1, 25.5, 22.6, 22.5, 19.0, 10.2. LRMS $[M+H]^+$ 577.6.

**Example 84**

*Preparation of $N^1$,$N^{1'}$-((5-isopropoxy-1,3-phenylene)bis(methylene))bis($N^3$-(3-aminopropyl)propane-1,3-diamine), hydrochloride salt* (CZ-93)

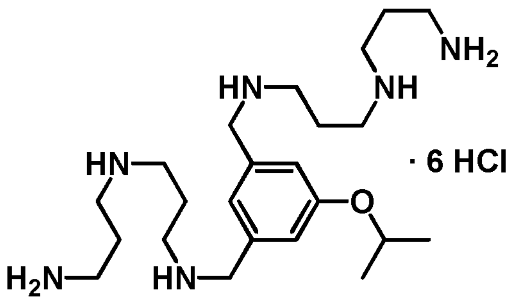

Example 84 was prepared in a similar fashion to Example 83 (CZ-91) from 5-isopropoxyisophthalaldehyde and tert-butyl (3-((3-aminopropyl)amino)propyl)carbamate. $^1H$ NMR (300 MHz, $D_2O$) δ 7.13 (s, 3H), 4.78-4.72 (m, 1H), 4.23 (s, 4H), 3.27-3.18 (m, 12H), 3.13 (t, J = 7.8 Hz, 4H), 2.23-2.18 (m, 4H), 2.18-2.10 (m, 4H), 1.36 (d, J = 10.2Hz, 6H).

**Example 85**

*Preparation of $N^1$,$N^{1'}$-((5-(cyclohexylmethoxy)-1,3-phenylene)bis(methylene))bis($N^3$-(3-(octylamino)propyl)propane-1,3-diamine), hydrochloride salt* (CZ-103)

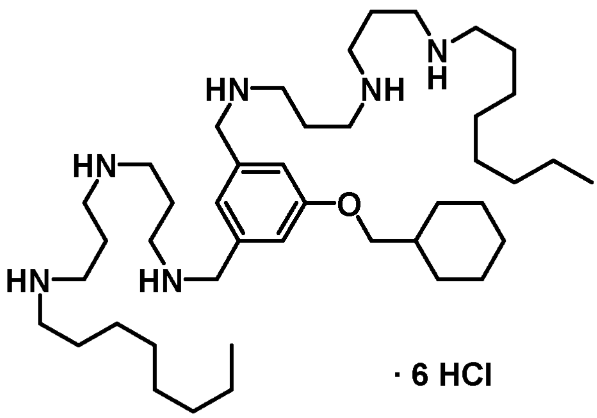

Example 85 was prepared in a similar fashion to Example 83 (CZ-91) from dimethyl 5-butoxyisophthalate, (bromomethyl)cyclohexane, tert-butyl (3-((3-aminopropyl)amino)propyl)carbamate and octanal. $^1H$ NMR (500 MHz, $D_2O$) δ 7.18 (s, 3H), 4.28 (s, 4H), 3.95 (s, 2H) 3.20-3.14 (m, 16H), 3.06 (t, J = 7.5 Hz, 4H), 2.22-2.10 (m, 8H), 1.88-1.80 (m, 3H), 1.76-1.64 (m, 8H), 1.41-1.20 (m, 22H), 1.12-1.03 (m, 2H), 0.89-0.83 (m, 6H). $^{13}C$ NMR (125 MHz, $D_2O$) δ 159.5, 133.0, 123.4, 117.3, 74.4, 50.8, 47.8, 44.6, 44.6, 44.2, 44.1, 36.8, 30.9, 29.1, 28.1, 28.0, 25.9, 25.6, 25.4, 25.2, 22.6, 22.6, 21.9, 13.3. LRMS $[M+H]^+$ 701.7.

**Example 86**

*Preparation of $N^1$,$N^{1'}$-((5-(cyclohexyloxy)-1,3-phenylene)bis(methylene))bis($N^3$-(3-(isobutylamino)propyljpropane-1,3-diamine), hydrochloride salt* (CZ-104)

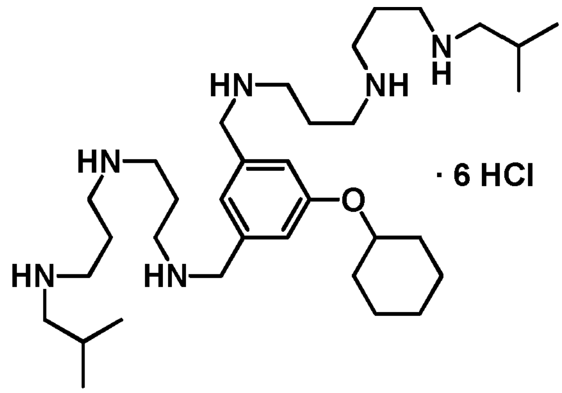

Example 86 was prepared in a similar fashion to Example 83 (CZ-91) from 5-(cyclohexyloxy)isophthalaldehyde and $N^1$-(3-aminopropyl)-$N^3$-isobutylpropane-1,3-diamine. $^1$H NMR (300 MHz, $D_2O$) δ 7.60 (s, 2H), 7.53 (s, 1H), 4.73 (t, J = 1.2 Hz, 1H), 4.42 (s, 4H), 3.26-3.13 (m, 16H), 2.84 (d, J = 6.9 Hz, 2H), 2.23-2.08 (m, 8H), 2.06-1.96 (m, 2H), 1.87-1.78 (m, 4H), 1.65-1.36 (m, 6H), 0.99 (d, J = 6.6 Hz, 12H). LRMS $[M+H]^+$ 575.5.

**Example 87**

*Preparation of $N^1$,$N^{1'}$-((5-(cyclohexyloxy)-1,3-phenylene)bis(methylene))bis($N^3$-(3-aminopropyl)propane-1,3-diamine), hydrochloride salt* (CZ-105)

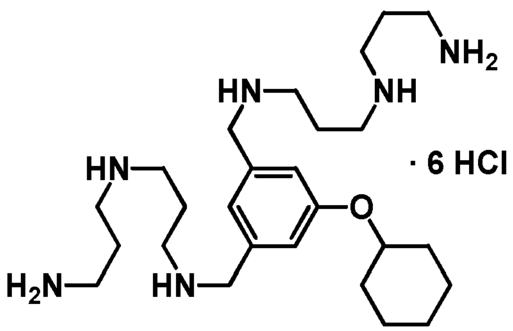

Example 87 was prepared in a similar fashion to Example 83 (CZ-91) from 5-(cyclohexyloxy)isophthalaldehyde and tert-butyl (3-((3-aminopropyl)amino)propyl)-carbamate. $^1$H NMR (300 MHz, $D_2O$) δ 7.22 (s, 2H), 7.18 (s, 1H), 4.68 (t, J = 1.2 Hz, 1H), 4.30 (s, 4H), 3.28-3.18 (m, 12H), 3.14 (t, J = 7.8 Hz, 4H), 2.21-2.06 (m, 8H), 1.84-1.75 (m, 4H), 1.63-1.34 (m, 6H). LRMS $[M+H]^+$ 463.5.

**Example 88**

*Preparation of $N^1$,$N^{1'}$-((5-(benzyloxy)-1,3-phenylene)bis(methylene))bis($N^3$-(3-(isobutylamino)propyl)propane-1,3-diamine), hydrochloride salt* (CZ-106)

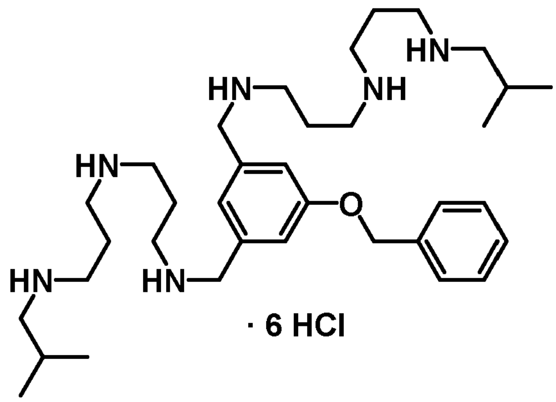

Example 88 was prepared in a similar fashion to Example 83 (CZ-91) from dimethyl 5-butoxyisophthalate, benzyl bromide, tert-butyl (3-((3-aminopropyl)amino)-propyl)carbamate and isobutyraldehyde.

**Example 89**

*Preparation of $N^1$,$N^{1'}$-(1,3-phenylenebis(methylene))bis($N^3$-(3-((cyclohexylmethyl)-amino)propyl)propane-1,3-diamine), hydrochloride salt* (CZ-97)

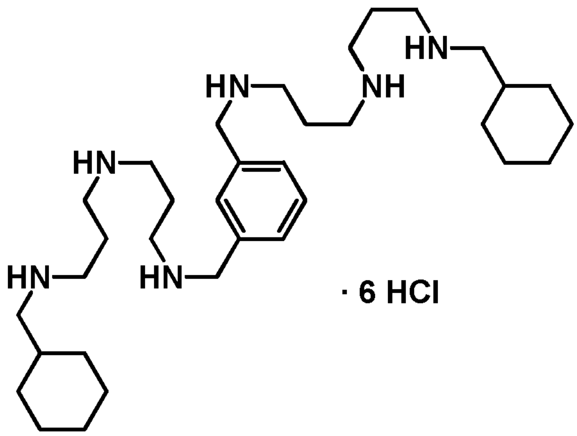

Step 1: *$N^1$,$N^{1'}$-(1,3-Phenylenebis(methylene))bis($N^3$-(3-((cyclohexylmethyl)-amino)propyl)propane-1,3-diamine).* Isophthalaldehyde (0.34 g, 2.57 mmol, 1) and MeOH (20 mL) were added to a round-bottom flask. To the solution was added $N^1$-(3-aminopropyl)-$N^3$-(cyclohexylmethyl)propane-1,3-diaminediamine (1.17 g, 5.14 mmol, 2) and the reaction mixture was stirred at rt for 24 h. Sodium borohydride (0.39 g, 10.28 mmol, 4) was added and the reaction mixture stirred for 1 h. The reaction mixture was concentrated under reduced pressure to afford a white solid. Aqueous NaOH (10%, 100 mL) and EtOAc (100 mL) were added and the reaction mixture stirred for 1 h. The layers were separated and the aqueous layer was extracted with EtOAc (100 mL). The organic layers were combined, dried over $Na_2SO_4$, filtered, and concentrated under reduced pressure to afford a clear oil which was used without further purification.

Step 2: *$N^1$,$N^{1'}$-(1,3-phenylenebis(methylene))bis($N^3$-(3-((cyclohexylmethyl)amino)-propyl)propane-1,3-diamine) hydrochloride salt.* To the crude $N^1$,$N^{1'}$-(1,3-phenylenebis-(methylene))bis($N^3$-(3-((cyclohexylmethyl)amino)propyl)propane-1,3-diamine) from Step 1 was added methanolic HCl (100 mL, 1.0M). The reaction mixture stirred at rt for 2 h. The reaction mixture was concentrated under reduced pressure and the solid was collected by vacuum filtration and washed with $Et_2O$ (50 mL) and hot MeOH (50 mL) to afford the desire product (1.3 g, 67%) as a white solid. $^1H$ NMR (500 MHz, $D_2O$) $\delta$ 7.57 (s, 4H), 4.30 (s, 4H), 3.22 (t, J = 7.5 Hz, 4H), 3.18-3.15 (m, 8H), 3.12 (t, J = 8.0 Hz, 4H), 2.91 (d, J = 7.0 Hz, 4H), 2.18-2.08 (m, 8H), 1.72-1.62 (m, 12H), 1.28-1.11 (m, 6H), 1.03-0.96 (m, 4H). $^{13}C$ NMR (125 MHz, $D_2O$) $\delta$ 131.4, 131.2, 131.0, 130.2, 53.7, 50.8, 48.8, 44.7, 44.6, 44.0, 34.5, 29.7, 25.3, 24.8, 22.6, 22.5. LRMS $[M+H]^+$ 557.5.

**Example 90**

*Preparation of N[1],N[1]'-(1,3-phenylenebis(methylene))bis(N[3]-(3-(octylamino)propyl)propane-1,3-diamine), hydrochloride salt* (CZ-100)

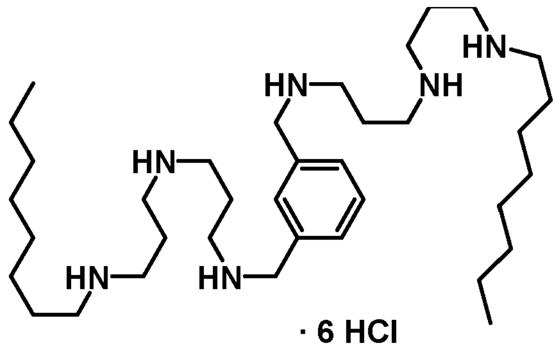

Example 90 was prepared in a similar fashion to Example 62 (CZ-76) from isophthaldehyde, tert-butyl (3-((3-aminopropyl)amino)propyl)carbamate and octanal. $^1$H NMR (500 MHz, $D_2O$) δ 7.58 (s, 4H), 4.32 (s, 4H), 3.10-2.98 (m, 16H), 2.91 (t, J = 8.0 Hz, 4H), 2.05-1.95 (m, 8H), 1.53 (p, J = 7 Hz, 4H), 1.24-1.13 (m, 20H), 0.71 (t, J = 7.0 Hz, 6H). $^{13}$C NMR (125 MHz, $D_2O$) δ 131.4, 131.2, 131.1, 130.2, 50.8, 47.9, 44.6, 44.6, 44.2, 44.1, 30.9, 28.1, 28.1, 25.6, 25.4, 22.6, 21.9, 13.3. LRMS $[M+H]^+$ 589.5.

**Example 91**

*Preparation of N[1],N[1]'-(1,3-phenylenebis(methylene))bis(N[3]-(3-(isobutylamino)propyl)propane-1,3-diamine), hydrochloride salt* (CZ-108)

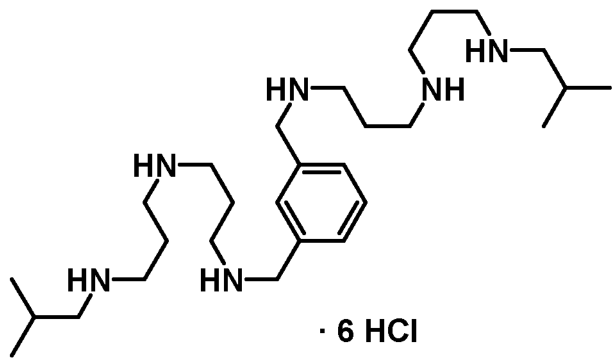

Example 91 was prepared in a similar fashion to Example 89 (CZ-97) from isophthaldehyde and *N*[1]-(3-aminopropyl)-*N*[3]-isobutylpropane-1,3-diamine. $^1$H NMR (300 MHz, $D_2O$) δ 7.54 (s, 4H), 4.28 (s, 4H), 3.19-3.08 (m, 16H), 2.88 (d, *J*= 7.5 Hz, 4H), 2.15-2.05 (m, 8H), 2.02-1.90 (m, 2H), 0.95 (d, *J*= 6.9 Hz, 12H). LRMS $[M+H]^+$ 477.4.

**Example 92**

*Preparation of N[1],N[1]'-((5-bromo-1,3-phenylene)bis(methylene))bis(N[3]-(3-(isobutylamino)propyl)propane-1,3-diamine), hydrochloride salt* (CZ-109)

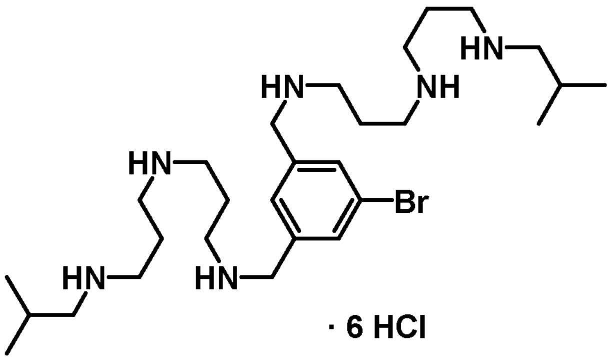

Example 92 was prepared in a similar fashion to Example 89 (CZ-97) from 4-bromo-isophthaldehyde and *N*1-(3-aminopropyl)-*N*3-isobutylpropane-1,3-diamine. $^1$H NMR (300 MHz, $D_2O$) δ 7.76 (s, 2H), 7.51 (s, 1H), 4.27 (s, 4H), 3.21-3.08 (m, 16H), 2.88 (d, *J* = 7.5 Hz, 4H), 2.16-2.06 (m, 8H), 2.02-1.93 (m, 2H), 0.96 (d, *J* = 6.6 Hz, 12H). LRMS $[M+H]^+$ 555.4, 557.4.

**Example 93**

*Preparation of N1,N1'-(1,3-phenylenebis(methylene))bis(N3-(3-(hexylamino)propyl)propane-1,3-diamine), hydrochloride salt* (CZ-113)

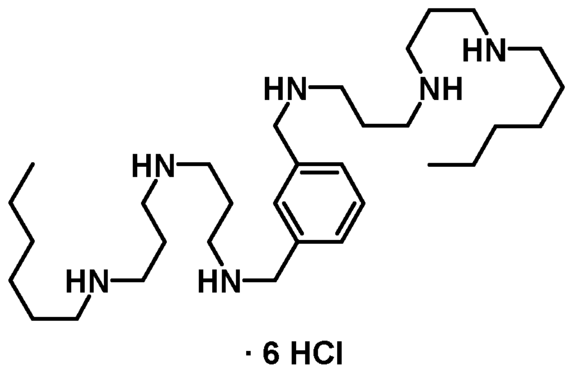

Example 93 was prepared in a similar fashion to Example 89 (CZ-97) from isophthaldehyde and *N*1-(3-aminopropyl)-*N*3-hexylpropane-1,3-diamine. $^1$H NMR (500 MHz, $D_2O$) δ 7.58 (s, 3H), 4.32 (s, 4H), 3.24-3.13 (m, 16H), 3.05 (t, J = 8.0 Hz, 4H), 2.20-2.09 (m, 8H), 1.68-1.63 (m, 4H), 1.38-1.33 (m, 4H), 1.32-1.26 (m, 8H), 0.85 (t, J = 7.5 Hz, 6H). $^{13}$C NMR (125 MHz, $D_2O$) δ 131.6, 131.5, 131.4, 130.4, 51.1, 48.1, 44.9, 44.8, 44.5, 44.3, 30.6, 25.6, 25.5, 22.8, 21.9, 13.5. LRMS $[M+H]^+$ 533.6.

**Example 94**

*Preparation of N1,N1'-(1,3-phenylenebis(methylene))bis(N4-(3-(hexylamino)propyl)butane-1,4-diamine), hydrochloride salt* (CZ-119)

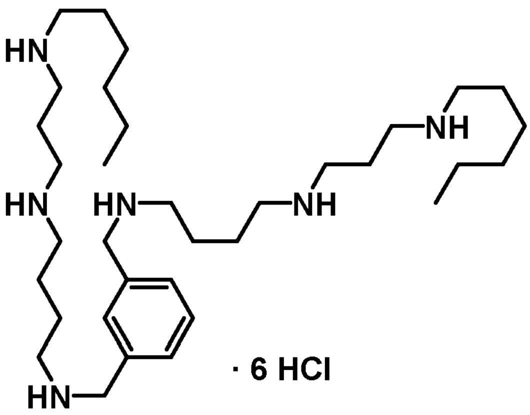

Example 94 was prepared in a similar fashion to Example 89 (CZ-97) from isophthaldehyde and *N*[1]-(3-aminopropyl)-*N*[4]-hexylbutane-1,4-diamine. $^1$H NMR (500 MHz, $D_2O$) δ 7.65 (s, 4H), 4.37 (s, 4H), 3.26-3.19 (m, 16H), 3.14 (t, *J* = 7.5 Hz, 4H), 2.20 (quint, *J* = 8.0 Hz, 4H), 1.88 (br s, 8H), 1.75 (quint, *J* = 8.0 Hz, 4H), 1.46-1.42 (m, 4H), 1.39-1.36 (m, 8H), 0.94 (t, *J* = 6.5 Hz, 6H). $^{13}$C NMR (125 MHz, $D_2O$) δ 134.2, 133.7, 133.5, 132.7, 53.3, 50.4, 49.6, 49.1, 47.1, 46.8, 32.9, 27.9, 27.8, 25.4, 25.3, 25.2, 24.2, 15.8. LRMS $[M+H]^+$ 561.5.

**Example 95**

*Preparation of N$^{1,}$N$^{1'}$,N$^{1''}$-([1,1'-biphenyl]-3,3',5-triyltris(methylene))tris(N$^3$-(3-(isobutylamino)propyl)propane-1,3-diamine), hydrochloride salt* (CZ-99)

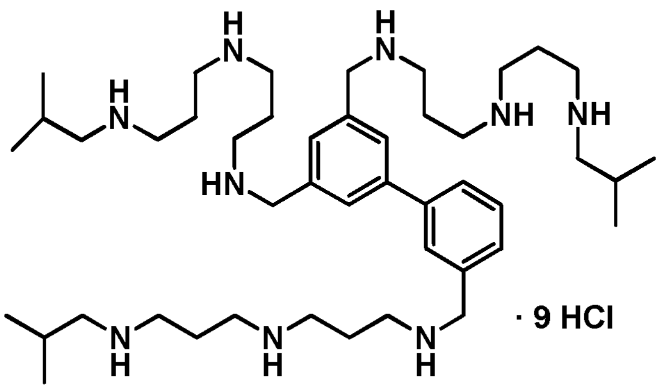

Step 1: *[1,1'-Biphenyl]-3,3',5-tricarbaldehyde.* A solution of 5-bromoisophthaldehyde (2.13 g, 10.0 mmol), 3-formylphenylboronic acid (1.49 g, 10.0 mmol) and potassium carbonate (2.76 g, 20.0 mmol) in DME/$H_2O$ (5:1 50 mL) was purged with $N_2$ for 5 min. Tetrakispalladium triphenylphosphine (0.12 g, 0.1 mmol) was added and the reaction mixture was heated to 90 °C and stirred for 16 h. The reaction mixture was cooled, filtered through a pad of Celite diatomaceous earth and the solvent evaporated under reduced pressure. Purification by column chromatography (10% EtOAc/hexanes) afforded the desired product (1.47 g, 62%) as a tan solid. $^1$H NMR (300 MHz, $CDCl_3$) δ ppm 10.20 (s, 2H), 10.10 (s, 1H), 8.42 (s, 3H), 8.21 (s, 1H), 7.98 (t, *J* = 5.4 Hz, 2H), 7.72 (t, *J* = 5.4 Hz, 2H).

Step 2: *N$^1$,N$^{1'}$,N$^{1''}$-([1,1'-Biphenyl]-3,3',5-triyltris(methylene))tris(N$^3$-(3-(isobutylamino)propyl)propane-1,3-diamine).* [1,1'-biphenyl]-3,3',5-tricarbaldehyde (0.24 g, 1.0 mmol) and MeOH (20 mL) were added to a round-bottom flask. To the solution was added *N*[1]-(3-aminopropyl)-*N*[3]-isobutylpropane-1,3-diamine (570 mg, 3.0 mmol, 3 equiv.) and the reaction mixture was stirred at rt for 24 h. Sodium borohydride (0.34 g, 9.0 mmol) was added and the reaction mixture stirred for 1 h. The reaction mixture was concentrated under reduced pressure to afford a white solid. Aqueous NaOH (10%, 100 mL) and EtOAc (100 mL) were added and the reaction mixture stirred for 1 h. The layers were separated and the aqueous layer was extracted with EtOAc (100 mL). The organic layers were combined, dried over $Na_2SO_4$, filtered, and concentrated under reduced pressure to afford a clear oil which was used without further purification.

Step 3: *N$^1$,N$^{1'}$,N$^{1''}$-([1,1'-Biphenyl]-3,3',5-triyltris(methylene))tris(N$^3$-(3-(isobutylamino)propyl)propane-1,3-di-*

*amine), hydrochloride salt.* *N*1,*N*1',*N*1''-([1,1'-biphenyl]-3,3',5-triyltris(methylene))tris(*N*3-(3-(isobutylamino)propyl)propane-1,3-diamine) from step 2 was subjected to acidification with methanolic HCl (100 mL, 1.0M). The reaction mixture stirred at rt for 2 h. The reaction mixture was concentrated under reduced pressure and the solid was collected by vacuum filtration and washed with $Et_2O$ (50 mL) and hot MeOH (50 mL) to afford the desired product (0.58 g, 52%) as a white solid. $^1H$ NMR (500 MHz, $D_2O$) $\delta$ ppm 7.91 (s, 1H), 7.85-7.82 (m, 3H), 7.65-7.56 (m, 3H), 4.42 (s, 4H), 4.39 (s, 2H), 3.29-3.16 (m, 24H), 2.93 (d, J = 6.0 Hz, 6H), 2.20-2.13 (m, 12H), 2.07-2.00 (m, 3H), 0.99 (d, J = 6.0 Hz, 18H). $^{13}$CNMR (125 MHz, $D_2O$) $\delta$ ppm 141.6, 139.8, 132.2, 131.3, 130.4, 130.3, 129.7, 129.5, 128.6, 128.4, 54.8, 54.8, 51.1, 50.8, 44.7, 44.6, 44.2, 44.0, 25.5, 22.7,22.5, 19.0. LRMS $[M+2H^+]^{2+}$ 376.8.

**Example 96**

*Preparation of N*1*,N*1*-((5-(3-(isoButylammonio)propoxy)-1,3-phenylene)bis(methylene))-bis(N*3*-(3-ammoniopropyl)propane-1,3-diaminium) chloride* (CZ-107)

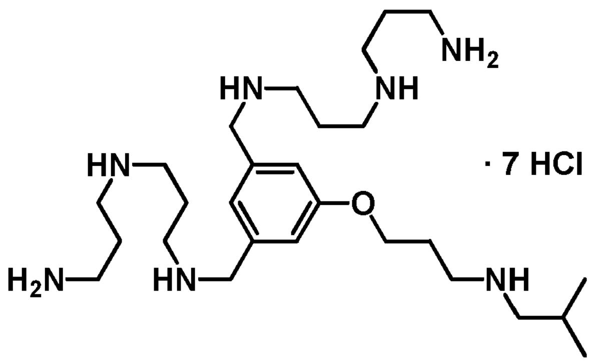

Step 1: *tert-Butyl (3-hydroxypropyl)(isobutyl)carbamate.* 3-(*iso*-Butylamino)propan-1-ol (7.92 g, 60.5 mmol) and THF (100 mL) were added to a round-bottom flask. To the solution was added aqueous NaOH (10%, 100 mL) followed by the slow addition of di-tert-butyl dicarbonate (11.86 g, 54.4 mmol). The reaction mixture was stirred for 12 h. The reaction mixture was extracted with $CH_2Cl_2$ (3 x 100 mL). The combined organics were dried over $Na_2SO_4$, filtered, and concentrated under reduced pressure to afford the desired product (12.0 g, 86%) which was used without further purification.

Step 2: *3-((tert-Butoxycarbonyl)(isobutyl)amino)propyl methanesulfonate.* *tert*-Butyl (3-hydroxypropyl)(isobutyl)carbamate (0.73 g, 3.15), triethylamine (0.64 g, 6.30 mmol) and $CH_2Cl_2$ (30 mL) were added to a round-bottom flask. To the solution was added methanesulfonyl chloride (0.54 g, 4.72 mmol) and the reaction mixture was stirred for 5 h. The reaction mixture was concentrated under reduced pressure and purified by column chromatography (hexanes/EtOAC) to afford the desired product (0.63 g, 64%) as a yellow oil.

Step 3: *Dimethyl 5-(3-((tert-butoxycarbonyl)(isobutyl)amino)propoxy)isophthalate.* Dimethyl 5-hydroxyisophthalate (0.30 g, 1.44 mmol) cesium carbonate (0.95 g, 2.88 mmol) and $CH_3CN$ (25 mL) were stirred for 30 min. 3-((*tert*-Butoxycarbonyl)(isobutyl)amino)-propyl methanesulfonate (0.63 g, 2.02 mmol) was added and the reaction mixture was stirred for 16 h. The reaction mixture was concentrated under reduced pressure and partitioned between EtOAc (50 mL) and $H_2O$ (50 mL). The layers were separated and the aqueous layer was extracted with EtOAc (50 mL). The combined organics were dried over $Na_2SO_4$, filtered, and concentrated under reduced pressure to afford the desired product, which was used without further purification.

Step 4: *tert-Butyl (3-(3,5-bis(hydroxymethyl)phenoxy)propyl)(isobutyl)carbamate.* To a solution of dimethyl 5-(3-((tert-butoxycarbonyl)(isobutyl)amino)propoxy)isophthalate (0.60 g, 1.44 mmol) in THF (10 mL) was added $LiAlH_4$ (0.30 g, 7.89 mmol). The reaction mixture was stirred for 8 h and subsequently quenched with 2N HCl (10 mL) and extracted with $Et_2O$ (2 x 25 mL) and EtOAc (2 x 25 mL). The combined organic layers were dried over $Na_2SO_4$, filtered, and concentrated under reduced pressure to afford the desired product (0.28 g, 54%) as an oil, which was used without further purification.

Step 5: *tert-Butyl (3-(3,5-diformylphenoxy)propyl)(isobutyl)carbamate.* *tert*-Butyl (3-(3,5-bis(hydroxymethyl)phenoxy)propyl)(isobutyl)carbamate (0.29 g, 0.78 mmol) and $CH_2Cl_2$ (20 mL) were added to a round-bottom flask. To the solution was added PCC (0.42 g, 1.94 mmol) and the reaction was stirred for 16 h. The reaction mixture was concentrated under reduced pressure. Purification by column chromatography (90% Hexanes/EtOAc) afforded the desired product (0.15 g, 20% 3 steps) as a white semi-solid, which was used without further purification.

Step 6: *$N^1$,$N^{1'}$-((5-(3-(iso-Butylamino)propoxy)-1,3-phenylene)bis(methylene))-bis($N^3$-(3-aminopropyl)propane-1,3-diamine). tert*-Butyl (3-(3,5-diformylphenoxy)propyl)-(isobutyl)carbamate (0.15 g, 0.42 mmol) and MeOH (10 mL) were added to a round-bottom flask. To the solution was added *tert*-butyl (3-((3-aminopropyl)amino)propyl)carbamate (0.20 g, 0.84 mmol) and the reaction mixture was stirred for 24 h. Sodium borohydride (0.06 g, 1.69 mmol) was added and the reaction mixture stirred for 1 h. The reaction mixture was concentrated under reduced pressure to afford a white solid. Aqueous NaOH (10%, 50 mL) and EtOAc (50 mL) were added, the layers separated and the aqueous layer was extracted with EtOAc (50 mL). The organic layers were combined, dried over $Na_2SO_4$, filtered, and concentrated under reduced pressure to afford the desired product as clear oil which was used without further purification.

Step 7: *$N^1$,$N^{1'}$-((5-(3-(iso-Butylammonio)propoxy)-1,3-phenylene)bis(methylene))-bis($N^3$-(3-ammoniopropyl)propane-1,3-diaminium) chloride.* To $N^1$,$N^{1'}$-((5-(3-(*iso*-butylamino)propoxy)-1,3-phenylene)bis(methylene))bis($N^3$-(3-aminopropyl)propane-1,3-diamine) from step 6 was added methanolic HCl (50 mL, 1.0M). The reaction mixture was stirred for 1 h, the reaction mixture was concentrated under reduced pressure and the solid collected by vacuum filtration. The solid was washed with $Et_2O$ (10 mL) and hot MeOH (10 mL) to afford the desired product (0.14 g, 43%) as a white solid. $^1$H NMR (500 MHz, $D_2O$) δ ppm 7.21 (s, 1H), 7.19 (s, 2H), 4.30 (s, 4H), 4.23 (t, $J$ = 5.5 Hz, 2H), 3.29 (t, $J$ = 7 Hz, 2H), 3.25-3.19 (m, 12H), 3.12 (t, $J$ = 7.5 Hz, 4H), 2.95 (d, $J$ = 7 Hz, 2H), 2.25-2.03 (m, 11H), 1.01 (d, $J$ = 7.5 Hz, 6H). $^{13}$C NMR (125 MHz, $D_2O$) 158.9, 133.0, 123.7, 117.1, 65.8, 54.8, 50.7, 45.7, 44.7, 44.6, 44.2, 36.7, 25.5, 25.2, 23.7, 22.6, 19.1. LRMS $[M+H]^+$ 494.4.

**Example 97**

*Preparation of $N^1$,$N^{1'}$-((5-(3-(isobutylamino)propoxy)-1,3-phenylene)bis(methylene))bis($N^3$-(3-(octylamino)propyl)propane-1,3-diamine), hydrochloride salt* (CZ-110)

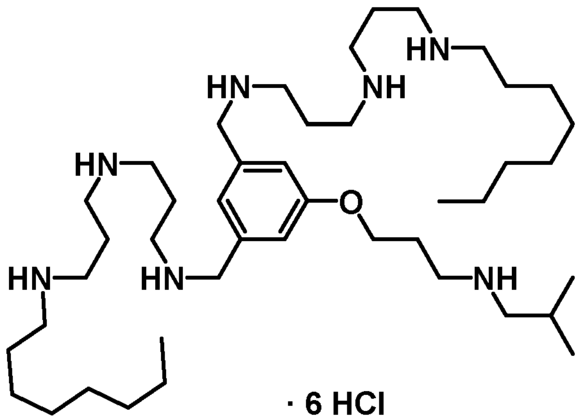

**· 6 HCl**

Step 1: *tert-Butyl (3-hydroxypropyl)(isobutyl)carbamate.* 3-(*iso*-Butylamino)propan-1-ol (7.92 g, 60.5 mmol) and THF (100 mL) were added to a round-bottom flask. To the solution was added aqueous NaOH (10%, 100 mL) followed by the slow addition of di-tert-butyl dicarbonate (11.86 g, 54.4 mmol). The reaction mixture was stirred for 12 h. The reaction mixture was extracted with $CH_2Cl_2$ (3 x 100 mL). The combined organics were dried over $Na_2SO_4$, filtered, and concentrated under reduced pressure to afford the desired product (12.0 g, 86%), which was used without further purification.

Step 2: *3-((tert-Butoxycarbonyl)(isobutyl)amino)propyl methanesulfonate. tert*-Butyl (3-hydroxypropyl)(isobutyl)carbamate (0.73 g, 3.15 ), triethylamine (0.64 g, 6.30 mmol) and $CH_2Cl_2$ (30 mL) were added to a round-bottom flask. To the solution was added methanesulfonyl chloride (0.54 g, 4.72 mmol) and the reaction mixture was stirred for 5 h. The reaction mixture was concentrated under reduced pressure and purified by column chromatography (hexanes/EtOAC) to afford the desired product (0.63 g, 64%) as a yellow oil.

Step 3: *Dimethyl 5-(3-((tert-butoxycarbonyl) (isobutyl)amino)propoxy)isophthalate*: Dimethyl 5-hydroxyisophthalate (0.30 g, 1.44 mmol) cesium carbonate (0.95 g, 2.88 mmol) and $CH_3CN$ (25 mL) were stirred for 30 min. 3-((*tert*-Butoxycarbonyl)(isobutyl)amino)-propyl methanesulfonate (0.63 g, 2.02 mmol) was added, and the reaction mixture was stirred for 16 h. The reaction mixture was concentrated under reduced pressure and partitioned between EtOAc (50 mL) and $H_2O$ (50 mL). The layers were separated, and the aqueous layer was extracted with EtOAc (50 mL). The combined organics were dried over $Na_2SO_4$, filtered, and concentrated under reduced pressure to afford the desired product, which was used without further purification.

Step 4: *tert-Butyl (3-(3,5-bis(hydroxymethyl)phenoxy)propyl)(isobutyl)carbamate.* To a solution of dimethyl 5-(3-((tert-butoxycarbonyl)(isobutyl)amino)propoxy)isophthalate (0.60 g, 1.44 mmol) in THF (10 mL) was added $LiAlH_4$ (0.30 g, 7.89 mmol). The reaction mixture was stirred for 8 h and subsequently quenched with 2N HCl (10 mL) and extracted with $Et_2O$ (2 x 25 mL) and EtOAc (2 x 25 mL). The combined organic layers were dried over $Na_2SO_4$, filtered, and concentrated under reduced pressure to afford the desired product (0.28 g, 54%) as an oil, which was used without further purification.

Step 5: *tert-Butyl (3-(3,5-diformylphenoxy)propyl)(isobutyl)carbamate. tert*-Butyl (3-(3,5-bis(hydroxymethyl)phenoxy)propyl)(isobutyl)carbamate (0.29 g, 0.78 mmol) and $CH_2Cl_2$ (20 mL) were added to a round-bottom flask. To the solution was added PCC (0.42 g, 1.94 mmol) and the reaction was stirred for 16 h. The reaction mixture was concentrated under reduced pressure. Purification by column chromatography (90% hexanes/EtOAc) afforded the desired product (0.15 g, 20% 3 steps) as a white semi-solid which was used without further purification.

Step 6: *$N^1$,$N^{1'}$-((5-(3-(iso-Butylamino)propoxy)-1,3-phenylene)bis(methylene))-bis($N^3$-(3-aminopropyl)propane-1,3-diamine). tert*-Butyl (3-(3,5-diformylphenoxy)propyl)-(isobutyl)carbamate (0.15 g, 0.42 mmol) and MeOH (10 mL) were added to a round-bottom flask. To the solution was added *tert*-butyl (3-((3-aminopropyl)amino)propyl)carbamate (0.20 g, 0.84 mmol), and the reaction mixture was stirred for 24 h. Sodium borohydride (0.06 g, 1.69 mmol) was added and the reaction mixture stirred for 1 h. The reaction mixture was concentrated under reduced pressure to afford a white solid. Aqueous NaOH (10%, 50 mL) and EtOAc (50 mL) were added, the layers separated, and the aqueous layer was extracted with EtOAc (50 mL). The organic layers were combined, dried over $Na_2SO_4$, filtered, and concentrated under reduced pressure to afford the desired product as a clear oil, which was used without further purification.

Step 7: *$N^1$,$N^{1'}$-((5-(3-(iso-Butylammonio)propoxy)-1,3-phenylene)bis(methylene))bis($N^3$-(3-ammoniopropyl)propane-1,3-diaminium) chloride.* To $N^1$,$N^{1'}$-((5-(3-(*iso*-Butylamino)propoxy)-1,3-phenylene)bis(methylene))bis($N^3$-(3-aminopropyl)propane-1,3-diamine) from step 6 was added methanolic HCl (50 mL, 1.0M). The reaction mixture was stirred for 1 h, the reaction mixture was concentrated under reduced pressure and the solid collected by vacuum filtration. The solid was washed with $Et_2O$ (10 mL) and hot MeOH (10 mL) to afford the desired product (0.14 g, 43%) as a white solid. $^1H$ NMR (500 MHz, $D_2O$) δ ppm 7.21 (s, 1H), 7.19 (s, 2H), 4.30 (s, 4H), 4.23 (t, *J* = 5.5 Hz, 2H), 3.29 (t, *J* = 7 Hz, 2H), 3.25-3.19 (m, 12H), 3.12 (t, *J* = 7.5 Hz, 4H), 2.95 (d, *J* = 7 Hz, 2H), 2.25-2.03 (m, 11H), 1.01 (d, *J* = 7.5 Hz, 6H). $^{13}C$ NMR (125 MHz, $D_2O$) 158.9, 133.0, 123.7, 117.1, 65.8, 54.8, 50.7, 45.7, 44.7, 44.6, 44.2, 36.7, 25.5, 25.2, 23.7, 22.6, 19.1. LRMS $[M+H]^+$ 494.4.

Step 8: To $N^1$,$N^{1'}$-((5-(3-(isoButylammonio)propoxy)-1,3-phenylene)bis-(methylene))bis($N^3$-(3-ammoniopropyl)propane-1,3-diaminium) hydrochloride salt was added aqueous NaOH (10%, 100 mL) and 75% $CHCl_3$/isopropanol (100 mL). The layers were separated, and the aqueous layer was extracted with 75% $CHCl_3$/isopropanol (4 x 100 mL). The organic layers were combined, dried over $Na_2SO_4$, filtered, and concentrated under reduced pressure to afford a clear oil, which was used without further purification.

Step 9: To a round-bottom flask was added the crude $N^1$,$N^{1'}$-((5-(3-(isobutylammonio)propoxy)-1,3-phenylene)bis(methylene))bis($N^3$-(3-ammoniopropyl)propane-1,3-diaminium) (0.91 g, 1.84 mmol) and MeOH (20 mL). To the solution was added octanal (0.47 g, 3.68 mmol) and the reaction mixture was stirred at rt for 24 h. Sodium borohydride (0.28 g, 7.36 mmol) was added and the reaction mixture was stirred for 1 h. The reaction mixture was concentrated under reduced pressure to afford a white solid. Aq. NaOH (10%, 100 mL) and EtOAc (100 mL) were added and the mixture stirred for 1 h. The layers were separated and the aqueous layer was extracted with EtOAc (100 mL). The combined organics were dried over $Na_2SO_4$, filtered, and concentrated under reduced pressure to afford the desired product as a clear oil which was used without further purification.

Step 10: *$N^1$,$N^{1'}$-((5-(3-(isobutylamino)propoxy)-1,3-phenylene)bis(methylene))-bis($N^3$-(3-(octylamino)propyl)propane-1,3-diamine), hydrochloride salt.* To the crude $N^1$,$N^{1'}$-((5-(3-(isobutylamino)propoxy)-1,3-phenylene)bis(methylene))bis($N^3$-(3-(octylamino)propyl)propane-1,3-diamine) was added methanolic HCl (100 mL, 1.0M). The reaction mixture was stirred at rt for 1 h. The reaction mixture was concentrated under reduced pressure, and the solid was collected by vacuum filtration and washed with $Et_2O$ (50 mL) and hot MeOH (50 mL) to afford the desired product as a white solid (0.75 g, 42%). 1H NMR (500 MHz, $D_2O$) δ 7.21 (s, 1H), 7.20 (s 2H), 4.30 (s, 4H), 4.24 (t, J = 5 Hz, 2H), 3.30 (t, J = 7 Hz, 2H), 3.26-3.14 (m, 16H), 3.07 (t, J = 7 Hz, 4H), 2.96 (d, J = 7.5 Hz, 2H), 2.26-2.03 (m, 11H), 1.69 (p, J = 6.5 Hz, 4H), 1.38-1.28 (m, 20H), 1.02 (d, J = 6.5 Hz, 6H), 0.87 (t, J = 5.5 Hz, 6H). 13C NMR (125 MHz, D2O) δ 158.9, 133.0, 123.7, 117.1, 65.8, 54.8, 50.8, 47.9, 45.7, 44.6, 44.6, 44.2, 44.1, 30.9, 28.1, 28.1, 25.6, 25.5, 25.4, 25.2, 22.6, 22.6, 19.1, 13.4.

**Example 98**

*Antimicrobial activity of polyamines in agar media*

A 0.5 McFarland standard of each bacteria was made, then individual lawns of bacteria spread onto the surface of tryptic soy agar (TSA). 500 uL of the suspended gel were placed on the center of each lawn. Plates were incubated

at 37 C for 24 hours, then imaged. Images of the resulting gels are shown in FIGS. 30A to 30I show the effects of the polyamines CZ-86, CZ-100, and CZ-110 on various bacterial cell cultures. The data shown indicated that when the polyamine compounds were mixed with the gel, the compounds diffused out of the gel and created zones of inhibition in the bacterial lawns. The gel alone showed no signs of antimicrobial activity (FIG. 30B).

The hydrogel that was used for those images is a LifeCore gel with 1% sodium hyaluronate (1% Sodium Hyaluronate Solution Part #82). The CZ compound was suspended in water at 1% (w/w) concentration and combined with the LifeCore gel 1:1 for a final product that contained 0.5% sodium hyaluronate and 0.5% CZ compound.

**Example 99**

*CZ-86 and CZ-90 polyamine hemolysis activity*

The polyamines CZ-86 and CZ-90 were tested for hemolytic activity. The difference between the hemolytic indexes of both polyamines and the negative control was 0.00 percent. This places both test articles in the non-hemolytic range according to the grade outlined below in Table 6A.

Table 6A: Hemolytic Index and Grade:

| Hemolytic Index | Hemolytic Grade |
|---|---|
| 0-2 | Non-Hemolytic |
| 2-5 | Slightly Hemolytic |
| >5 | Hemolytic |

All test method acceptance criteria were met. The test procedures listed below were followed without deviation.

Table 6B: Results

| Test Article / Control | Optical Density | Average Optical Density | Hemolytic Index | Average Hemolytic Index (% Hemolysis) | Corrected Hemolytic Index (% Hemolysis) |
|---|---|---|---|---|---|
| CZ-86 | 0.003<br>0.003<br>0.003 | 0.003 | 0.764<br>0.764<br>0.764 | 0.76 | 0.00 |
| CZ-90 | 0.003<br>0.003<br>0.003 | 0.003 | 0.764<br>0.764<br>0.764 | 0.76 | 0.00 |
| Negative Control | 0.003<br>0.003<br>0.003 | 0.003 | 0.764<br>0.764<br>0.764 | 0.76 | 0.00 |
| Positive Control | 0.430<br>0.468<br>0.453 | 0.450 | 97.068<br>105.638<br>102.255 | 101.7 | 100.8 |
| Phosphate Buffered Saline (PBS) Blank | 0.005<br>0.003<br>0.002 | 0.003 | 1.215<br>0.764<br>0.539 | 0.84 | N/A |

Table 6C: Hemoglobin Standard:

| Regression Output | |
|---|---|
| Constant | 0.00056 |
| Standard Error of Y Estimate | 0.00604 |

(continued)

| Regression Output | |
|---|---|
| $R^2$ | 0.99961 |
| Degrees of Freedom | 6 |
| X Coefficient(s) | 1.44547 |
| Standard Error of Coefficient | 0.01164 |

Acceptance Criteria: The negative control must produce a corrected hemolytic index of less than 2%. The positive control must produce a corrected hemolytic index of greater than 5%.

Procedure: The PBS used in testing was calcium and magnesium free. The method has been validated using human blood from one donor. This is in compliance with ISO 10993-4 which states due to differences in blood activity, human blood should be used where possible. Furthermore, pooling unmatched human blood from multiple donors may cause red blood cell agglutination which in turn could cause hemolysis; this supports the use of a single donor.

The blood was drawn using vacutainers containing 0.1 M sodium citrate at a ratio of 9:1 (3.2% anticoagulant to blood). The blood in this test was used within four hours of blood draw. The collected blood was refrigerated until testing was performed.

A hemoglobin standard was diluted with Drabkin's reagent to give solutions at concentrations of 0.80, 0.60, 0.40, 0.30, 0.20, 0.10, 0.02, and 0.01 mg/ml. These solutions were allowed to stand at room temperature for a minimum of five minutes. The absorbance was read on a spectrophotometer at 540 nanometers (nm). A standard curve was determined with the absorbance values and the standard concentrations of hemoglobin.

Human blood was centrifuged at 700-800 x g for 15 minutes. A 1 mL aliquot of the plasma was added into 1 mL of Drabkin's reagent, and placed at room temperature for a minimum of 15 minutes. The absorbance was read on a spectrophotometer at 540 nm. The hemoglobin concentration was determined from the standard curve and then multiplied by a factor of 2 to obtain the total plasma free hemoglobin. The plasma free hemoglobin was less than 2 mg/mL (actual value 0.221 mg/mL). A 20 µL aliquot of blood was added to 5 mL of Drabkin's reagent in duplicate and allowed to stand at room temperature for a minimum of 15 minutes. The absorbance was read on a spectrophotometer at 540 nm then multiplied by 251 to account for the dilution.

Based on the plasma hemoglobin and the blood absorbance against Drabkin's reagent, the blood was diluted out to 10 ± 1 mg/mL with PBS. To verify the blood dilution, a 300 µL aliquot of this blood was added to 4.5 mL of Drabkin's reagent in triplicate and allowed to stand at room temperature for a minimum of 15 minutes. The absorbance was read on a spectrophotometer at 540 nm then multiplied by 16 to account for the dilution.

Glass test tubes were labeled appropriately. Both samples were prepared the same way. The samples were allowed to thaw prior to testing. To each test tube, 7 mL of the test article and 1 mL of the diluted blood were added. The controls consisted of the appropriate amount of control material, 7 mL of PBS and 1 mL of blood. Three tubes were prepared for each test article and control. The tubes were incubated at 37 ± 2°C for 3 hours ± 5 minutes. Tubes were gently inverted twice at 30 minute intervals throughout the incubation period. A non-hemolytic negative control, a hemolytic positive control and a PBS blank were included.

After incubation, test articles were centrifuged at 700-800 x g for 15 minutes and 1 mL of the supernatant fluid was combined with 1 mL of Drabkin's reagent and allowed to stand at room temperature for a minimum of 15 minutes. Following the centrifugation phase, the supernatant of both test articles visually appeared clear and were particulate free. The PBS blank and the negative control supernatant visually appeared clear and were particulate free. The supernatant of the positive control visually appeared red and were particulate free. The test articles and controls were read at 540 nm in a spectrophotometer.

The hemolytic index (percent hemolysis) was interpreted using the following equation:

$$\text{Hemolytic Index} = \frac{\text{Hemoglobin Released (mg/mL)}}{\text{Hemoglobin Present (mg/mL)}} x\ 100$$

Where:

$$\text{Hemoglobin Released (mg/mL)} = (\text{Optical Density x X Coefficient} + \text{Constant}) \text{ x } 16$$

$$\text{Hemoglobin Present (mg/ml)} = \text{Diluted Blood } 10 \pm 1 \text{ mg/mL}$$

The corrected hemolytic index was calculated by subtracting the hemolytic index of the PBS blank solution from the hemolytic index of the test article and controls.

The test article is compared to the negative control by subtracting the hemolytic index of the negative control from the hemolytic index of the test article.

Table 6D: Test Parameters:

| | |
|---|---|
| Blood Type Used: | Human, Citrated |
| Positive Control: | Nitrile Glove Material, tested at 3 $cm^2$/mL |
| Negative Control: | Polypropylene Pellets tested at 0.2 grams/mL |
| Total Hemoglobin Kit: | Stanbio, 80 mg/dL |
| Incubation Time: | 3 hours ± 5 minutes |
| Incubation Temperature: | 37 ± 2°C |

**Example 100**

*Minimum Elution Media (MEM) elution test of CZ-25*

Summary: The Minimal Essential Media (MEM) Elution test was designed to determine the cytotoxicity of extractable substances. The test article was added to cell monolayers and incubated. The cell monolayers were examined and scored based on the degree of cellular destruction. All test method acceptance criteria were met. The test procedure(s) listed above were followed without deviation.

Table 7A: Results of Test

| Dilution of CZ-25 | Results Pass/Fail | Scores | | | |
|---|---|---|---|---|---|
| | | #1 | #2 | #3 | Average |
| Initial Concentration 2.5 mg/mL | Fail | 4 | 4 | 4 | 4 |
| 1:2 | Fail | 4 | 4 | 4 | 4 |
| 1:4 | Fail | 4 | 4 | 4 | 4 |
| 1:8 | Fail | 4 | 4 | 4 | 4 |
| 1:16 | Fail | 4 | 4 | 4 | 4 |

Table 7B: Controls:

| Identification | Scores | | | | Extraction Ratio | Amount Tested / Extraction Solvent Amount |
|---|---|---|---|---|---|---|
| | #1 | #2 | #3 | Average | | |
| Negative Control - Polypropylene Pellets | 0 | 0 | 0 | 0 | 0.2 g/mL | 4 g / 20 mL |
| Media Control | 0 | 0 | 0 | 0 | N/A | 20 ml |
| Positive Control - Latex Natural Rubber | 4 | 4 | 4 | 4 | 0.2 g/mL | 4 g / 20 mL |

Acceptance Criteria: The United States Pharmacopeia & National Formulary (USP <87>) states that the test article meets the requirements, or receives a passing score (Pass) if the reactivity grade is not greater than grade 2 or a mild reactivity. The ANSI/AAMI/ISO 10993-5 standard states that the achievement of a numerical grade greater than 2 is considered a cytotoxic effect, or a failing score (Fail).

Acceptance criteria were based upon the negative and media controls receiving "0" reactivity grades and positive controls receiving a 3-4 reactivity grades (moderate to severe). The test was considered valid as the control results were within acceptable parameters.

The cell monolayers were examined microscopically. The wells were scored as to the degree of discernable morphological cytotoxicity on a relative scale of 0 to 4:

Table 7C: Standards for Grades

| Conditions of All Cultures | Reactivity | Grade |
|---|---|---|
| No cell lysis, intracytoplasmic granules. | None | 0 |
| Not more than 20% rounding, occasional lysed cells. | Slight | 1 |
| Not more than 50% rounding, no extensive cell lysis. | Mild | 2 |
| Not more than 70% rounding and lysed cells. | Moderate | 3 |
| Nearly complete cell destruction. | Severe | 4 |

The results from the three wells were averaged to give a final cytotoxicity score.

Procedure: The test article was added to IX Minimal Essential Media + 5% bovine serum at an initial concentration of 2.5 mg/ml. This initial concentration was then diluted 1: 2, 1:4, 1:8, and 1:16. Multiple well cell culture plates were seeded with a verified quantity of industry standard L-929 cells (ATCC CCL-1) and incubated until approximately 80% confluent. The test articles and control extracts were added to the cell monolayers in triplicate. The cells were incubated at 37 ± 1°C with 5 ± 1% $CO_2$ for 48 ± 3 hours.

## Example 101

*Minimum Elution Media (MEM) elution test of CZ-86*

Summary: The polyamine compound CZ-86 was added to cell monolayers and incubated according to the procedure described in Example 100.

Table 8A: Results

| Dilution of CZ-86 | Results Pass/Fail | Scores | | | |
|---|---|---|---|---|---|
| | | #1 | #2 | #3 | Average |
| 400 μg/mL | Fail | 4 | 4 | 4 | 4 |
| 1:2 | Fail | 4 | 4 | 4 | 4 |
| 1:4 | Fail | 3 | 3 | 3 | 3 |
| 1:8 | Pass | 0 | 0 | 0 | 0 |
| 1:16 | Pass | 0 | 0 | 0 | 0 |

Table 8B: Controls:

| Identification | Scores | | | | Extraction Ratio | Amount Tested / Extraction Solvent Amount |
|---|---|---|---|---|---|---|
| | #1 | #2 | #3 | Average | | |
| Negative Control - Polypropylene Pellets | 0 | 0 | 0 | 0 | 0.2 g/mL | 4 g / 20 mL |
| Media Control | 0 | 0 | 0 | 0 | N/A | 20 ml |
| Positive Control - Latex Natural Rubber | 4 | 4 | 4 | 4 | 0.2 g/mL | 4 g / 20 mL |

The cell monolayers were examined microscopically. The wells were scored as to the degree of discernable

morphological cytotoxicity on a relative scale of 0 to 4:

Table 8C: Standards for Grades

| Conditions of All Cultures | Reactivity | Grade |
|---|---|---|
| No cell lysis, intracytoplasmic granules. | None | 0 |
| Less than or equal to 20% rounding, occasional lysed cells. | Slight | 1 |
| Greater than 20% to less than or equal to 50% | Mild | 2 |
| rounding, no extensive cell lysis. | | |
| Greater than 50% to less than 70% rounding and lysed cells. | Moderate | 3 |
| Nearly complete destruction of the cell layers. | Severe | 4 |

**Example 102**

*Minimum Elution Media (MEM) elution test of CZ-52* and *CZ-100*

Summary: The test articles shown below was added to cell monolayers and incubated according to the procedure of Example 100.

Table 9A: Results

| Identification | | Results Pass/Fail | Scores | | | |
|---|---|---|---|---|---|---|
| | | | #1 | #2 | #3 | Average |
| 1 | 25 μg/mL | Fail | 4 | 4 | 4 | 4 |
| | 12.5 μg/mL | Pass | 2 | 2 | 2 | 2 |
| | 6.25 μg/mL | Pass | 0 | 0 | 0 | 0 |
| | 3.125 μg/mL | Pass | 0 | 0 | 0 | 0 |
| | 1.563 | Pass | 0 | 0 | 0 | 0 |
| 2 | 25 μg/mL | Fail | 4 | 4 | 4 | 4 |
| | 12.5 μg/mL | Fail | 4 | 4 | 4 | 4 |
| | 6.25 μg/mL | Fail | 3 | 3 | 3 | 3 |
| | 3.125 μg/mL | Pass | 1 | 1 | 1 | 1 |
| | 1.563 | Pass | 0 | 0 | 0 | 0 |
| 3 | 25 μg/mL | Fail | 4 | 4 | 4 | 4 |
| | 12.5 μg/mL | Fail | 4 | 4 | 4 | 4 |
| | 6.25 μg/mL | Fail | 4 | 4 | 4 | 4 |
| | 3.125 μg/mL | Pass | 2 | 2 | 2 | 2 |
| | 1.563 | Pass | 0 | 0 | 0 | 0 |
| 4 | 25 μg/mL | Fail | 4 | 4 | 4 | 4 |
| | 12.5 μg/mL | Pass | 2 | 2 | 2 | 2 |
| | 6.25 μg/mL | Pass | 0 | 0 | 0 | 0 |
| | 3.125 μg/mL | Pass | 0 | 0 | 0 | 0 |
| | 1.563 | Pass | 0 | 0 | 0 | 0 |

(continued)

| Identification | | Results Pass/Fail | Scores | | | |
|---|---|---|---|---|---|---|
| | | | #1 | #2 | #3 | Average |
| 5 | 25 µg/mL | Pass | 0 | 0 | 0 | 0 |
| | 12.5 µg/mL | Pass | 0 | 0 | 0 | 0 |
| | 6.25 µg/mL | Pass | 0 | 0 | 0 | 0 |
| | 3.125 µg/mL | Pass | 0 | 0 | 0 | 0 |
| | 1.563 | Pass | 0 | 0 | 0 | 0 |
| 6 | 25 µg/mL | Fail | 4 | 4 | 4 | 4 |
| | 12.5 µg/mL | Fail | 4 | 4 | 4 | 4 |
| | 6.25 µg/mL | Fail | 4 | 4 | 4 | 4 |
| | 3.125 µg/mL | Fail | 4 | 4 | 4 | 4 |
| | 1.563 | Fail | 3 | 3 | 3 | 3 |
| Code for Test Article: | | 1) Chlorhexidine gluconate<br>2) Glutaraldehyde<br>3) CZ-52<br>4) CZ-100<br>5) Vancomycin<br>6) Benzethonium chloride | | | | |

Table 9B: Controls:

| Identification | Scores | | | | Extraction Ratio | Amount Tested / Extraction Solvent Amount |
|---|---|---|---|---|---|---|
| | #1 | #2 | #3 | Average | | |
| Negative Control - Polypropylene Pellets | 0 | 0 | 0 | 0 | 0.2 g/mL | 4 g / 20 mL |
| Media Control | 0 | 0 | 0 | 0 | N/A | 20 ml |
| Positive Control - Latex Natural Rubber | 4 | 4 | 4 | 4 | 0.2 g/mL | 4 g / 20 mL |

The cell monolayers were examined microscopically. The wells were scored as to the degree of discernable morphological cytotoxicity on a relative scale of 0 to 4:

Table 9C: Standards for Grades

| Conditions of All Cultures | Reactivity | Grade |
|---|---|---|
| No cell lysis, intracytoplasmic granules. | None | 0 |
| Not more than 20% rounding, occasional lysed cells. | Slight | 1 |
| Not more than 50% rounding, no extensive cell lysis. | Mild | 2 |
| Not more than 70% rounding and lysed cells. | Moderate | 3 |
| Nearly complete cell destruction. | Severe | 4 |

**Example 103**

*Minimum Elution Media (MEM) elution test of CZ-58, CZ-62, CZ-65* and *CZ-66*

Summary: The test articles shown below was added to cell monolayers and incubated according to the procedure of Example 100.

Table 10A: Results

| Identification | Dilution | Results Pass/Fail | Scores | | | |
|---|---|---|---|---|---|---|
| | | | #1 | #2 | #3 | Average |
| CZ-58 | 25 μg/mL | Fail | 4 | 4 | 4 | 4 |
| | 1:2 | Fail | 4 | 4 | 4 | 4 |
| | 1:4 | Pass | 2 | 2 | 2 | 2 |
| | 1:8 | Pass | 1 | 1 | 1 | 1 |
| | 1:16 | Pass | 1 | 1 | 1 | 1 |
| CZ-62 | 25 μg/mL | Fail | 4 | 4 | 4 | 4 |
| | 1:2 | Fail | 4 | 4 | 4 | 4 |
| | 1:4 | Fail | 3 | 3 | 3 | 3 |
| | 1:8 | Pass | 1 | 1 | 1 | 1 |
| | 1:16 | Pass | 1 | 1 | 1 | 1 |
| CZ-65 | 25 μg/mL | Fail | 4 | 4 | 4 | 4 |
| | 1:2 | Fail | 4 | 4 | 4 | 4 |
| | 1:4 | Pass | 2 | 2 | 2 | 2 |
| | 1:8 | Pass | 1 | 1 | 1 | 1 |
| | 1:16 | Pass | 1 | 1 | 1 | 1 |
| CZ-66 | 25 μg/mL | Fail | 4 | 4 | 4 | 4 |
| | 1:2 | Fail | 4 | 4 | 4 | 4 |
| | 1:4 | Pass | 2 | 2 | 2 | 2 |
| | 1:8 | Pass | 1 | 1 | 1 | 1 |
| | 1:16 | Pass | 1 | 1 | 1 | 1 |

Table 10B: Controls:

| Identification | Scores | | | | Extraction Ratio | Amount Tested / Extraction Solvent Amount |
|---|---|---|---|---|---|---|
| | #1 | #2 | #3 | Average | | |
| Negative Control - Polypropylene Pellets | 0 | 0 | 0 | 0 | 0.2 g/mL | 4 g / 20 mL |
| Media Control | 0 | 0 | 0 | 0 | N/A | 20 ml |
| Positive Control - Latex Natural Rubber | 4 | 4 | 4 | 4 | 0.2 g/mL | 4 g / 20 mL |

The cell monolayers were examined microscopically. The wells were scored as to the degree of discernable morphological cytotoxicity on a relative scale of 0 to 4:

Table 10C: Standards for Grades

| Conditions of All Cultures | Reactivity | Grade |
|---|---|---|
| No cell lysis, intracytoplasmic granules. | None | 0 |
| Not more than 20% rounding, occasional lysed cells. | Slight | 1 |
| Not more than 50% rounding, no extensive cell lysis. | Mild | 2 |
| Not more than 70% rounding and lysed cells. | Moderate | 3 |
| Nearly complete cell destruction. | Severe | 4 |

**Example 104**

*Minimum Elution Media (MEM) elution test of CZ-92, CZ-96 and CZ-99*

Summary: The test articles shown below was added to cell monolayers and incubated according to the procedure of Example 100.

Table 11A: Results

| Identification | Dilution | Results Pass/Fail | Scores | | | |
|---|---|---|---|---|---|---|
| | | | #1 | #2 | #3 | Average |
| CZ-92 | 25 µg/mL | Fail | 4 | 4 | 4 | 4 |
| | 1:2 | Fail | 4 | 4 | 4 | 4 |
| | 1:4 | Fail | 4 | 4 | 4 | 4 |
| | 1:8 | Fail | 3 | 3 | 2 | 3 |
| | 1:16 | Pass | 1 | 1 | 1 | 1 |
| CZ-96 | 25 µg/mL | Pass | 2 | 2 | 2 | 2 |
| | 1:2 | Pass | 0 | 0 | 0 | 0 |
| | 1:4 | Pass | 0 | 0 | 0 | 0 |
| | 1:8 | Pass | 0 | 0 | 0 | 0 |
| | 1:16 | Pass | 0 | 0 | 0 | 0 |
| CZ-99 | 25 µg/mL | Pass | 0 | 0 | 0 | 0 |
| | 1:2 | Pass | 0 | 0 | 0 | 0 |
| | 1:4 | Pass | 0 | 0 | 0 | 0 |
| | 1:8 | Pass | 0 | 0 | 0 | 0 |
| | 1:16 | Pass | 0 | 0 | 0 | 0 |

Table 11B: Controls:

| Identification | Scores | | | | Extraction Ratio | Amount Tested / Extraction Solvent Amount |
|---|---|---|---|---|---|---|
| | #1 | #2 | #3 | Average | | |
| Negative Control - Polypropylene Pellets | 0 | 0 | 0 | 0 | 0.2 g/mL | 4 g / 20 mL |
| Media Control | 0 | 0 | 0 | 0 | N/A | 20 ml |
| Positive Control - Latex Natural Rubber | 4 | 4 | 4 | 4 | 0.2 g/mL | 4 g / 20 mL |

The cell monolayers were examined microscopically. The wells were scored as to the degree of discernable morphological cytotoxicity on a relative scale of 0 to 4:

Table 11C: Standards for Grades

| **Conditions of All Cultures** | **Reactivity** | **Grade** |
|---|---|---|
| No cell lysis, intracytoplasmic granules. | None | 0 |
| Less than or equal to 20% rounding, occasional lysed cells. | Slight | 1 |
| Greater than 20% to less than or equal to 50% rounding, no extensive cell lysis. | Mild | 2 |
| Greater than 50% to less than 70% rounding and lysed cells. | Moderate | 3 |
| Nearly complete destruction of the cell layers. | Severe | 4 |

## Claims

1. A non-therapeutic method for dispersing or killing a biofilm, the method comprising a step of treating the biofilm with an anti-biofilm composition, thereby effectively dispersing or killing the biofilm;
wherein the anti-biofilm composition comprises a polyamine compound selected from the group consisting of

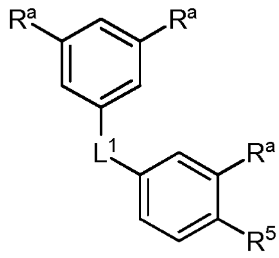 , 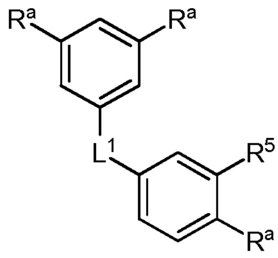 , 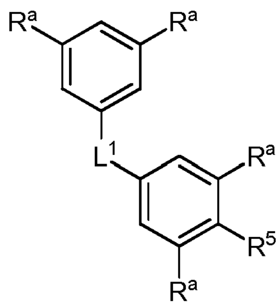 ,

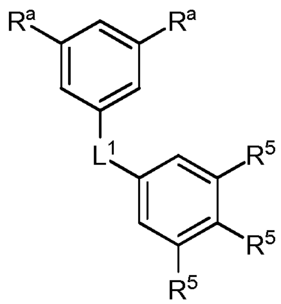 ,

and a salt thereof;
wherein:

each $L^1$ is selected from the group consisting of a bond and -O-;
each $R^a$ is $-CH_2[NH(CH_2)_n]_pNHR^4$;
each n is an integer independently selected from 3 to 12;
each p is an integer independently selected from 1 to 3;
each $R^4$ is a member independently selected from the group consisting of hydrogen, alkyl, arylalkyl, and cycloalkylalkyl; and
each $R^5$ is a member independently selected from the group consisting of hydrogen, alkyl, hydroxyl, alkoxy, halo, fluoroalkyl, fluoroalkyloxy, and hydroxyalkyl,
wherein the polyamine compound comprises at least four primary or secondary amino groups.

2. The method of claim 1, wherein n is 3.

**3.** The method of claim 1 or claim 2, wherein each $R^4$ is a member independently selected from the group consisting of hydrogen and alkyl.

**4.** The method of any one of claims 1 to 3, wherein each $R^a$ is $-CH_2[NH(CH_2)_n]_pNH_2$.

**5.** The method according to any one of claims 1 to 4, wherein the polyamine compound is selected from the group consisting of

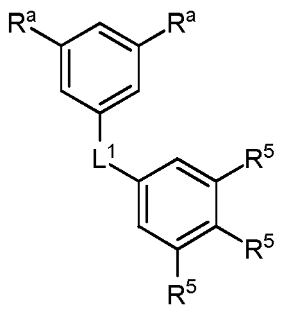

and a salt thereof.

**6.** The method of claim 1, wherein the polyamine compound is selected from the group consisting of

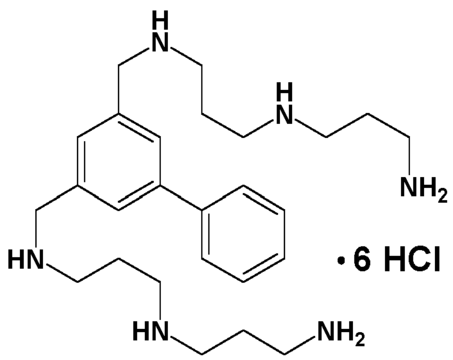

,

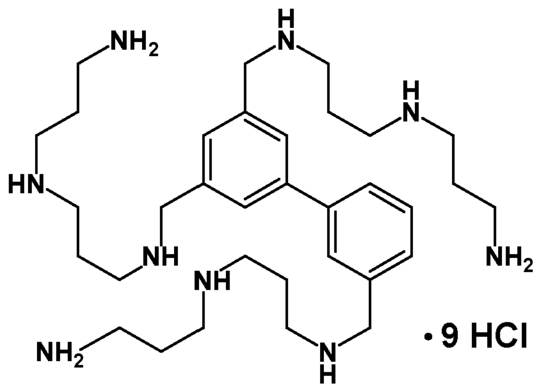

,

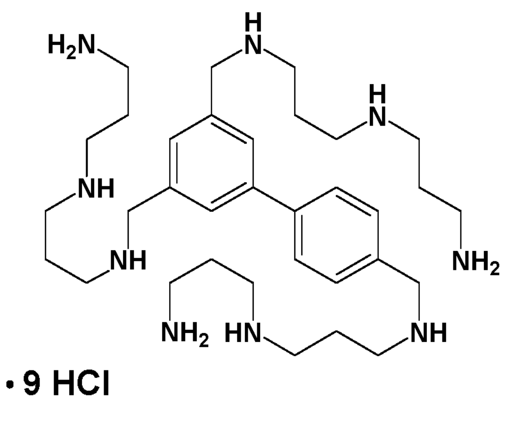

,

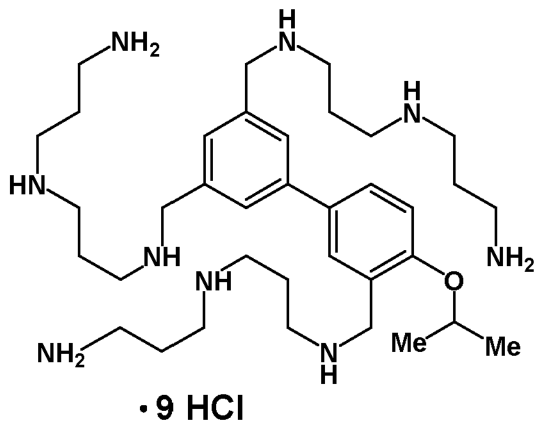

,

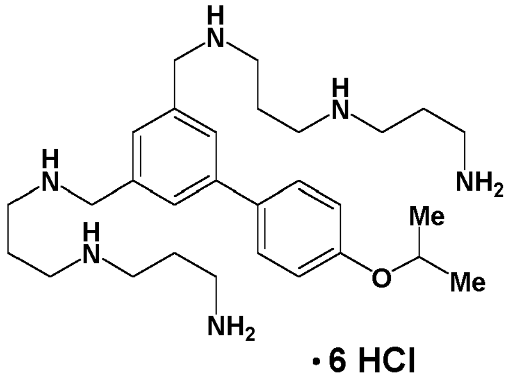

•6 HCl ,

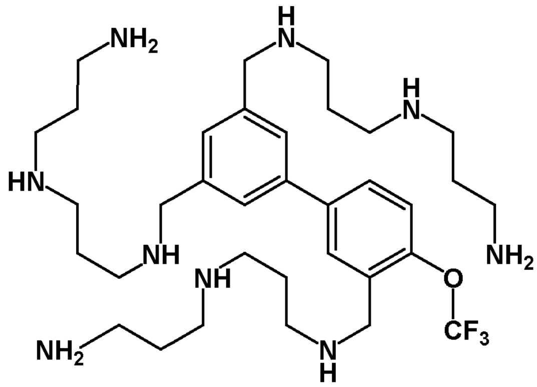

•9 HCl ,

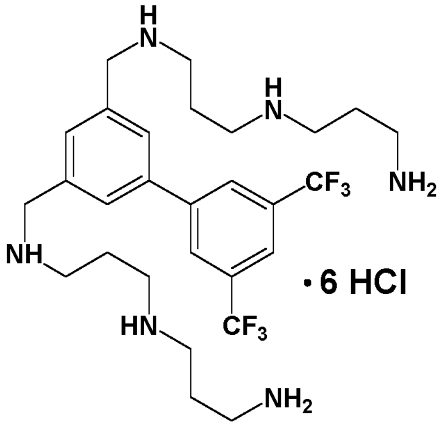

•6 HCl ,

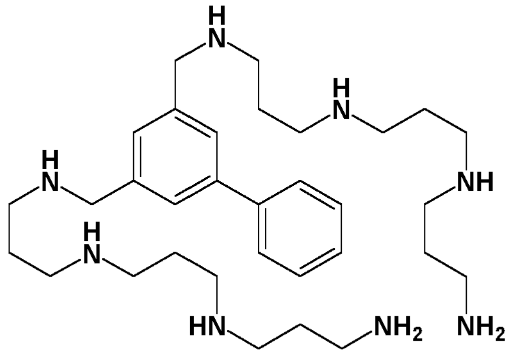

•8 HCl ,

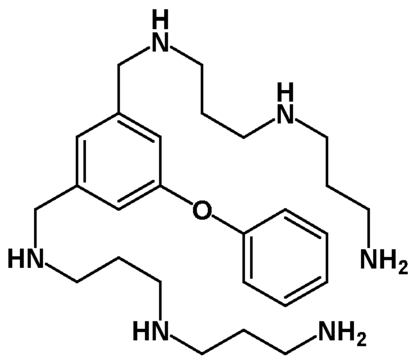

•6 HCl ,

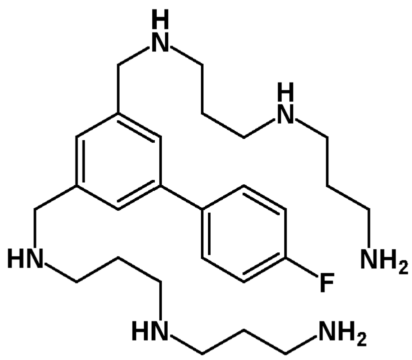

•6 HCl ,

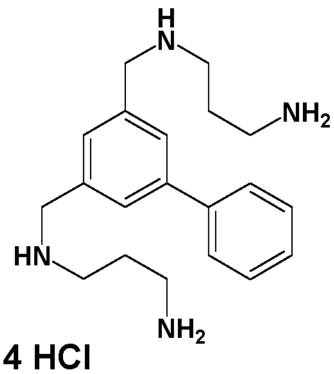

• 4 HCl ,

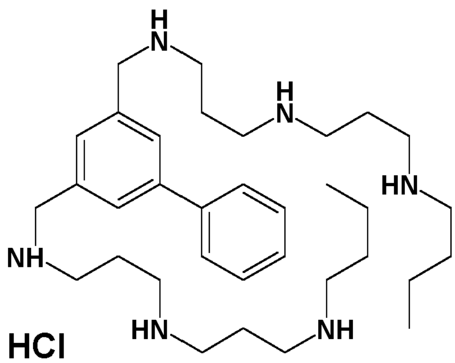

• 6 HCl ,

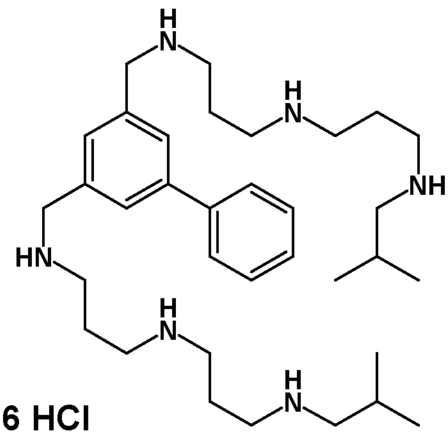

• 6 HCl ,

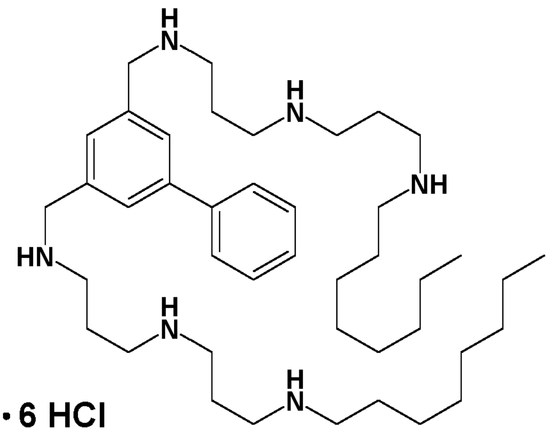

• 6 HCl ,

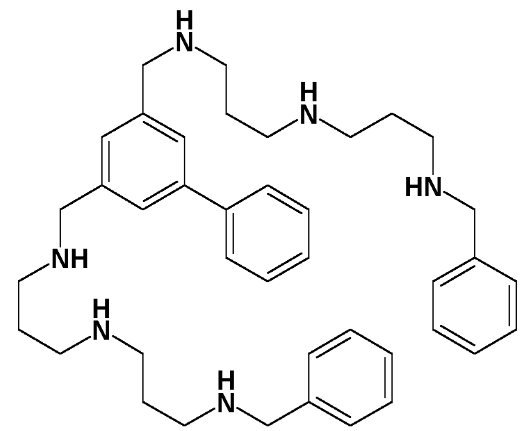

• 6 HCl ,

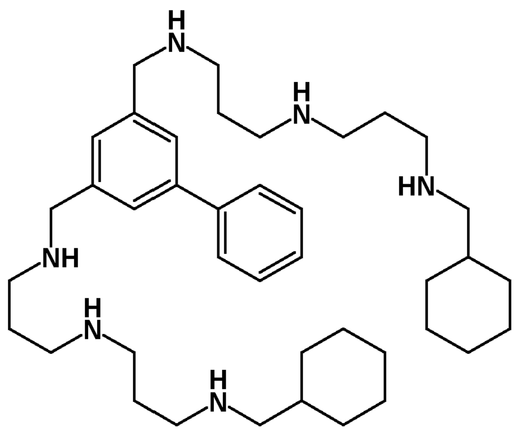

• 6 HCl ,

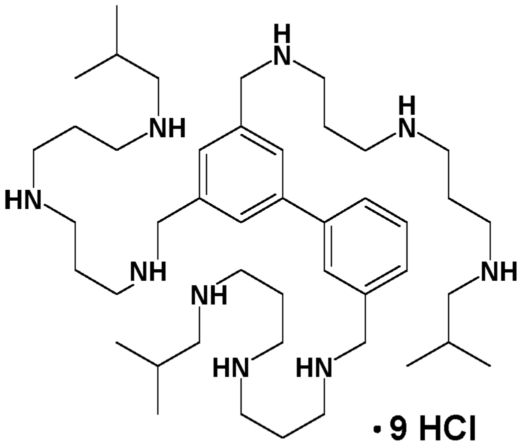
• 9 HCl ,

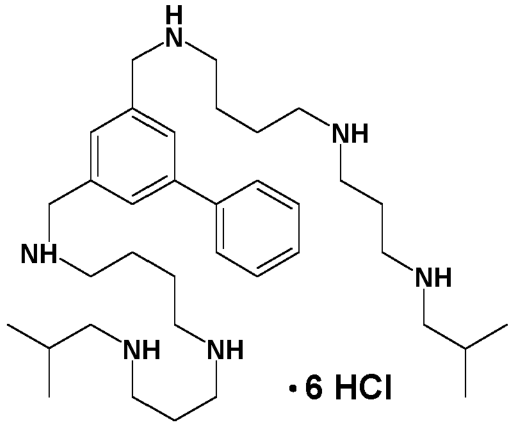
• 6 HCl ,

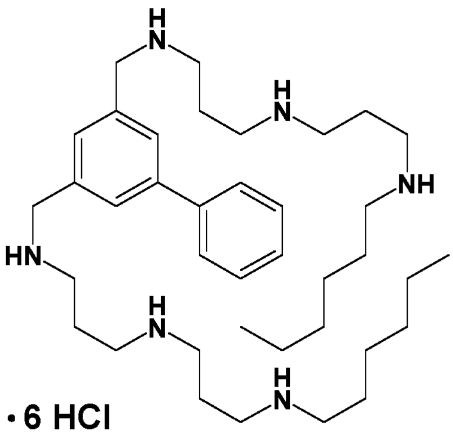
• 6 HCl ,

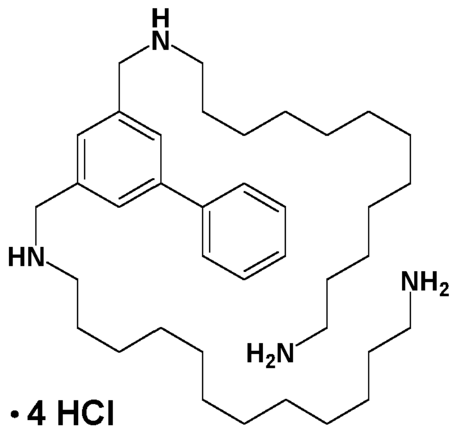
• 4 HCl ,

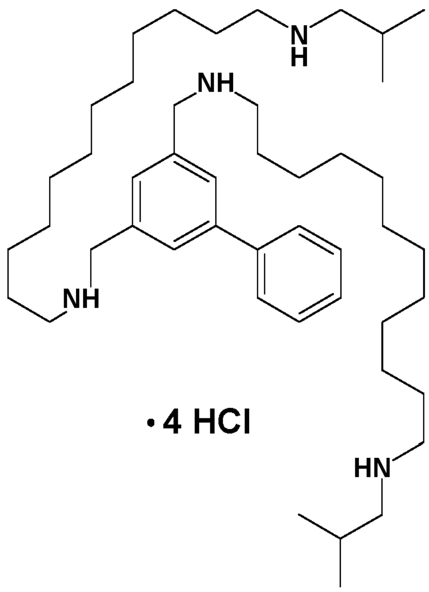
• 4 HCl ,

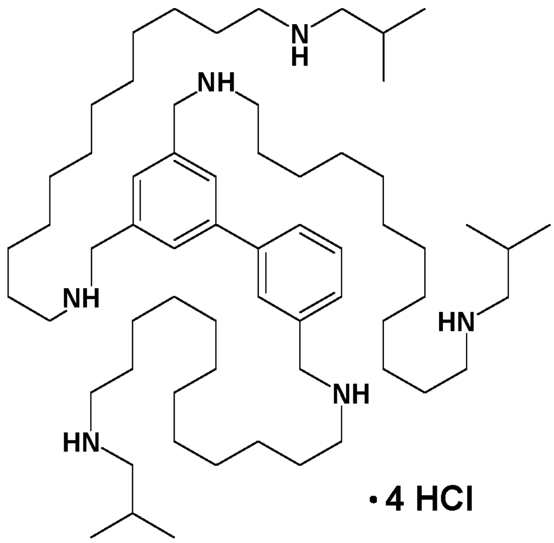
• 4 HCl ,

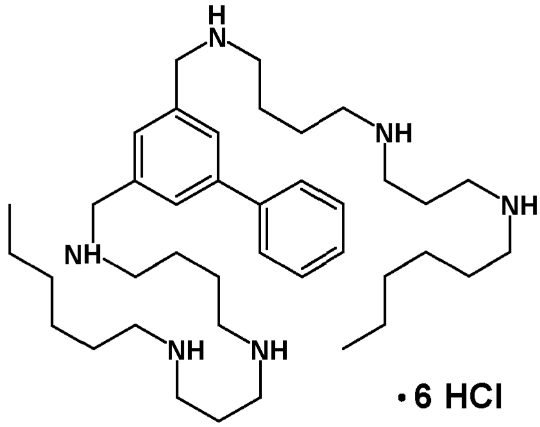

• 6 HCl ,

or selected from the group consisting of

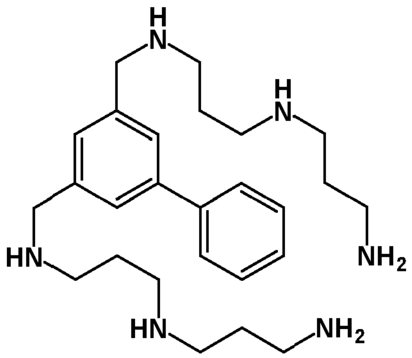 , 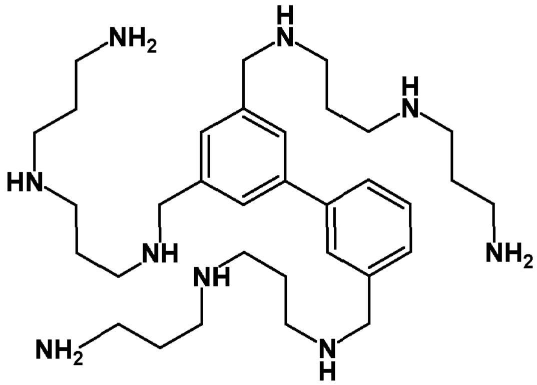 ,

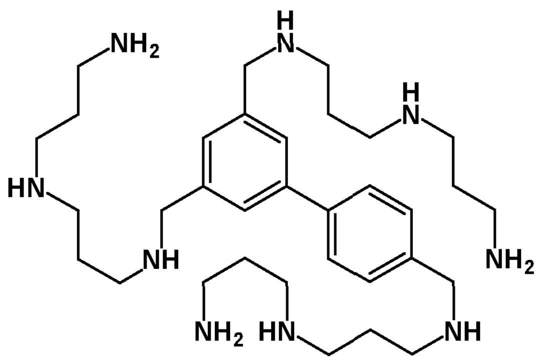 , 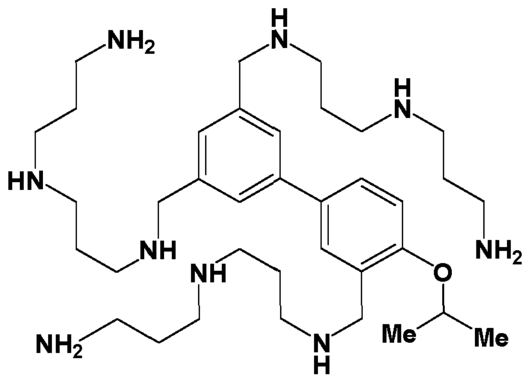 ,

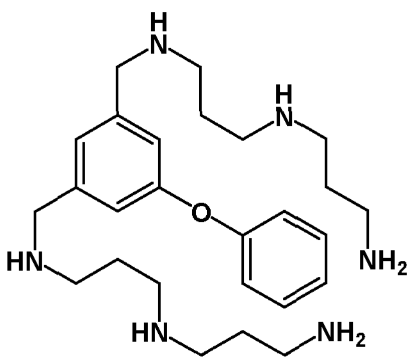 , 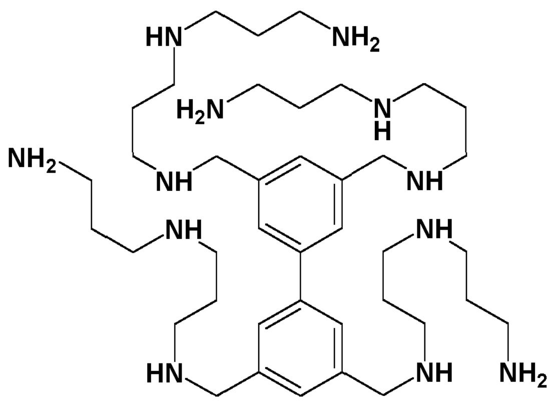 ,

and a salt thereof.

**7.** The method of claim 1 or claim 2, wherein the polyamine compound is selected from the group consisting of

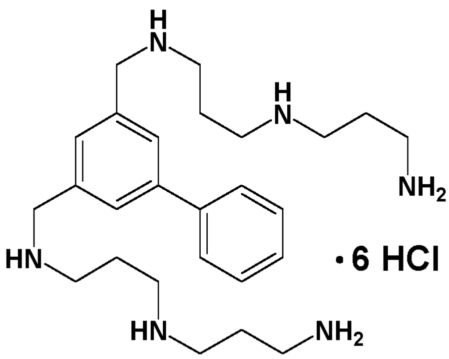

,

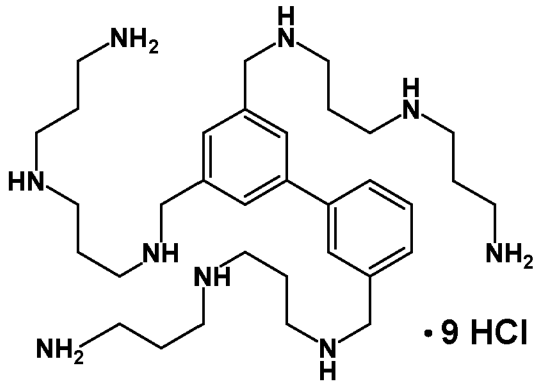

,

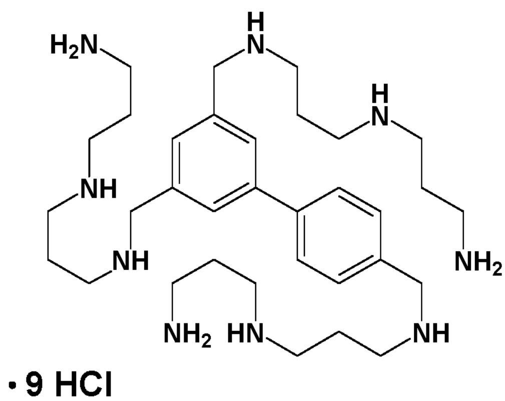

,

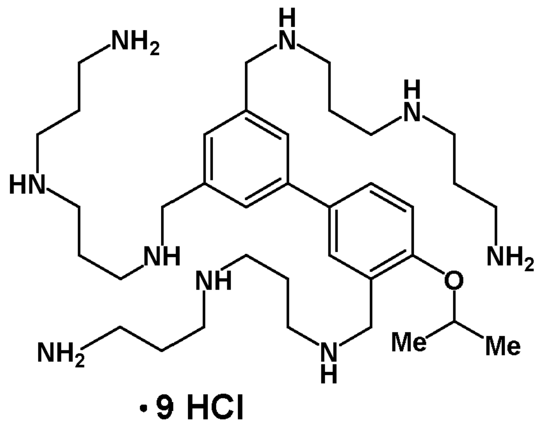

,

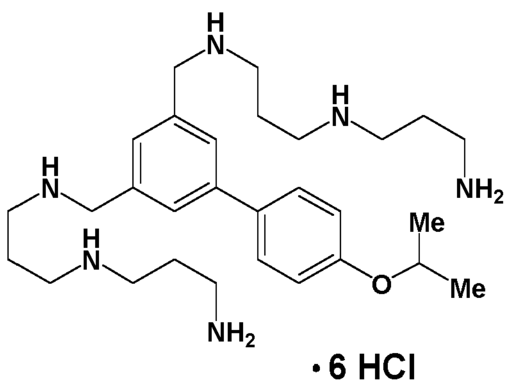

,

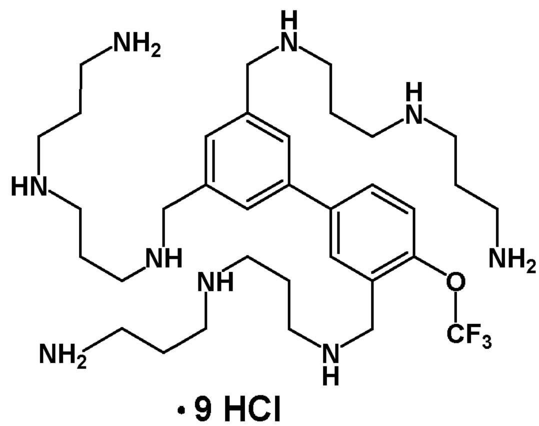

,

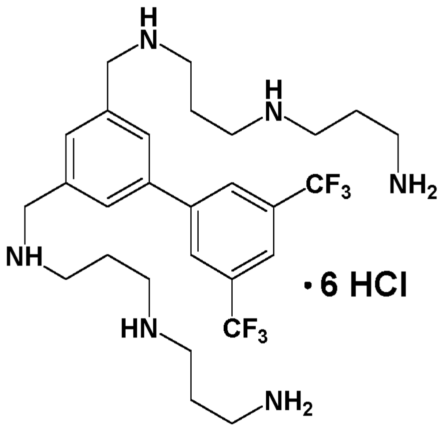
,
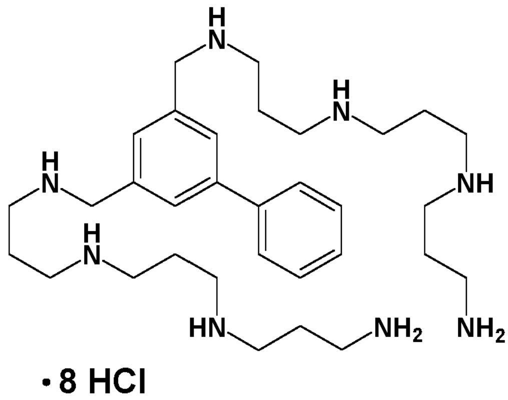
,

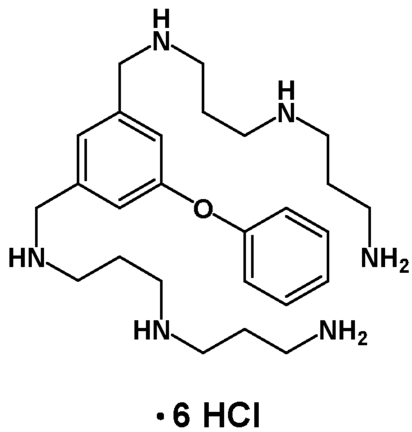
,
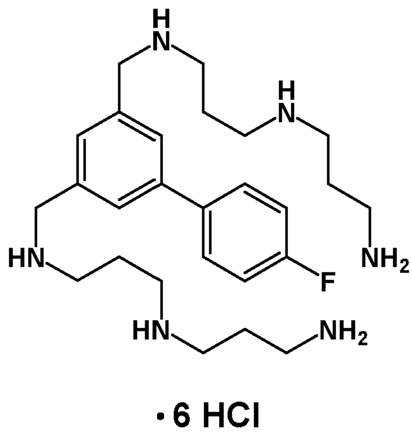
,

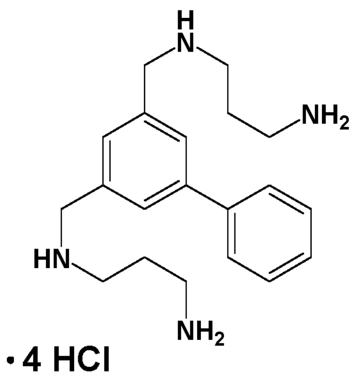
,
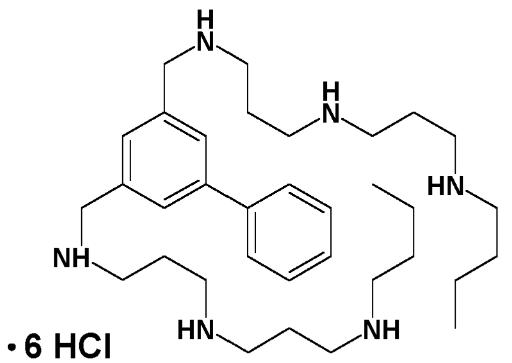
,

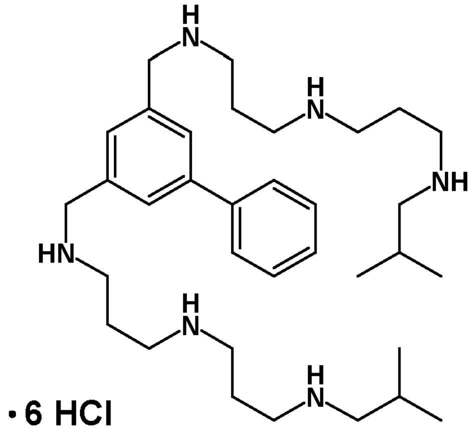

• 6 HCl ,

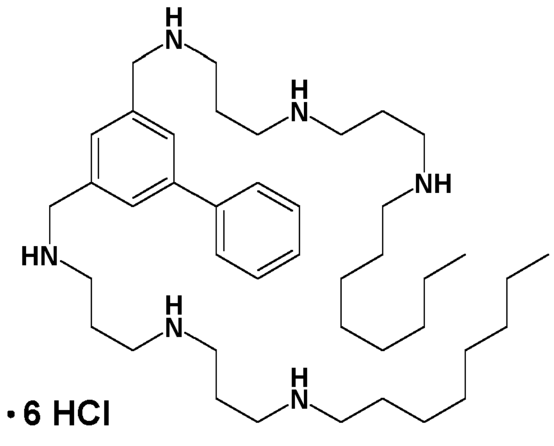

• 6 HCl ,

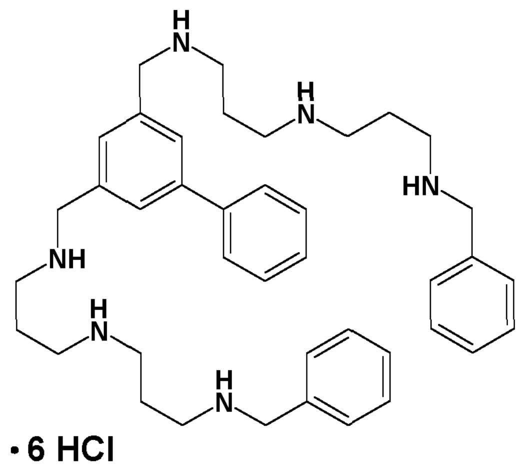

• 6 HCl ,

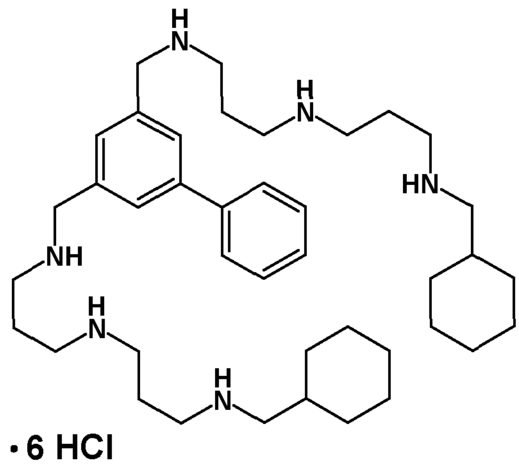

• 6 HCl ,

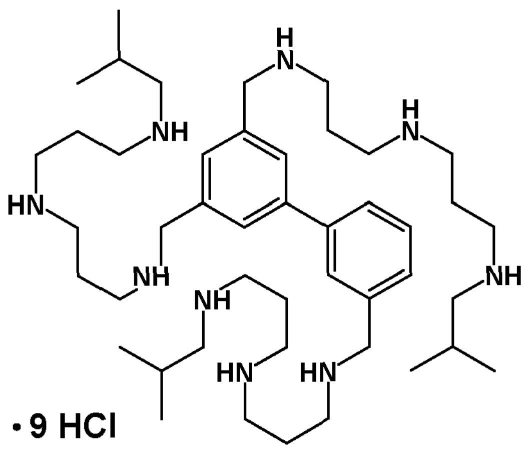

• 9 HCl ,

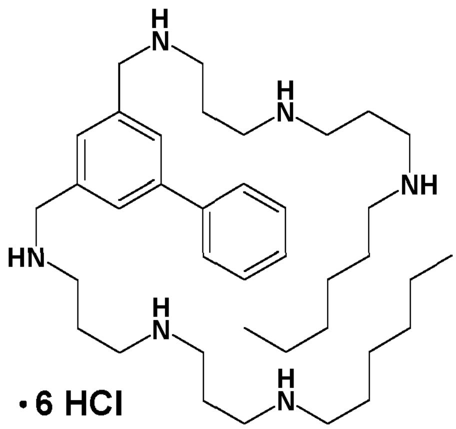

• 6 HCl ,

or selected from the group consisting of

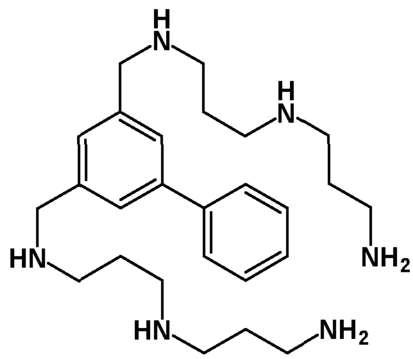,
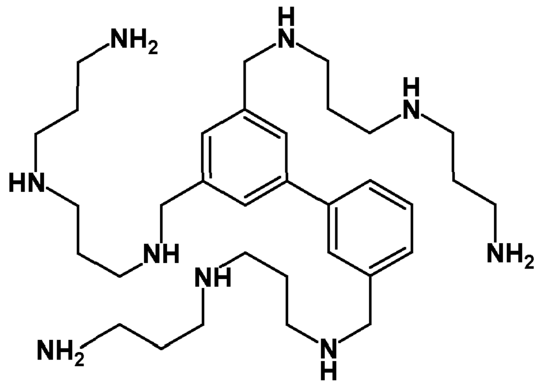,

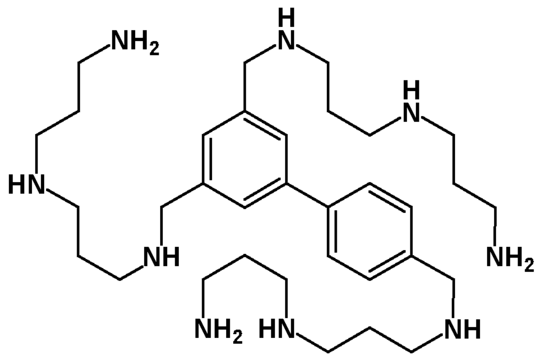,
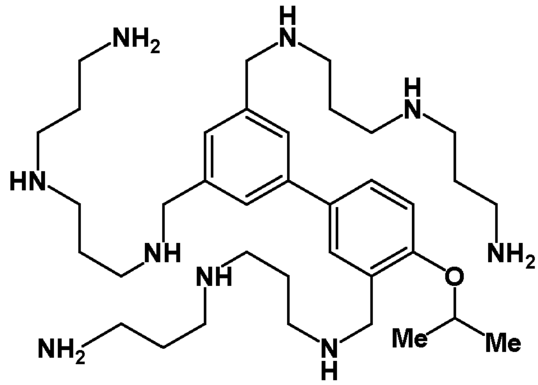,

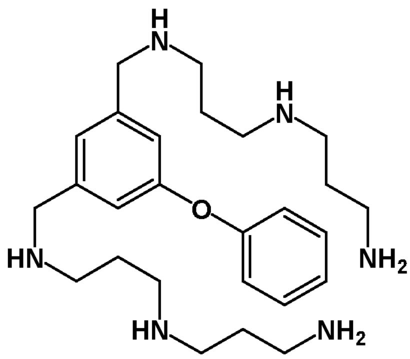,
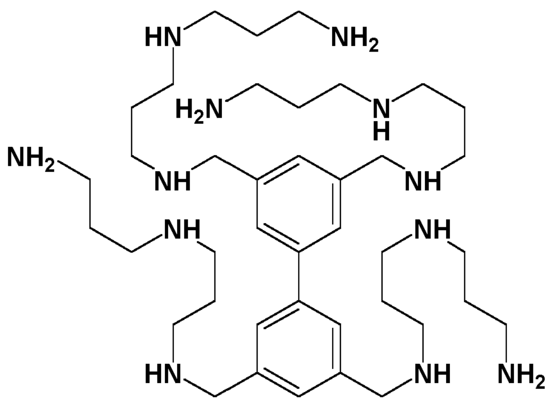,

and a salt thereof.

**8.** The method of any one of claims 1 to 4, wherein the polyamine compound is selected from the group consisting of

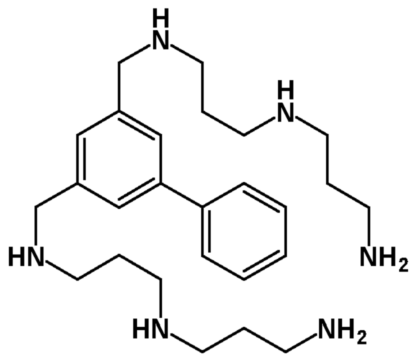
,
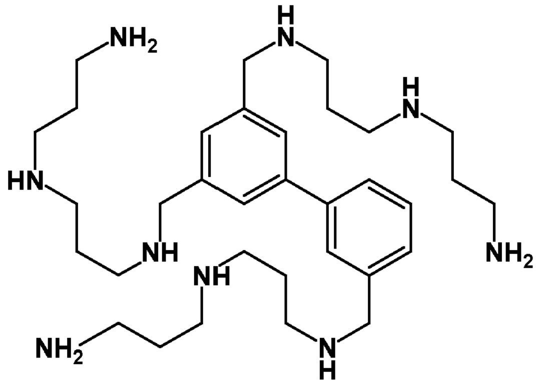
,
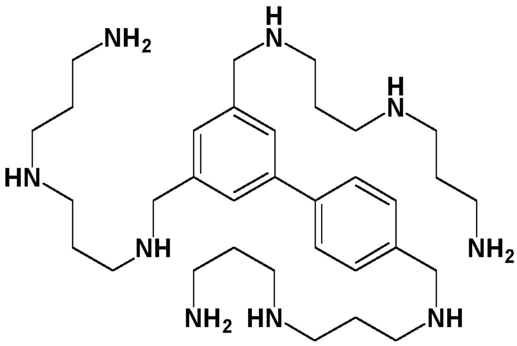
,
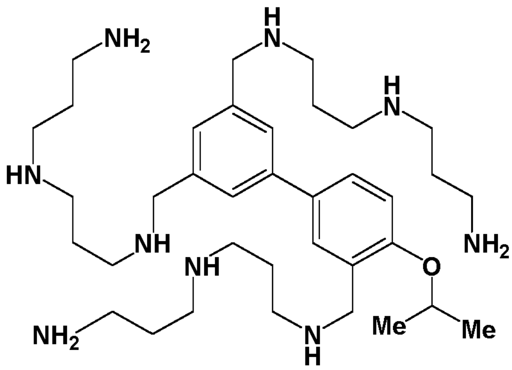
,
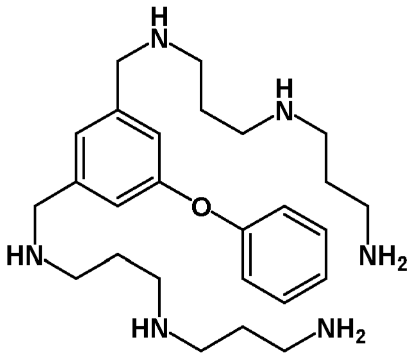
,
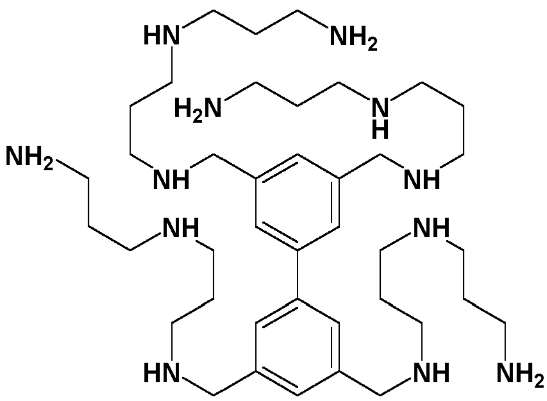
,

and a salt thereof.

**9.** The method of one of claims 1 to 8, wherein the biofilm comprises an antibiotic-resistant bacterial species.

**10.** The method of any one of claims 1 to 9, wherein the method comprises a step of:

(a) treating a contact lens with the anti-biofilm composition;
(b) treating a pipe with the anti-biofilm composition;
(c) treating a heating or cooling tower with the anti-biofilm composition; or
(d) coating an object with the anti-biofilm composition.

**11.** A non-therapeutic method for inhibiting formation of a biofilm, the method comprising a step of treating planktonic bacteria with the anti-biofilm composition used any one of claims 1 to 10, thereby inhibiting incorporation of the planktonic bacteria into the biofilm.

**12.** An anti-biofilm composition, the composition comprising the polyamine compound used in any one of claims 1 to 8.

**13.** The anti-biofilm composition of claim 12, wherein the polyamine compound is of formula

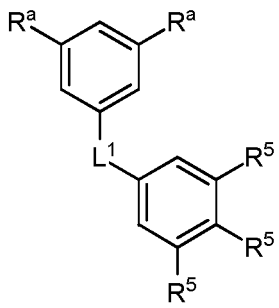

or a salt thereof.

**14.** A polyamine compound for use in therapeutic dispersing or killing of a biofilm, wherein the polyamine compound is selected from the group consisting of

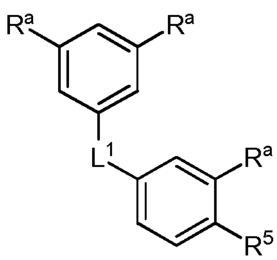,
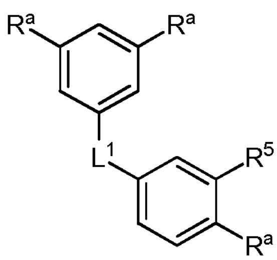,
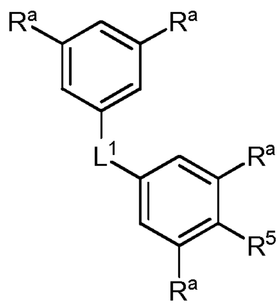,

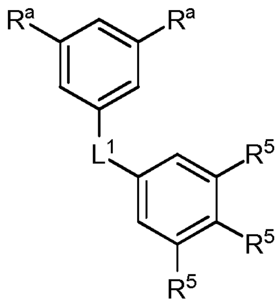,

and a salt thereof;
wherein:

each $L^1$ is selected from the group consisting of a bond and -O-;
each $R^a$ $-CH_2[NH(CH_2)_n]_pNHR^4$;
each n is an integer independently selected from 3 to 12;
each p is an integer independently selected from 1 to 3;
each $R^4$ is a member independently selected from the group consisting of hydrogen, alkyl, arylalkyl and cycloalkylalkyl; and
each $R^5$ is a member independently selected from the group consisting of hydrogen, alkyl, hydroxyl, alkoxy, halo, fluoroalkyl, fluoroalkyloxy, and hydroxyalkyl,
wherein the polyamine compound comprises at least four primary or secondary amino groups.

**15.** The polyamine compound of claim 14, further defined as in any of claims 2 - 8.

## Patentansprüche

1. Nicht-therapeutisches Verfahren zum Dispergieren oder Töten eines Biofilms, wobei das Verfahren einen Schritt des Behandelns des Biofilms mit einer Antibiofilmzusammensetzung umfasst, wodurch der Biofilm effektiv dispergiert oder getötet wird; wobei die Antibiofilmzusammensetzung eine Polyaminverbindung umfasst, die ausgewählt aus der Gruppe bestehend aus

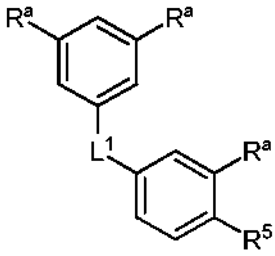,
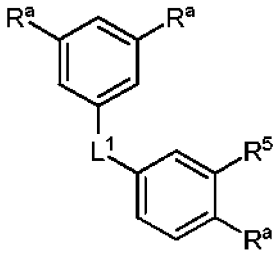,
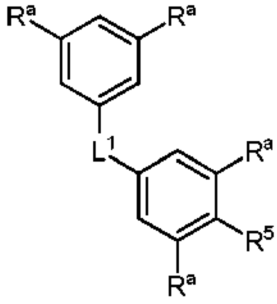,
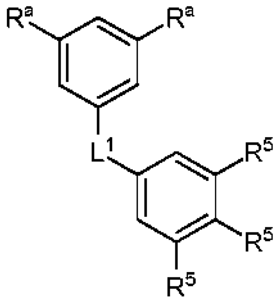

und einem Salz davon;
wobei:

jedes $L^1$ ausgewählt ist aus der Gruppe bestehend aus einer Bindung und -O-;
jedes $R^a$ $-CH_2[NH(CH_2)_n]_pNHR^4$ ist;
jedes n eine ganze Zahl ist, die unabhängig ausgewählt ist aus 3 bis 12;
jedes p eine ganze Zahl ist, die unabhängig ausgewählt ist aus 1 bis 3;
jedes $R^4$ ein Element ist, das unabhängig ausgewählt ist aus der Gruppe bestehend aus Wasserstoff, Alkyl, Arylalkyl und Cycloalkylalkyl; und
jedes $R^5$ ein Element ist, das unabhängig ausgewählt ist aus der Gruppe bestehend aus Wasserstoff, Alkyl, Hydroxyl, Alkoxy, Halogen, Fluoralkyl, Fluoralkyloxy und Hydroxyalkyl,
wobei die Polyaminverbindung mindestens vier primäre oder sekundäre Aminogruppen umfasst.

2. Verfahren nach Anspruch 1, wobei n 3 ist.

3. Verfahren nach Anspruch 1 oder Anspruch 2, wobei jedes $R^4$ ein Element ist, das unabhängig ausgewählt ist aus der Gruppe bestehend aus Wasserstoff und Alkyl.

4. Verfahren nach einem der Ansprüche 1 bis 3, wobei jedes $R^a$ $-CH_2[NH(CH_2)_n]_pNH_2$ ist.

5. Verfahren nach einem der Ansprüche 1 bis 4, wobei die Polyaminverbindung ausgewählt ist aus der Gruppe bestehend aus

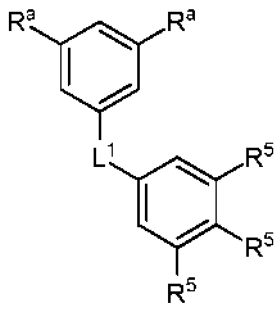

und einem Salz davon.

**6.** Verfahren nach Anspruch 1, wobei die Polyaminverbindung ausgewählt ist aus der Gruppe bestehend aus:

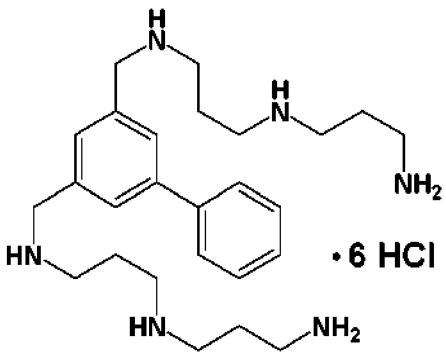

• 6 HCl ,

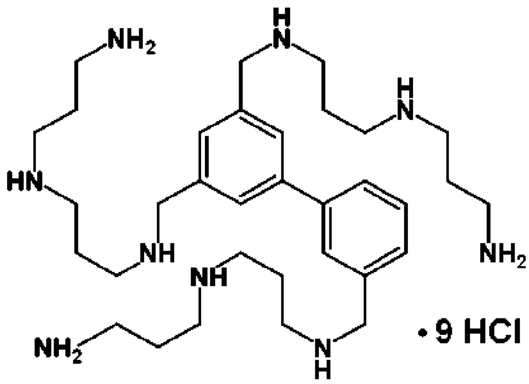

• 9 HCl ,

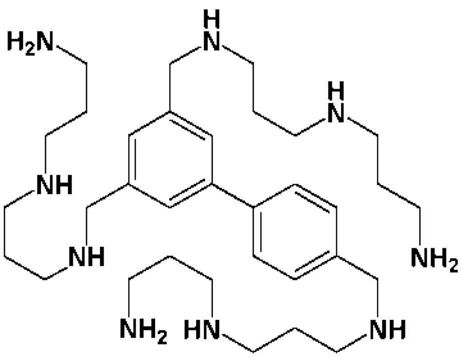

• 9 HCl ,

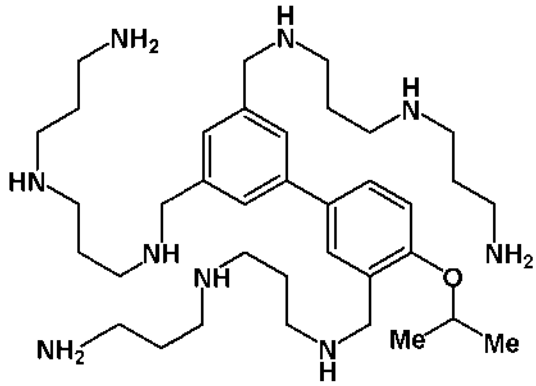

• 9 HCl ,

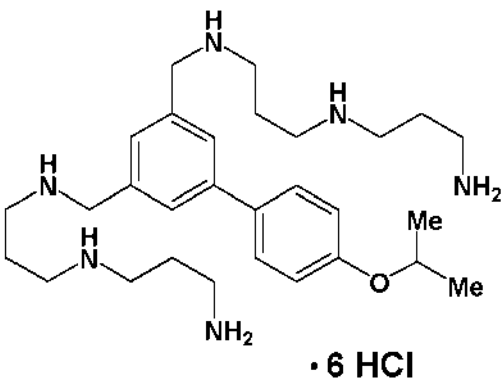

• 6 HCl ,

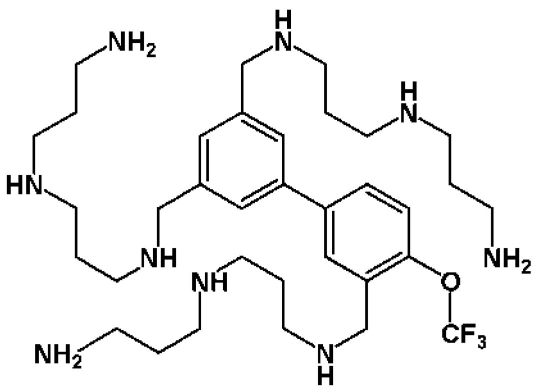

• 9 HCl ,

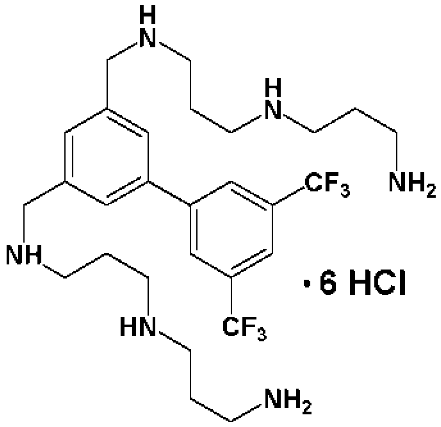

• 6 HCl

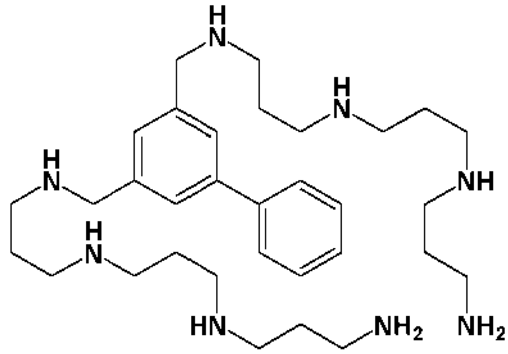

• 8 HCl ,

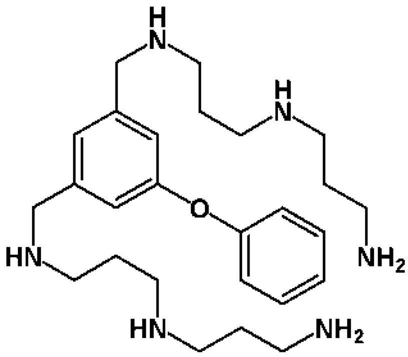

• 6 HCl ,

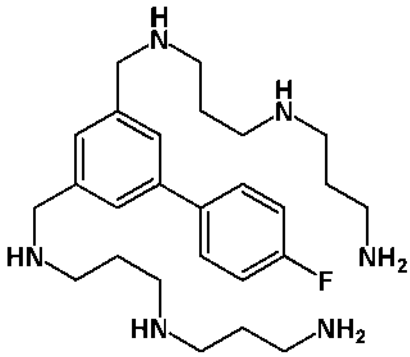

• 6 HCl ,

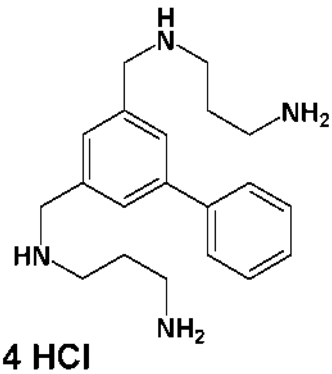

• 4 HCl ,

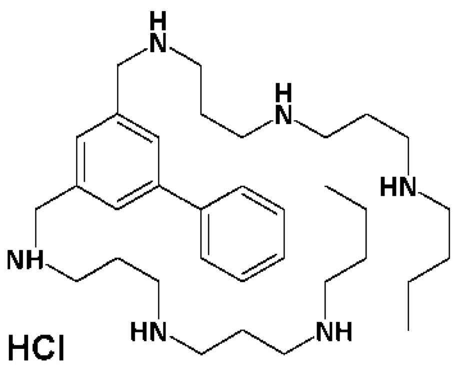

• 6 HCl ,

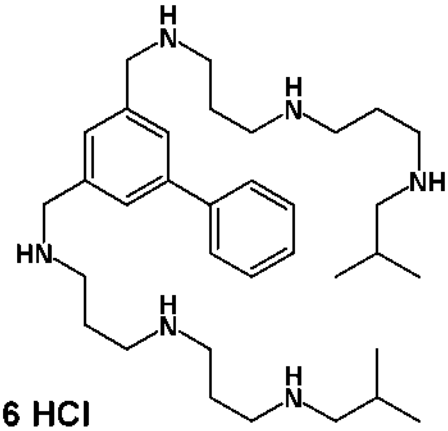

•6 HCl ,

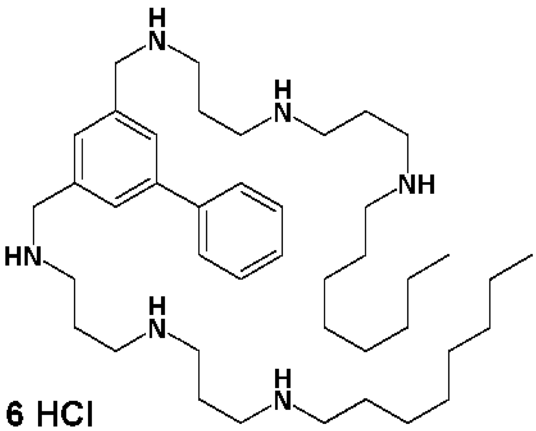

•6 HCl ,

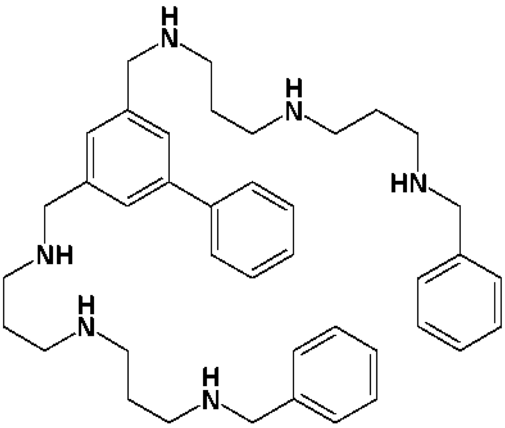

•6 HCl ,

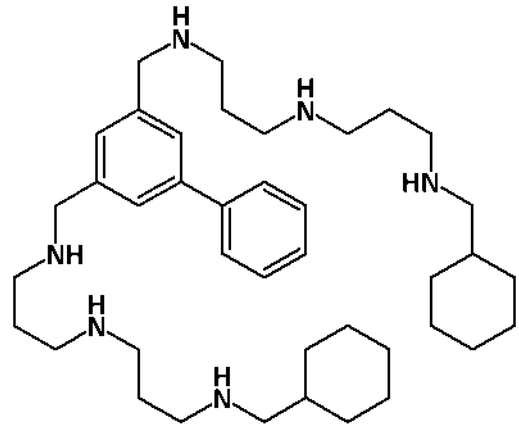

•6 HCl ,

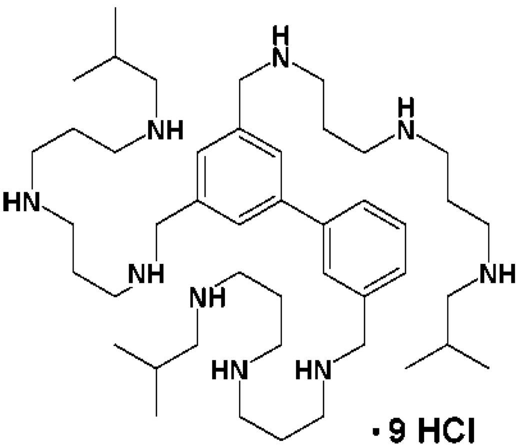

•9 HCl ,

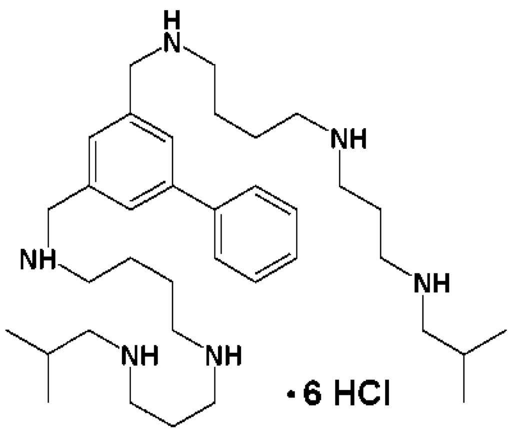

•6 HCl ,

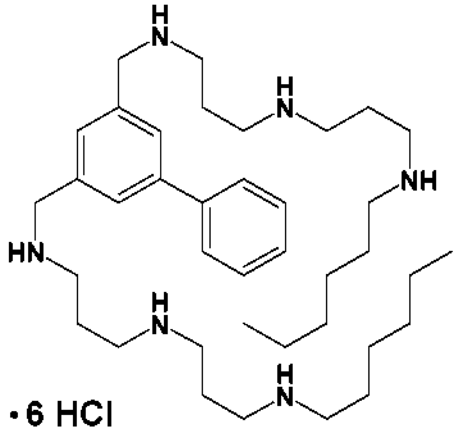

• 6 HCl ,

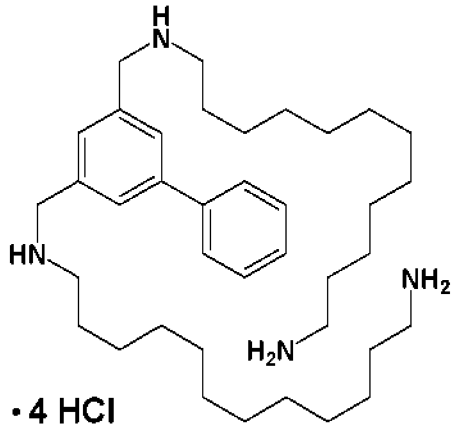

• 4 HCl ,

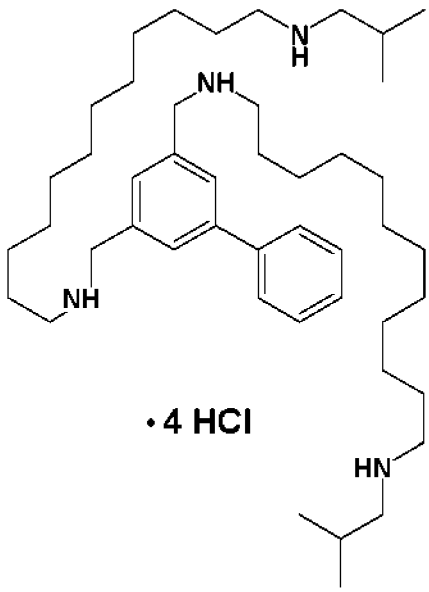

• 4 HCl ,

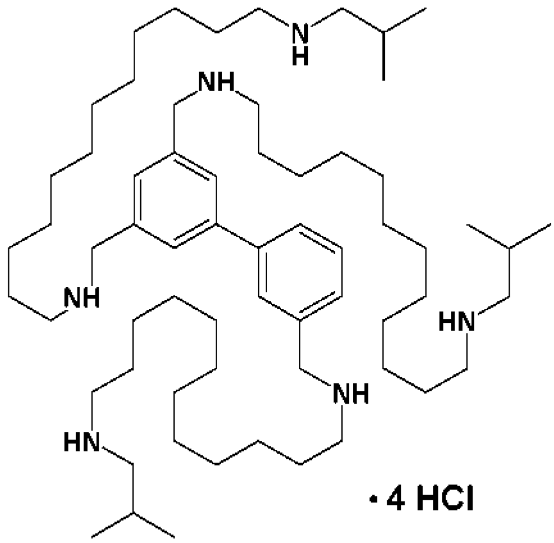

• 4 HCl ,

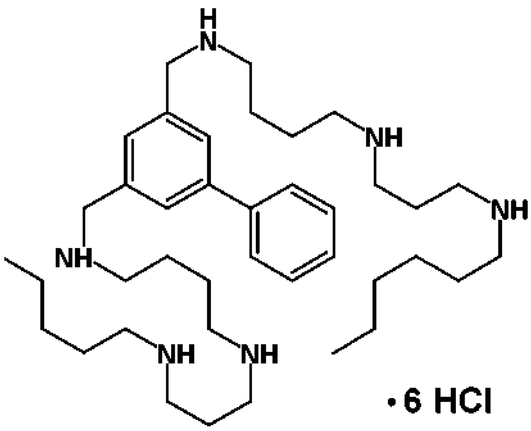

• 6 HCl .

oder ausgewählt aus der Gruppe bestehend aus

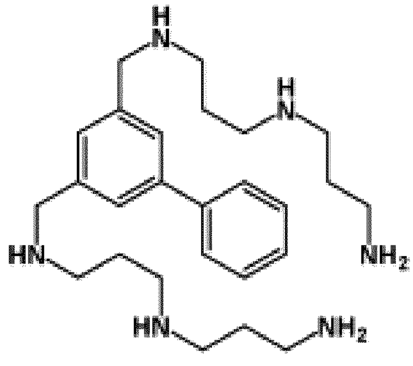
,
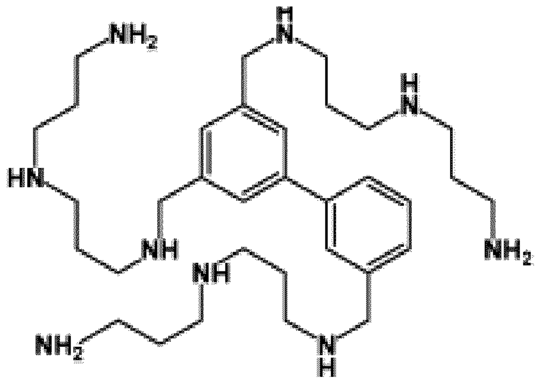
,

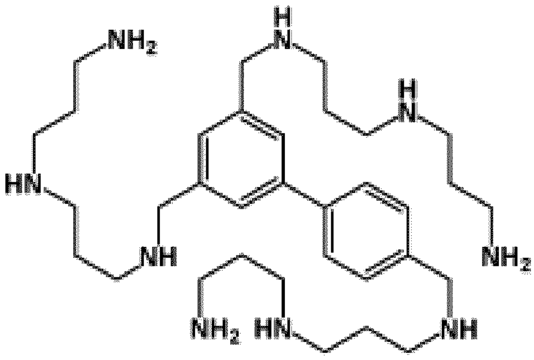
,
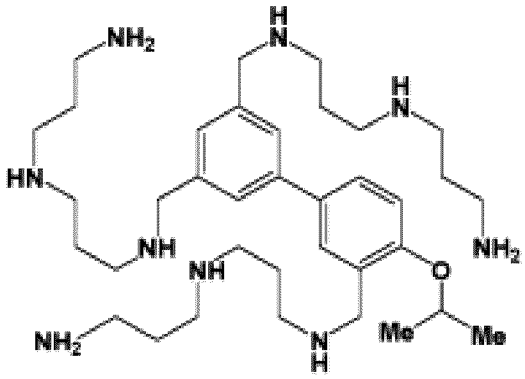
,

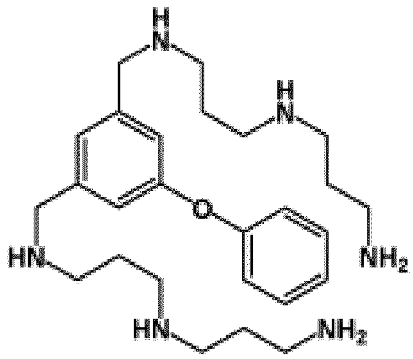
,
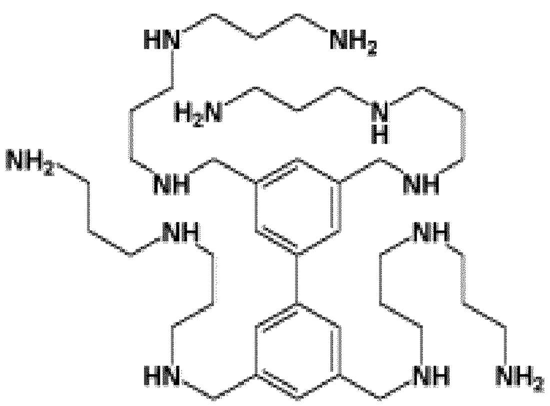
,

und einem Salz davon.

7. Verfahren nach Anspruch 1 oder Anspruch 2, wobei die Polyaminverbindung ausgewählt ist aus der Gruppe bestehend aus:

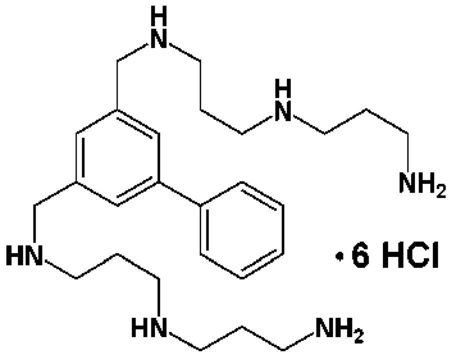

• 6 HCl ,

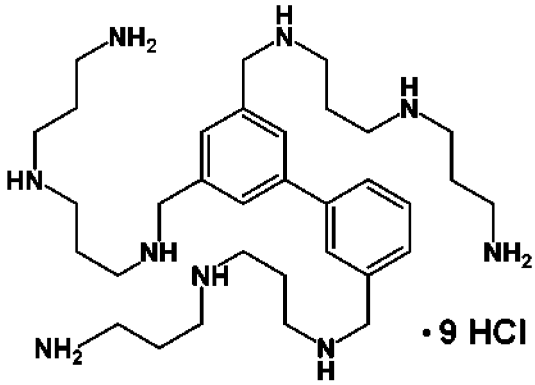

• 9 HCl ,

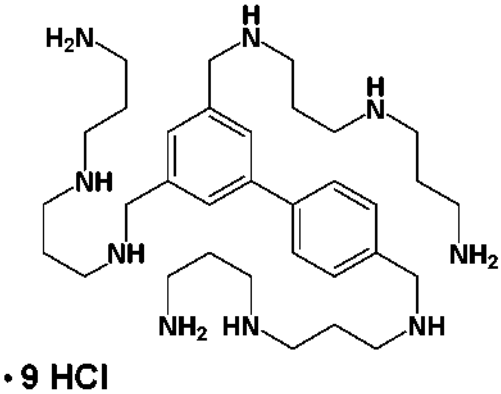

• 9 HCl ,

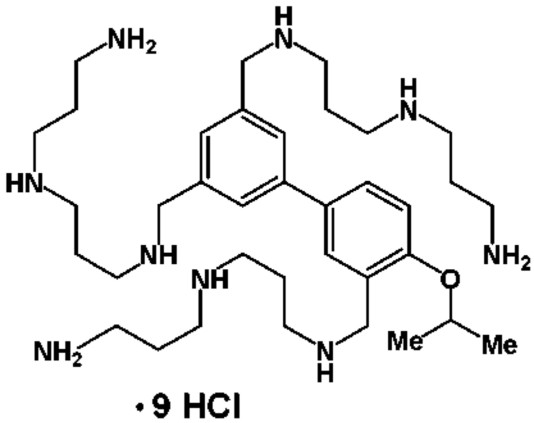

• 9 HCl ,

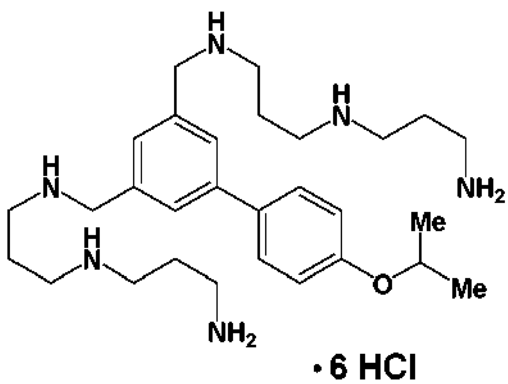

• 6 HCl ,

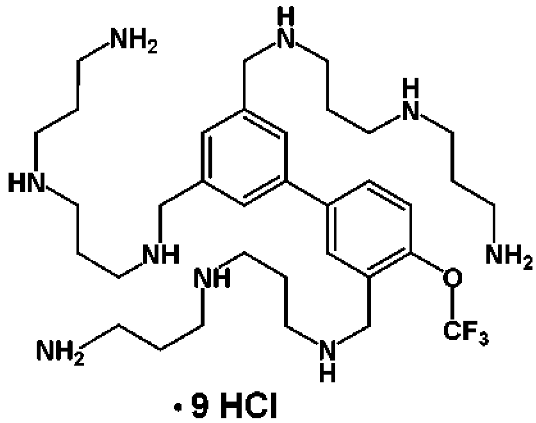

• 9 HCl ,

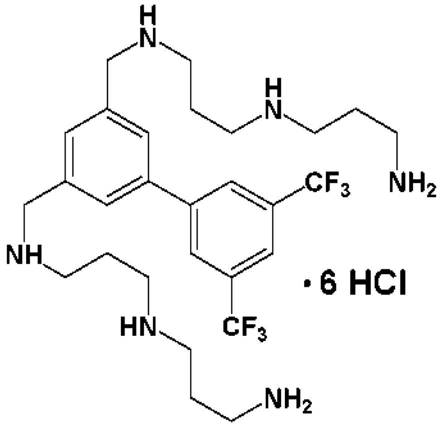

• 6 HCl ,

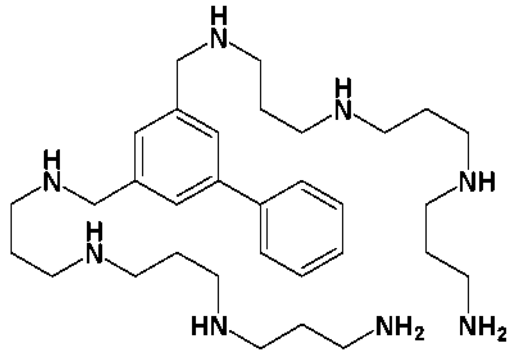

• 8 HCl ,

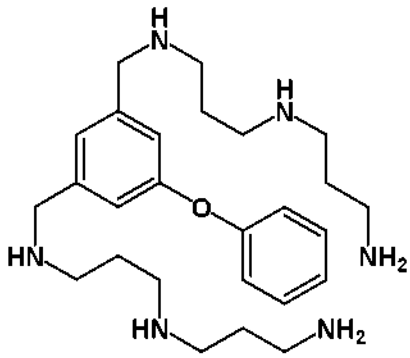

• 6 HCl ,

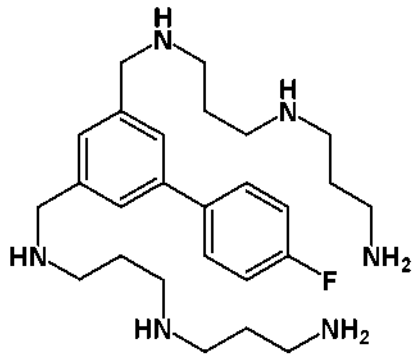

• 6 HCl ,

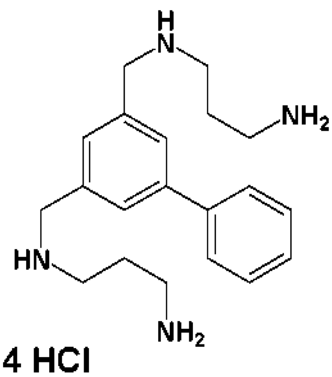

• 4 HCl ,

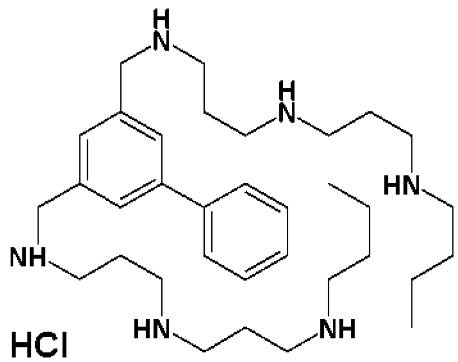

• 6 HCl ,

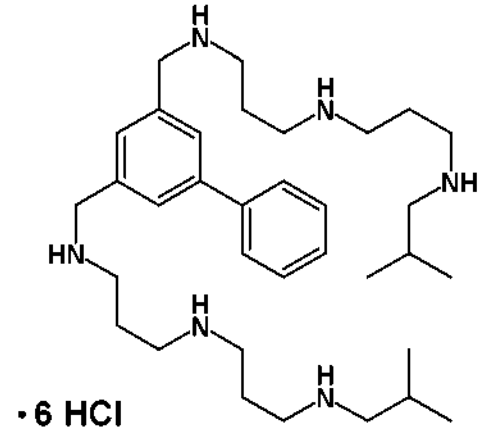

• 6 HCl ,

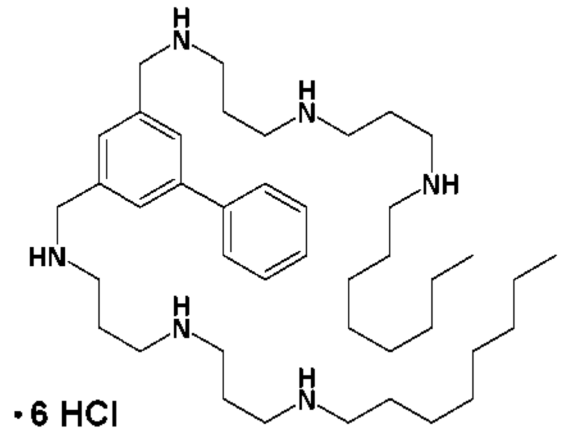

• 6 HCl ,

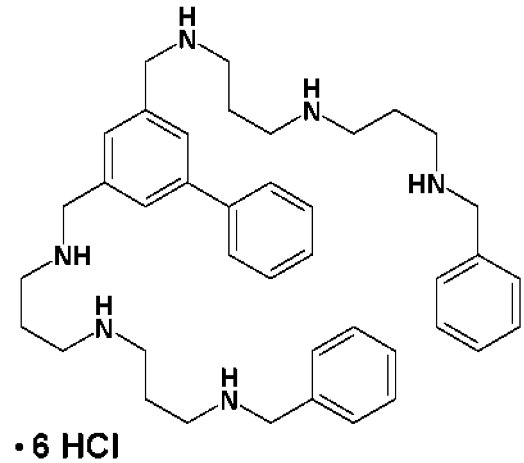

• 6 HCl ,

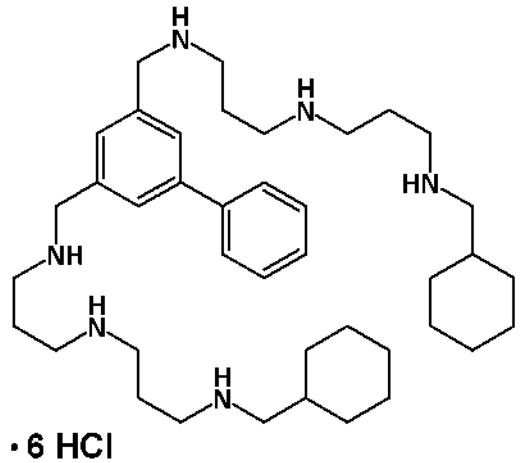

• 6 HCl ,

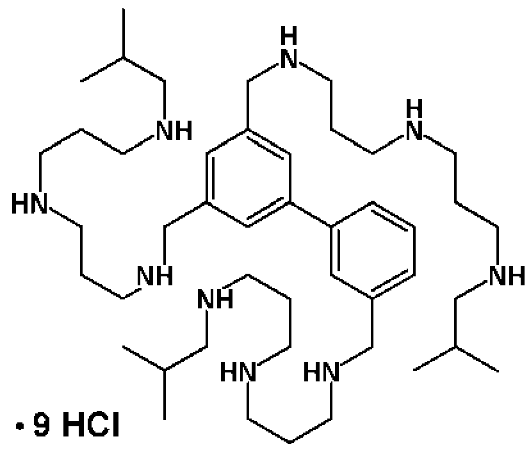

• 9 HCl ,

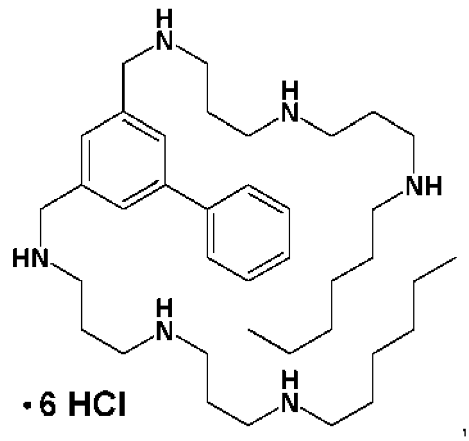

• 6 HCl ,

oder ausgewählt aus der Gruppe bestehend aus

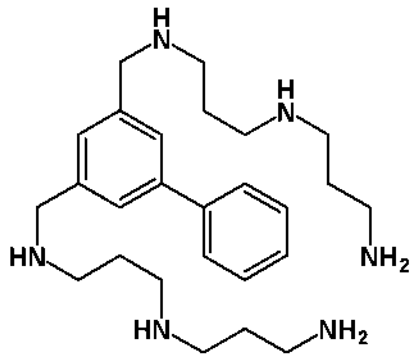

,

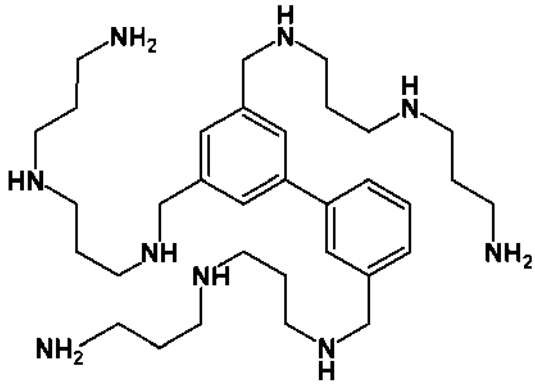

,

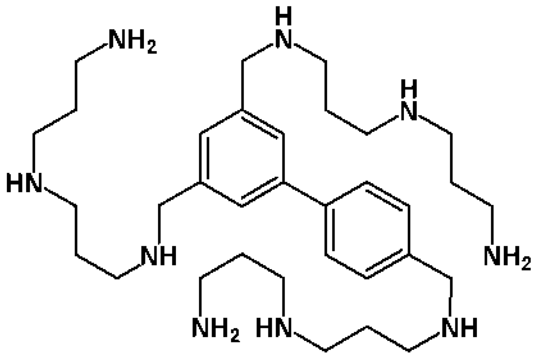

,

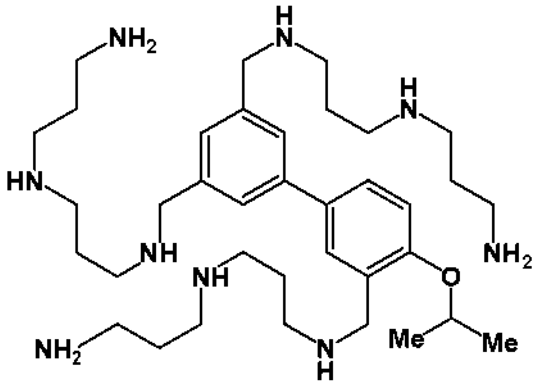

,

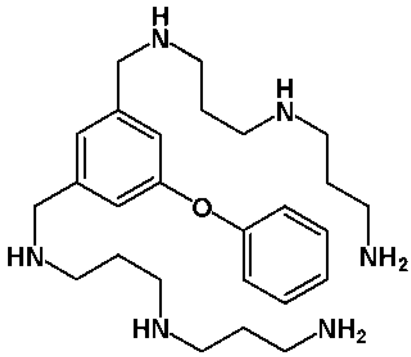

,

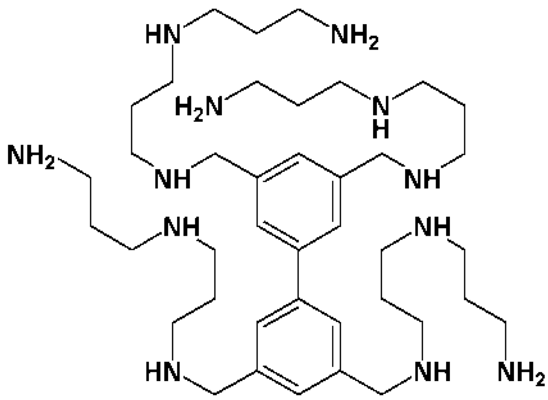

,

und einem Salz davon.

**8.** Verfahren nach einem der Ansprüche 1 bis 4, wobei die Polyaminverbindung ausgewählt ist aus der Gruppe bestehend aus

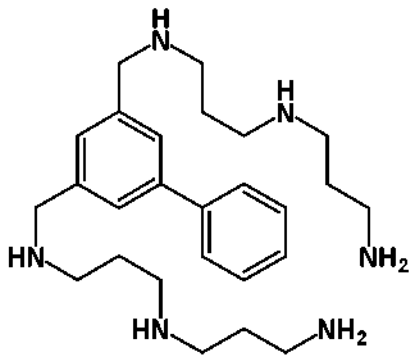

,

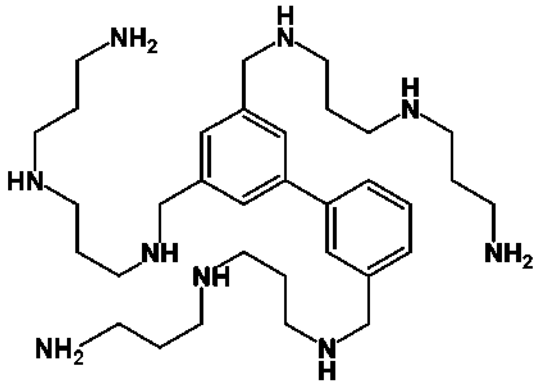

,

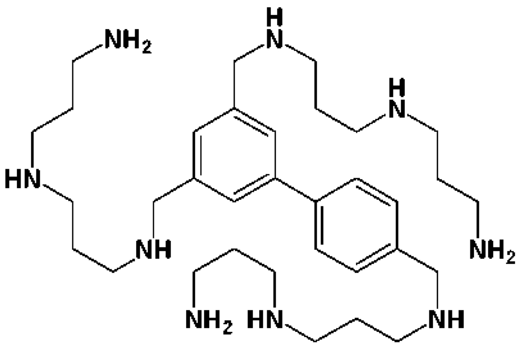

,

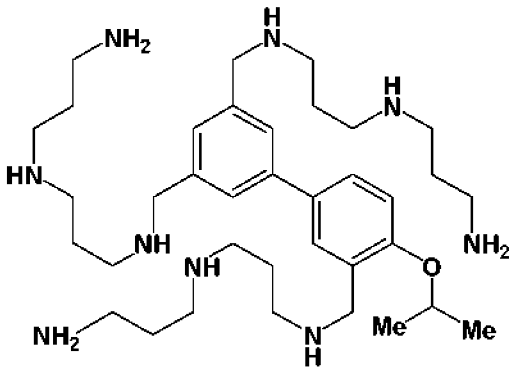

,

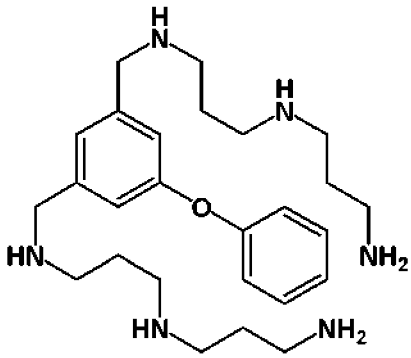

,

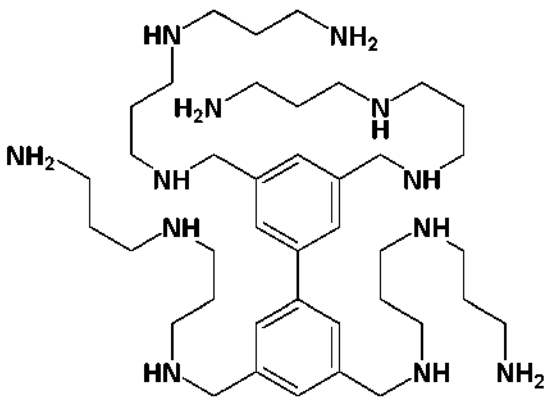

,

und einem Salz davon.

9. Verfahren nach einem der Ansprüche 1 bis 8, wobei der Biofilm eine antibiotikaresistente Bakterienspezies umfasst.

10. Verfahren nach einem der Ansprüche 1 bis 9, wobei das Verfahren einen Schritt wie folgt umfasst:

(a) Behandeln einer Kontaktlinse mit der Antibiofilmzusammensetzung;
(b) Behandeln eines Rohrs mit der Antibiofilmzusammensetzung;
(c) Behandeln eines Heiz- oder Kühlturms mit der Antibiofilmzusammensetzung; oder
(d) Beschichten eines Objekts mit der Antibiofilmzusammensetzung.

11. Nicht-therapeutisches Verfahren zum Hemmen der Bildung eines Biofilms, wobei das Verfahren einen Schritt des Behandelns von planktonischen Bakterien mit der Antibiofilmzusammensetzung umfasst, die in einem der Ansprüche 1 bis 10 verwendet wurde, wodurch der Einbau der planktonischen Bakterien in den Biofilm gehemmt wird.

12. Antibiofilmzusammensetzung, wobei die Zusammensetzung die Polyaminverbindung umfasst, die in einem der

Ansprüche 1 bis 8 verwendet wurde.

13. Antibiofilmzusammensetzung nach Anspruch 12, wobei die Polyaminverbindung die folgende Formel hat

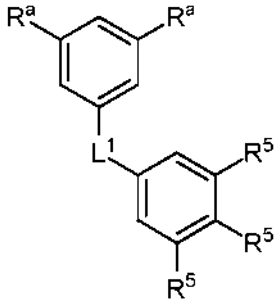

oder ein Salz davon ist.

14. Polyaminverbindung zur Verwendung im therapeutischen Dispergieren oder Töten eines Biofilms, wobei die Polyaminverbindung ausgewählt ist aus der Gruppe bestehend aus:

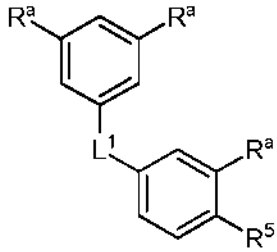
,
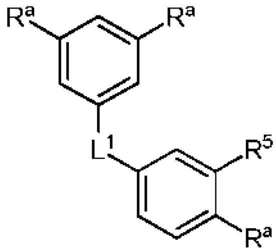
,
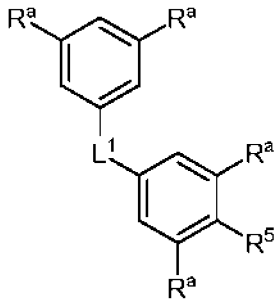
,
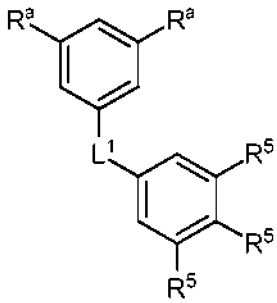

und einem Salz davon; wobei:

jedes $L^1$ ausgewählt ist aus der Gruppe bestehend aus einer Bindung und -O-;
jedes $R^a$ $-CH_2[NH(CH_2)_n]_pNHR^4$ ist;
jedes n eine ganze Zahl ist, die unabhängig ausgewählt ist aus 3 bis 12;
jedes p eine ganze Zahl ist, die unabhängig ausgewählt ist aus 1 bis 3;
jedes $R^4$ ein Element ist, das unabhängig ausgewählt ist aus der Gruppe bestehend aus Wasserstoff, Alkyl, Arylalkyl und Cycloalkylalkyl; und
jedes $R^5$ ein Element ist, das unabhängig ausgewählt ist aus der Gruppe bestehend aus Wasserstoff, Alkyl, Hydroxyl, Alkoxy, Halogen, Fluoralkyl, Fluoralkyloxy und Hydroxyalkyl,
wobei die Polyaminverbindung mindestens vier primäre oder sekundäre Aminogruppen umfasst.

15. Polyaminverbindung nach Anspruch 14, die des Weiteren wie in einem der Ansprüche 2 bis 8 definiert ist.

## Revendications

**1.** Procédé non thérapeutique pour la dispersion ou la destruction d'un biofilm, le procédé comprenant une étape de traitement du biofilm avec une composition anti-biofilm, dispersant ou détruisant ainsi efficacement le biofilm ; la composition anti-biofilm comprenant un composé de type polyamine choisi dans le groupe constitué par

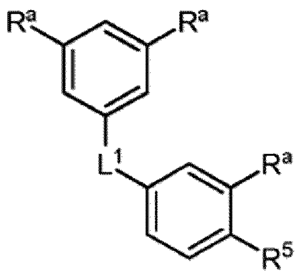,
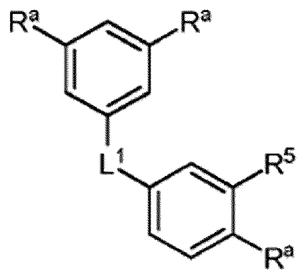,
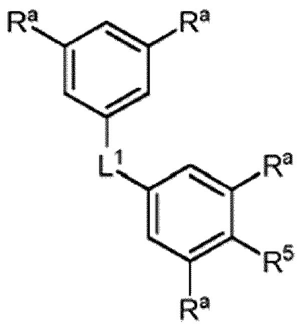,
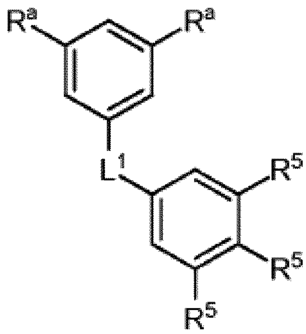,

et un sel correspondant ;

chaque $L^1$ étant choisi dans le groupe constitué par une liaison et -O- ;
chaque $R^a$ étant $-CH_2[NH(CH_2)_n]_pNHR^4$
chaque n étant un entier indépendamment choisi parmi 3 à 12 ;
chaque p étant un entier indépendamment choisi parmi 1 à 3 ;
chaque $R^4$ étant un élément indépendamment choisi dans le groupe constitué par hydrogène, alkyle, arylalkyle et cycloalkylalkyle ; et
chaque $R^5$ étant un élément indépendamment choisi dans le groupe constitué par hydrogène, alkyle, hydroxyle, alcoxy, halogéno, fluoroalkyle, fluoroalkyloxy et hydroxyalkyle,
le composé de type polyamine comprenant au moins quatre groupes amino primaire ou secondaire.

**2.** Procédé selon la revendication 1, n étant 3.

**3.** Procédé selon la revendication 1 ou la revendication 2, chaque $R^4$ étant un membre indépendamment choisi dans le groupe constitué par hydrogène et alkyle.

**4.** Procédé selon l'une quelconque des revendications 1 à 3, chaque $R^a$ étant $-CH_2[NH(CH_2)_n]_pNH_2$

**5.** Procédé selon l'une quelconque des revendications 1 à 4, le composé de type polyamine étant choisi dans le groupe constitué par

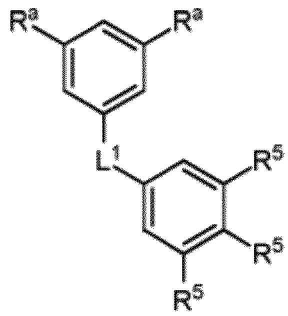

et un sel correspondant.

6. Procédé selon la revendication 1, le composé de type polyamine étant choisi dans le groupe constitué par

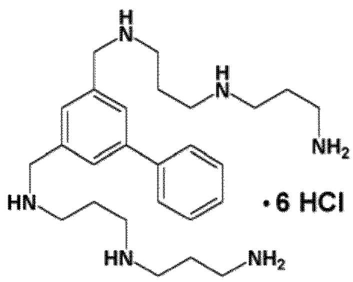

• 6 HCl ,

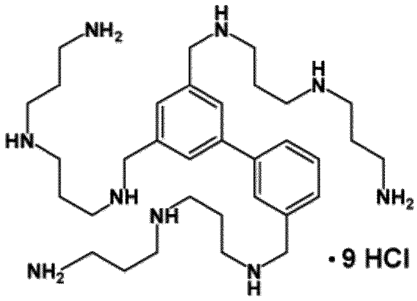

• 9 HCl ,

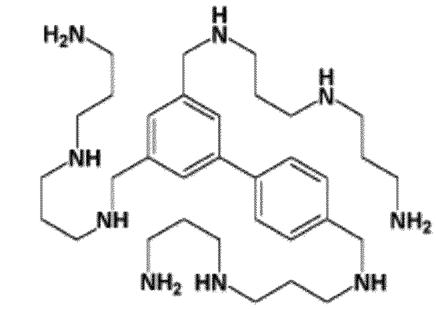

• 9 HCl ,

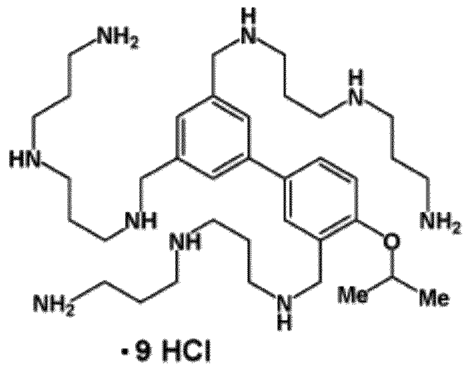

• 9 HCl ,

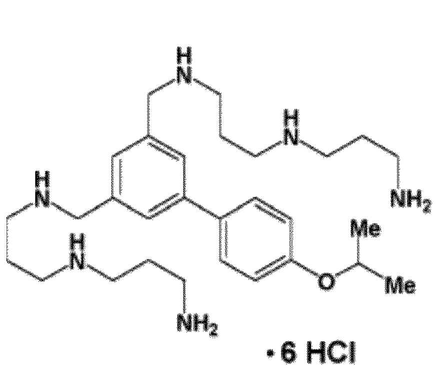

• 6 HCl ,

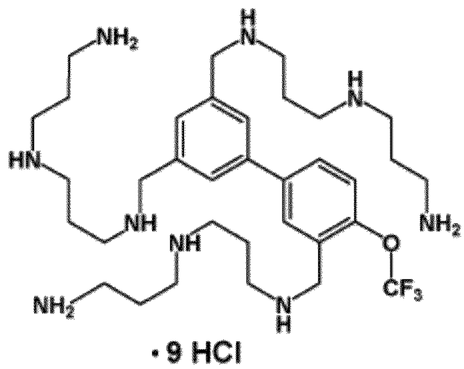

• 9 HCl ,

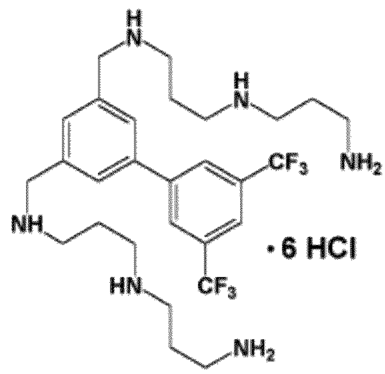
,
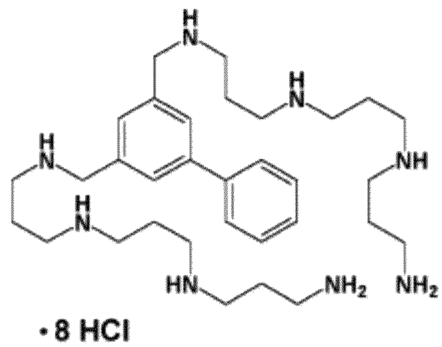
,

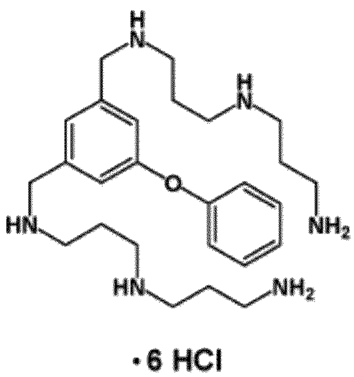
,
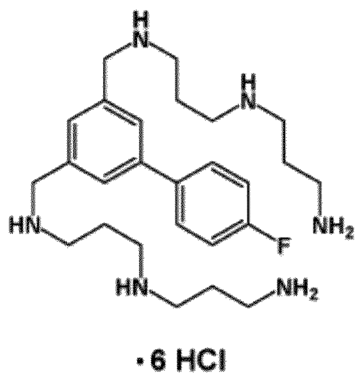
,

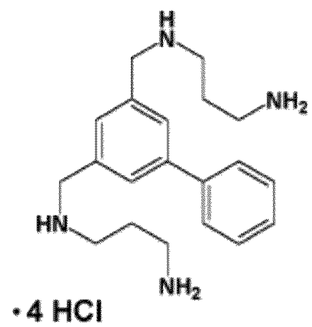
,
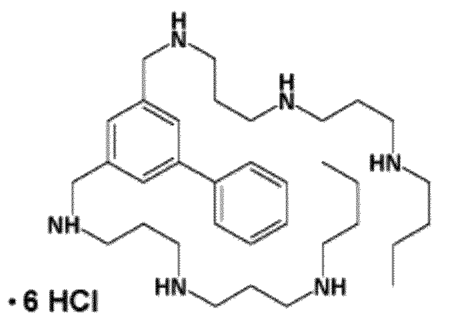
,

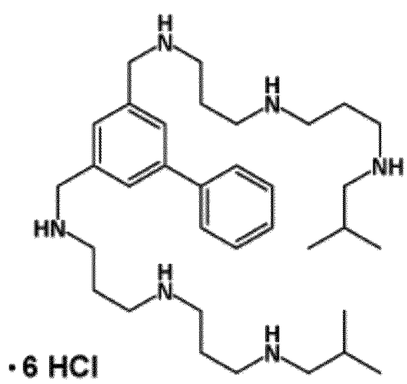
,
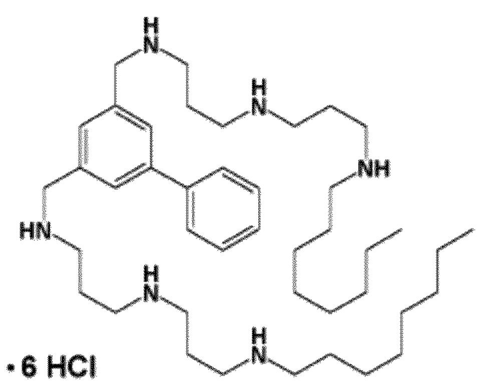
,

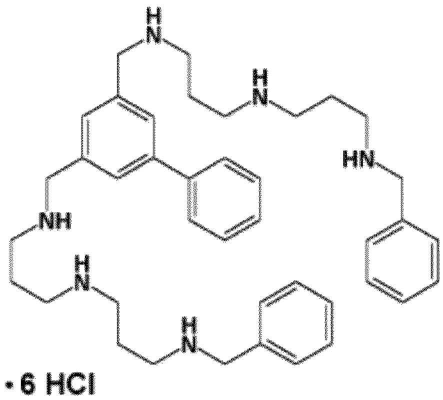

· 6 HCl ,

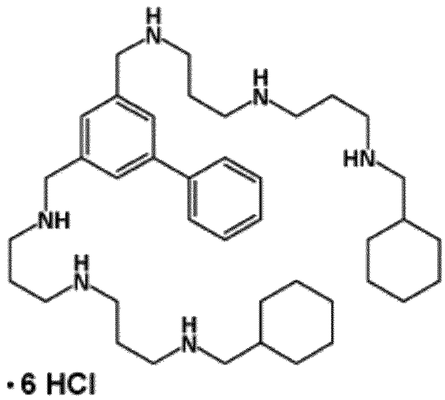

· 6 HCl ,

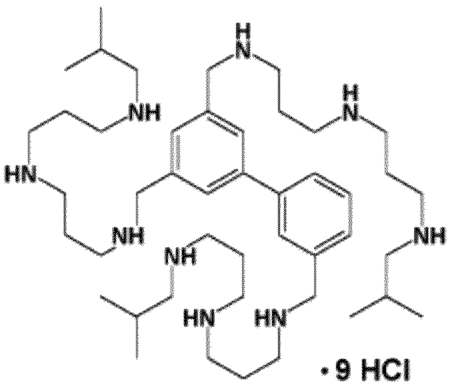

· 9 HCl ,

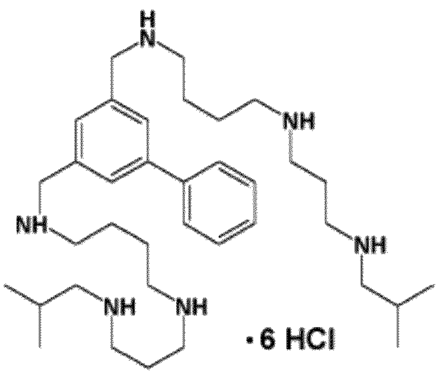

· 6 HCl ,

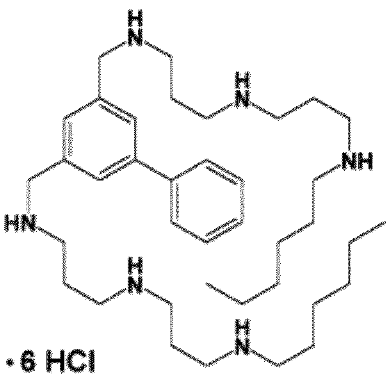

· 6 HCl ,

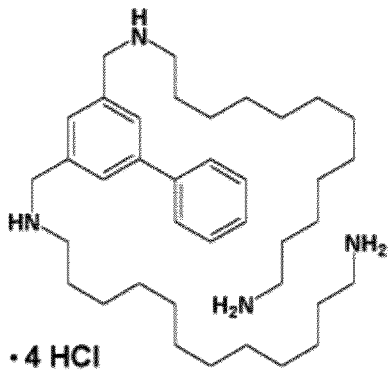

· 4 HCl ,

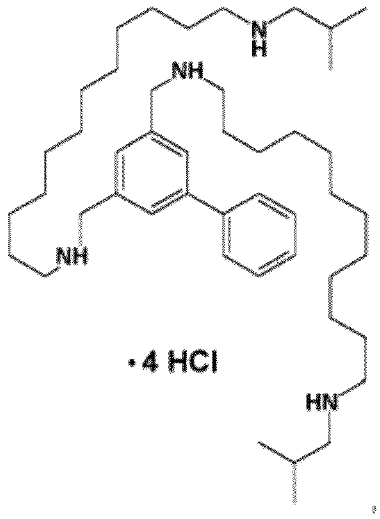

• 4 HCl ,

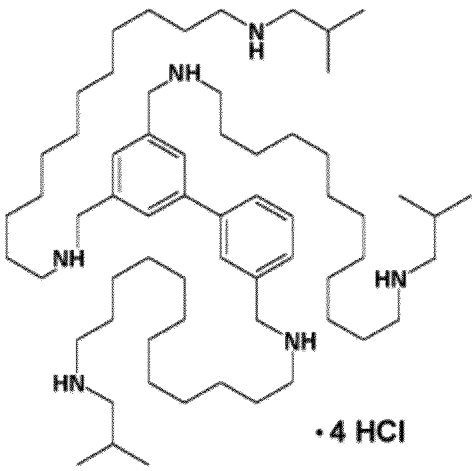

• 4 HCl ,

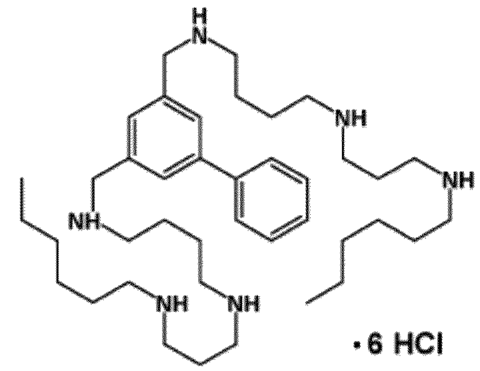

• 6 HCl ,

ou choisi dans le groupe constitué par

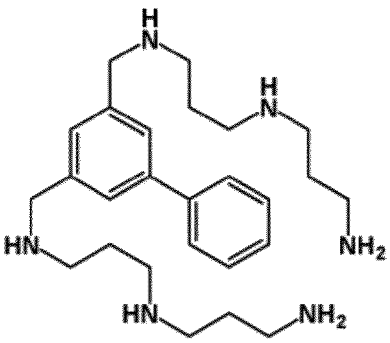

,

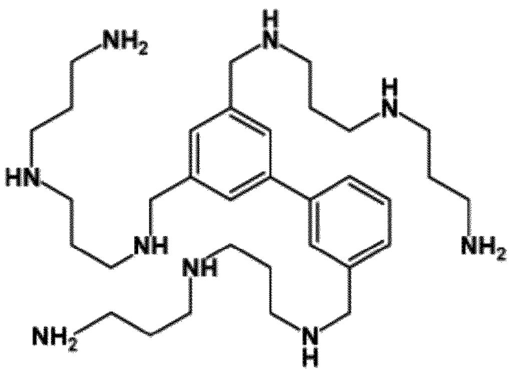

,

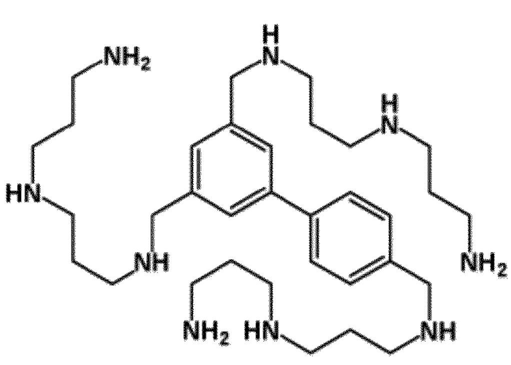
,
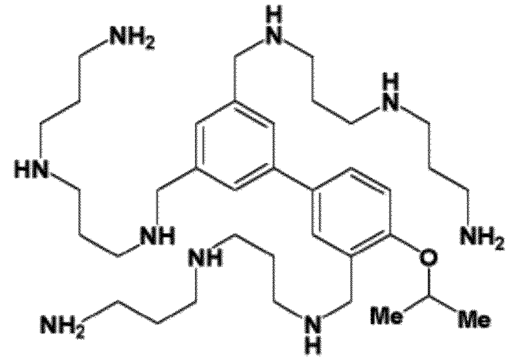
,
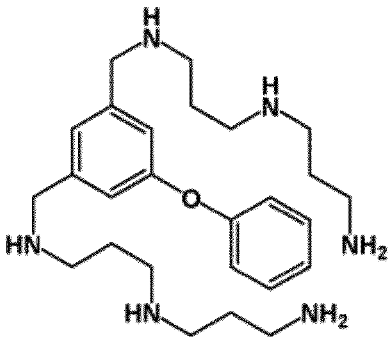
,
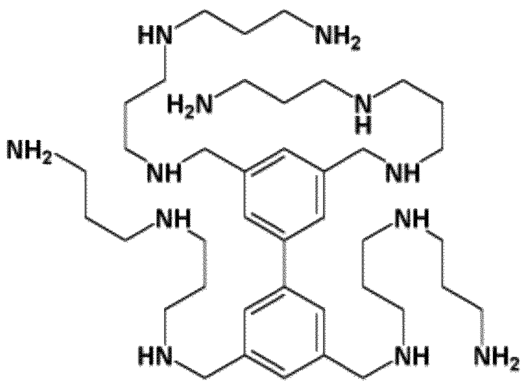
,

et un sel correspondant.

7. Procédé selon la revendication 1 ou la revendication 2, le composé de type polyamine étant choisi dans le groupe constitué par

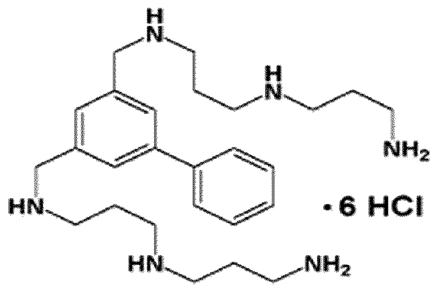
• 6 HCl
,
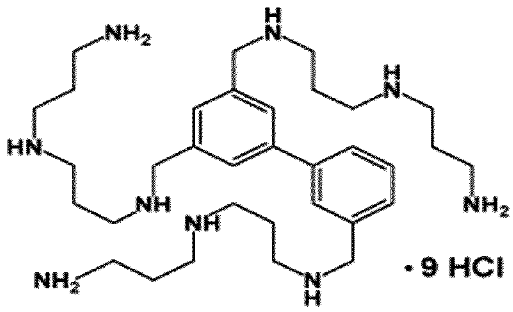
• 9 HCl
,
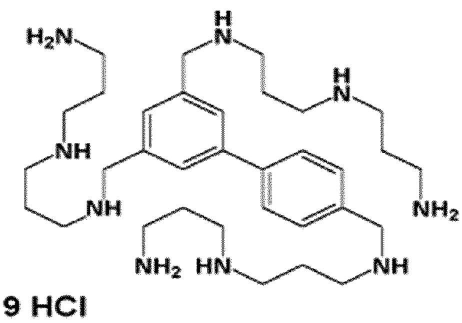
• 9 HCl
,
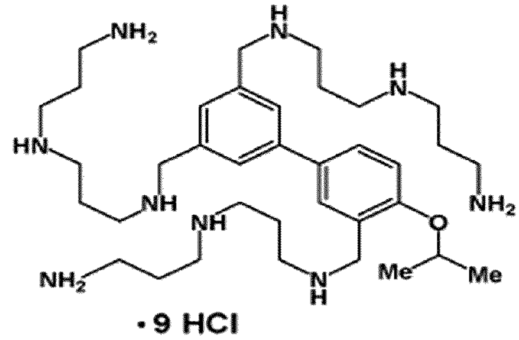
• 9 HCl
,

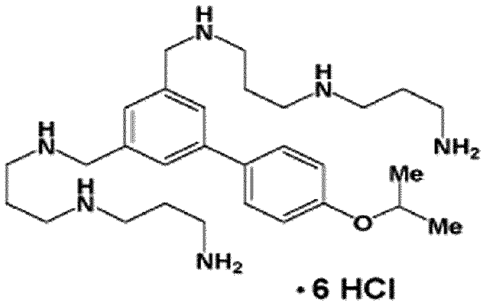

•6 HCl ,

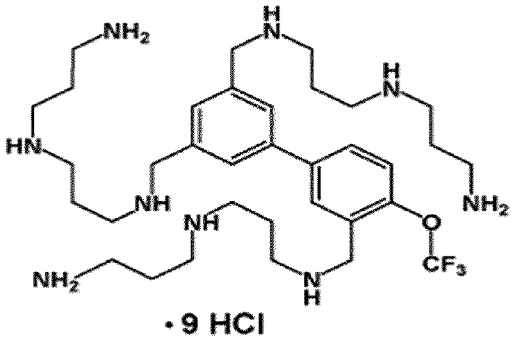

•9 HCl ,

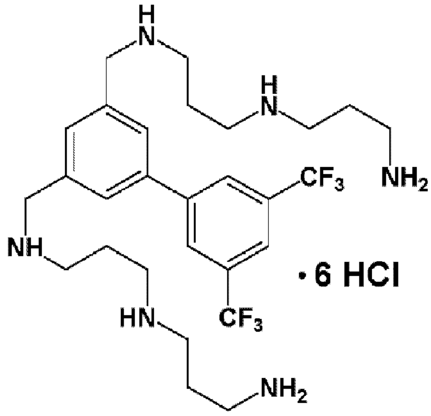

•6 HCl ,

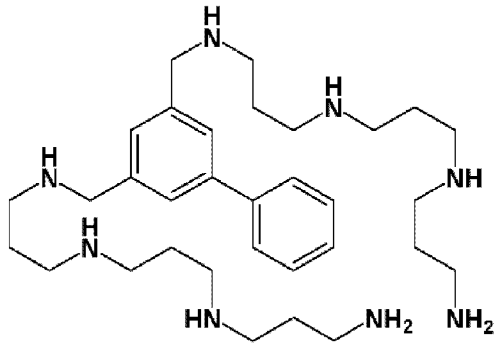

•8 HCl ,

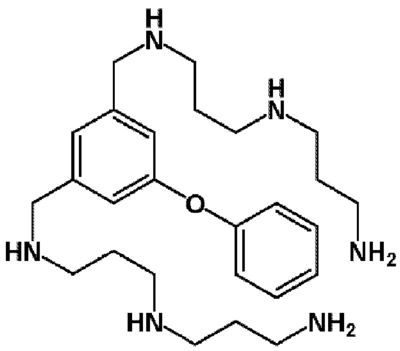

•6 HCl ,

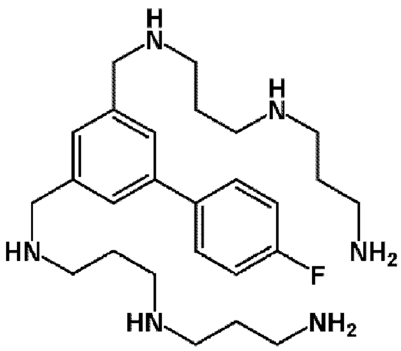

•6 HCl ;

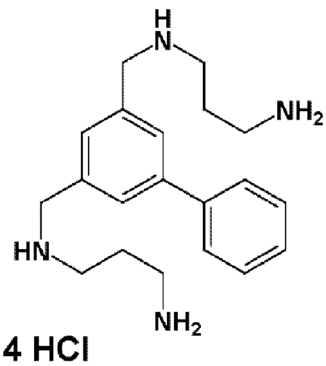

•4 HCl ,

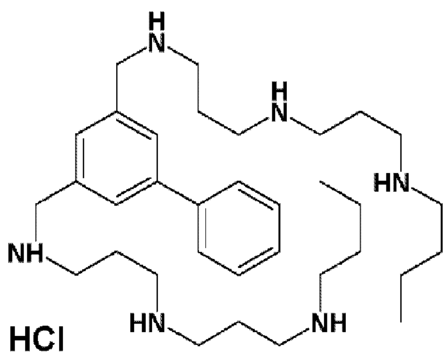

•6 HCl ,

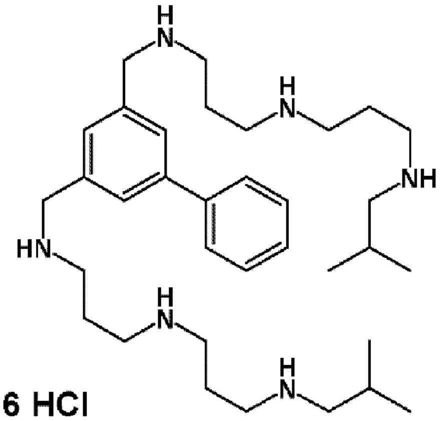

• 6 HCl ;

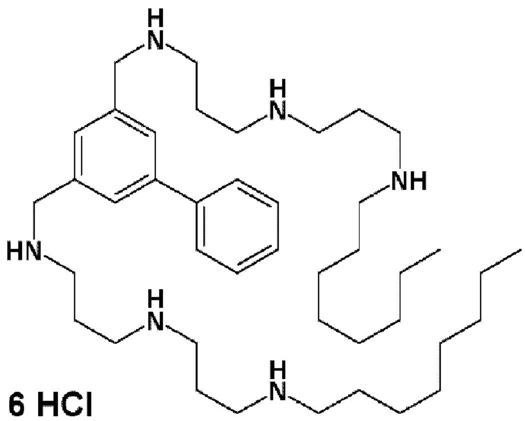

• 6 HCl ,

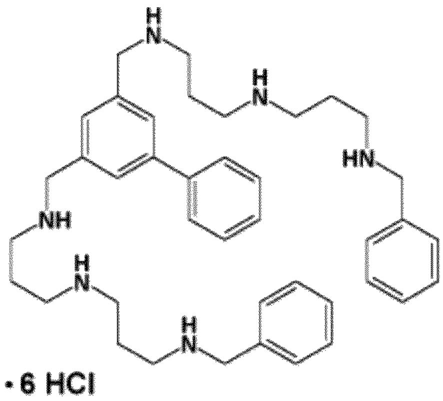

• 6 HCl ,

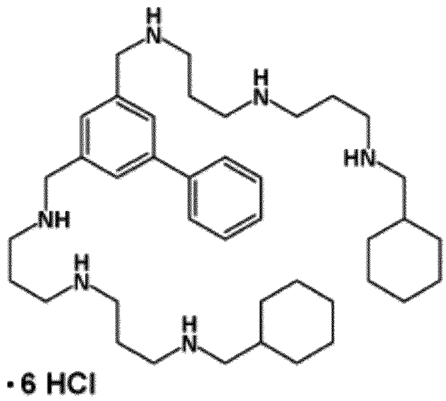

• 6 HCl ,

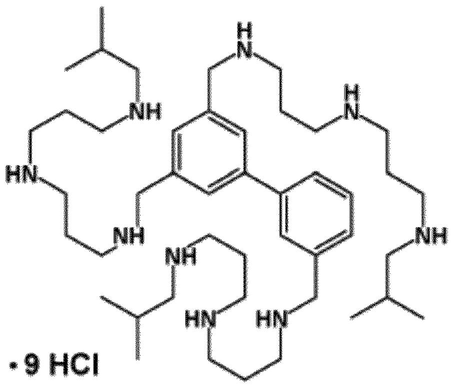

• 9 HCl ,

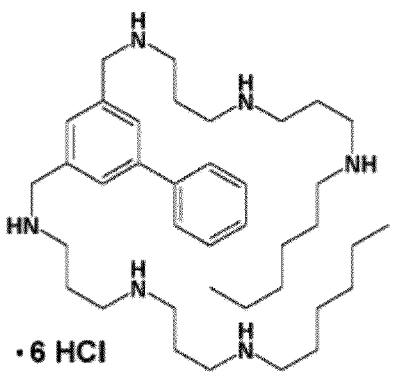

• 6 HCl ,

ou choisi dans le groupe constitué par

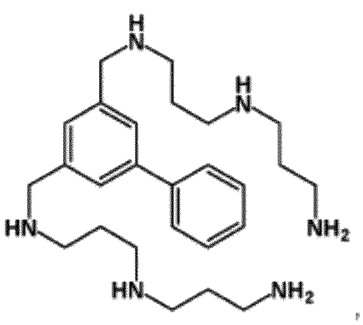

,

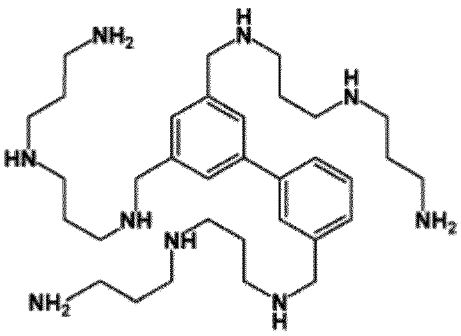

,

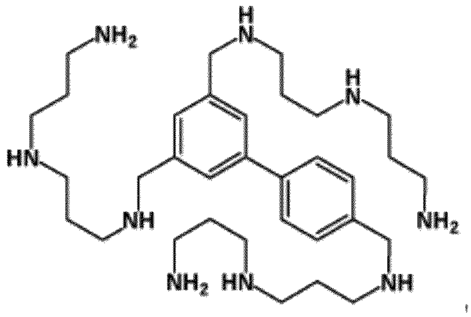 , 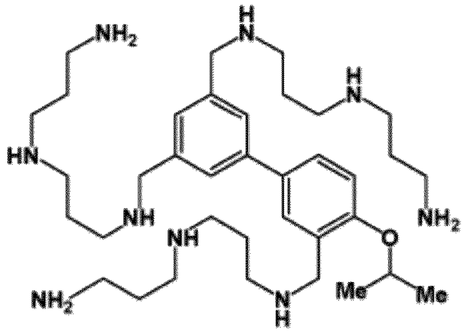 ,

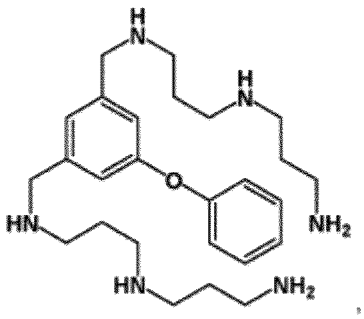 , 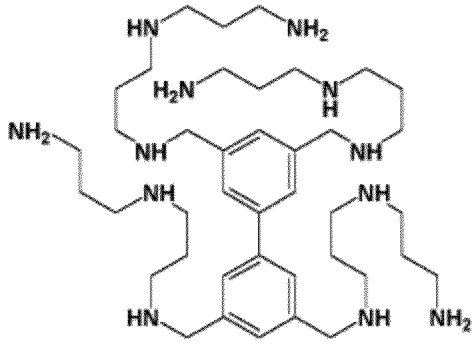 ,

et un sel correspondant.

**8.** Procédé selon l'une quelconque des revendications 1 à 4, le composé de type polyamine étant choisi dans le groupe constitué par

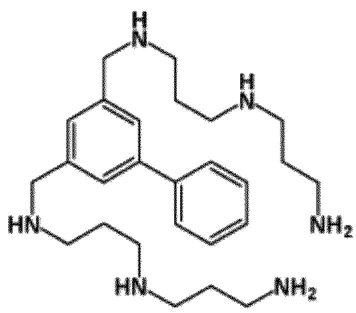 , 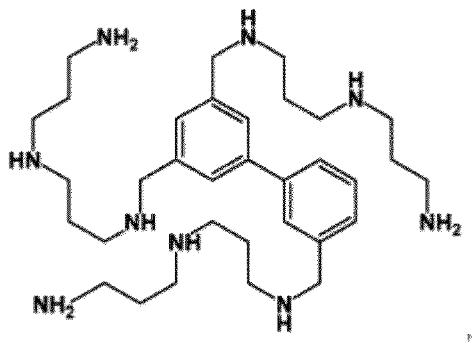 ,

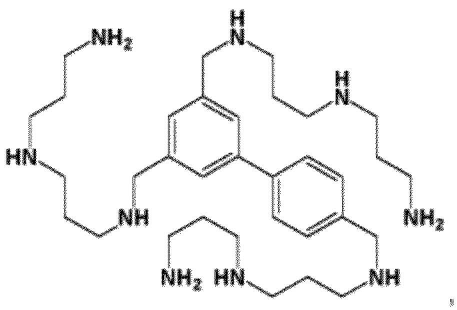 , 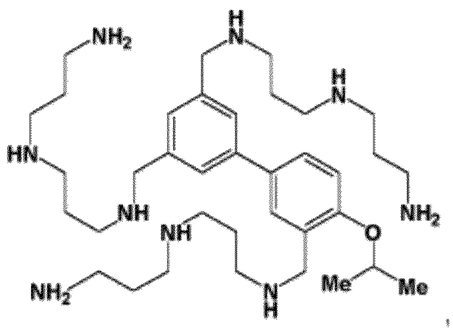 ,

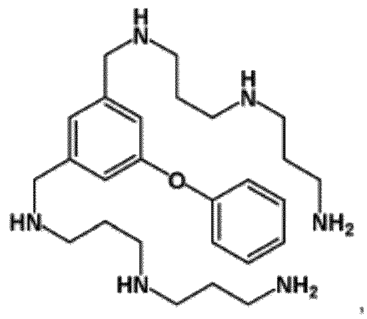,

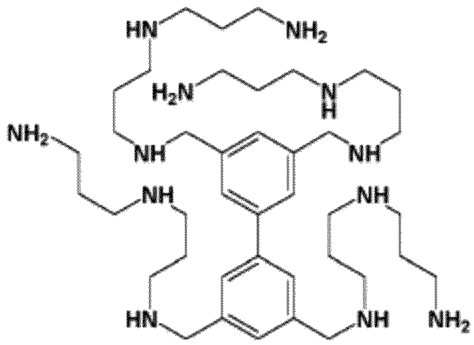,

et un sel correspondant.

**9.** Procédé selon l'une des revendications 1 à 8, le biofilm comprenant une espèce bactérienne résistante à un antibiotique.

**10.** Procédé selon l'une quelconque des revendications 1 à 9, le procédé comprenant une étape de :

(a) traitement d'une lentille de contact avec la composition anti-biofilm ;
(b) traitement d'un tuyau avec la composition anti-film ;
(c) traitement d'une tour de chauffage ou de refroidissement avec la composition anti-biofilm ; ou
(d) revêtement d'un objet avec la composition anti-biofilm.

**11.** Procédé non thérapeutique pour l'inhibition de la formation d'un biofilm, le procédé comprenant une étape de traitement de bactéries planctoniques avec la composition anti-biofilm utilisée selon l'une quelconque des revendications 1 à 10, inhibant ainsi l'incorporation des bactéries planctoniques dans le biofilm.

**12.** Composition anti-biofilm, la composition comprenant le composé de type polyamine utilisé dans l'une quelconque des revendications 1 à 8.

**13.** Composition anti-biofilm selon la revendication 12, le composé de type polyamine étant de formule

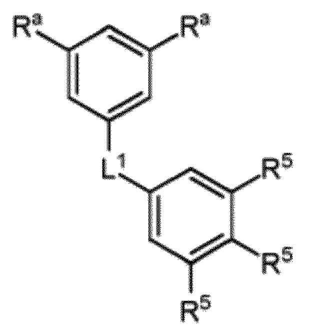

ou un sel correspondant.

**14.** Composé de type polyamine pour une utilisation dans la dispersion ou destruction thérapeutique d'un biofilm,

le composé de type polyamine étant choisi dans le groupe constitué par

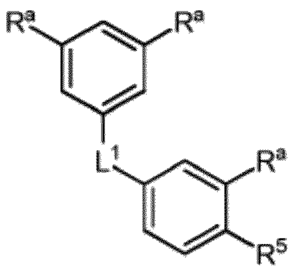,
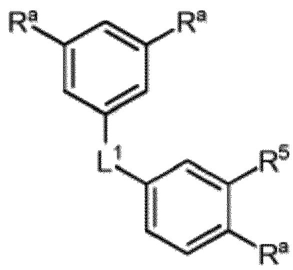,
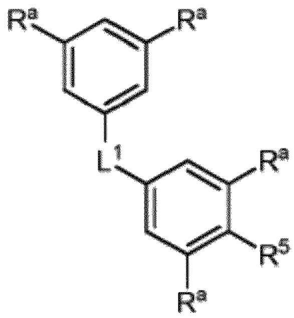,

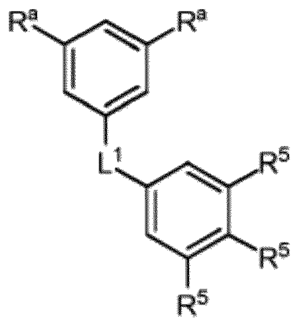,

et un sel correspondant ;
chaque $L^1$ étant choisi dans le groupe constitué par une liaison et -O- ;
chaque $R^a$ étant $-CH_2[NH(CH_2)_n]_pNHR^4$
chaque n étant un entier indépendamment choisi parmi 3 à 12 ;
chaque p étant un entier indépendamment choisi parmi 1 à 3 ;
chaque $R^4$ étant un élément indépendamment choisi dans le groupe constitué par hydrogène, alkyle, arylalkyle et cycloalkylalkyle ; et
chaque $R^5$ étant un élément indépendamment choisi dans le groupe constitué par hydrogène, alkyle, hydroxyle, alcoxy, halogéno, fluoroalkyle, fluoroalkyloxy et hydroxyalkyle,
le composé de type polyamine comprenant au moins quatre groupes amino primaire ou secondaire.

**15.** Composé de type polyamine selon la revendication 14, en outre défini comme dans l'une quelconque des revendications 2 à 8.

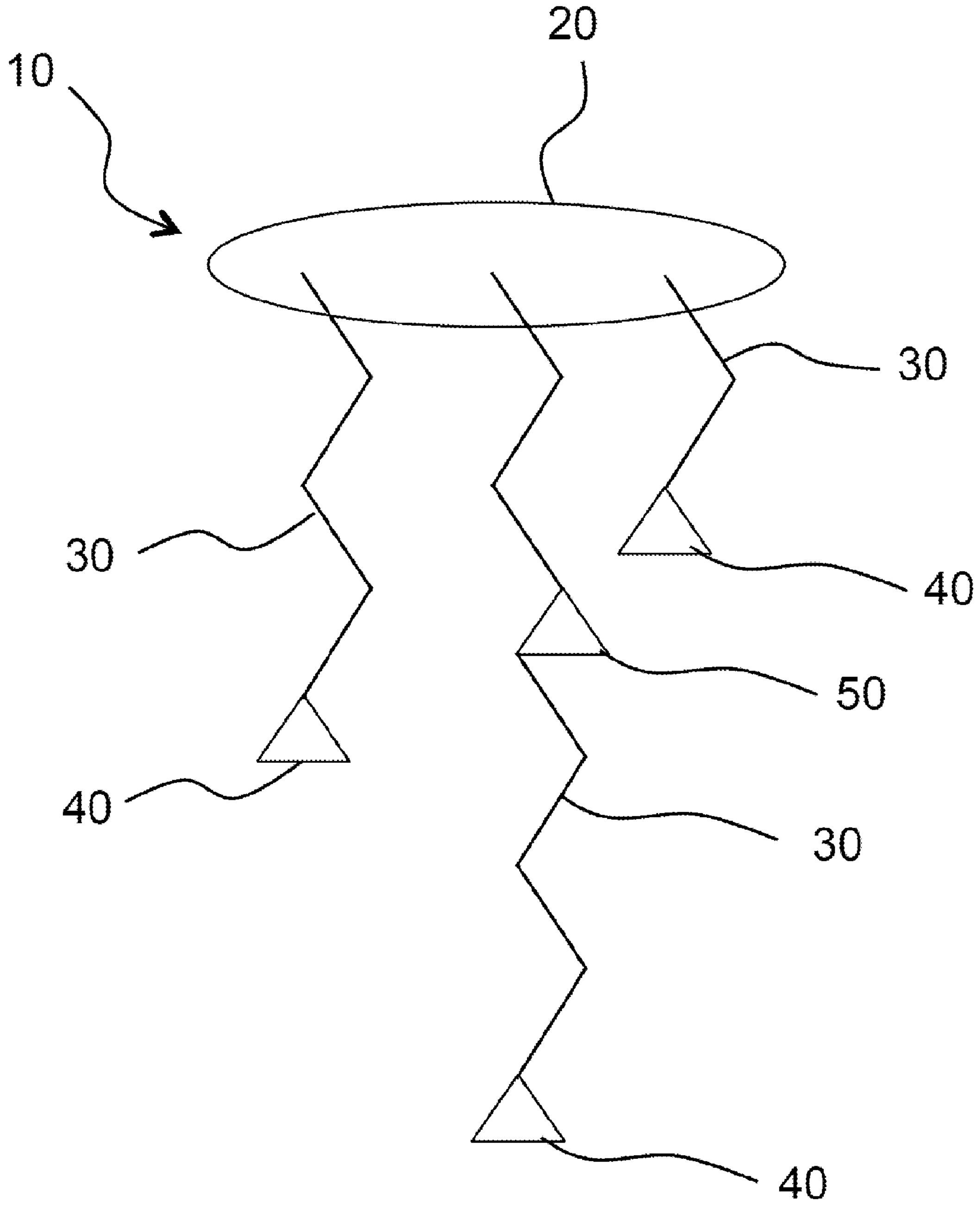


FIG. 1

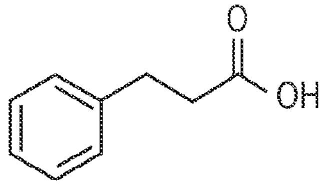

FIG. 2A
(PRIOR ART)

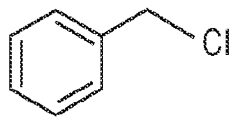

FIG. 2B
(PRIOR ART)

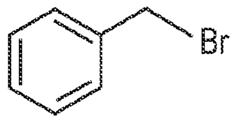

FIG. 2C
(PRIOR ART)

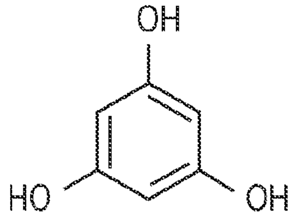

FIG. 2D
(PRIOR ART)

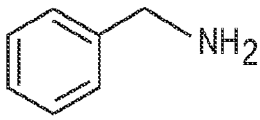

FIG. 2E
(PRIOR ART)

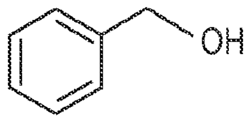

FIG. 2F
(PRIOR ART)

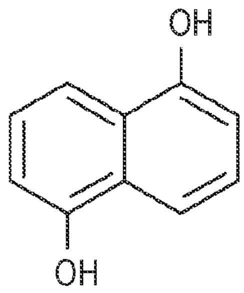

FIG. 2G
(PRIOR ART)

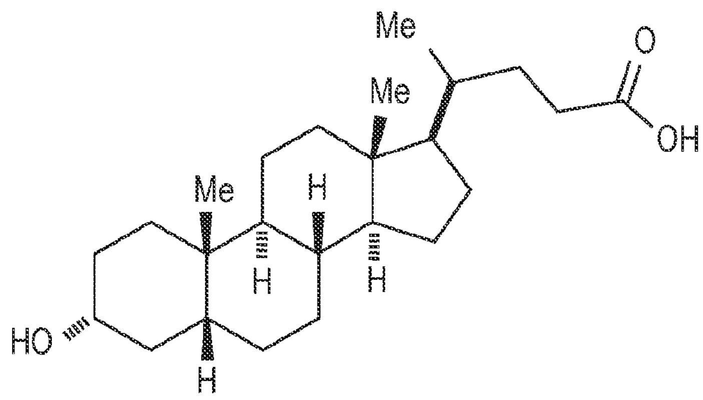

FIG. 2H
(PRIOR ART)

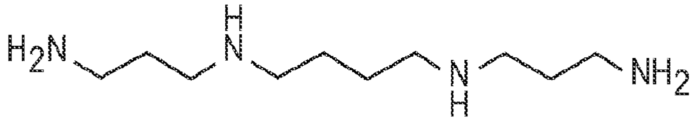

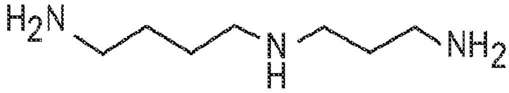

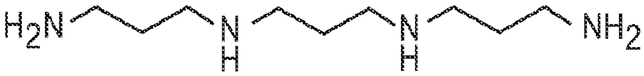

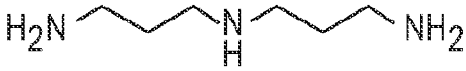

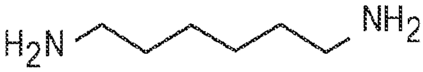

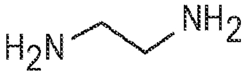

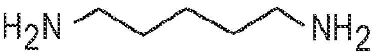

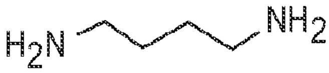

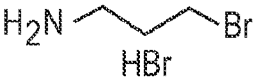

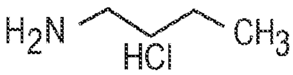

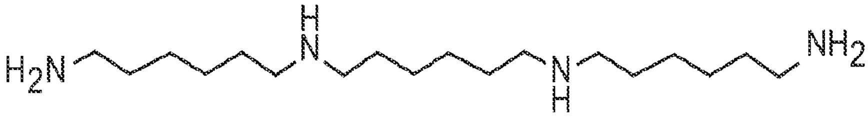

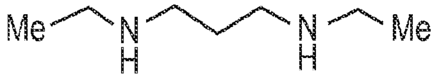

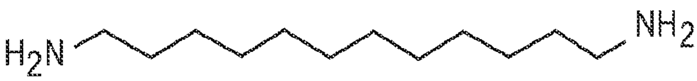

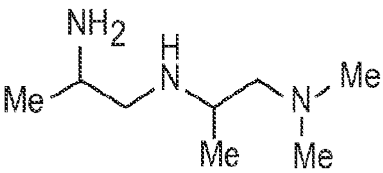

**FIG. 3**
**(PRIOR ART)**

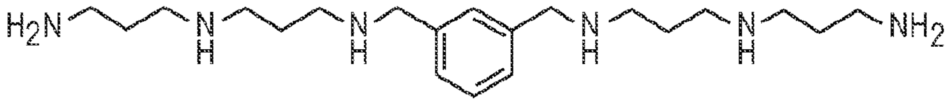

FIG. 4A

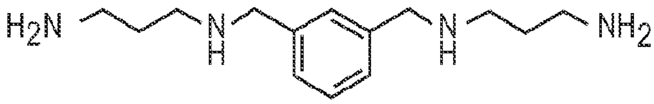

FIG. 4B

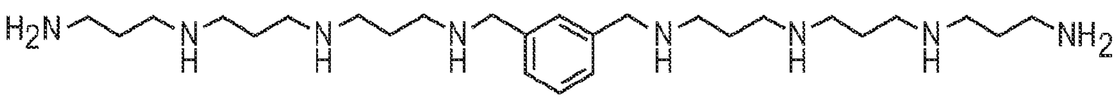

FIG. 4C

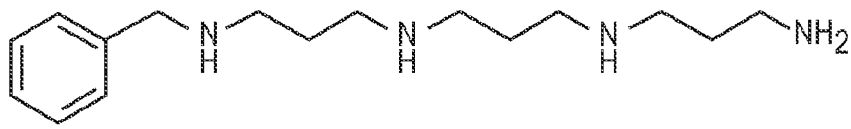

FIG. 4D

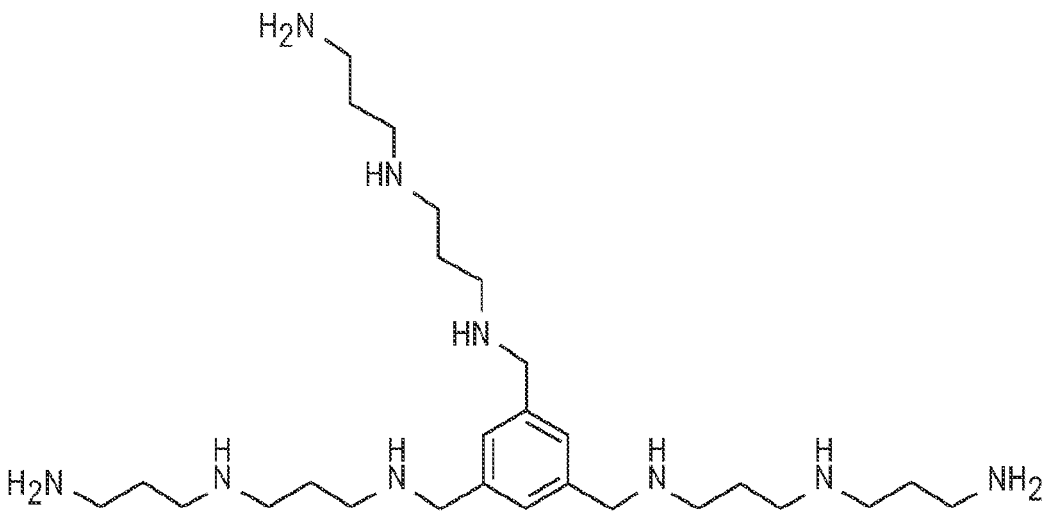

FIG. 4E

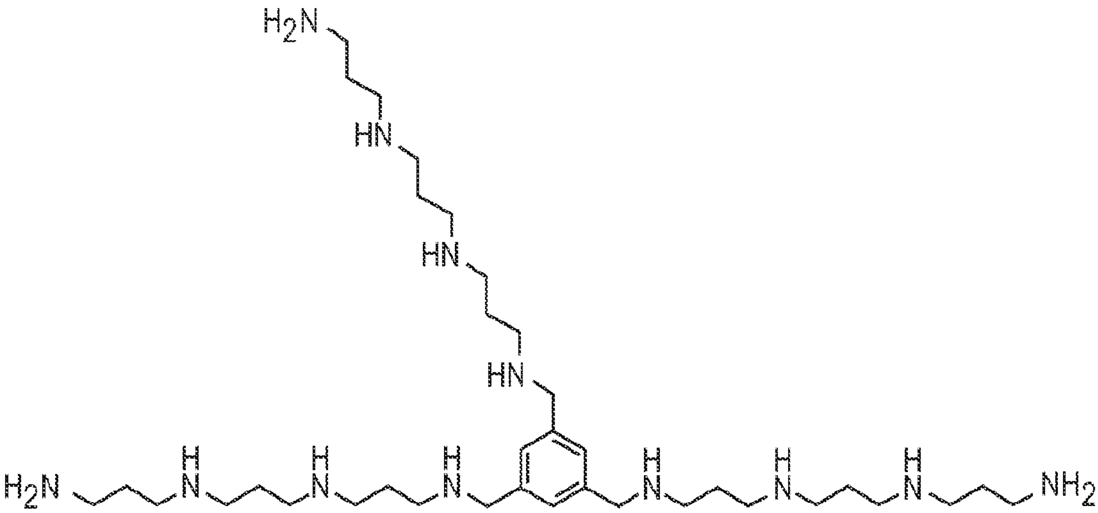

FIG. 4F

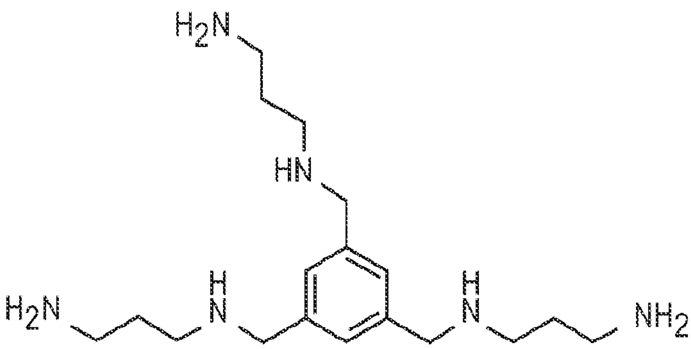

FIG. 4G

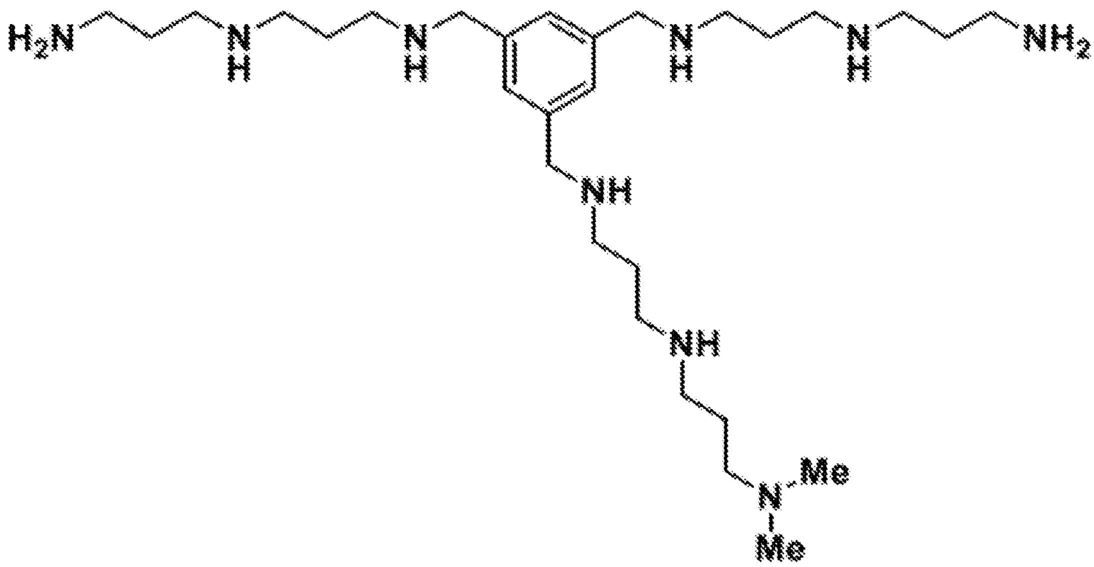

FIG. 4H

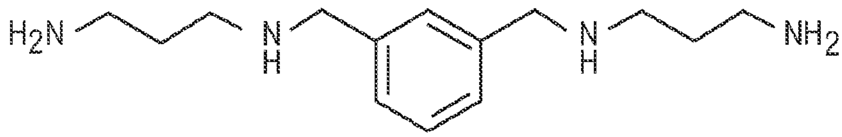

FIG. 4I

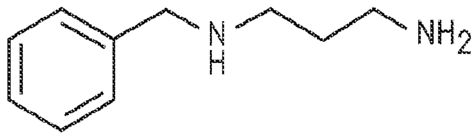

FIG. 4J

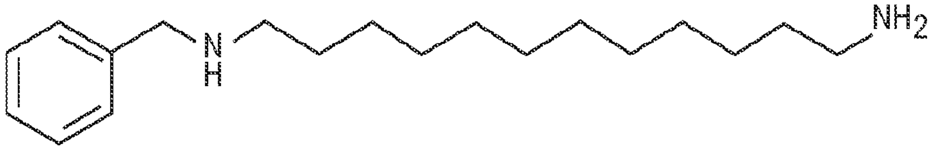

FIG. 4K

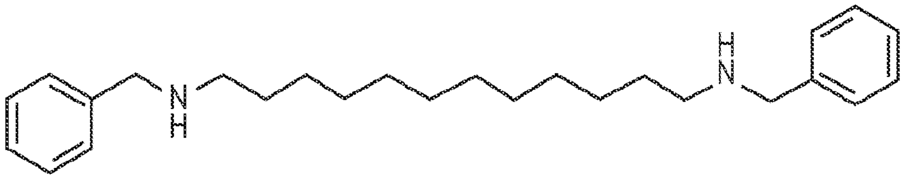

FIG. 4L

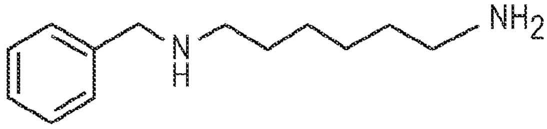

FIG. 4M

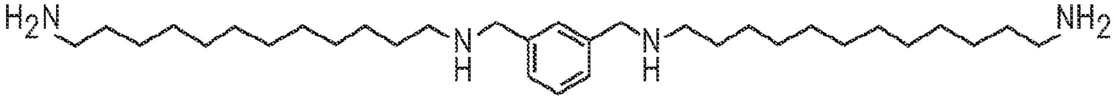

FIG. 4N

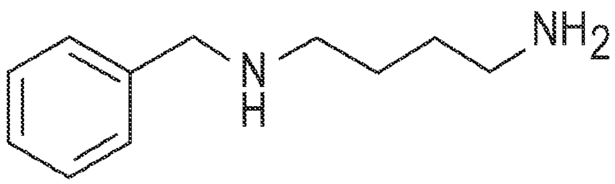

FIG. 4O

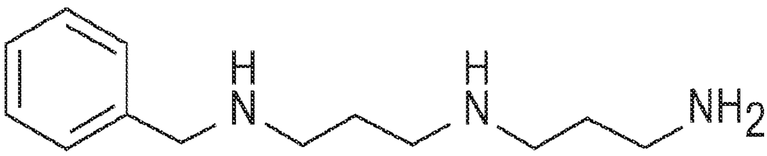

FIG. 4P

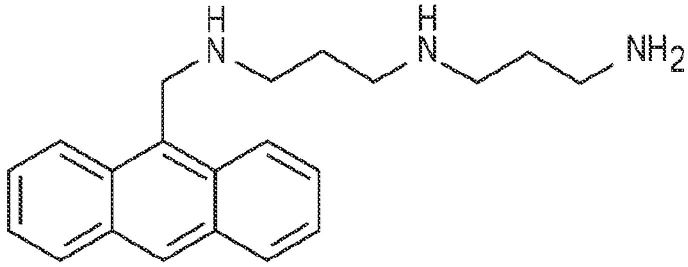

FIG. 5A

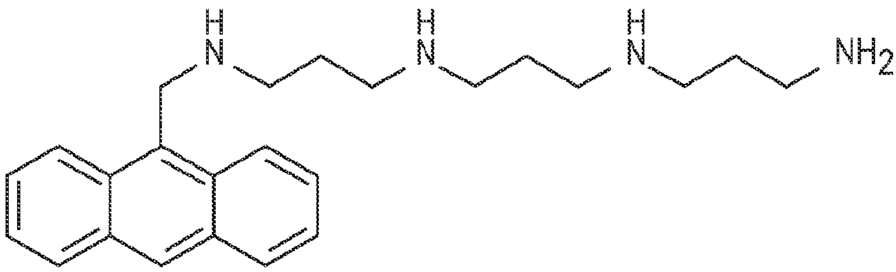

FIG. 5B

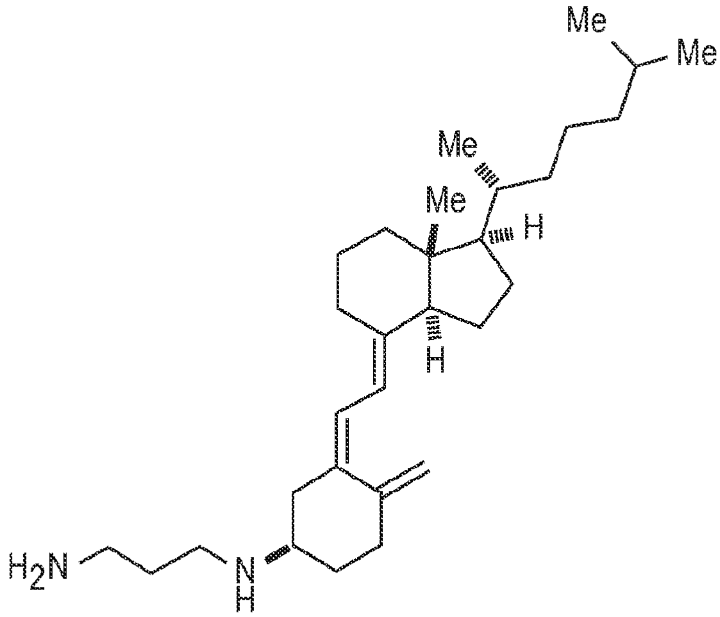

FIG. 6A

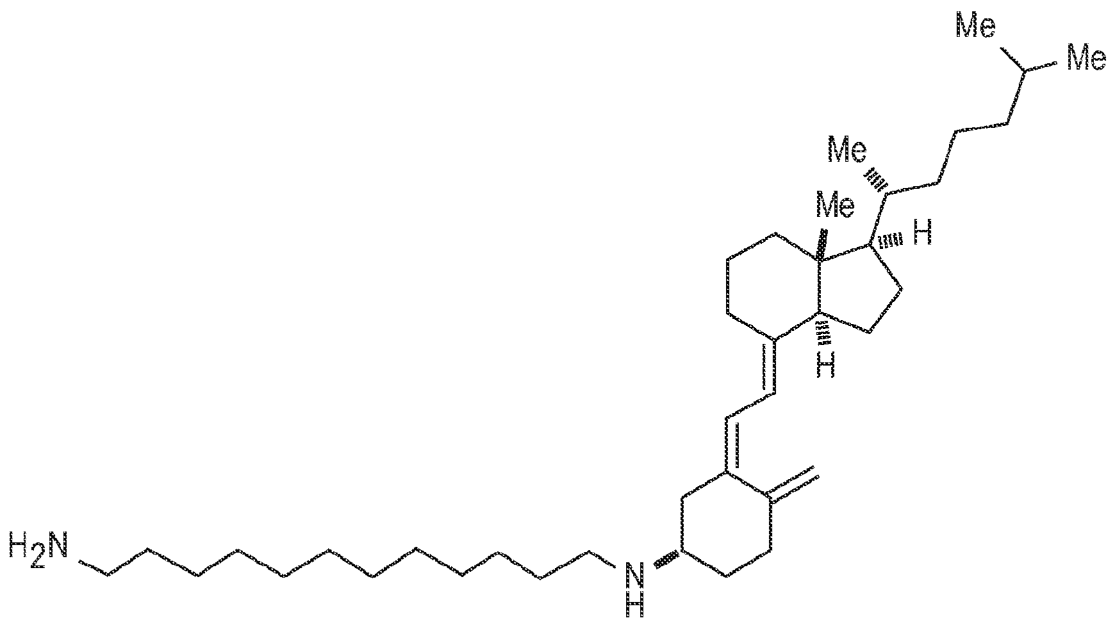

FIG. 6B

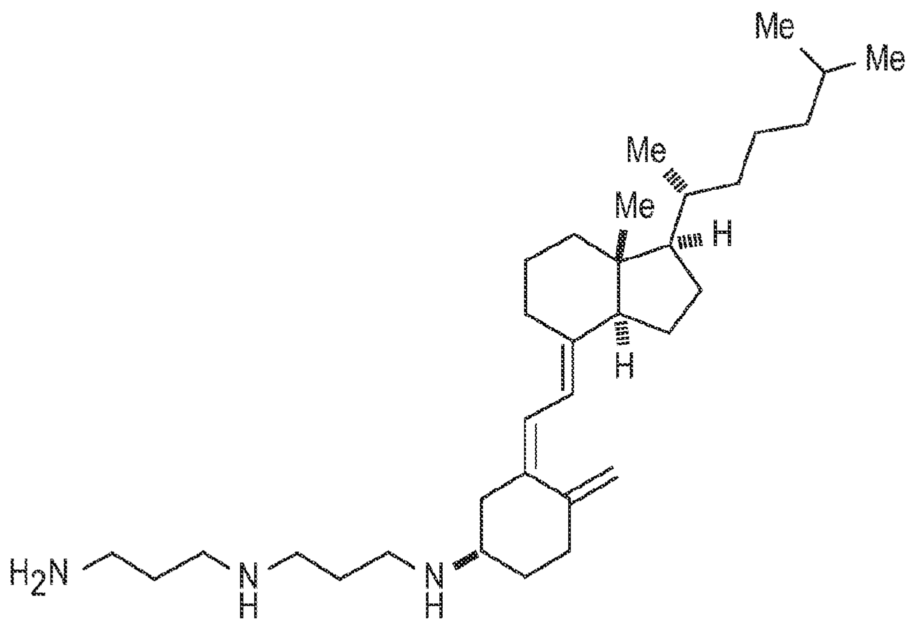

FIG. 6C

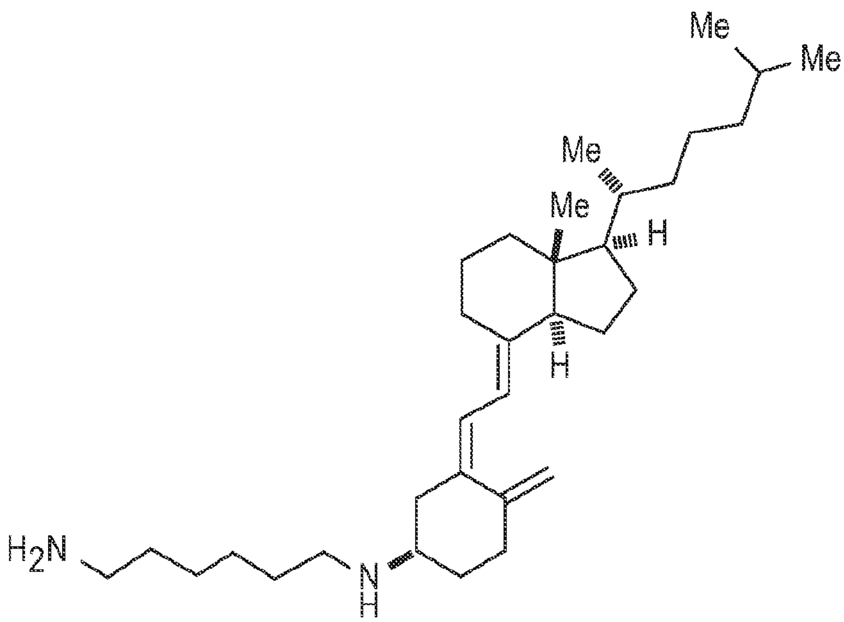

FIG. 6D

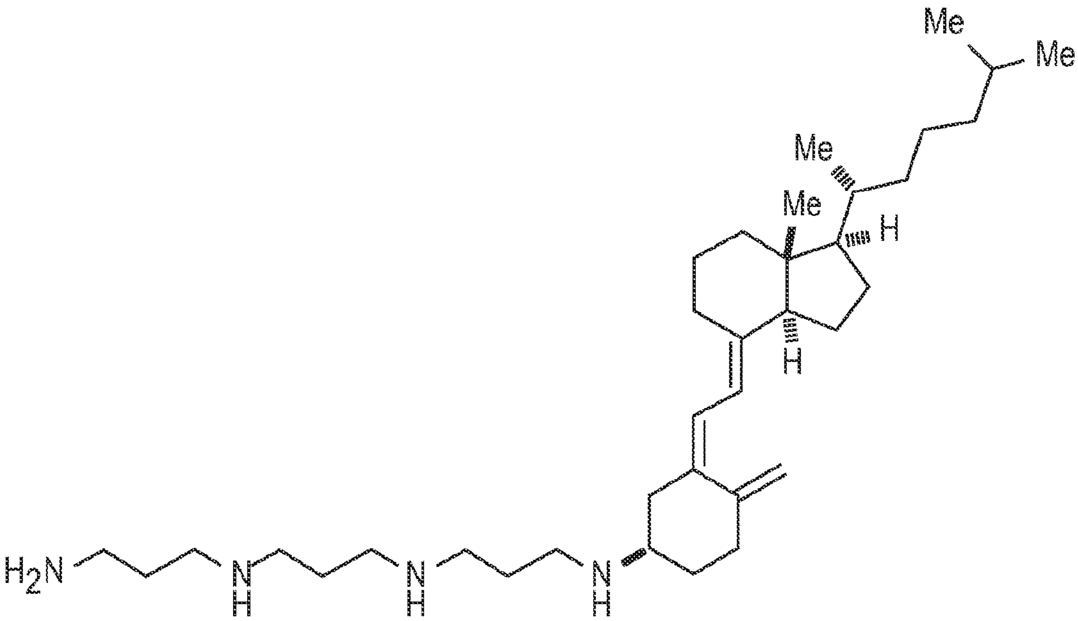

FIG. 6E

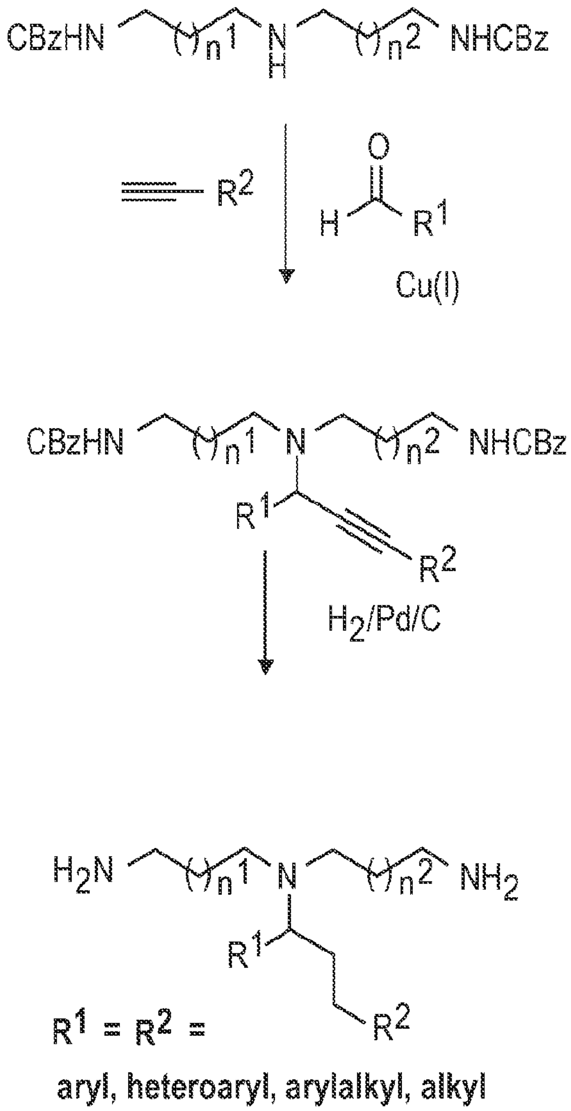


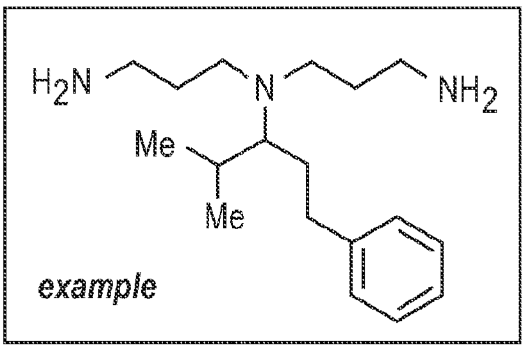
*example*

**FIG. 7**

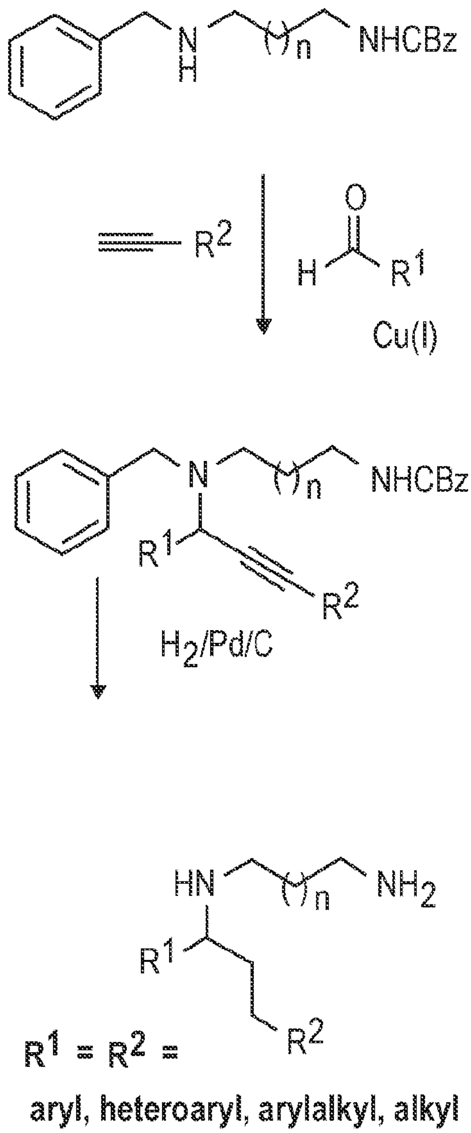


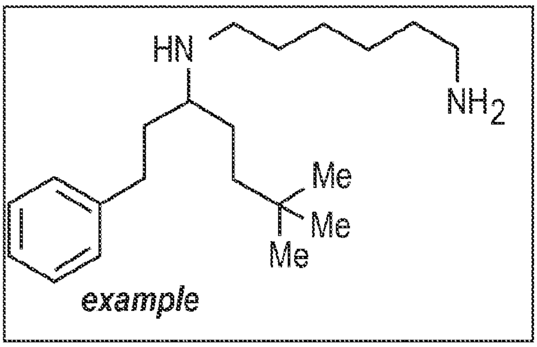


# FIG. 8

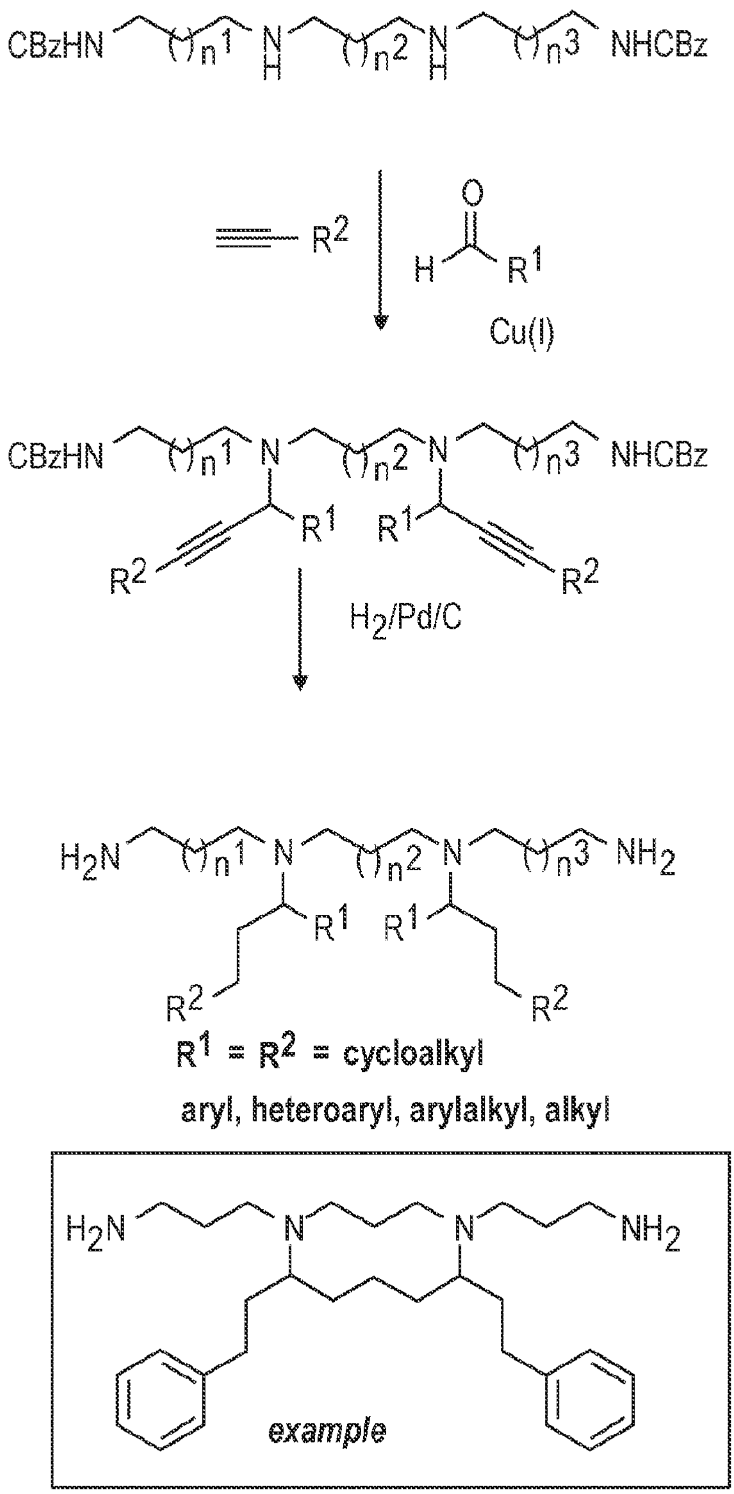
Cu(I)
$H_2$/Pd/C
$R^1$ = $R^2$ = cycloalkyl
aryl, heteroaryl, arylalkyl, alkyl
example

FIG. 9

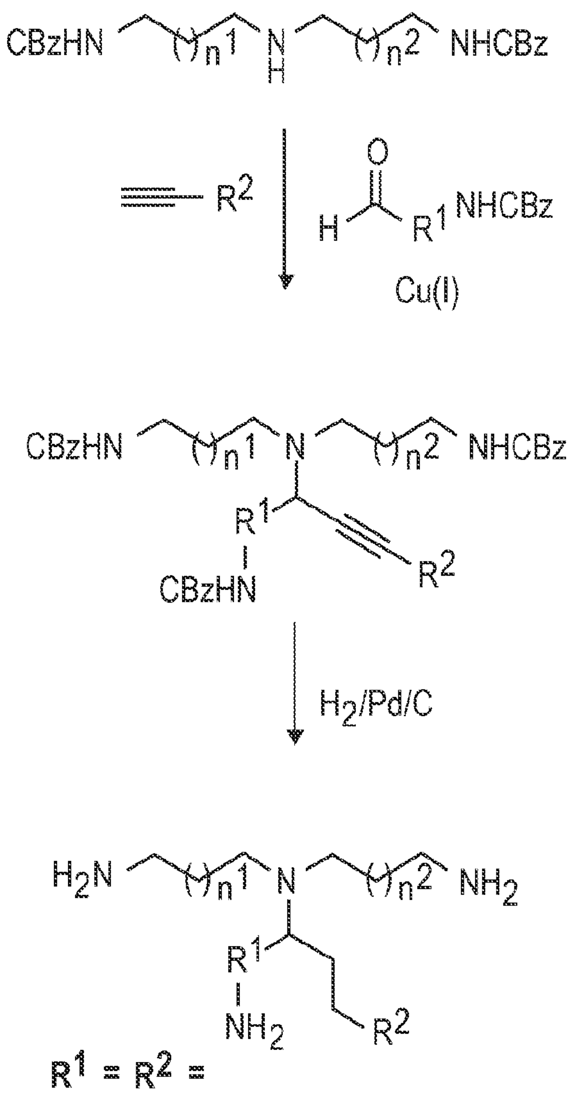


**$R^1 = R^2 =$**

**aryl, heteroaryl, arylalkyl, alkyl**

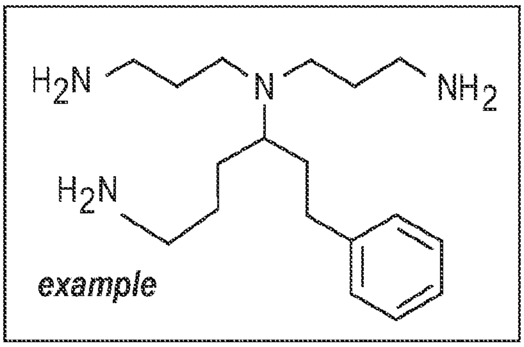


# FIG. 10

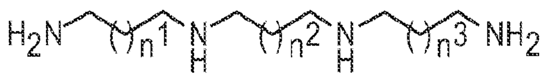

***norspermine*** $n^1 = n^2 = n^3 = 1$

MIC = 800

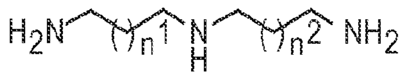

***norspermidine*** $n^1 = n^2 = 1$

MIC = 900

All MIC are in µg/mL

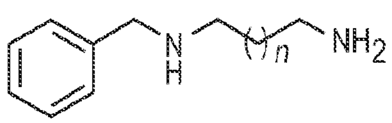

n = 1; MIC = 1,300

n = 10; MIC = 100

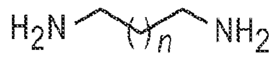

n = 1; MIC = 1,175

n = 2; MIC = 1,200

n = 4; MIC = >1,400

n = 10; MIC = 950

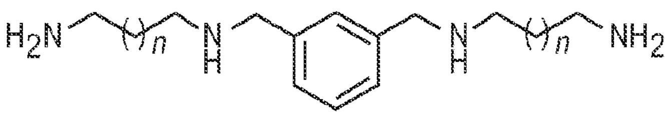

n = 1; MIC = 300

n = 10; MIC = 50

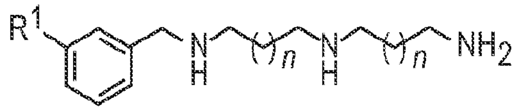

$R^1$ = H; n = 1; MIC = >1,000

$R^1 = CH_2NH(CH_2)_3NH(CH_2)_3NH_2$ n = 1; MIC = 50

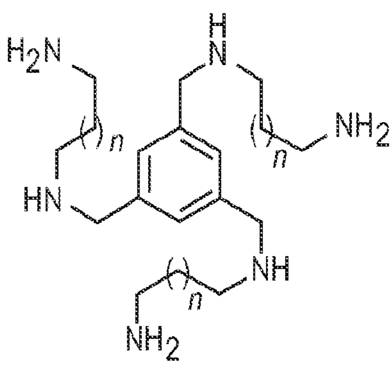

n = 1; MIC = 75

n = 2; MIC = 50

**FIG. 11**

| Compound | Structure |
|---|---|
| 1 | 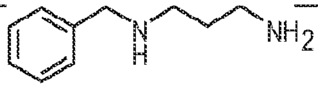 |
| 2 | 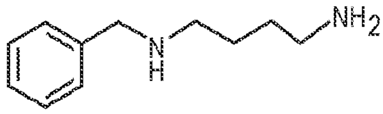 |
| 3 | 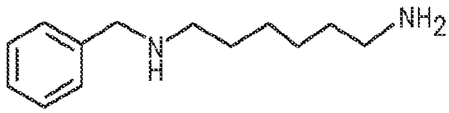 |
| 4 | 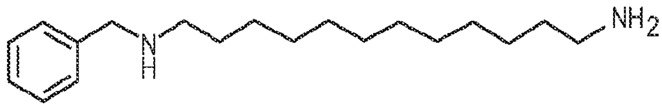 |
| 5 | 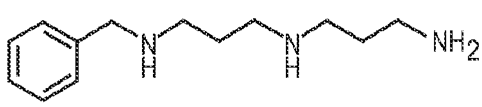 |
| 6 | 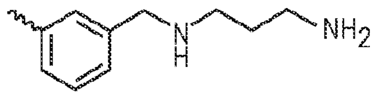 |
| 7 | 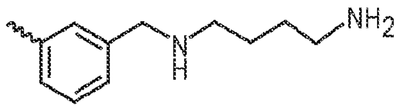 |
| 8 | 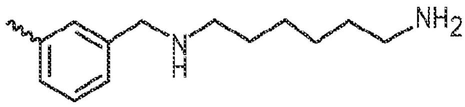 |
| 9 | 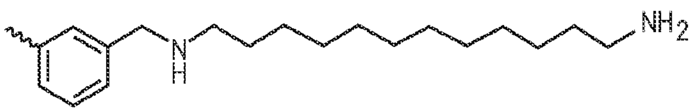 |
| 10 | 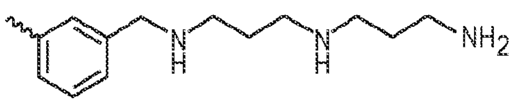 |
| 11 | 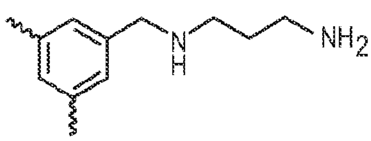 |
| 12 | 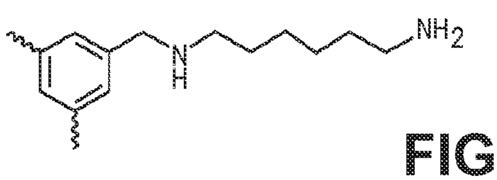 |

FIG. 12A

| Compound | EBEC (µg/mL) | EBEC (µM) |
|---|---|---|
| 1 | | |
| 2 | | |
| 3 | | |
| 4 | | |
| 5 | 1,000 | |
| 6 | 800 | |
| 7 | | |
| 8 | ~750 | |
| 9 | ~750 | |
| 10 | ~750 | |
| 11 | | |
| 12 | | |
| Norspermidine | >1,000 | |
| Spermine | <1,600 | |
| Spermidine | <1,600 | |
| Vancomycin | >3,000 | |

FIG. 12B

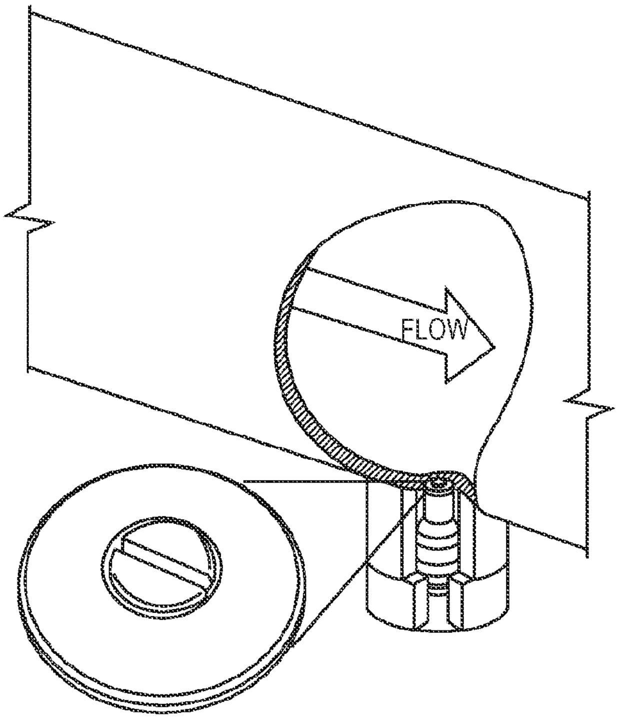


FIG. 13A
(PRIOR ART)

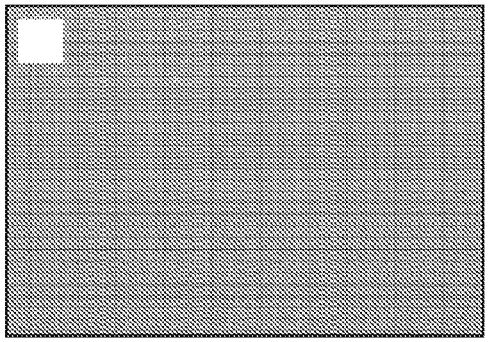

FIG. 13B
(PRIOR ART)

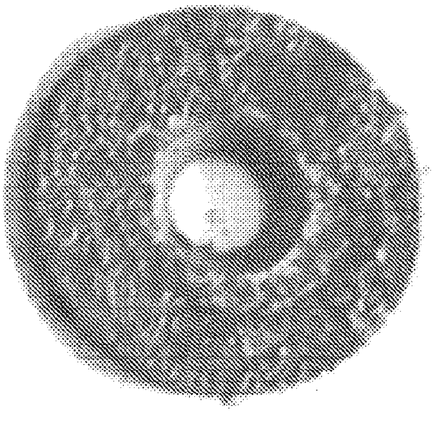

FIG. 13C
(PRIOR ART)

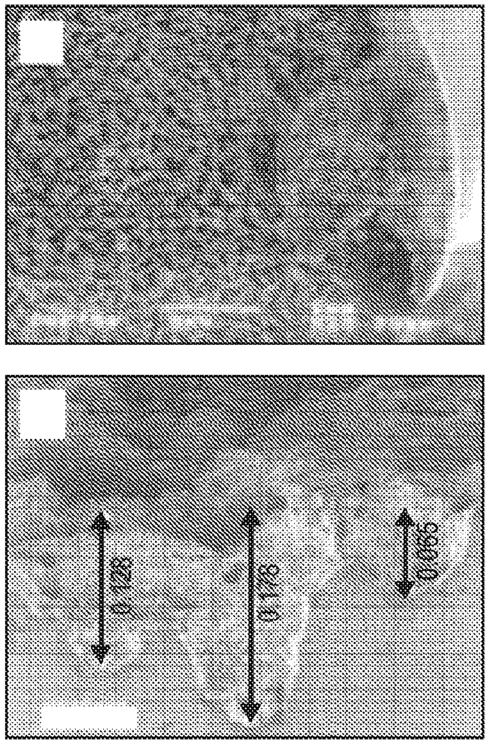


FIG. 13D
(PRIOR ART)

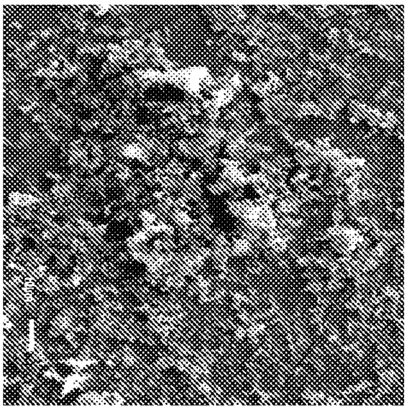

FIG. 14A
– Prior Art –

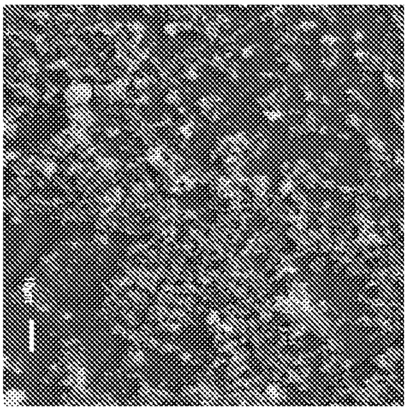

FIG. 14B
– Prior Art –

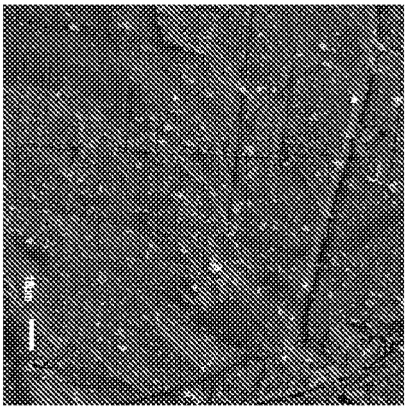

FIG. 14C

Biofilms of *Alcanivorax borkumensis* were grown on the surface of galvanized steel for 48 hours at 30 C. After biofilms were grown, they were either untreated or treated for 2 hrs with PBC-25

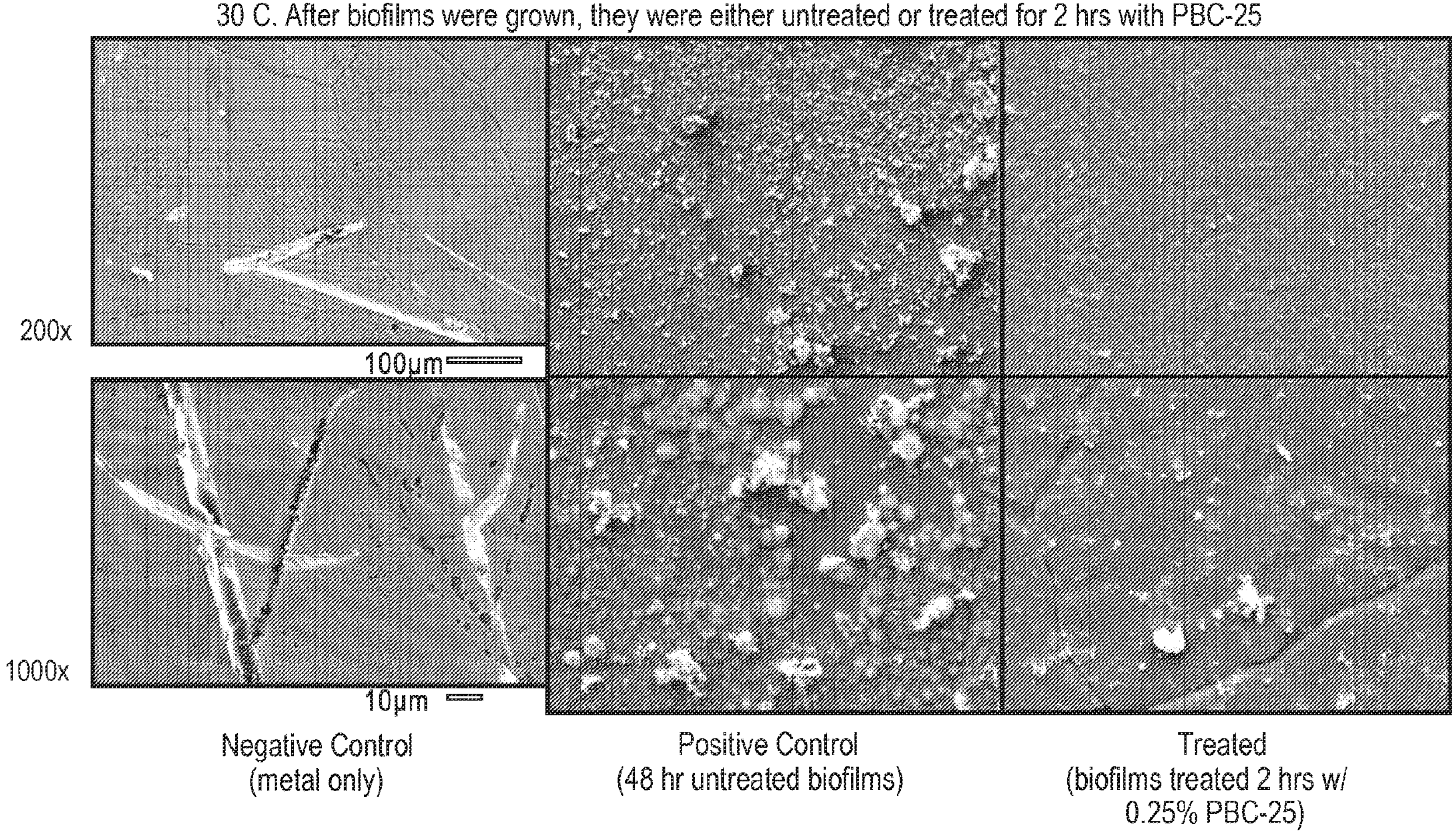


FIG. 15

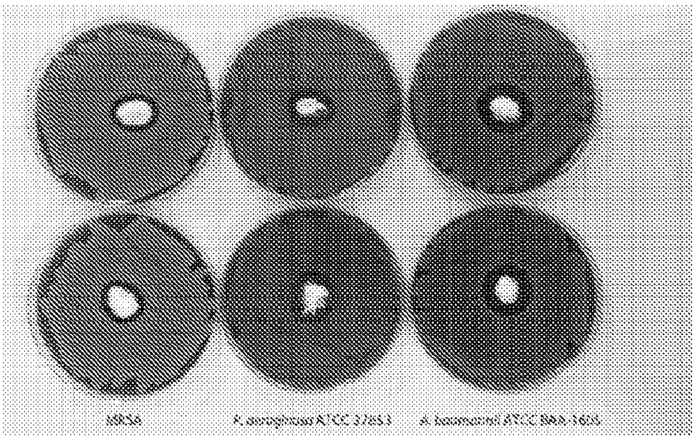


FIG. 16

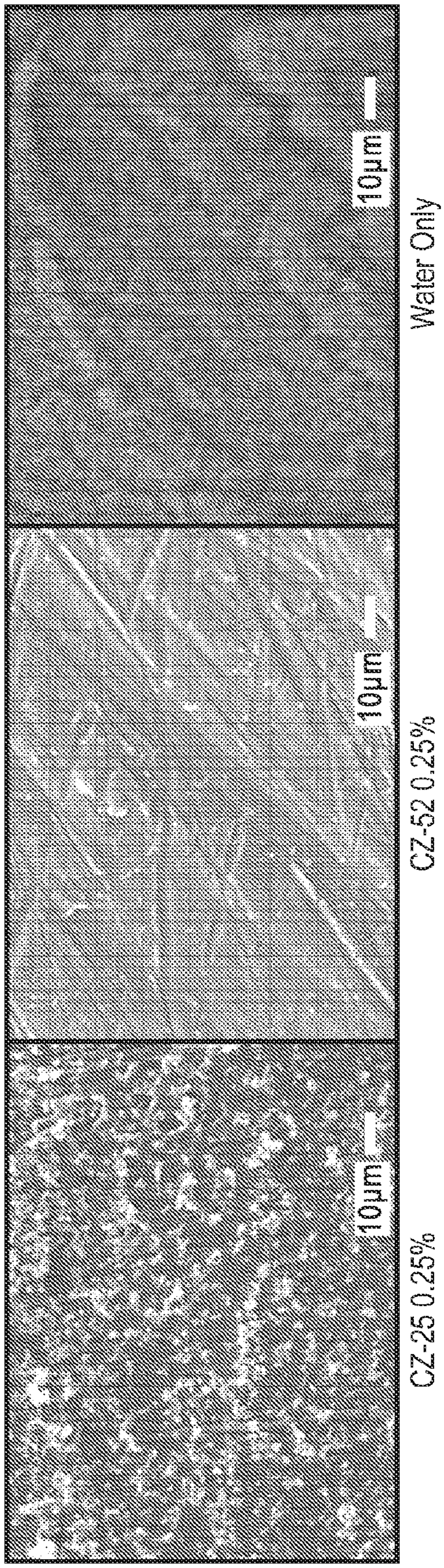


FIG. 17

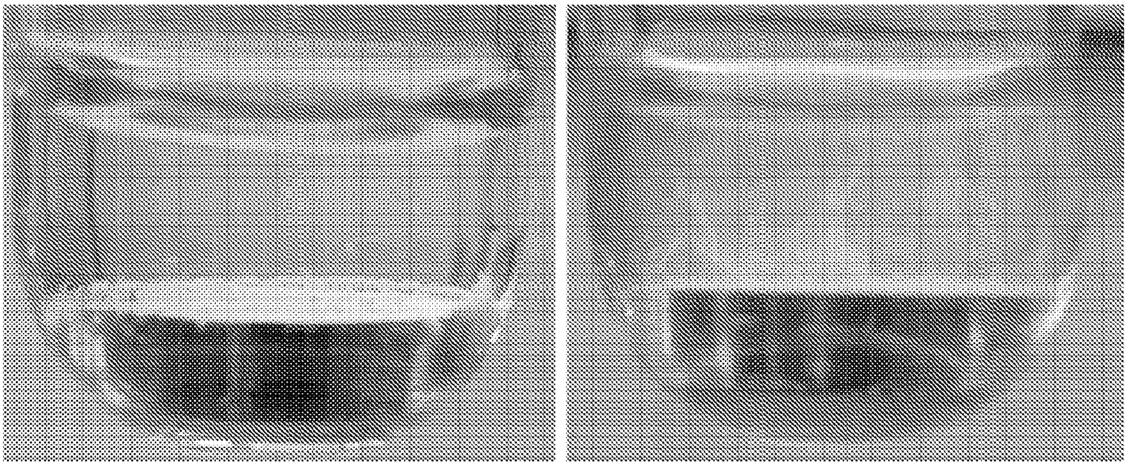

FIG. 18

Biofilms of *Alcanivorax borkumensis* were grown on the surface of galvanized steel for 48 hours at 30 C. After biofilms were grown, they were either untreated or treated for 2 hrs with PBC-25

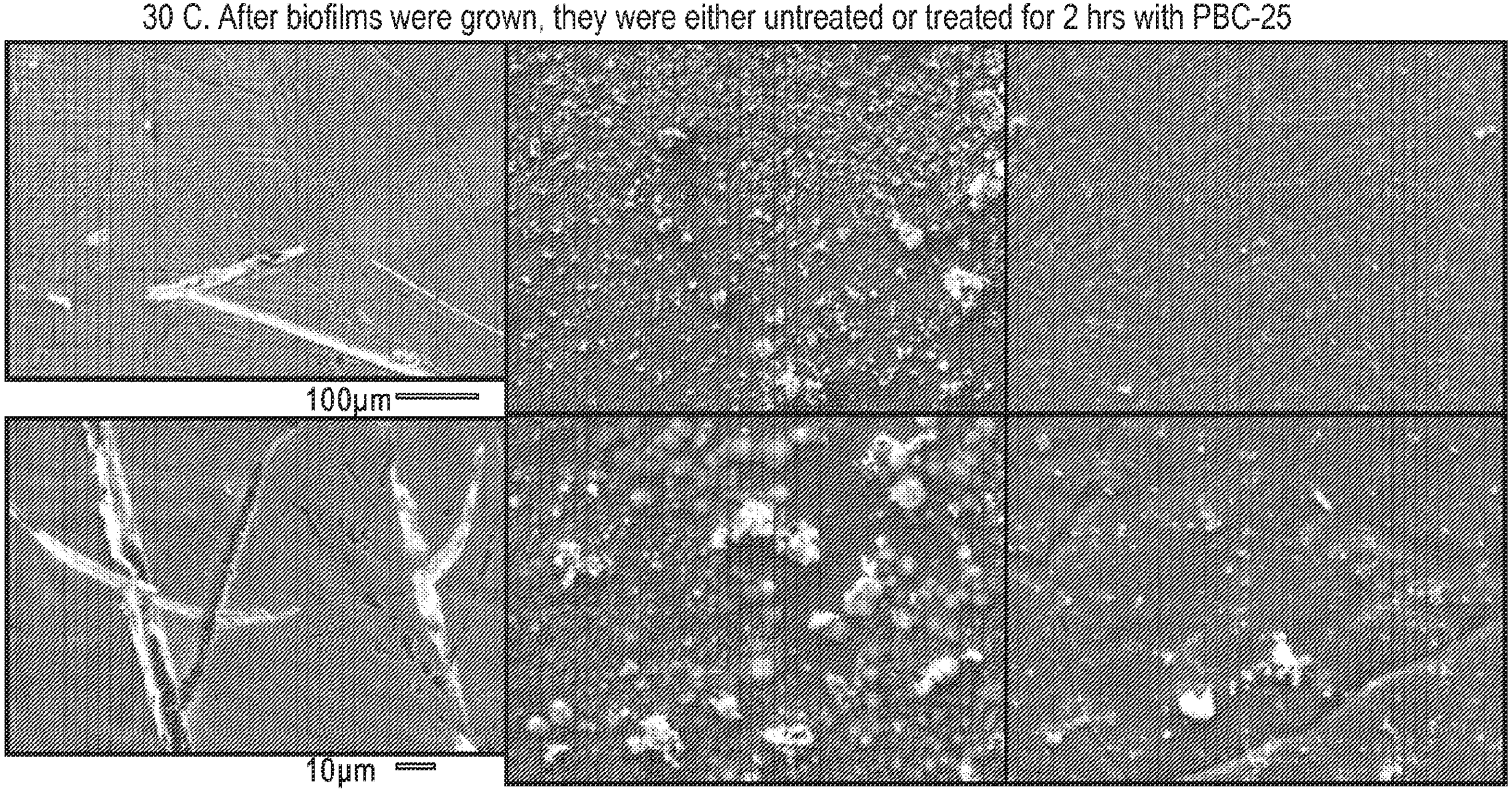


**FIG. 19**

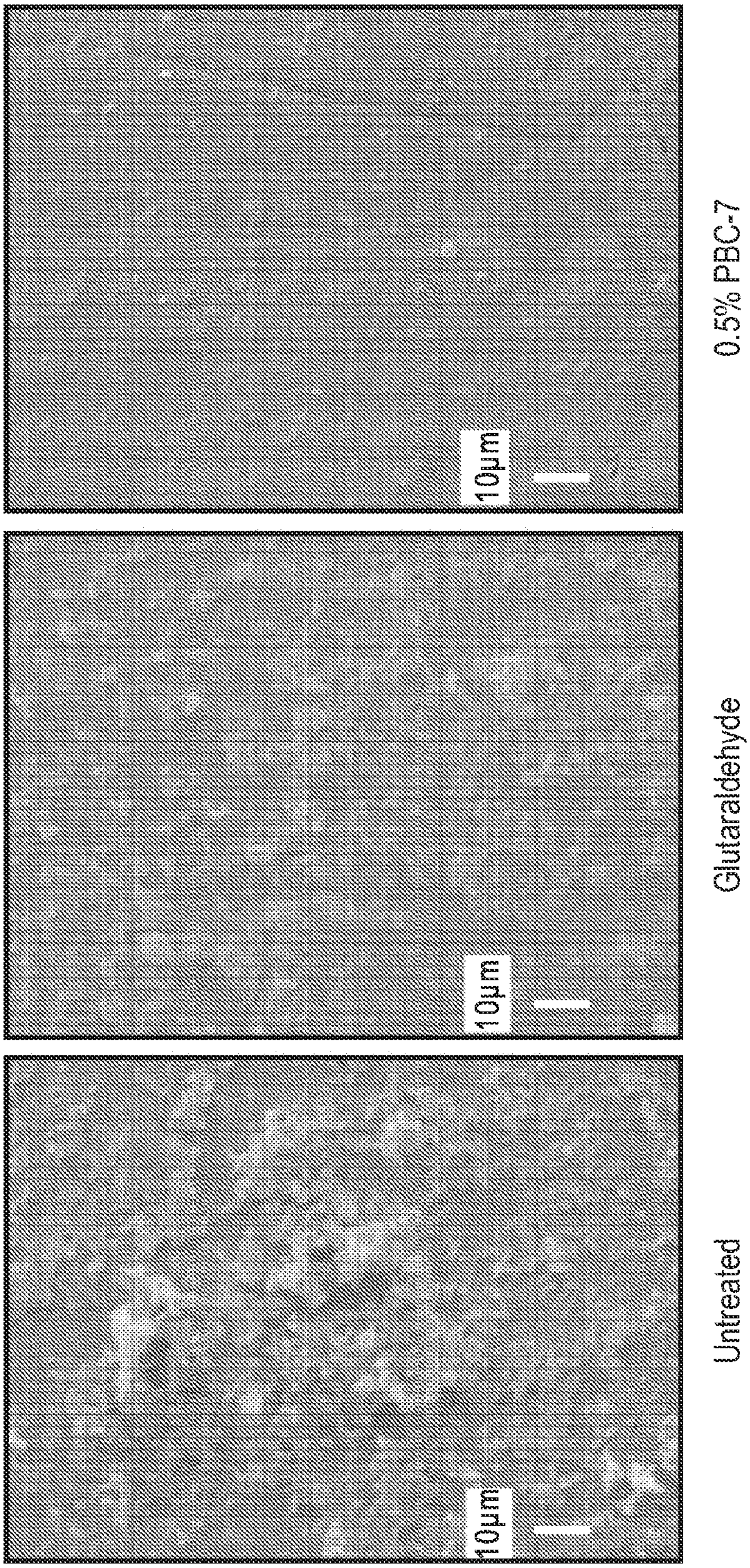


FIG. 20

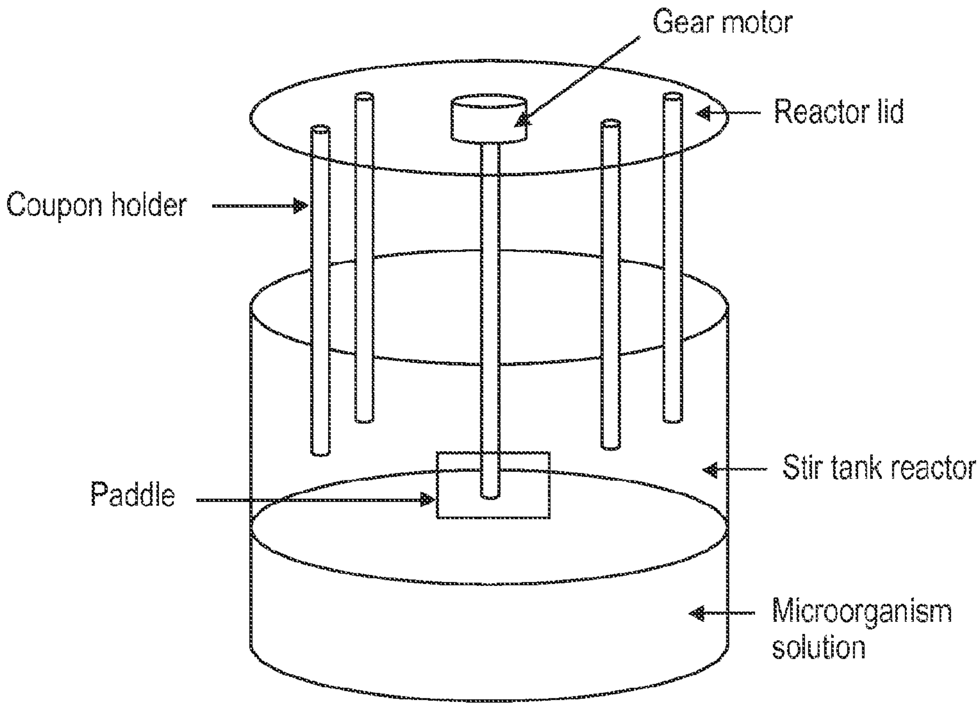


FIG. 21
(PRIOR ART)

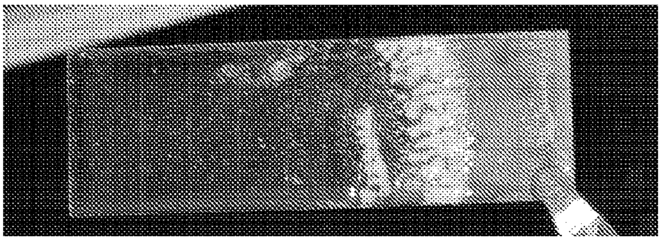

FIG. 22

A

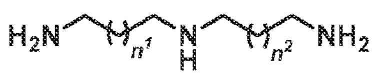

***spermidine*** $n^1 = 1$, $n^2 = 2$

*MBIC = 1 mM*

***norspermidine*** $n^1 = n^2 = 1$

*MBIC = 25 μM*

C

*"second-generation" spermidine analogues*

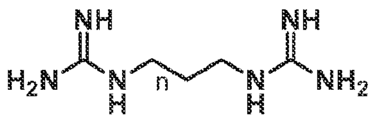

*MBICs ~ 2 μM*

B

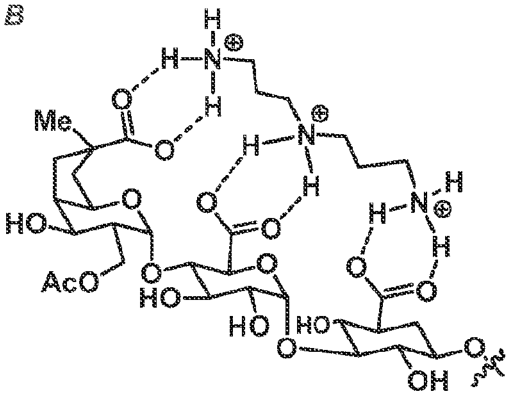

*hypothetical binding of spermidine to exopolysaccharides*

Discovery of polyamines as biofilm disruptors.

FIG. 23

A

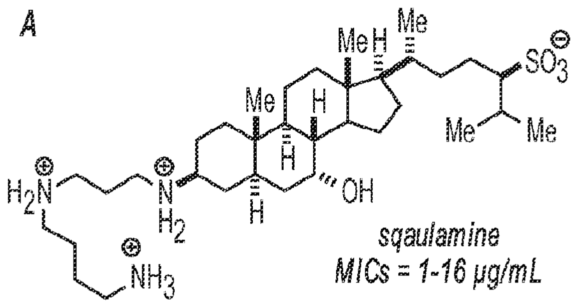

*sqaulamine*
*MICs = 1-16 μg/mL*

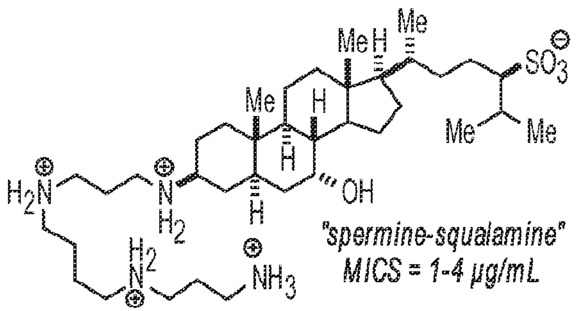

*"spermine-squalamine"*
*MICS = 1-4 μg/mL*

B

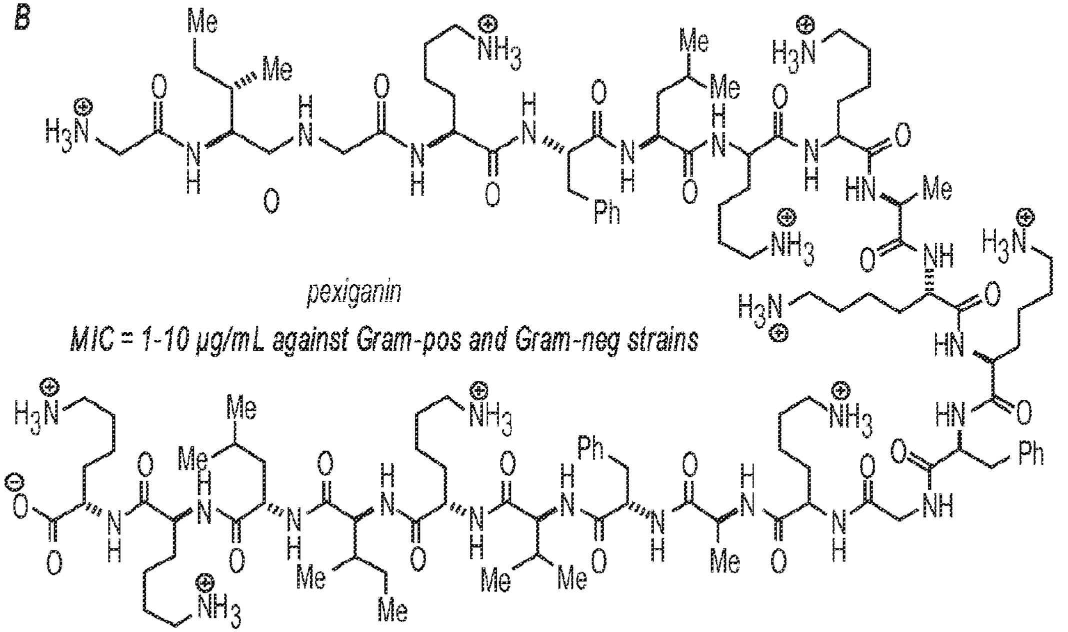

*pexiganin*
*MIC = 1-10 μg/mL against Gram-pos and Gram-neg strains*

Polyamine natural products with known anti-microbial activity.

**FIG. 24**
**(PRIOR ART)**

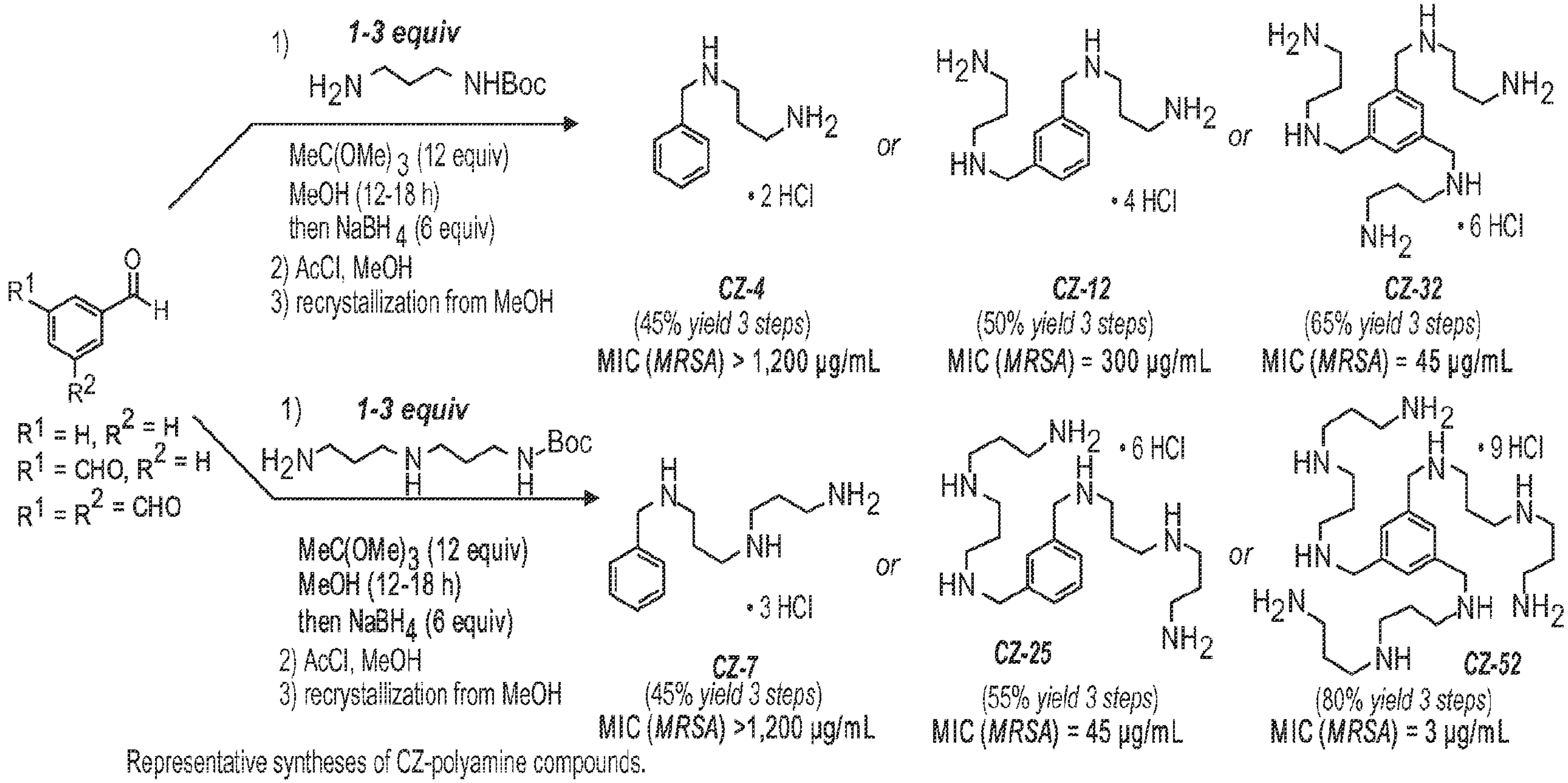


$R^1$ = H, $R^2$ = H
$R^1$ = CHO, $R^2$ = H
$R^1$ = $R^2$ = CHO

• 2 HCl or • 4 HCl or • 6 HCl

**CZ-4**
(45% *yield* 3 *steps*)
MIC (*MRSA*) > 1,200 μg/mL

**CZ-12**
(50% *yield* 3 *steps*)
MIC (*MRSA*) = 300 μg/mL

**CZ-32**
(65% *yield* 3 *steps*)
MIC (*MRSA*) = 45 μg/mL

• 3 HCl or • 6 HCl or • 9 HCl

**CZ-7**
(45% *yield* 3 *steps*)
MIC (*MRSA*) >1,200 μg/mL

**CZ-25**
(55% *yield* 3 *steps*)
MIC (*MRSA*) = 45 μg/mL

**CZ-52**
(80% *yield* 3 *steps*)
MIC (*MRSA*) = 3 μg/mL

Representative syntheses of CZ-polyamine compounds.

**FIG. 25**

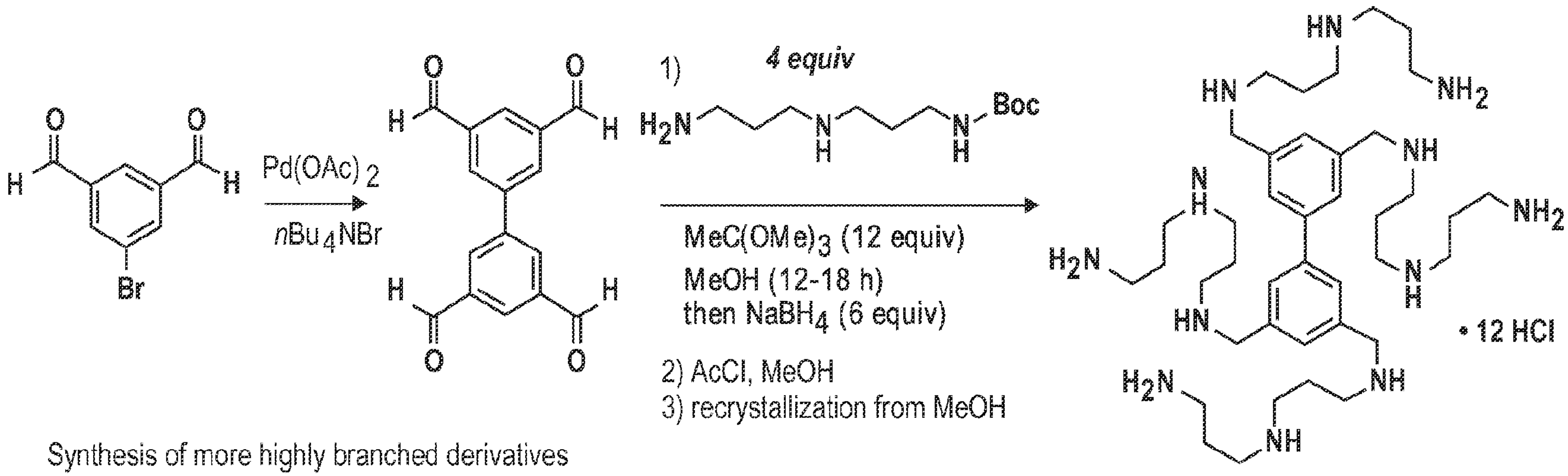


Synthesis of more highly branched derivatives

# FIG. 26

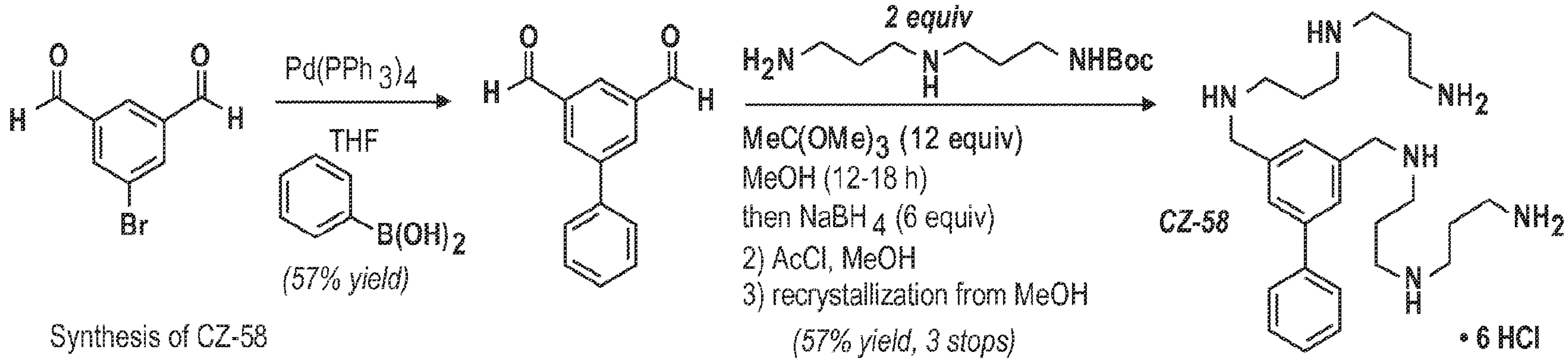


Synthesis of CZ-58

CZ-58

FIG. 27

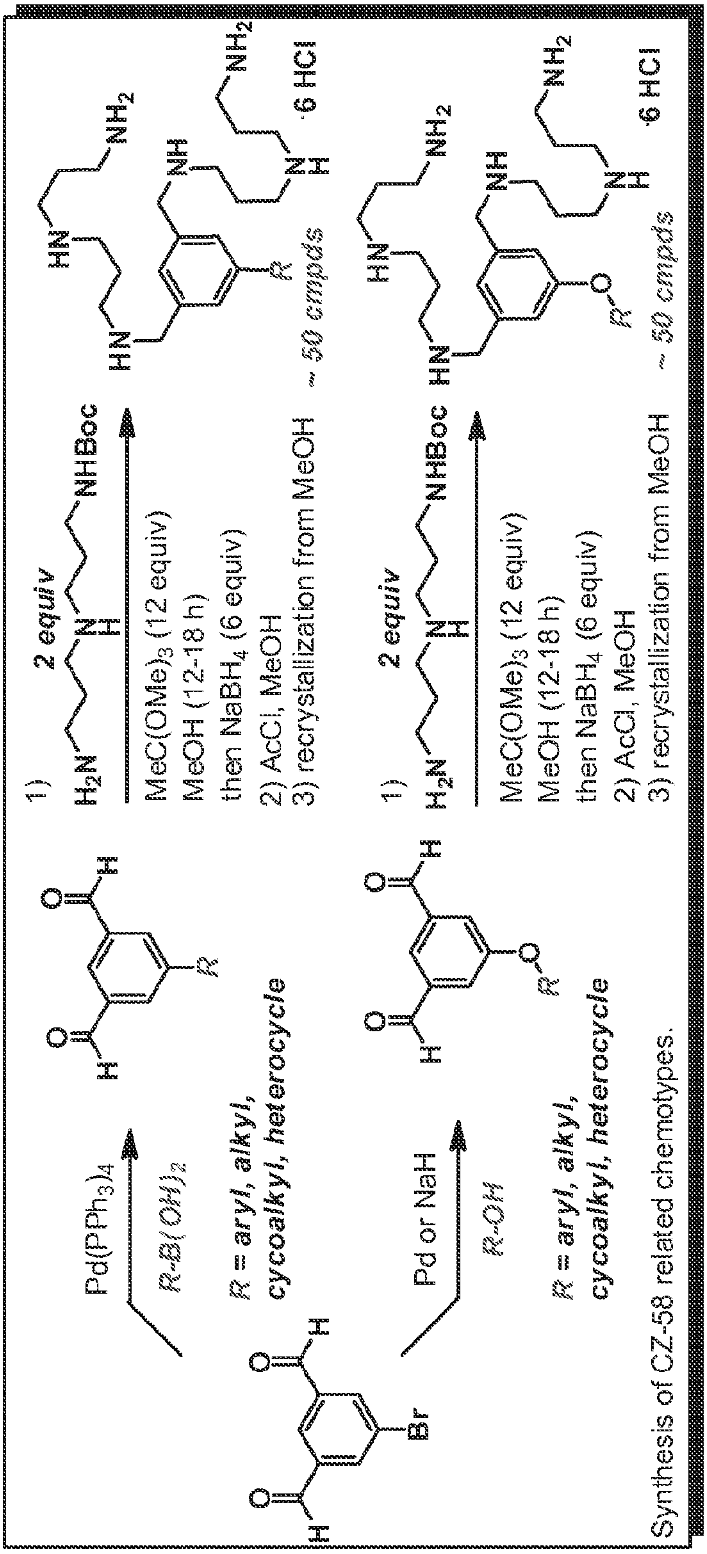

Synthesis of CZ-58 related chemotypes.

FIG. 28

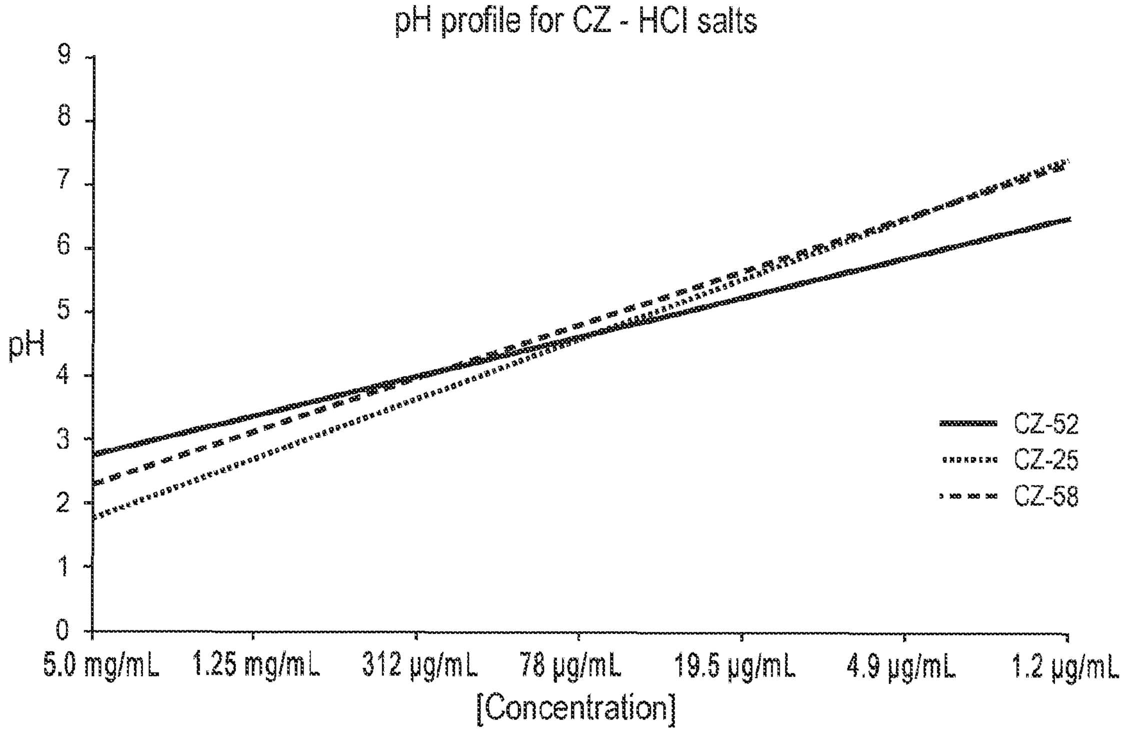


pH profile of CZ-HCl salts as a function of concentration in deionized water

**FIG. 29**

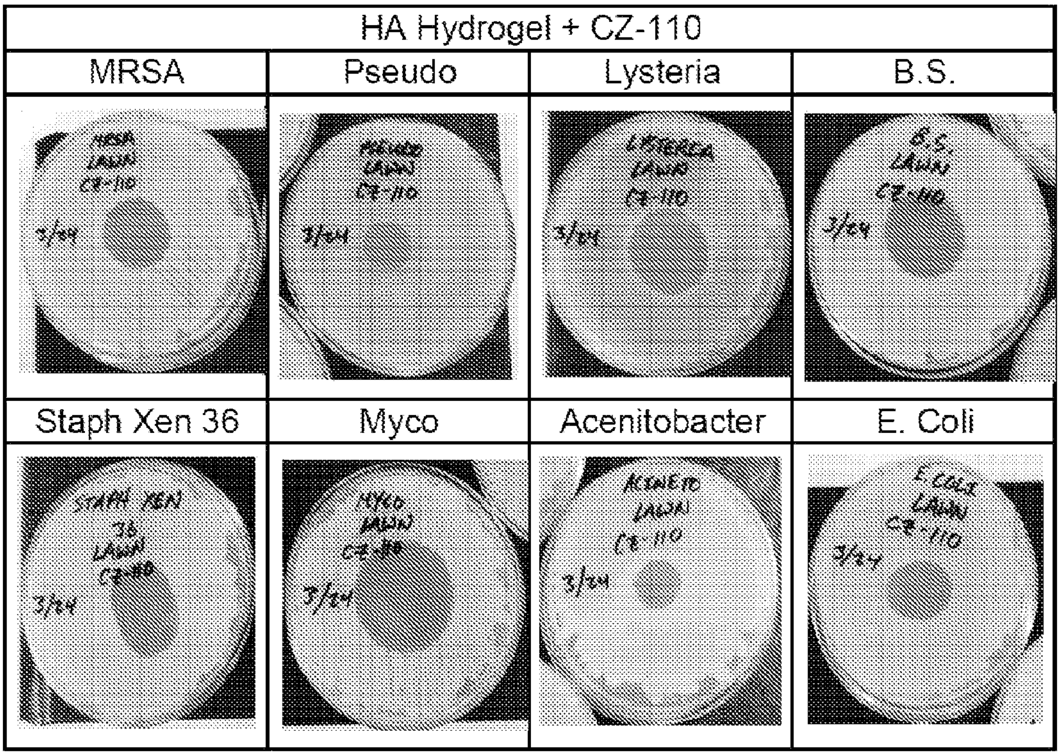


FIG. 30A

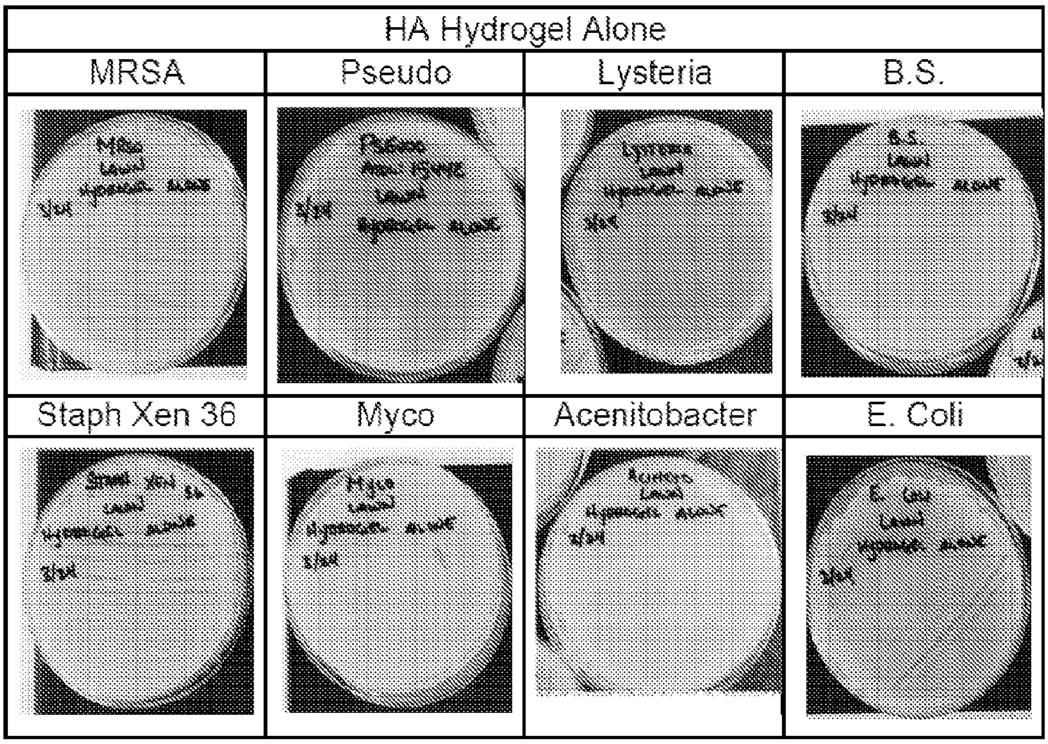


FIG. 30B

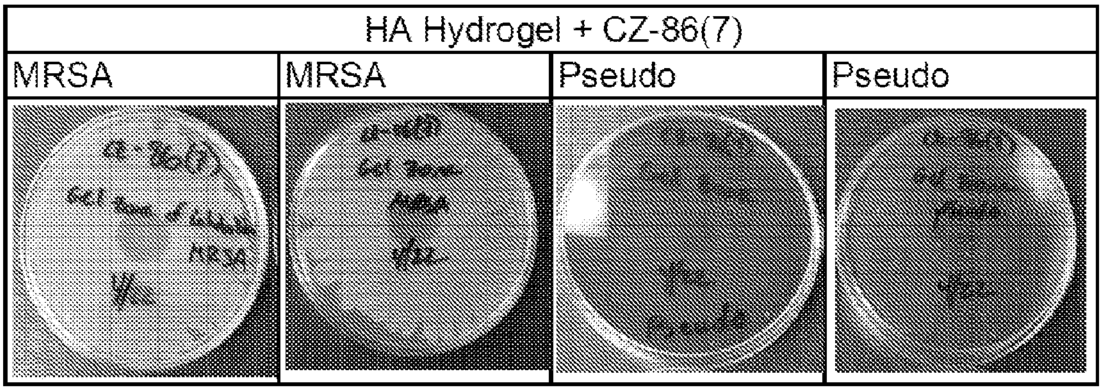


FIG. 30C

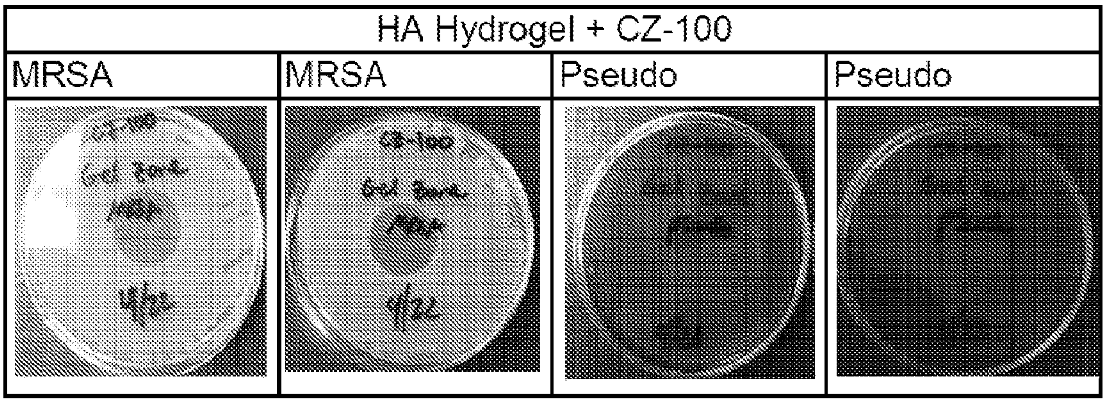


FIG. 30D

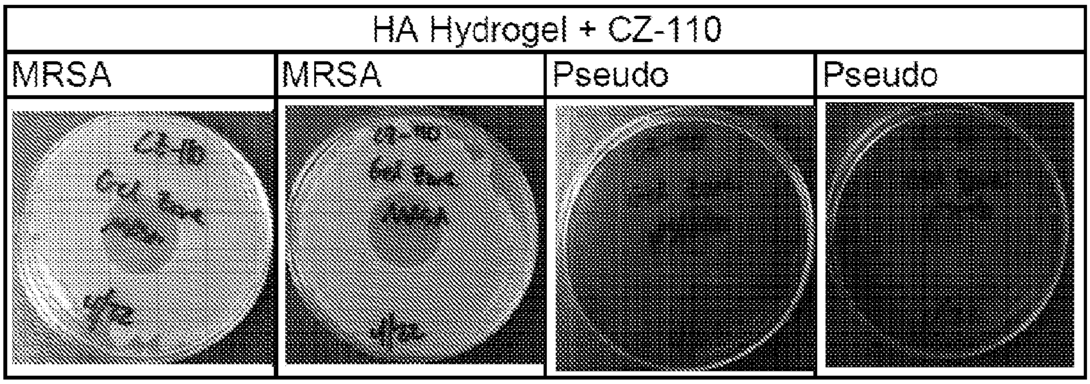


FIG. 30E

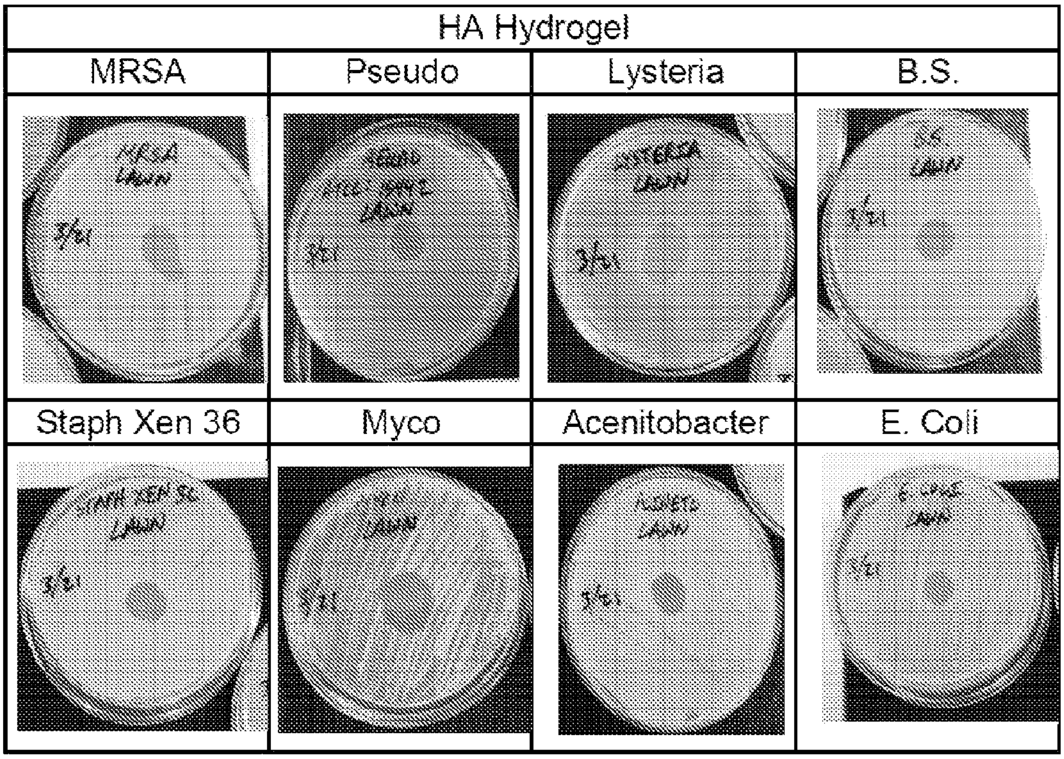


FIG. 30F

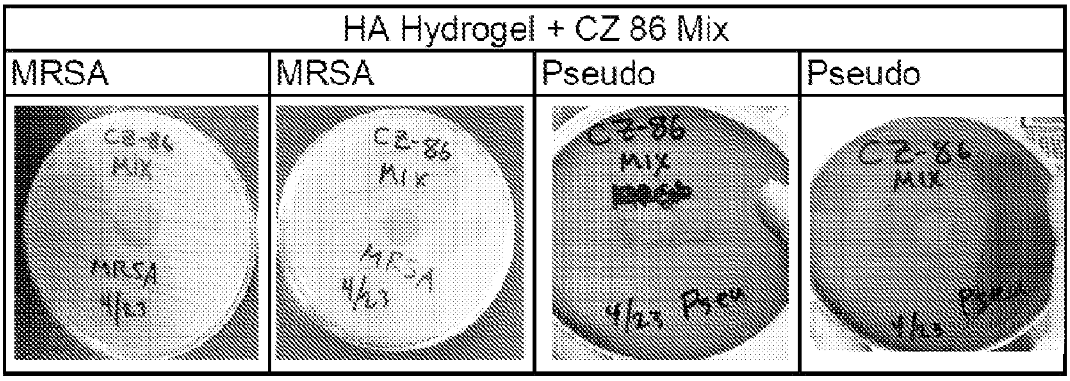


FIG. 30G

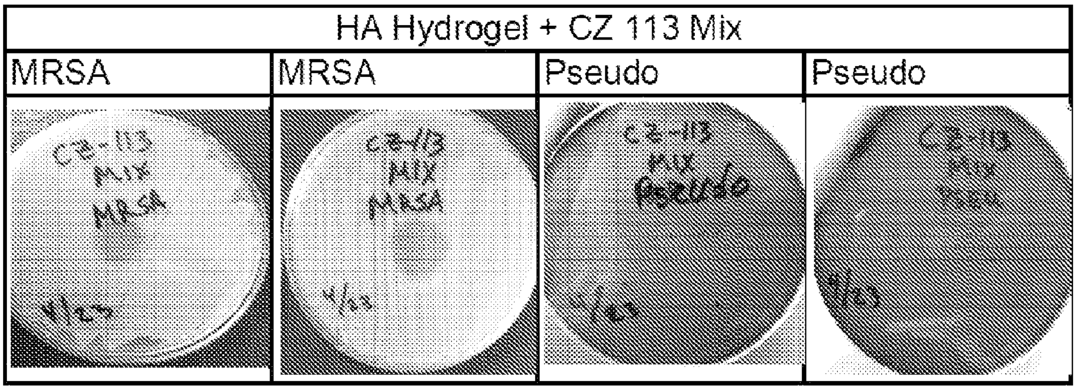


FIG. 30H

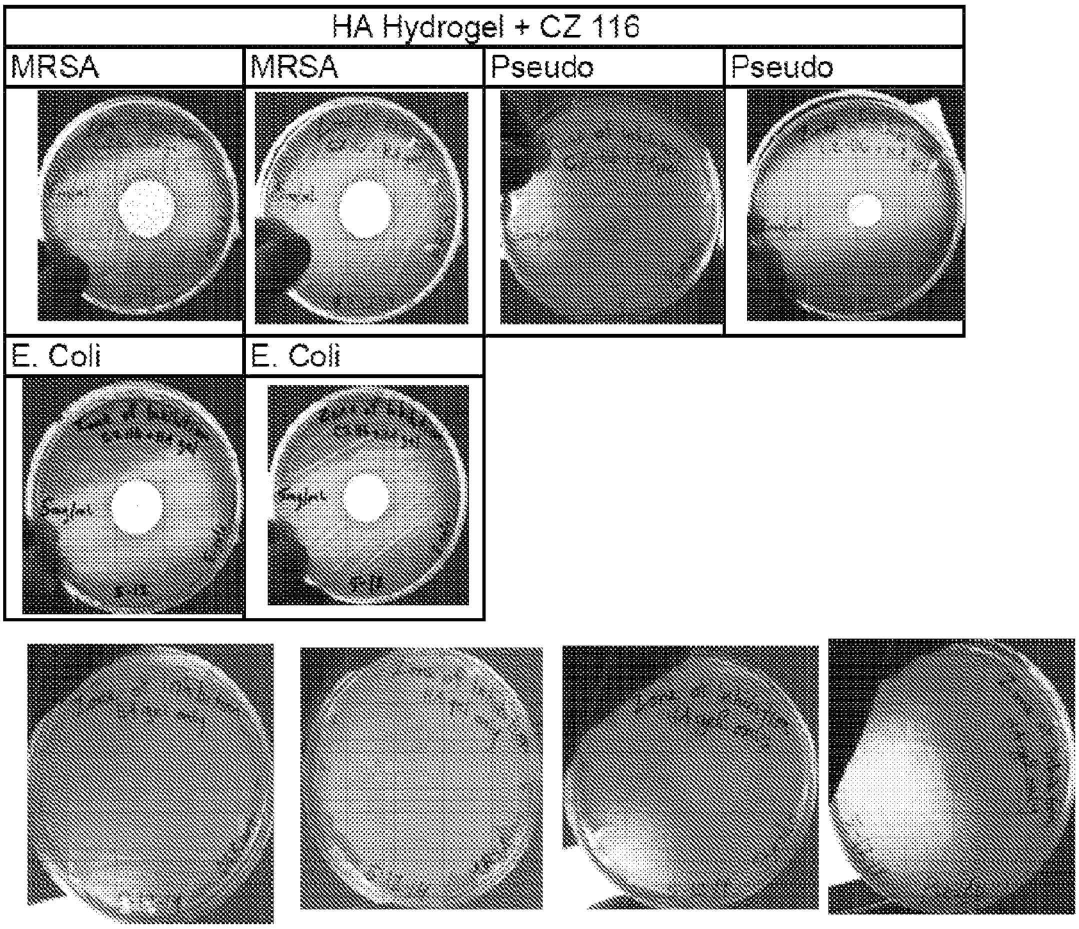


FIG. 30I

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012151554 A
- US 61482522
- US 61482523
- US 61591601
- US 61616944
- US 61826453
- US 61826761
- US 61836555
- US 61834149
- US 13379191
- US 14076143
- US 14076149
- WO 2010148390 A
- WO 2012151555 A
- WO 2013148230 A
- US 4522811 A


### Non-patent literature cited in the description


- **ZHANG et al.** Antibiotic resistance as a global threat: Evidence from China, Kuwait and the United States. *Global Health,* 2006, vol. 2, 6
- **BALABAN et al.** Control of Biofilm Infections by Signal Manipulation. Springer, 2008, 1-11
- **LESLIE.** Germs Take a Bite Out of Antibiotics. *Science,* 2008, vol. 320, 33
- *Europe in the Grip of Drug-Resistant Superbugs,* 2011
- **MELNICK, M.** *TIME,* 2010
- **ARIAS et al.** The rise of the Enterococcus: beyond vancomycin resistance. *Nat Rev Microbiol,* 2012, vol. 10, 266-278
- **JAIN, R. et al.** Veterans affairs initiative to prevent methicillin-resistant Staphylococcus aureus infections. *N Engl J Med,* 2011, vol. 364, 1419-1430
- **NORDMANN et al.** The real threat of Klebsiella pneumoniae carbapenemase-producing bacteria. *Lancet Infect Dis,* 2009, vol. 9, 228-236
- **ALOUSH et al.** Multidrug-resistant Pseudomonas aeruginosa: risk factors and clinical impact. *Antimicrob Agents Chem,* 2006, vol. 50, 43-48
- **CALHOUN et al.** Multidrug-resistant Organisms in Military Wounds from Iraq and Afghanistan. *Clinical Orthopaedics and Related Research,* 2008, vol. 466, 1356-1362
- **MURRAY et al.** Bacteriology of War Wounds at the Time of Injury. *Military Medicine,* 2006, vol. 171, 826-829
- **HUJER et al.** Analysis of Antibiotic Resistance Genes in Multidrug-Resistant Acinetobacter sp. Isolates from Military and Civilian Patients Treated at the Walter Reed Army Medical Center. *Antmicrobial Agents and Chemotherapy,* 2006, vol. 50, 4114-4123
- **MORITA et al.** Antibiotic Inducibility of the MexXY Multidrug Efflux System of Pseudomonas aeruginosa: Involvement of the Antibiotic-Inducible PA5471 Gene Product. *Journal of Bacteriology,* 2006, vol. 188, 1847-1855
- **TRAN et al.** Heat-Shock Protein ClpL/HSP 100 Increases Penicillin Tolerance in Streptococcus pneumoniae. *Advances in Oto-rhino-laryngology,* 2011, vol. 72, 126-128
- **LIVORSI et al.** Virulence Factors of Gram-Negative Bacteria in Sepsis With a Focus on Neisseria meningitidis. *Contributions to Microbiology,* 2011, vol. 17, 31-47
- **NOSTRO et al.** Specific Ion Effects on the Growth Rates of Staphylococus aureus and Pseudomonas aeruginosa. *Physical Biology,* 2005, vol. 2, 1-7
- **COSTERTON et al.** How bacteria stick. *Sci Am,* 1978, vol. 238, 86-95
- **LAWRENCE et al.** Optical sectioning of microbial biofilms. *J Bacteriol,* 1991, vol. 173, 6558-6567
- **ZOBELL.** The Effect of Solid Surfaces upon Bacterial Activity. *Journal of Bacteriology,* 1943, vol. 46, 39-56
- **LEID et al.** The Exopolysacharide Alginate Protects Pseudomonas aeruginosa Biofilm Bacteria from IFN-γ-Mediated Macrophage Killing. *The Journal of Immunology,* 2005, vol. 175, 7512-7518
- **COSTERTON et al.** The Bacterial Glycocalyx in Nature and Disease. *Annual Review of Microbiology,* 1981, vol. 35, 299-324
- **LUJAN et al.** Disrupting Antibiotic Resistance Propagation by Inhibiting the Conjugative DNA Relaxase. *PNAS,* 2007, vol. 104, 12282-12287
- **LEDERBERG et al.** Gene Recombination in Escherichia coli. *Nature,* 1946, vol. 158, 529-564

- **HAUSNER et al.** High Rates of Conjugation in Bacterial Biofilms as Determined by Quantitative In Situ Analysis. *Applied and Environmental Microbiology,* 1999, vol. 65, 3710-3713
- **WALTERS et al.** Contributions of Antibiotic Penetration, Oxygen Limitation, and Low Metabolic Activity to Tolerance of Pseudomonas aeruginosa biofilms to Ciprofloxacin and Tobramycin. *Antimicrobial Agents and Chemotherapy,* 2003, vol. 47, 317-323
- **BORRIELLO et al.** Oxygen Limitation Contributes to Antibiotic Tolerance of Pseudomonas aeruginosa in Biofilms. *Antimicrobial Agents and Chemotherapy,* 2004, vol. 48, 2659-2664
- **MANDELL.** Interaction of Intraleukocytic Bacteria and Antibiotics. *The Journal of Clinical Investigation,* 1973, vol. 52, 1673-1673
- **GILBERT et al.** Influence of Growth Rate on Susceptibility to Antimicrobial Agents: Biofilms, Cell Cycle, Dormancy, and Stringent Response. *Antimicrobial Agents and Chemotherapy,* 1990, vol. 34, 1865-1868
- **STOODLEY et al.** Liquidflow in biofilm systems. *App Env Microbiol,* 1994, vol. 60, 2711-2716
- **KOLODKIN-GAL, I. et al.** A self-produced trigger for biofilm disassemby that targets exopolysaccharide. *Cell,* 2012, 149
- **BAXTER, E. W. ; REITZ, A. B.** Reductive Aminations of Carbonyl Compounds with Borohydride and Borane Reducing Agents. *Org Reac,* 2004, vol. 1, 59
- Remington: The Science and Practice of Pharmacy. Lippincott Williams & Wilkins, 2006
- **TAN et al.** *Pharm. Res.,* 2007, vol. 24, 2297-2308
- **WILLIAMS et al.** Vivo Efficacy of a Silicone - Cationic Steroid Antimicrobial Coating to Prevent Implant-Related Infection. *Biomaterials,* 2012, vol. 33, 8641-8656
- **WILLIAMS et al.** Use of delrin plastic in a modified CDC biofilm reactor. *Res J Microbiol,* 2011, vol. 6, 425-429
- **WILLIAMS et al.** Experimental model of biofilm implant-related osteomyelitis to test combination biomaterials using biofilms as initial inocula. *J Biomed Mat Res A,* 2012, vol. 100, 1888-1900
- **WILLIAMS et al.** A modified CDC biofilm reactor to produce mature biofilms on the surface of PEEK membranes for an in vivo animal model application. *Curr Microbiol,* 2011, vol. 62, 1657-1663
- **BAKKEN.** Separation and Purification of Bacteria from Soil. *Appl Environ Microbiol,* 1985, vol. 49, 1482-1487
- **TORSVIK et al.** High Diversity in DNA of Soil Bacteria. *Applied and Environmental Microbiology,* 1990, vol. 56, 782-787
- **WILLIAMS, D. L. ; HAYMOND, B. S. ; BLOEBAUM, R. D.** Use of delrin plastic in a modified CDC biofilm reactor. *Res J Microbiol,* 2011, vol. 6, 425-429
- **WILLIAMS, D. L. ; WOODBURY, K. L. ; HAYMOND, B. S. ; PARKER, A. E. ; BLOEBAUM, R. D.** A modified CDC biofilm reactor to produce mature biofilms on the surface of PEEK membranes for an in vivo animal model application. *Curr Microbiol,* 2011, vol. 62, 1657-1663
- **WILLIAMS, D. L. et al.** Vivo Efficacy of a Silicone - Cationic Steroid Antimicrobial Coating to Prevent Implant-Related Infection. *Biomaterials,* 2012, vol. 33, 8641-8656
- **WILLIAMS, D. L. et al.** Experimental model of biofilm implant-related osteomyelitis to test combination biomaterials using biofilms as initial inocula. *JBiomed-Mat Res A,* 2012, vol. 100, 1888-1900
- *Med. Chem. Commun.,* 2012, vol. 3, 763-770